# EUROPEAN PATENT SPECIFICATION

(11) **EP 2 121 600 B1**
(45) Date of publication and mention of the grant of the patent: **18.07.2012**
(21) Application number: 08709013.0
(22) Date of filing: 15.02.2008
(51) Int. Cl.: C07D 209/04, C07D 401/04, C07D 401/14, C07D 403/04, C07D 403/14, A61K 31/404, A61K 31/416, A61P 3/10

(54) **SUBSTITUTED ARYLSULPHONYLGLYCINES, THE PREPARATION THEREOF AND THE USE THEREOF AS PHARMACEUTICAL COMPOSITIONS**
SUBSTITUIERTE ARYLSULPHONYLGLYCINE, IHRE HERSTELLUNG UND IHRE VERWENDUNG ALS PHARMAZEUTISCHE ZUSAMMENSETZUNGEN
NOUVELLES ARYLSULPHONYLGLYCINES SUBSTITUÉES, LEUR PROCÉDÉ DE PRÉPARATION ET LEUR UTILISATION EN TANT QUE COMPOSITIONS PHARMACEUTIQUES

(30) Priority: 16.02.2007 DE 102007007751
(43) Date of publication of application: 25.11.2009
(73) Proprietor: Boehringer Ingelheim International GmbH, 55216 Ingelheim am Rhein (DE)
(72) Inventor: WAGNER, Holger, 55216 Ingelheim am Rhein (DE); LANGKOPF, Elke, 55216 Ingelheim am Rhein (DE); STREICHER, Ruediger, 55216 Ingelheim am Rhein (DE); ECKHARDT, Matthias, 55216 Ingelheim am Rhein (DE); SCHULER-METZ, Annette, 55216 Ingelheim am Rhein (DE); PAUTSCH, Alexander, 55216 Ingelheim am Rhein (DE); SCHOELCH, Corinna, 55216 Ingelheim am Rhein (DE)
(74) Representative: Hammann, Heinz
(86) International application number: PCT/EP2008/051824
(87) International publication number: WO 2008/099000

(56) References cited:
- WO-A-2005/013976
- WO-A-2005/013977
- WO-A-2005/013978

## Description

The present invention relates to substituted arylsulphonylglycines of general formula I 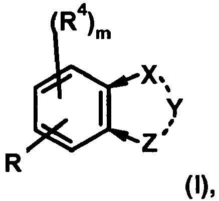 wherein the groups R, R⁴, m, X, Y and Z are defined as hereinafter, including the tautomers, stereoisomers, mixtures thereof and salts thereof. This invention further relates to pharmaceutical compositions containing a compound of formula I according to the invention as well as the use of a compound according to the invention for preparing a pharmaceutical composition for the treatment of metabolic disorders, particularly type 1 or type 2 diabetes mellitus. The invention also relates to processes for preparing a pharmaceutical composition as well as a compound according to the invention.

Compounds of formula I are suitable for preventing the inhibiting effect of glycogen phosphorylase on the activity of glycogen synthase by stopping the interaction of glycogen phosphorylase a with the G_{L} subunit of glycogen-associated protein phosphatase 1 (PP1). Compounds with these properties stimulate glycogen synthesis and are proposed for the treatment of metabolic disorders, particularly diabetes (P. Cohen, Nature Reviews Molecular Cell Biology 2006, 7, 867-874).

### Aim of the invention

The aim of the present invention is to provide new arylsulphonylglycines that suppress the interaction of glycogen phosphorylase a with the G_{L} subunit of glycogen-associated protein phosphatase 1 (PP1).

A further aim of the present invention is to provide new pharmaceutical compositions that are suitable for the prevention and/or treatment of metabolic disorders, particularly diabetes.

Another aim of this invention is to provide a process for preparing the compounds according to the invention.

Other aims of the present invention will become directly apparent to the skilled man from the foregoing remarks and those that follow.

### Object of the invention

In a first aspect the present invention relates to new substituted arylsulphonylglycines of general formula 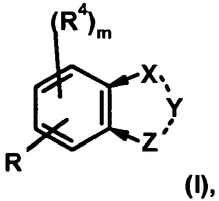 wherein
R denotes a group of formula 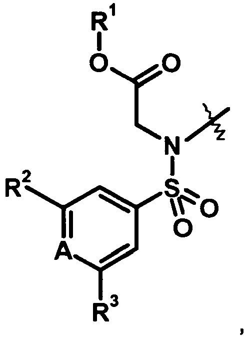 wherein
- R¹: denotes H, C₁-₆-alkyl or a group of formula 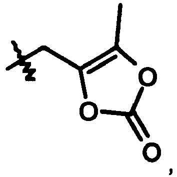 wherein the C₁₋₆-alkyl group mentioned hereinbefore for R¹ may be substituted by C₁₋₆-alkyl-carbonyloxy, C₁₋₆-alkoxy-carbonyloxy, C₁₋₆-alkoxy, hydroxy, amino, C₁₋₃-alkyl-amino, di-(C₁₋₃-alkyl)-amino, pyrrolidin-1-yl, piperidin-1-yl, morpholin-4-yl, piperazin-1-yl, 4-(C₁₋₃-alkyl)-piperazin-1-yl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, tetrahydrofuran-3-yl-oxy, C₁₋₃-alkylamino-C₁₋₃-alkyloxy, di-(C₁₋₃-alkyl)-amino- C₁₋₃-alkyloxy, pyrrolidin-1-yl-C₁₋₃-alkyloxy, piperidin-1-yl-C₁₋₃-alkyloxy, morpholin-4-yl-C₁₋₃-alkyloxy, piperazin-1-yl-C₁₋₃-alkyloxy or 4-(C₁₋₃-alkyl)-piperazin-1-yl-C₁₋₃-alkyloxy,
R² and R³ independently of one another denote H, halogen, C₁₋₃-alkyl, C₁₋₃-perfluoralkyl, C₁₋₃-perfuoralkoxy, C₁₋₃-alkoxy, cyano, nitro or hydroxy,
and
A denotes CH or N,
- m: denotes 0, 1 or 2,
- R⁴: denotes halogen, C₁₋₃-alkyl, C₁₋₃-perfuoroalkyl, C₁₋₃-perfluoroalkoxy, cyano, hydroxy or C₁₋₃-alkoxy, while, if m denotes the number 2, the groups R⁴ may be identical or different,
and the heterocyclic group 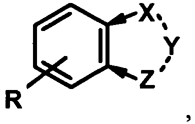 which may be substituted by R⁴ as described hereinbefore denotes a group of formula 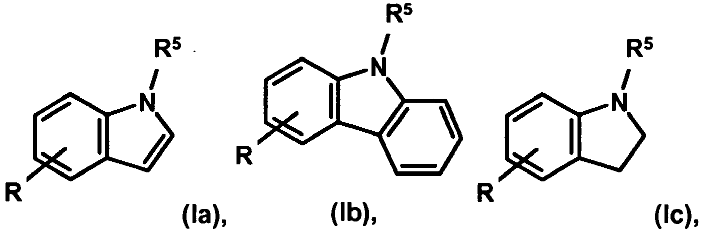 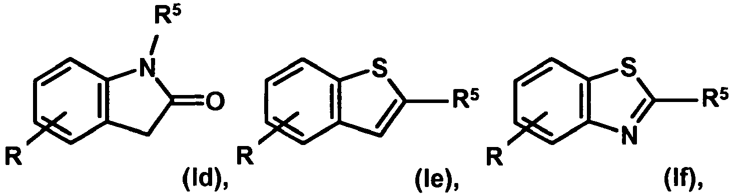 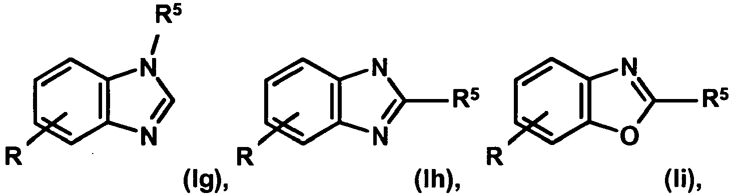 or 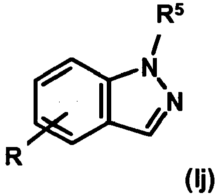
wherein the above-mentioned heterocycles of formulae (Ia), (Ic), (Id), (Ie), (Ig) and (Ij) may each optionally be substituted at the carbon atoms of the 5-ring by one or two groups selected from among halogen, C₁₋₃-alkyl, cyano, C₁₋₃-perfuoroalkyl, C₃₋₆-cycloalkyl, C₂₋₄-alkynyl, C₂₋₄-alkenyl, C₁₋₃-alkyl-carbonyl, C₁₋₃-perfuoroalkyl-carbonyl, carboxyl, aminomethyl, C₁₋₃-alkyl-aminomethyl, di-(C₁₋₃-alkyl)-aminomethyl, aminocarbonyl, C₁₋₃-alkylaminocarbonyl or di-(C₁₋₃-alkyl)-aminocarbonyl, wherein the groups may be identical or different and each carbon atom may carry only one group, and
R⁵ denotes a 1H-pyrimidin-2,4-dionyl, 2H-pyridazin-3-onyl or 1H-pyridin-2-onyl group optionally mono- or disubstituted by one or two methyl groups or
a mono- or bicyclic 5- to 14-membered ring system which may contain 0 to 4 heteroatoms selected from among N, O or S, wherein not more than one oxygen atom and/or one sulphur atom may be present, is aromatic, saturated or partially unsaturated and may be mono-, di- or trisubstituted independently of one another by a group selected from among
   halogen, cyano, nitro,
   C₁₋₆-alkyl, C₃₋₆-cycloalkyl, C₁₋₃-perfuoroalkyl, C₂₋₆-alkynyl, C₂₋₆-alkenyl,
   hydroxy, C₁₋₆-alkoxy, C₃₋₆-cycloalkoxy, C₁₋₃-perfluoroalkoxy,
   carboxyl, C₁₋₃-alkyl-carbonyl, C₁₋₄-alkoxy-carbonyl, C₃₋₆-cycloalkoxy-carbonyl, aminocarbonyl, C₁₋₆-alkyl-aminocarbonyl, di-(C₁₋₆-alkyl)-aminocarbonyl, N-(C₁₋₆-alkyl)-N-(C₁₋₆-alkoxy)-aminocarbonyl, C₃₋₆-cycloalkyl-aminocarbonyl, N-(C₃₋₆-cycloalkyl)-N-(C₁₋₆-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, 4-(C₁₋₃-alkyl-carbonyl)-piperazin-1-yl-carbonyl, 4-(C₃₋₆-cycloalkyl-carbonyl)-piperazin-1-yl-carbonyl, 4-(C₁₋₃ -alkylsulphonyl)-piperazin-1-yl-carbonyl, 4-(cos-cycloalkylsulphonyl)-piperazin-1-yl-carbonyl,
   C₁₋₃-alkylsulphanyl, C₃-6-cycloalkylsulphanyl, C₁₋₃-alkylsulphinyl, C₃₋₆ -cycloalkylsulphinyl, C₁₋₃-alkylsulphonyl, C₃₋₆-cycloalkylsulphonyl,
   amino, C₁₋₆-alkyl-amino, di-(C₁₋₆-alkyl)-amino, C₃₋₆-cycloalkylamino, N-(C₃₋₆-cycloalkyl)-N-(C₁₋₆-alkyl)-amino, (C₁₋₄-alkylcarbonyl)-amino, (C₃₋₆-cycloalkyl-carbonyl)-amino, (C₁₋₄-alkylcarbonyl)-(C₁₋₃-alkyl)-amino, (C₃₋₆-cycloalkyl-carbonyl)-(C₁₋₃-alkyl)-amino,
   pyrrolidin-1-yl, piperidin-1-yl, 3-amino-piperidin-1-yl, 4-aminopiperidin-1-yl, morpholin-4-yl, thiomorpholin-4-yl, 1-oxo-1λ⁴-thiomorpholin-4-yl, 1,1-dioxo-1λ⁶-thiomorpholin-4-yl, piperazin-1-yl, homopiperazin-1-yl, 4-(C₁₋₃-alkyl)-piperazin-1-yl, 4-(C₁₋₃-alkylcarbonyl)-piperazin-1-yl, 4-(C₃₋₆-cycloalkyl-carbonyl)-piperazin-1-yl, 4-(C₁₋₃-alkylsulphonyl)-piperazin-1-yl, 4-(C₃₋₆-cycloalkyl-sulphonyl)-piperazin-1-yl,
   tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidin-1-yl, 2-oxo-tetrahydropyrimidin-1-yl and heteroaryl,
      wherein the C₁₋₆-alkyl, C₃₋₆-cycloalkyl, C₂₋₆-alkynyl, C₂₋₆-alkenyl, C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl, C₃₋₆-cycloalkylsulphonyl, C₁₋₆-alkoxy, C₃₋₆-cycloalkoxy, C₁₋₆-alkyl-amino, di-(C₁₋₆-alkyl)-amino, C₃₋₆-cycloalkyl-amino-N-(C₃₋₆-cycloalkyl)-N-(C₁₋₆-alkyl)-amino, (C₁₋₄-alkyl-carbonyl)-amino, (C₃₋₆-cycloalkyl-carbonyl)-amino, (C₁₋₄-alkylcarbonyl)-(C₁₋₃-alkyl)-amino- and (C₃₋₆-cycloalkyl-carbonyl)-(C₁₋₃-alkyl)-amino groups mentioned above in the definition of R⁵ may each be mono- or disubstituted independently of one another in the carbon skeleton by a group selected from among
      cyano, hydroxy, C₁₋₃-alkoxy, tetrahydro-pyran-2-yloxy,
      amino, C₁₋₆-alkyl-amino, di-(C₁₋₆-alkyl)-amino, C₃₋₆-cycloalkyl-amino, N-(C₃₋₆-cycloalkyl)-N-(C₁₋₆-alkyl)-amino, (C₁₋₄-alkyl-carbonyl)-amino, (C₃₋₆-cycloalkyl-carbonyl)-amino, (C₁₋₄-alkyl-carbonyl)-(C₁₋₃-alkyl)-amino, (C₃₋₆-cycloalkylcarbonyl)-(C₁₋₃-alkyl)-amino, (C₁₋₄-alkylsulphonyl)-amino, (C₃₋₆ -cycloalkylsulphonyl)-amino, (C₁₋₄-alkylsulphonyl)-(C₁₋₃-alkyl)-amino, (C₃₋₆-cycloalkylsulphonyl)-(C₁₋₃-alkyl)-amino, pyrrolidin-1-yl, piperidin-1-yl, morpholin-4-yl, piperazin-1-yl, 4-(C₁₋₃-alkyl)-piperazin-1-yl,
      carboxyl, aminocarbonyl, C₁₋₆-alkyl-aminocarbonyl, di-(C₁₋₆-alkyl)-aminocarbonyl, C₁₋₂-alkoxy-carbonyl, C₃₋₆-cycloalkyl-aminocarbonyl, N-(C₃₋₆-cycloalkyl)-N-(C₁₋₆-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl,
      C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl, and C₃₋₆-cycloalkylsulphonyl, wherein the substituents must not be bound to a common carbon atom, and
   wherein the pyrrolidin-1-yl and piperidin-1-yl groups mentioned above in the definition of R⁵ may be substituted by amino or hydroxy, and
   wherein the C₁₋₆-alkyl-aminocarbonyl, di-(C₁₋₆-alkyl)-aminocarbonyl, C₃₋₆-cycloalkyl-aminocarbonyl, N-(C₃₋₆-cycloalkyl)-N-(C₁₋₆-alkyl)-aminocarbonyl groups mentioned hereinbefore for R⁵ may each be mono- or disubstituted independently of one another in the carbon skeleton by a group selected from among
      cyano, hydroxy, C₁₋₃-alkoxy,
      amino, C₁₋₃-alkyl-amino, di-(C₁₋₃-alkyl)-amino, C₃₋₆-cycloalkyl-amino, N-(C₃₋₆-cycloalkyl)-N-(C₁₋₆-alkyl)-amino, (C₁₋₄-alkyl-carbonyl)-amino, (C₃₋₆-cycloalkyl-carbonyl)-amino, (C₁₋₄-alkyl-carbonyl)-(C₁₋₃-alkyl)-amino, (C₃₋₆-cycloalkylcarbonyl)-(C₁₋₃-alkyl)-amino, (C₁₋₄-alkylsulphonyl)-amino, (C₃₋₆ -cycloalkylsulphonyl)-amino, (C₁₋₄-alkylsulphonyl)-(C₁₋₃-alkyl)-amino, (C₃₋₆-cycloalkylsulphonyl)-(C₁₋₃-alkyl)-amino
      pyrrolidin-1-yl, piperidin-1-yl, morpholin-4-yl, piperazin-1-yl, 4-(C₁₋₃-alkyl)-piperazin-1-yl,
      C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl and C₃₋₆-cycloalkylsulphonyl, wherein the substituents must not be bound to a common carbon atom, and
wherein the heteroaryl group mentioned hereinbefore for R⁵ denotes a monocyclic five-membered aromatic system with 1 to 4 heteroatoms selected from among N, O or S, wherein not more than one oxygen atom and/or one sulphur atom may be present, or a six-membered aromatic system with 1 to 3 nitrogen atoms, and may be mono- or disubstituted independently of one another by halogen, cyano, hydroxy, amino, C₁₋₃-alkyl or C₁₋₃-alkyloxycarbonyl, and
wherein in the above-mentioned morpholin-4-yl and piperazin-1-yl groups in each case a methylene unit may be replaced by a carbonyl or sulphonyl group.

The invention also relates to the tautomers, stereoisomers, mixtures and salts, particularly the physiologically acceptable salts, of the compounds according to the invention.

The compounds of general formula I according to the invention and the physiologically acceptable salts thereof have valuable pharmacological properties, in particular they suppress the interaction of glycogen phosphorylase a with the G_{L}-subunit of glycogen-associated protein phosphatase 1 (PP1).

Therefore this invention also relates to the use of the compounds according to the invention, including the physiologically acceptable salts, as pharmaceutical compositions.

This invention further relates to pharmaceutical compositions containing at least one compound according to the invention or a physiologically acceptable salt according to the invention, optionally together with one or more inert carriers and/or diluents.

A further object of this invention is the use of at least one compound according to the invention or a physiologically acceptable salt of such a compound for preparing a pharmaceutical composition that is suitable for the treatment or prevention of diseases or conditions that can be influenced by suppressing the interaction of glycogen phosphorylase a with the G_{L}-subunit of glycogen-associated protein phosphatase 1 (PP1).

The invention also relates to the use of at least one compound according to the invention for preparing a pharmaceutical composition which is suitable for the treatment of metabolic disorders, for example type I or II diabetes mellitus.

The invention also relates to the use of at least one compound according to the invention for preparing a pharmaceutical composition for suppressing the interaction of glycogen phosphorylase a with the G_{L}-subunit of glycogen-associated protein phosphatase 1 (PP1).

A further object of this invention is a process for preparing a pharmaceutical composition according to the invention, characterised in that a compound according to the invention is incorporated in one or more inert carriers and/or diluents by a non-chemical method.

The present invention also relates to a process for preparing the compounds of general formula I according to the invention.

### Detailed description of the invention

Unless stated otherwise, the groups, radicals and substituents, particularly R, R¹ to R⁵, m, X, Y, Z and A have the meanings given hereinbefore and hereinafter.

If groups, substituents or radicals occur more than once in a compound, they may have the same or different meanings.

Preferred compounds of the above general formula I are those wherein
R denotes a group of the above-mentioned formula wherein
- R¹: denotes H, C₁₋₆-alkyl or a group of formula 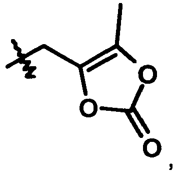 wherein the C₁₋₆-alkyl group mentioned hereinbefore for R¹ may be substituted by C₁₋₆-alkyl-carbonyloxy, C₁₋₆-alkoxy-carbonyloxy, C₁₋₆-alkoxy, hydroxy, amino, C₁₋₃-alkyl-amino, di-(C₁₋₃-alkyl)-amino, pyrrolidin-1-yl, piperidin-1-yl, morpholin-4-yl, piperazin-1-yl, 4-(C₁₋₃-alkyl)-piperazin-1-yl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl or 4-(C₁₋₃ -alkyl)-piperazin-1-yl-carbonyl,
- R² and R³: independently of one another denote halogen, C₁₋₃-alkyl, C₁₋₃-perfluoroalkyl, C₁₋₂-alkoxy or cyano and
- A: denotes CH or N,
- m: denotes 0, 1 or 2,
- R⁴: denotes halogen, C₁₋₃-alkyl, trifluoromethyl or cyano, while, if m denotes the number 2, the groups R⁴ may be identical or different,
and the heterocyclic group 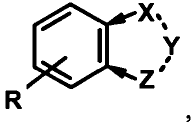 which may be substituted by R⁴ as described hereinbefore, denotes a group of formula 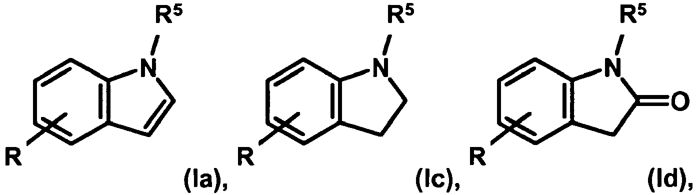 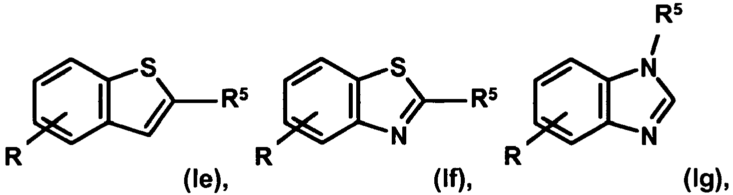 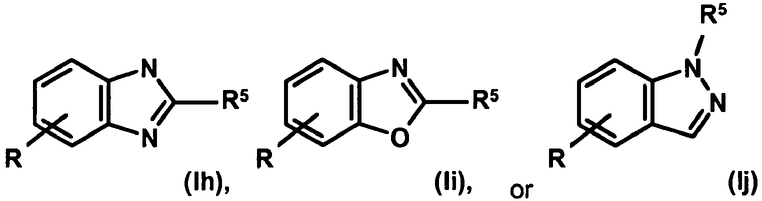
wherein the above-mentioned heterocycles of formulae (Ia), (Ic), (Id), (Ie), (Ig) and (Ij) may optionally be substituted at the carbon atoms of the 5 ring by one or two groups selected from among halogen, C₁₋₃-alkyl, cyano, C₁₋₃ -perfluoroalkyl, C₃₋₆-cycloalkyl, C₁₋₃-alkyl-carbonyl, C₁₋₃-perfluoroalkylcarbonyl, aminocarbonyl, C₁₋₃-alkyl-aminocarbonyl or di-(C₁₋₃-alkyl)-aminocarbonyl, wherein the groups may be identical or different and each carbon atom carries at most one group, and
wherein R⁵ denotes 1,3-dimethyl-1H-pyrimidin-2,4-dion-5-yl, 1H-pyrimidin-2,4-dion-6-yl, 1H-pyrimidin-2,4-dion-5-yl, 2H-pyridazin-3-on-6-yl, 1H-pyridin-2-on-3-yl, 1H-pyridin-2-on-5-yl, 1H-pyridin-2-on-4-yl or
a mono- or bicyclic 5- to 14-membered ring system which may contain 0 to 4 heteroatoms selected from among N, O or S, wherein not more than one oxygen atom and/or one sulphur atom may be present, is aromatic, saturated or partially unsaturated and may be mono-, di- or trisubstituted independently of one another by a group selected from among
   halogen, cyano,
   C₁₋₆-alkyl, C₃₋₆-cycloalkyl, C₁₋₃-perfluoroalkyl, C₂₋₆-alkynyl, C₂₋₆-alkenyl,
   hydroxy, C₁₋₆-alkoxy, C₃₋₆-cycloalkoxy, trifluoromethoxy,
   carboxyl, C₁₋₃-alkyl-carbonyl, C₁₋₄-alkoxy-carbonyl, cyclopropoxycarbonyl, aminocarbonyl, C₁₋₆-alkyl-aminocarbonyl, di-(C₁₋₆-alkyl)-aminocarbonyl, N-(C₁₋₃-alkyl)-N-(C₁₋₃-alkoxy)-aminocarbonyl, C₃₋₆-cycloalkyl-aminocarbonyl, N-(C₃₋₆-cycloalkyl)-N-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, 4-(C₁₋₃-alkyl-carbonyl)-piperazin-1-yl-carbonyl, 4-(C₃₋₆-cycloalkyl-carbonyl)-piperazin-1-yl-carbonyl, 4-(C₁₋₃ -alkylsulphonyl)-piperazin-1-yl-carbonyl, 4-(C₃₋₆-cycloalkylsulphonyl)-piperazin-1-yl-carbonyl,
   C₁₋₃-alkylsulphanyl, C₃₋₆-cycloalkylsulphanyl, C₁₋₃-alkylsulphinyl, C₃₋₆ -cycloalkylsulphinyl, C₁₋₃-alkylsulphonyl, C₃₋₆-cycloalkylsulphonyl,
   amino, C₁₋₆-alkyl-amino, di-(C₁₋₆-alkyl)-amino, C₃₋₆-cycloalkylamino, N-(C₃₋₆-cycloalkyl)-N-(C₁₋₃-alkyl)-amino, (C₁₋₄-alkylcarbonyl)-amino, (C₃₋₆-cycloalkyl-carbonyl)-amino, (C₁₋₄-alkylcarbonyl)-(C₁₋₃-alkyl)-amino, (C₃₋₆-cycloalkyl-carbonyl)-(C₁₋₃-alkyl)-amino, pyrrolidin-1-yl, piperidin-1-yl, 3-amino-piperidin-1-yl, 4-amino-piperidin-1-yl, morpholin-4-yl, thiomorpholin-4-yl, 1-oxo-1λ⁴-thiomorpholin-4-yl, 1,1-dioxo-1λ⁶-thiomorpholin-4-yl, piperazin-1-yl, homopiperazin-1-yl, 4-(C₁₋₃-alkyl)-piperazin-1-yl, 4-(C₁₋₃-alkylcarbonyl)-piperazin-1-yl, 4-(C₃₋₆-cycloalkyl-carbonyl)-piperazin-1-yl, 4-(C₁₋₃-alkylsulphonyl)-piperazin-1-yl, 4-(C₃₋₆-cycloalkylsulphonyl)-piperazin-1-yl,
   tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, 2-oxo-tetrahydropyrimidinyl and heteroaryl,
   wherein the C₁₋₆-alkyl, C₃₋₆-cycloalkyl, C₂₋₆-alkynyl, C₂₋₆-alkenyl, C₁₋₃ -alkylsulphanyl, C₃₋₆-cycloalkylsulphanyl, C₁₋₃-alkylsulphinyl, C₃₋₆-cycloalkylsulphinyl, C₁₋₃-alkylsulphonyl, C₃₋₆-cycloalkylsulphonyl, C₁₋₆-alkoxy, C₃₋₆-cycloalkoxy, C₁₋₆-alkyl-amino, di-(C₁₋₆-alkyl)-amino, C₃₋₆-cycloalkyl-amino, N-(C₃₋₆-cycloalkyl)-N-(C₁₋₃-alkyl)-amino, (C₁₋₄ -alkyl-carbonyl)-amino, (C₃₋₆-cycloalkyl-carbonyl)-amino, (C₁₋₄-alkyl-carbonyl)-(C₁₋₃-alkyl)-amino- and (C₃₋₆-cycloalkyl-carbonyl)-(C₁₋₃-alkyl)-amino groupsmentioned above in the definition of R⁵ may each be mono- or disubstituted independently of one another in the carbon skeleton by a group selected from among
      cyano, hydroxy, C₁₋₃-alkoxy, tetrahydro-pyran-2-yloxy,
      amino, C₁₋₆-alkyl-amino, di-(C₁₋₆-alkyl)-amino, C₃₋₆-cycloalkyl-amino, N-(C₃₋₆-cycloalkyl)-N-(C₁₋₃-alkyl)-amino, (C₁₋₄-alkyl-carbonyl)-amino, (C₃₋₆-cycloalkyl-carbonyl)-amino, (C₁₋₄-alkyl-carbonyl)-(C₁₋₃-alkyl)-amino, (C₃₋₆-cycloalkylcarbonyl)-(C₁₋₃-alkyl)-amino, (C₁₋₄-alkylsulphonyl)-amino, (C₃₋₆ -cycloalkylsulphonyl)-amino, (C₁₋₄-alkylsulphonyl)-(C₁₋₃-alkyl)-amino, (C₃₋₆-cycloalkylsulphonyl)-(C₁₋₃-alkyl)-amino
      pyrrolidin-1-yl, piperidin-1-yl, morpholin-4-yl, piperazin-1-yl, 4-(C₁₋₃-alkyl)-piperazin-1-yl,
      carboxyl, C₁₋₂-alkoxy-carbonyl, aminocarbonyl, C₁₋₆-alkylaminocarbonyl, di-(C₁₋₆-alkyl)-aminocarbonyl, C₃₋₆-cycloalkylaminocarbonyl, N-(C₃₋₆-cycloalkyl)-N-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl,
      C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl and C₃₋₆-cycloalkylsulphonyl, wherein the substituents must not be bound to a common carbon atom, and
   wherein the pyrrolidin-1-yl and piperidin-1-yl groups mentioned above in the definition of R⁵ may be substituted by amino or hydroxy, and
   wherein the C₁₋₆-alkyl-aminocarbonyl, di-(C₁₋₆-alkyl)-aminocarbonyl, C₃₋₆-cycloalkyl-aminocarbonyl, N-(C₃₋₆-cycloalkyl)-N-(C₁₋₃-alkyl)-aminocarbonyl groups mentioned above in the definition of R⁵ may each be mono- or disubstituted independently of one another in the carbon skeleton by a group selected from among
      amino, hydroxy, C₁₋₃-alkoxy, C₁₋₃-alkyl-amino, di-(C₁₋₃-alkyl)-amino, C₃₋₆-cycloalkyl-amino, N-(C₃₋₆-cycloalkyl)-N-(methyl)-amino, (C₁₋₄-alkyl-carbonyl)-amino, (C₃₋₆-cycloalkylcarbonyl)-amino, (C₁₋₄-alkyl-carbonyl)-(methyl)-amino, (C₃₋₆-cycloalkyl-carbonyl)-(methyl)-amino, (C₁₋₄-alkylsulphonyl)-amino, (C₃₋₆-cycloalkylsulphonyl)-amino, (C₁₋₄-alkylsulphonyl)-(methyl)-amino or (C₃₋₆-cycloalkylsulphonyl)-(methyl)-amino,
      pyrrolidin-1-yl, piperidin-1-yl, morpholin-4-yl, piperazin-1-yl, 4-(C₁₋₃-alkyl)-piperazin-1-yl,
      C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl and C₃₋₆-cycloalkylsulphonyl, wherein the substituents must not be bound to a common carbon atom, and
wherein the heteroaryl group mentioned hereinbefore for R⁵ denotes a monocyclic five-membered aromatic system with 1 to 3 heteroatoms selected from among N, O or S, wherein not more than one oxygen atom and/or one sulphur atom may be present, or denotes a monocyclic five-membered aromatic system with 4 nitrogen atoms or a six-membered aromatic system with 1 to 3 nitrogen atoms and may be mono- or disubstituted independently of one another by fluorine, chlorine, cyano, C₁₋₃-alkyl or C₁₋₃-alkyloxy-carbonyl, and
wherein in the above-mentioned morpholin-4-yl and piperazin-1-yl groups in each case a methylene unit may be replaced by a carbonyl or sulphonyl group.

Particularly preferred are those compounds of the above general formula I, wherein R denotes a group of the above-mentioned formula wherein
- R¹: denotes H, C₁₋₄-alkyl or a group of formula 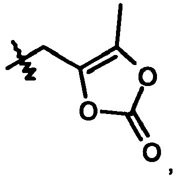 wherein the C₁₋₄-alkyl group mentioned hereinbefore for R¹ may be substituted by C₁₋₄-alkoxy, hydroxy, di-(C₁₋₃-alkyl)-amino, pyrrolidin-1-yl, piperidin-1-yl, morpholin-4-yl, piperazin-1-yl or 4-(methyl)-piperazin-1-yl,
R² and R³ independently of one another denote chlorine, bromine or C₁₋₂-alkyl and
A denotes CH or N,
- m: denotes 0 or 1,
- R⁴: denotes fluorine, chlorine, methyl or ethyl,
and the heterocyclic group 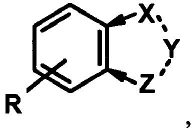 which may be substituted by R⁴ as described hereinbefore, denotes a group of formula 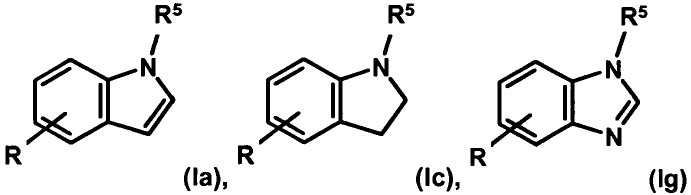 or 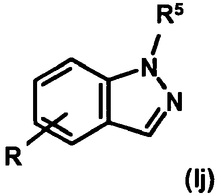
while the heterocycles mentioned hereinbefore may optionally be substituted at the carbon atoms of the 5-ring by one or two groups selected from among chlorine, bromine, iodine, C₁₋₃-alkyl, cyano and trifluoromethyl, wherein the groups may be identical or different and each carbon atom carries at most one group, and
wherein R⁵ denotes 1,3-dimethyl-1H-pyrimidin-2,4-dion-5-yl, 1H-pyrimidin-2,4-dion-6-yl, 1H-pyrimidin-2,4-dion-5-yl, 2H-pyridazin-3-on-6-yl, 1H-pyridin-2-on-3-yl, 1H-pyridin-2-on-5-yl or 1H-pyridin-2-on-4-yl,
phenyl, pyridazin-3-yl, pyrimidin-2-yl, pyrimidin-4-yl, pyrimidin-5-yl, pyrazin-2-yl, 1.3,5-triazin-2-yl, pyridin-2-yl, pyridin-4-yl, imidazol-2-yl, imidazol-4-yl, pyrazol-3-yl, pyrazol-4-yl, thiazol-2-yl, [1.3.4]thiadiazol-2-yl, thiophen-2-yl, thiophen-3-yl, naphthalin-1-yl, naphthalin-2-yl, purin-6-yl, purin-2-yl, 1-imidazo[1,2-a]pyrazin-6-yl, quinolin-6-yl, quinolin-8-yl, quinolin-2-yl or isoquinolin-1-yl, which may each be mono- or disubstituted independently of one another by a group selected from among
   fluorine, chlorine, C₁₋₄-alkyl, cyclopropyl, trifluoromethyl, cyano, hydroxy, C₁₋₃-alkoxy, cyclopropoxy,
   carboxyl, C₁₋₂-alkyl-carbonyl, C₁₋₂-alkoxy-carbonyl, aminocarbonyl, C₁₋₄-alkyl-aminocarbonyl, di-(C₁₋₂-alkyl)-aminocarbonyl, N-methoxy-N-methyl-aminocarbonyl, cyclopropyl-aminocarbonyl, N-( cyclopropyl)-N-(methyl)-aminocarbonyl, pyrrolidin-1-yl-carbonyl, piperidin-1-yl-carbonyl, morpholin-4-yl-carbonyl, piperazin-1-yl-carbonyl, 4-(methyl)-piperazin-1-yl-carbonyl, 4-(C₁₋₃-alkyl-carbonyl)-piperazin-1-yl-carbonyl, 4-(C₃₋₆-cycloalkyl-carbonyl)-piperazin-1-yl-carbonyl, 4-(C₁₋₃-alkylsulphonyl)-piperazin-1-yl-carbonyl, 4-(C₃₋₆-cycloalkylsulphonyl)-piperazin-1-yl-carbonyl,
   C₁₋₂-alkylsulphanyl, cyclopropylsulphanyl, C₁₋₂-alkylsulphinyl, cyclopropylsulphinyl, C₁₋₂-alkylsulphonyl, cyclopropylsulphonyl,
   amino, C₁₋₄-alkyl-amino, di-(C₁₋₃-alkyl)-amino, cyclopropyl-amino, N-(cyclopropyl)-N-(methyl)-amino, C₁₋₃-alkyl-carbonyl-amino,
   pyrrolidin-1-yl, piperidin-1-yl, 3-amino-piperidin-1-yl, 4-amino-piperidin-1-yl, morpholin-4-yl, thiomorpholin-4-yl, 1-oxo-1λ⁴-thiomorpholin-4-yl, 1,1-dioxo-1λ⁶-thiomorpholin-4-yl, piperazin-1-yl, homopiperazin-1-yl, 4-(methyl)-piperazin-1-yl, 4-(C₁₋₂-alkylcarbonyl)-piperazin-1-yl, 4-(C₁₋₂-alkylsulphonyl)-piperazin-1-yl,
   tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, 2-oxotetrahydropyrimidinyl, imidazol-2-yl, 1-methyl-imidazol-2-yl, thiazol-2-yl, 4-ethoxycarbonyl-thiazol-2-yl, 3-ethoxycarbonylisoxazol-5-yl, oxazol-2-yl, 2,4-dihydroxy-pyrimidin-5-yl.1.2,4-triazol-3-yl and tetrazol-5-yl, or
   wherein the C₁₋₄-alkyl, C₁₋₃-alkoxy, C₁₋₄-alkyl-amino, di-(C₁₋₃-alkyl)-amino- and C₁₋₃-alkyl-carbonyl-amino groups mentioned hereinbefore for R⁵ may each be mono- or disubstituted independently of one another in the carbon skeleton by a group selected from among
      cyano, hydroxy, C₁₋₂-alkoxy, tetrahydro-pyran-2-yloxy,
      amino, C₁₋₃-alkyl-amino, di-(C₁₋₃-alkyl)-amino, (C₁₋₃-alkylcarbonyl)-amino, (C₁₋₃-alkylsulphonyl)-amino,
      pyrrolidin-1-yl, piperidin-1-yl, morpholin-4-yl, piperazin-1-yl, 4-(methyl)-piperazin-1-yl,
      carboxyl, C₁₋₂-alkoxy-carbonyl, aminocarbonyl, C₁₋₃-alkylaminocarbonyl, di-(C₁₋₃-alkyl)-aminocarbonyl and C₃₋₆-cycloalkyl-aminocarbonyl, wherein the substituents must not be bound to a common carbon atom, and
   wherein the mentioned hereinbefore for R⁵ erwähnten C₁₋₄-alkylaminocarbonyl- and di-(C₁₋₂-alkyl)-aminocarbonyl groups in the carbon skeleton may each be mono- or disubstituted independently of one another by amino, hydroxy, C₁₋₃-alkoxy, C₁₋₃-alkyl-amino or di-(C₁₋₃-alkyl)-amino, wherein the substituents must not be bound to a common carbon atom, and
wherein in the above-mentioned morpholin-4-yl and piperazin-1-yl groups in each case a methylene unit may be replaced by a carbonyl group,
but particularly those compounds of the above general formula I wherein
- R: denotes a group of the above-mentioned formula wherein
R¹ denotes H or a C₁₋₃-alkyl group optionally substituted by a di-(C₁₋₃-alkyl)-amino group,
R² and R³ independently of one another denote chlorine, bromine or methyl and
A denotes CH or N,
m denotes 0 or 1,
R⁴ denotes chlorine, methyl or ethyl,
and the heterocyclic group 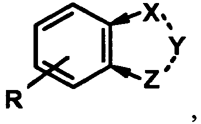 which may be substituted by R⁴as described hereinbefore may be substituted, denotes a group of formula 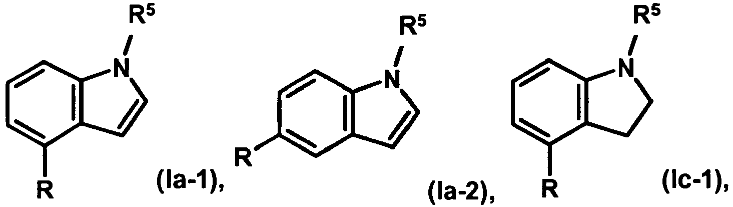 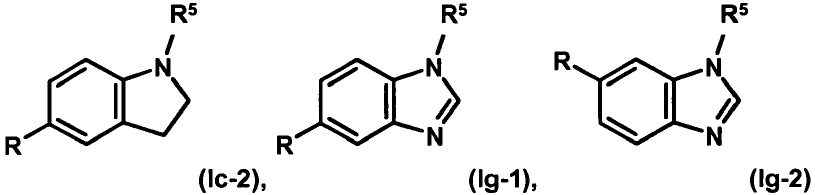 or 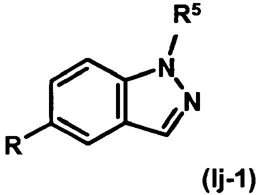 while the heterocycles mentioned hereinbefore may optionally be substituted at the carbon atoms of the 5 ring by one or two groups selected from among chlorine, bromine, iodine, C₁₋₂-alkyl, cyano and trifluoromethyl, wherein the groups may be identical or different and each carbon atom carries at most one group, and
R⁵ is as hereinbefore defined.

Most particularly preferred are those compounds of the above general formula I, wherein R denotes a group of the above-mentioned formula wherein
- R¹: denotes H, methyl, ethyl or 2-dimethylamino-ethyl,
R² and R³ independently of one another denote chlorine, bromine or methyl and
A denotes CH or N,
- m: denotes 0 or 1,
- R⁴: denotes chlorine, methyl or ethyl,
and the heterocyclic group 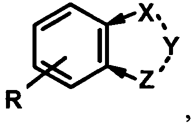 which may be substituted by R⁴ as described hereinbefore denotes a group of formula 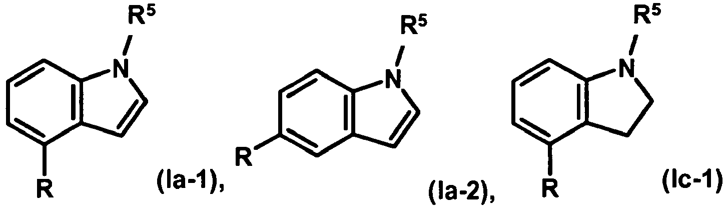 or 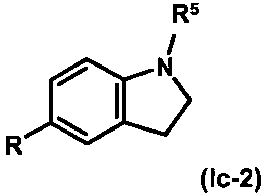
while the heterocycles mentioned hereinbefore may optionally be substituted at the carbon atoms of the 5 ring by one or two methyl or ethyl groups, wherein the groups may be identical or different and each carbon atom carries at most one group, and
wherein R⁵ denotes 1,3-dimethyl-1H-pyrimidin-2,4-dion-5-yl, 1H-pyrimidin-2,4-dion-6-yl, 1H-pyrimidin-2,4-dion-5-yl, 2H-pyridazin-3-on-6-yl, 1H-pyridin-2-on-3-yl, 1H-pyridin-2-on-5-yl, 1H-pyridin-2-on-4-yl or phenyl, pyridazin-3-yl, pyrimidin-2-yl, pyrimidin-4-yl, pyrimidin-5-yl, pyrazin-2-yl, pyridin-2-yl, pyridin-4-yl, imidazol-2-yl, imidazol-4-yl, pyrazol-3-yl, pyrazol-4-yl, 1.3,5-triazin-2-yl, thiazol-2-yl, [1.3.4]thiadiazol-2-yl, thiophen-2-yl, thiophen-3-yl, purin-6-yl, purin-2-yl or 1-imidazo[1,2-a]pyrazin-6-yl, which may be mono- or disubstituted independently of one another by a group selected from among
   chlorine, cyano, methyl, aminomethyl, morpholin-4-ylmethyl, hydroxymethyl, 3-hydroxypropyl, trifluoromethyl,
   hydroxy, methoxy, 2-hydroxyethoxy, 2-aminoethoxy, 2-dimethylaminoethoxy, 2-methylsulphonylamino-ethoxy, 2-acetylamino-ethoxy, 2,3-dihydroxy-propoxy,
   carboxyl, acetyl, ethylcarbonyl, aminocarbonyl, methyl-aminocarbonyl, dimethyl-aminocarbonyl, N-methoxy-N-methyl-aminocarbonyl, 2-dimethylamino-ethyl-aminocarbonyl, 2-hydroxy-ethyl-aminocarbonyl, 2-methoxy-ethyl-aminocarbonyl, cyclopropyl-aminocarbonyl,
   morpholin-4-yl-carbonyl, piperazin-1-yl-carbonyl, methoxy-carbonyl,
   methylsulphanyl, methylsulphinyl, ethylsulphinyl, methylsulphonyl,
   amino, methyl-amino, acetylamino, 2-aminoethyl-amino, 2-dimethylaminoethyl-amino, 2-hydroxyethyl-amino, 2-(methylamino)-ethyl-amino, N-carboxymethyl-N-(2-dimethylamino-ethyl)-amino, 2-(acetylamino)ethyl-amino, 2-(methylsulphonylamino)-ethyl-amino, 2-(pyrrolidin-1-yl)-ethyl-amino, 2-(piperidin-1-yl)-ethyl-amino, 2-(tetrahydro-pyran-2-yloxy)-ethylamino, 3-aminopropyl-amino, 3-(methylamino)-propyl-amino, 2-amino-2-methyl-propyl-amino, 1,3-dihydroxy-2-propyl-amino, 3-acetylaminopropyl-amino, 3-(methylsulphonylamino)-propyl-amino, dimethyl-amino, N-methyl-N-2-aminoethyl-amino, N,N-bis-2-(hydroxyethyl)-amino, N-methyl-N-(3-aminopropyl)-amino, N-methyl-N-[3-(acetylamino)-propyl]-amino, N-methyl-N-[3-(methylsulphonylamino)-propyl]-amino, cyclopropyl-amino,
   morpholin-4-yl, thiomorpholin-4-yl, 1-oxo-1λ⁴-thiomorpholin-4-yl, 1,1-dioxo-1λ⁶-thiomorpholin-4-yl, 3-amino-piperidin-1-yl, piperazin-1-yl, homopiperazin-1-yl , 4-acetyl-piperazin-1-yl, 4-methylsulphonyl-piperazin-1-yl,
   tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, imidazol-2-yl, 1-methyl-imidazol-2-yl, thiazol-2-yl, 4-ethoxycarbonylthiazol-2-yl, 3-ethoxycarbonyl-isoxazol-5-yl, and oxazol-2-yl,
wherein in the above-mentioned morpholin-4-yl and piperazin-1-yl groups in each case a methylene unit may be replaced by a carbonyl group,
but particularly those compounds of the above general formula I wherein
- R: denotes a group of the above-mentioned formula wherein
R¹ denotes hydrogen,
R² and R³ in each case denote chlorine and
A denotes CH,
- m: denotes 0 and
and the heterocyclic group 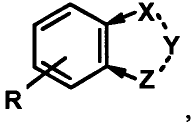 denotes a group of formula 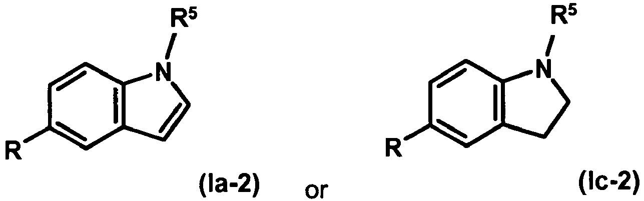 while the heterocycles mentioned hereinbefore may optionally be substituted at the carbon atoms of the 5-ring by one or two methyl groups, while each carbon atom carries at most one group, and wherein
R⁵ denotes phenyl, pyridazin-3-yl, pyrimidin-2-yl, pyrimidin-4-yl, pyrimidin-5-yl, pyrazin-2-yl, pyridin-2-yl or pyridin-4-yl, which may be substituted by a group selected from among
   cyano, methyl, aminomethyl, hydroxymethyl, 3-hydroxypropyl, trifluoromethyl,
   hydroxy, methoxy, 2-hydroxyethoxy, 2-aminoethoxy, 2-(dimethylamino)-ethoxy, 2-(methylsulphonylamino)-ethoxy, 2-(acetylamino)-ethoxy, 2,3-dihydroxy-propoxy,
   carboxyl, acetyl, ethylcarbonyl, aminocarbonyl, methyl-aminocarbonyl, dimethyl-aminocarbonyl, N-methoxy-N-methyl-aminocarbonyl, 2-(dimethylamino)-ethyl-aminocarbonyl, 2-hydroxy-ethyl-aminocarbonyl, 2-methoxy-ethyl-aminocarbonyl, cyclopropyl-aminocarbonyl, morpholin-4-yl-carbonyl, piperazin-1-yl-carbonyl, methoxy-carbonyl,
   methylsulphanyl, methylsulphinyl, ethylsulphinyl, methylsulphonyl,
   amino, methyl-amino, acetylamino, 2-aminoethyl-amino, 2-(dimethylamino)-ethyl-amino, 2-hydroxyethyl-amino, 2-(methylamino)-ethyl-amino, N-carboxymethyl-N-[2-(dimethylamino)-ethyl]-amino, 2-(acetylamino)-ethyl-amino, 2-(methylsulphonylamino)-ethyl-amino, 2-(pyrrolidin-1-yl)-ethyl-amino, 2-(piperidin-1-yl)-ethyl-amino, 3-aminopropyl-amino, 3-(methylamino)-propyl-amino, 3-(acetylamino)-propyl-amino, 3-(methylsulphonylamino)-propyl-amino, dimethyl-amino, N-methyl-N-2-aminoethyl-amino, N,N-bis-2-(hydroxyethyl)-amino, N-methyl-N-3-aminopropyl-amino, N-methyl-N-[3-(acetylamino)-propyl]-amino, N-methyl-N-[3-(methylsulphonylamino)-propyl]-amino, cyclopropyl-amino,
   morpholin-4-yl, thiomorpholin-4-yl, 1-oxo-1λ⁴-thiomorpholin-4-yl, 1,1-dioxo-1λ⁶-thiomorpholin-4-yl, 3-amino-piperidin-1-yl, piperazin-1-yl, homopiperazin-1-yl, 4-acetyl-piperazin-1-yl, 4-methylsulphonyl-piperazin-1-yl,
   tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, imidazol-2-yl, 1-methyl-imidazol-2-yl, thiazol-2-yl, 4-ethoxycarbonyl-thiazol-2-yl, 3-ethoxycarbonyl-isoxazol-5-yl and oxazol-2-yl.

The following preferred compounds are mentioned by way of example:
(1) ((3,5-dichloro-phenylsulphonyl)-{1-[5-(2-dimethylamino-ethylamino)-pyrazin-2-yl]-1H-indol-5-yl}-amino)-acetic acid
(2) {(3,5-dichloro-phenylsulphonyl)-[1-(6-piperazin-1-yl-pyridazin-3-yl)-2,3-dihydro-1H-indol-5-yl]-amino}-acetic acid
(3) ((3,5-dichloro-phenylsulphonyl)-{1-[6-(2-dimethylamino-ethylamino)-pyridazin-3-yl]-1H-indol-5-yl}-amino)-acetic acid
(4) {(3,5-dichloro-phenylsulphonyl)-[1-(6-methyl-pyridazin-3-yl)-1H-indol-5-yl]-amino}-acetic acid
(5) ((3-bromo-5-methyl-phenylsulphonyl)-{1-[6-(2-dimethylamino-ethylamino)-pyridazin-3-yl]-1H-indol-5-yl}-amino)-acetic acid
(6) {(3,5-dichloro-phenylsulphonyl)-[1-(6-piperazin-1-yl-pyridazin-3-yl)-1H-indol-5-yl]-amino}-acetic acid
(7) [[1-(6-[1,4]diazepan-1-yl-pyridazin-3-yl)-1H-indol-5-yl]-(3,5-dichlorophenylsulphonyl)-amino]-acetic acid
(8) {(3,5-dichloro-phenylsulphonyl)-[1-(4-methyl-pyridin-2-yl)-1H-indol-5-yl]-amino}-acetic acid
(9) ((3,5-dichloro-phenylsulphonyl)-{1-[6-(2-dimethylamino-ethylamino)-pyridazin-3-yl]-3-methyl-1H-indol-5-yl}-amino)-acetic acid
(10) [(3,5-dichloro-phenylsulphonyl)-(1-{6-[methyl-(tetrahydro-pyran-4-yl)-amino]-pyridazin-3-yl}-1H-indol-5-yl)-amino]-acetic acid
(11) ((3,5-dichloro-phenylsulphonyl)-{1-[5-(3-oxo-piperazin-1-yl)-pyrazin-2-yl]-1H-indol-5-yl}-amino)-acetic acid
(12) [[6-ethyl-1-(6-methyl-pyridazin-3-yl)-1H-indol-5-yl]-(3,5-dichlorophenylsulphonyl)-amino]-acetic acid
(13) [[1-(4-amino-pyridin-2-yl)-1H-indol-5-yl]-(3,5-dichloro-phenylsulphonyl)-amino]-acetic acid
(14) ((3,5-dichloro-phenylsulphonyl)-{1-[6-(3-hydroxy-propyl)-pyridazin-3-yl]-1H-indol-5-yl}-amino)-acetic acid
(15) [{1-[6-(4-acetyl-piperazin-1-yl)-pyridazin-3-yl]-1H-indol-5-yl}-(3,5-dichlorophenylsulphonyl)-amino]-acetic acid
(16) {(3,5-dichloro-phenylsulphonyl)-[1-(4-methylcarbamoyl-pyridin-2-yl)-1H-indol-5-yl]-amino}-acetic acid
(17) {(3,5-dichloro-phenylsulphonyl)-[3-methyl-1-(6-methyl-pyridazin-3-yl)-1H-indol-5-yl]-amino}-acetic acid
(18) {(3,5-dichloro-phenylsulphonyl)-[1-(4-methanesulphinyl-pyridin-2-yl)-1H-indol-5-yl]-amino}-acetic acid
(19) [[1-(4-cyclopropylcarbamoyl-pyridin-2-yl)-1H-indol-5-yl]-(3,5-dichlorophenylsulphonyl)-amino]-acetic acid
(20) {(2,6-dichloro-pyridin-4-sulphonyl)-[1-(4-methylcarbamoyl-pyridin-2-yl)-1H-indol-5-yl]-amino}-acetic acid
the enantiomers, the mixtures thereof and the salts thereof.

Some terms used hereinbefore and hereinafter to describe the compounds according to the invention are defined more specifically below.

The term halogen denotes an atom selected from among F, Cl, Br and I, particularly F, Cl and Br.

The term C₁₋ₙ-alkyl, wherein n may have a value as defined hereinbefore or hereinafter, denotes a saturated, branched or unbranched hydrocarbon group with 1 to n C atoms. Examples of such groups include methyl, ethyl, n-propyl, iso-propyl, butyl, iso-butyl, sec-butyl, tert-butyl, n-pentyl, iso-pentyl, neo-pentyl, tert-pentyl, n-hexyl, iso-hexyl, etc..

The term C₂₋ₙ-alkynyl, wherein n has a value as defined hereinbefore, denotes a branched or unbranched hydrocarbon group with 2 to n C atoms and a C≡C triple bond. Examples of such groups include ethynyl, 1-propynyl, 2-propynyl, iso-propynyl, 1-butynyl, 2-butynyl, 3-butynyl, 2-methyl-1-propynyl, 1-pentynyl, 2-pentynyl, 3-pentynyl, 4-pentynyl, 3-methyl-2-butynyl, 1-hexynyl, 2-hexynyl, 3-hexynyl, 4-hexynyl, 5-hexynyl etc..

The term C₂₋ₙ-alkenyl, wherein n has a value as defined hereinbefore, denotes a branched or unbranched hydrocarbon group with 2 to n C atoms and a C=C double bond. Examples of such groups include ethenyl, 1-propenyl, 2-propenyl, iso-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 2-hexenyl, 3-hexenyl, 4-hexenyl, 5-hexenyl etc..

The term C₁₋ₙ-alkoxy or C₁₋ₙ-alkyloxy denotes a C₁₋ₙ-alkyl-O group, wherein C₁₋ₙ-alkyl is as hereinbefore defined. Examples of such groups include methoxy, ethoxy, n-propoxy, iso-propoxy, n-butoxy, iso-butoxy, sec-butoxy, tert-butoxy, n-pentoxy, iso-pentoxy, neo-pentoxy, tert-pentoxy, n-hexoxy, iso-hexoxy etc..

The term C₁₋ₙ-alkyl-carbonyl denotes a C₁₋ₙ-alkyl-C(=O) group, wherein C₁₋ₙ-alkyl is as hereinbefore defined. Examples of such groups include methylcarbonyl, ethylcarbonyl, n-propylcarbonyl, iso-propylcarbonyl, n-butylcarbonyl, iso-butylcarbonyl, sec-butylcarbonyl, tert-butylcarbonyl, n-pentylcarbonyl, iso-pentylcarbonyl, neo-pentylcarbonyl, tert-pentylcarbonyl, n-hexylcarbonyl, iso-hexylcarbonyl, etc..

The term C₃₋ₙ-cycloalkyl denotes a saturated mono-, bi-, tri- or spirocarbocyclic group with 3 to n C atoms. Examples of such groups include cyclopropyl, cyclobutyl, cyclopentyl, cyclohexyl, cycloheptyl, cyclooctyl, cyclononyl, cyclododecyl, bicyclo[3.2.1.]octyl, spiro[4.5]decyl, norpinyl, norbornyl, norcaryl, adamantyl, etc.. Preferably the term C₃₋₇-cycloalkyl includes saturated monocyclic groups.

The term C₃₋ₙ-cycloalkyloxy or C₃₋ₙ-cycloalkoxy d a C₃₋ₙ-cycloalkyl-O group, wherein C₃₋ₙ-cycloalkyl is as hereinbefore defined. Examples of such groups include cyclopropyloxy, cyclobutyloxy, cyclopentyloxy, cyclohexyloxy, cycloheptyloxy, etc..

The term C₁₋ₙ-alkoxy-carbonyl denotes a C₁₋ₙ-alkyl-O-C(=O) group, wherein C₁₋ₙ-alkyl is as hereinbefore defined.

The term C₃₋ₙ-cycloalkyl-carbonyl denotes a C₃₋ₙ-cycloalkyl-C(=O) group, wherein C₃₋ₙ-cycloalkyl is as hereinbefore defined.

The terms C₁₋ₙ-alkyl-amino and di-(C₁₋ₙ-alkyl)-amino denote a C₁₋ₙ-alkyl-NH-or a di-(C₁₋ₙ-alkyl)-N group, respectively, wherein C₁₋ₙ-alkyl is as hereinbefore defined.

The term C₃₋ₙ-cycloalkyl-amino denotes a C₃₋ₙ-cycloalkyl-NH group, wherein C₃₋ₙ -cycloalkyl is as hereinbefore defined.

The term N-(C₃₋ₙ-cycloalkyl)-N-(C₁₋ₙ-alkyl)-amino denotes an N-(C₃₋ₙ-cycloalkyl)-N-(C₁₋ₙ-alkyl)-N group, wherein C₃₋ₙ-cycloalkyl and C₁₋ₙ-alkyl are as hereinbefore defined.

The terms C₁₋ₙ-alkyl-aminocarbonyl and di-(C₁₋ₙ-alkyl)-aminocarbonyl denote a C₁₋ₙ-alkyl-NH-C(=O)- or a di-(C₁₋ₙ-alkyl)-N-C(=O) group, respectively, wherein C₁₋ₙ-alkyl is as hereinbefore defined.

The term C₃₋ₙ-cycloalkyl-aminocarbonyl denotes a C₃₋ₙ-cycloalkyl-NH-C(=O) group, wherein C₃₋ₙ-cycloalkyl is as hereinbefore defined.

The term N-(C₃₋ₙ-cycloalkyl)-N-(C₁₋ₙ-alkyl)-amino denotes an N-(C₃₋ₙ-cycloalkyl)-N-(C₁₋ₙ-alkyl)-N-C(=O) group, wherein C₃₋ₙ-cycloalkyl and C₁₋ₙ-alkyl are as hereinbefore defined.

The terms di-(C₁₋ₙ-alkyl)amino and di-(C₁₋ₙ-alkyl)aminocarbonyl, wherein n has a value as defined hereinbefore, encompass amino groups which have the same or two different alkyl groups.

The term C₁₋ₙ-perfluoroalkyl denotes a F-(CF2)ₙ group. Examples of such groups include trifluoromethyl, pentafluoroethyl, heptafluoro-n-propyl, heptafluoro-iso-propyl etc., but preferably trifluoromethyl, pentafluoroethyl.

The term C₁₋ₙ-perfluoroalkoxy denotes a F-(CF2)ₙ-O group. Examples of such groups include trifluoromethoxy, pentafluoroethoxy, heptafluoro-n-propoxy, heptafluoro-iso-propoxy etc., but preferably trifluoromethoxy, pentafluoroethoxy.

The term C₁₋ₙ-alkylsulphanyl denotes a C₁₋ₙ-alkyl-S group, wherein C₁₋ₙ-alkyl is as hereinbefore defined.
The term C₁₋ₙ-alkylsulphinyl denotes a C₁₋ₙ-alkyl-S(=O) group, wherein C₁₋ₙ-alkyl is as hereinbefore defined.
The term C₁₋ₙ-alkylsulphonyl denotes a C₁₋ₙ-alkyl-S(=O)₂ group, wherein C₁₋ₙ-alkyl is as hereinbefore defined.

The term C₃₋ₙ-cycloalkylsulphanyl denotes a C₃₋ₙ-cycloalkyl-S group, wherein C₃₋ₙ-cycloalkyl is as hereinbefore defined.
The term C₃₋ₙ-cycloalkylsulphinyl denotes a C₃₋ₙ-cycloalkyl-S(=O) group, wherein C₃₋ₙ-cycloalkyl is as hereinbefore defined.
The term C₃₋ₙ-cycloalkylsulphonyl denotes a C₃₋ₙ-cycloalkyl-S(=O)₂ group, wherein C₃₋ₙ-cycloalkyl is as hereinbefore defined.

The compounds according to the invention may be obtained using methods of synthesis that are known in principle. Preferably the compounds are obtained by methods of preparation according to the invention that are described more fully hereinafter.

The preparation of compounds of general formula I may be carried out according to Process a) according to the invention shown in Scheme 1, wherein X, Y, Z, R¹, R², R³, A, m and R⁴ are as hereinbefore defined, starting from a compound of general formula II.

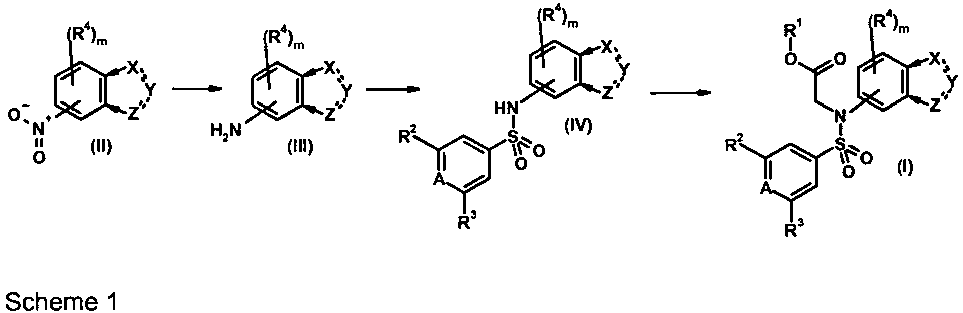

Here, compounds of general formula III are obtained by reacting a compound of general formula II with a reducing agent.
A suitable reducing agent is for example hydrogen in the presence of a catalyst, such as palladium on charcoal, palladium hydroxide on charcoal or Raney nickel, while palladium on charcoal is particularly suitable. The hydrogenation is carried out in a suitable solvent such as methanol, ethanol, isopropanol, tetrahydrofuran, dichloromethane or ethyl acetate, but preferably methanol, ethanol or tetrahydrofuran, at a pressure between 0.5 and 7 bar, but preferably at a pressure between 0.5 and 3 bar, and at a temperature between 0°C and 60°C, but preferably at a temperature between 15°C and 40°C.
Also suitable for the reduction is tin dichloride hydrate in lower alcoholic solvents such as methanol or ethanol at a temperature between ambient temperature and 80°C.
Alternatively titanium trichloride may be used as reducing agent. Suitable solvents are mixtures of acetone and water. The reaction is carried out between 0°C and 60°C, but preferably between 15°C and 40°C and in the presence of ammonium acetate.

Compounds of general formula IV are obtained by sulphonylation of compounds of general formula III.
The sulphonylation is carried out with aromatic sulphonyl chlorides in the presence of a base, such as triethylamine, N,N-diisopropyl-N-ethyl-amine, pyridine, or 4-dimethylamino-pyridine, but preferably pyridine. The reaction may be carried out in suitable solvents, such as diethyl ether, tetrahydrofuran, toluene, pyridine, dichloromethane, or chloroform, but preferably dichloromethane. The temperature may be between 0°C and 60°C, but preferably between 15°C and 40°C.

Compounds of general formula I are obtained from compounds of general formula IV by alkylation.
Suitable alkylating agents are acetic acid derivatives that contain a leaving group such as chlorine, bromine, iodine, p-tolylsulphonate, methylsulphonate, or trifluoromethylsulphonate in the 2-position. The alkylation is carried out in a solvent such as dimethylformamide, dimethylacetamide, tetrahydrofuran, acetonitrile, N-methylpyrrolidone or dimethylsulphoxide, but preferably in dimethylformamide, in the presence of a base such as sodium carbonate, potassium carbonate or caesium carbonate, but preferably potassium carbonate, and at a temperature between 0°C and 100°C, but preferably between 15°C and 50°C.
If acetic acid derivatives with a tert.-butyl ester unit are used as alkylating agents, compounds of general formula I are obtained wherein R¹ = tert.-butyl. The cleaving of the tert.-butyl group is preferably carried out by treatment with an acid such as trifluoroacetic acid or hydrochloric acid or by treatment with iodotrimethylsilane optionally using a solvent such as methylene chloride, dioxane, methanol or diethyl ether.

Compounds of general formula II, wherein R⁵ is bound to X and X denotes nitrogen may be obtained by Process b) according to the invention shown in Scheme 2 from compounds of general formula V, wherein m and R⁴ are as hereinbefore defined and -Y^{...}Z→ has the meaning -CH=CH→, -CH₂-CH₂→ or - N=CH→, but preferably has the meaning -CH=CH→ or -CH₂-CH₂→, while the carbon atoms therein may be substituted as hereinbefore defined and R⁵ denotes an aryl group.

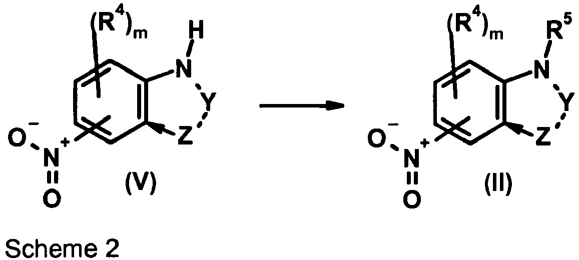

Aryl groups may be introduced by reacting with nitrogen-containing aromatic groups which contain at the carbon atom adjacent to the nitrogen a leaving group such as fluorine, chlorine, bromine, iodine, alkylsulphanyl, arylsulphanyl, alkylsulphinyl, arylsulphinyl, alkylsulphonyl or arylsulphonyl, but preferably chlorine, bromine or iodine. The reaction may be carried out without a solvent, at temperatures between 70°C and 220°C, but preferably between 120°C and 190°C.
Alternatively the reaction may be carried out in a dipolar-aprotic solvent such as dimethylformamide, dimethylacetamide, tetrahydrofuran, acetonitrile, N-methylpyrrolidone or dimethylsulphoxide, but preferably in dimethylformamide or N-methylpyrrolidone, in the presence of a base such as triethylamine, N,N-diisopropyl-N-ethyl-amine, sodium carbonate, potassium carbonate, caesium carbonate, sodium hydride, potassium-tert.-butoxide or potassium-hexamethyldisilazide, but preferably sodium hydride, potassium carbonate or potassium-tert.-butoxide, and at a temperature between 0°C and 150°C, but preferably between 15°C and 100°C.

The arylation reaction may also be carried out for compounds of general formula V wherein -Y^{...}Z→ has the meaning -CH=CH→, while the carbon atoms therein may be substituted as hereinbefore defined, according to the process described in J. Am. Chem. Soc. 2002, 124, 11684-11688, to obtain compounds of general formula II wherein -Y^{...}Z→ has the meaning -CH=CH→, wherein the carbon atoms therein may be substituted as hereinbefore defined. Compounds of general formula V are reacted with arylbromides or aryliodides. The reaction is carried out in toluene or dioxane in the presence of potassium phosphate as base, catalytic amounts of a copper-(I) salt, but preferably copper-(I)-iodide and catalytic amounts of a 1,2-diamino ligand such as for example ethylenediamine, N,N-ethylenediamine, N,N'-ethylenediamine, cis-cyclohexane-1,2-diamine, trans-cyclohexane-1,2-diamine, N,N'-dimethyl-cis-cyclohexane-1,2-diamine or N,N'-dimethyl-trans-cyclohexane-1,2-diamine, but preferably N,N'-dimethyl-trans-cyclohexane-1,2-diamine, at a temperature between 70°C and 130°C, but preferably between 90°C and 110°C.

End compounds of general formula VII which contain an indole scaffold may be obtained according to Process c) according to the invention shown in Scheme 3, wherein R¹, R², R³, A, R⁴ and m are as hereinbefore defined and the carbon atoms of the 5 ring may be substituted as hereinbefore defined, from compounds of general formula VI.

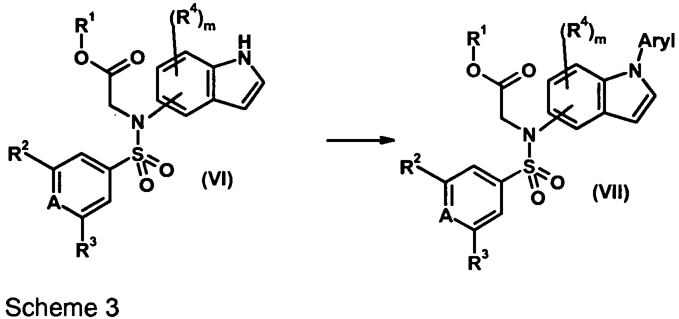

The arylation reaction is carried out according to methods known from the literature, as described for example in J. Am. Chem. Soc. 2002, 124, 11684-11688. Compounds of general formula V are reacted with arylbromides or aryliodides. The reaction is carried out in toluene or dioxane in the presence of potassium phosphate as base, catalytic amounts of a copper-(I) salt, but preferably copper-(I)-iodide and catalytic amounts of a 1,2-diamino ligand such as for example ethylenediamine, N,N-ethylenediamine, N,N'-ethylenediamine, cis-cyclohexane-1,2-diamine, trans-cyclohexane-1,2-diamine, N,N'-dimethyl-cis-cyclohexane-1,2-diamine or N,N'-dimethyl-trans-cyclohexane-1,2-diamine, but preferably N,N'-dimethyl-trans-cyclohexane-1,2-diamine, at a temperature between 70°C and 130°C, but preferably between 90°C and 110°C.

If compounds of general formula VI wherein R¹ = tert.-butyl are used, compounds of general formula VII are obtained wherein R¹ = tert.-butyl. The cleaving of the tert.-butyl group is then preferably carried out by treatment with an acid such as trifluoroacetic acid or hydrochloric acid or by treatment with iodotrimethylsilane, optionally using a solvent such as methylene chloride, dioxane, methanol or diethyl ether.

Central scaffold components of the general formulae II or III, which are not commercially obtainable, may be obtained by methods known from the literature. Indoles, for example, may be obtained by converting 4-nitrophenyl-hydrazine into a hydrazone with subsequent Fischer indole synthesis as described in Organic Preparations and Procedures International 1991, 23(3), 357-363. Alternatively, indole components may be obtained starting from substituted 4-nitroanilines analogously to a process as described in Tetrahedron 2003, 59, 1571-1587. The starting compounds of general formula II may also be prepared by nitration (Houben-Weyl, Methoden der organischen Chemie, Volume X/1, 463-890) using methods known *per se*, from commercially obtainable compounds.

Indoline components may be obtained starting from the indoles. The indole is dissolved in acetic acid, optionally with the addition of trifluoroacetic acid, or in trifluoroacetic acid and reacted with a reducing agent such as for example sodium cyanoborohydride or sodium triacetoxyborohydride at temperatures between - 20°C and 100°C, but preferably at between 0°C and 60°C.

Cyano functionalities may in each case be prepared from primary amides obtained in the syntheses. Suitable methods for this transformation are, for example, reaction with thionyl chloride and optionally catalytic amounts of dimethylformamide in a solvent such as dichloromethane, 1,2-dichloroethane, toluene or acetone at temperatures between 0°C and 100°C, reaction with trifluoroacetic anhydride or trichloroacetic anhydride, a base such as for example pyridine, triethylamine or N,N-diisopropyl-N-ethyl-amine in a solvent such as for example dichloromethane, 1,2-dichloroethane, tetrahydrofuran, 1,4-dioxane or toluene at temperatures between -10°C and 100°C, as well as reaction with phosphorus oxychloride and optionally a base such as pyridine or N,N-dimethylaniline in the presence or absence of a solvent such as for example dichloromethane, 1,2-dichloroethane, tetrahydrofuran, 1,4-dioxane or toluene, at temperatures between -10°C and 120°C.

Sulphonyl chlorides may be prepared from anilines. For this, the aniline is first diazotised by reacting with sodium nitrite in hydrochloric acid at temperatures between - 30°C and 10°C. The diazonium salt solution thus prepared is then added dropwise to copper-II-chloride and water in a 30% sulphur dioxide solution in glacial acetic acid at temperatures between - 30°C and 10°C. Then it is left to warm up to temperatures between 5°C and 50°C. Alternatively the sulphonyl chlorides may be prepared from aryl metal compounds such as aryl lithium or aryl magnesium chloride compounds. Aryl lithium compounds are obtained from the aryl bromides or aryl iodides by reacting with n-butyllithium, sec-butyllithium or tert.-butyllithium in a solvent such as diethyl ether or tetrahydrofuran at temperatures between
- 60°C and - 85°C. Arylmagnesium chloride compounds are obtained by a process as described in Angew. Chem. 2006, 118, 3024-3027. The aryl metal compounds thus obtained are further reacted at temperatures between -78°C and -20°C by piping sulphur dioxide through. This produces metal sulphinates, which can optionally be precipitated by the addition of hexane. The metal sulphinates are dissolved in dichloromethane and combined with N-chlorosuccinimide at temperatures between -20°C and 30°C. After the reaction the solid is filtered off, to obtain a dichloromethane solution of the sulphonyl chloride.

Heteroaryliodides, which are needed for the synthesis of the compounds of general formula I, may be prepared from the corresponding heteroaryl chlorides or heteroaryl bromides. For this the heteroaryl chlorides or heteroaryl bromides are reacted with concentrated hydriodic acid at temperatures between ambient temperature between 50°C and 180°C. Alternatively heteroarylbromides may be reacted with sodium iodide, potassium iodide or tetrabutylammonium iodide in a solvent such as tetrahydrofuran, dioxane, dimethylethyleneglycol or toluene in the presence of catalytic amounts of copper-I-iodide as well as a ligand such as N,N'-dimethyl-trans-cyclohexandiamine at temperatures between 60°C and 150°C to obtain the heteroaryl iodides.

In the reactions described hereinbefore, any reactive groups present such as carboxy, hydroxy, amino or alkylamino groups may be protected during the reaction by conventional protecting groups which are cleaved again after the reaction.

For example, a protecting group for a carboxy group may be a methyl, ethyl, tert.butyl or benzyl group.

For example, a protecting group for a hydroxy group may be an acetyl, benzyl or tetrahydropyranyl group.

Protecting groups for an amino or alkylamino may be a formyl, acetyl, trifluoroacetyl, ethoxycarbonyl, tert.butoxycarbonyl, benzyloxycarbonyl, benzyl, methoxybenzyl or 2,4-dimethoxybenzyl group.

A carboxymethyl or carboxyethyl unit is cleaved for example by hydrolysis in an aqueous solvent, e.g. In water, methanol/water, isopropanol/water, acetic acid/water, tetrahydrofuran/water or dioxane/water, but preferably in methanol/water, in the presence of an acid such as trifluoroacetic acid, hydrochloric acid or sulphuric acid or in the presence of an alkali metal base such as lithium hydroxide, sodium hydroxide or potassium hydroxide, but preferably sodium hydroxide, or aprotically, e.g. In the presence of iodotrimethylsilane, at temperatures between 0 and 120°C, preferably at temperatures between 10 and 100°C.

A benzyl, methoxybenzyl or benzyloxycarbonyl group is advantageously cleaved by hydrogenolysis, e.g. with hydrogen in the presence of a catalyst such as palladium on charcoal in a suitable solvent such as methanol, ethanol, ethyl acetate or glacial acetic acid, optionally with the addition of an acid such as hydrochloric acid, at temperatures between 0 and 100°C, but preferably at temperatures between 20 and 60°C, and under a hydrogen pressure of 1 to 7 bar, but preferably 1 to 3 bar. However, a 2,4-dimethoxybenzyl group is preferably cleaved in trifluoroacetic acid in the presence of anisole.

A tert.-butyl or tert.-butyloxycarbonyl group is preferably cleaved by treating with an acid such as trifluoroacetic acid or hydrochloric acid or by treating with iodotrimethylsilane, optionally using a solvent such as methylene chloride, dioxane, methanol or diethyl ether.

Moreover, the compounds of general formula I obtained, or intermediate products from the synthesis of compounds of general formula I, as already mentioned hereinbefore, may be resolved into their enantiomers and/or diastereomers. Thus, for example, cis/trans mixtures may be resolved into their cis and trans isomers, and compounds with at least one stereocentre may be resolved into their enantiomers.

Thus, for example, the cis/trans mixtures may be resolved by chromatography into the cis and trans isomers thereof, the compounds of general formula I obtained, or intermediate products from the synthesis of compounds of general formula I, which occur as racemates may be separated by methods known per se (cf. Allinger N. L. And Eliel E. L. In "Topics in Stereochemistry", Vol. 6, Wiley Interscience, 1971) into their optical antipodes and compounds of general formula I, or intermediate products from the synthesis of compounds of general formula I, with at least 2 asymmetric carbon atoms may be resolved into their diastereomers on the basis of their physical-chemical differences using methods known per se, e.g. by chromatography and/or fractional crystallisation, and, if these compounds are obtained in racemic form, they may subsequently be resolved into the enantiomers as mentioned above.

The enantiomers are preferably separated by chromatography on chiral phases or by recrystallisation from an optically active solvent or by reacting with an optically active substance which forms salts or derivatives such as e.g. esters or amides with the racemic compound, particularly acids and the activated derivatives or alcohols thereof, and separating the diastereomeric mixture of salts or derivatives thus obtained, e.g. on the basis of their differences in solubility, whilst the free antipodes may be released from the pure diastereomeric salts or derivatives by the action of suitable agents. Optically active acids in common use are e.g. The D- and L-forms of tartaric acid or dibenzoyltartaric acid, di-O-p-toluoyltartaric acid, malic acid, mandelic acid, camphorsulphonic acid, glutamic acid, aspartic acid or quinic acid. An optically active alcohol may be for example (+) or (-)-menthol and an optically active acyl group in amides, for example, may be a (+)-or (-)-menthyloxycarbonyl.

Furthermore, the compounds of formula I obtained, or intermediate products from the synthesis of compounds of general formula I, may be converted into the salts thereof, particularly for pharmaceutical use into the physiologically acceptable salts thereof with inorganic or organic acids. Acids which may be used for this purpose include for example hydrochloric acid, hydrobromic acid, sulphuric acid, methanesulphonic acid, phosphoric acid, fumaric acid, succinic acid, lactic acid, citric acid, tartaric acid or maleic acid.

Moreover, the new compounds of general formula I obtained, or intermediate products from the synthesis of compounds of general formula I, if they contain a carboxy group, may subsequently, if desired, be converted into the salts thereof with inorganic or organic bases, particularly for pharmaceutical use into the physiologically acceptable salts thereof. Suitable bases for this purpose include for example sodium hydroxide, potassium hydroxide, arginine, cyclohexylamine, ethanolamine, diethanolamine and triethanolamine.

The compounds of general formula I are inhibitors of the interaction between human liver glycogen phosphorylase (HLGP) and protein PPP1R3 (G_{L}-subunit of glycogen-associated protein phosphatase 1 (PP1)). The effect of the compounds on the binding of the protein PPP1R3 and the glycogen phosphorylase activated by phosphorylation is determined in a binding test based on SPA technology (Amersham Pharmacia). The binding of the substances inhibits the interaction of the glycogen phosphorylase with the protein PPP1 R3B. All measurements were made in triplicate in the 384-well format (Optiplate, Perkin Elmer).
Human glycogen phosphorylase is recombinantly expressed in E. Coli and purified. The isolated non-phosphorylated HLGP is radioactively labelled in a marking reaction with phosphorylase kinase (200-500 U/ mg, P2014, Sigma) and ³³P-gamma ATP (110 TBq/ mmol, Hartmann Analytic) (Ref.: Cohen et al., Methods Enzymol. 1988, Vol 159 pp 390). In a binding test, in a volume of 100 µl (test buffer: 50 mM Tris/HCl pH 7.0, 0.1 mM EGTA, 0.1% mercaptoethanol), different amounts of a test substance (final concentration: 1 nM to 30 µM) are incubated at ambient temperature for 16 hours with 100000 cpm of labelled HLGP, 375 µg streptavidin-SPA Beads (RPNQ 0007, Amersham Pharmacia), 0.1 µg GL-peptide (Biotin-FPEWPSYLGYEKLGPYY). After centrifuging for 5 minutes at 500 g the plate is measured (Topcount, Packard). The cpm values measured are used to calculate the IC₅₀ values specified. The basal value is determined in the absence of the peptide and the maximum value is determined in the absence of the test substance.

The compounds of general formula I have IC₅₀ values in the range from 9 nM to 15 µM.

In view of their ability to suppress the interaction of glycogen phosphorylase a with the GL-subunit of glycogen-associated protein phosphatase 1 (PP1), the compounds of general formula I according to the invention and the corresponding pharmaceutically acceptable salts thereof are theoretically suitable for treating and/or preventatively treating all those conditions or diseases that can be influenced by inhibiting the interaction of glycogen phosphorylase a with the GL-subunit of glycogen-associated protein phosphatase 1 (PP1). Therefore the compounds according to the invention are particularly suitable for the prevention or treatment of diseases, particularly metabolic disorders, or conditions such as type 1 and type 2 diabetes mellitus, complications of diabetes (such as e.g. retinopathy, nephropathy or neuropathies, diabetic foot, ulcers, macroangiopathies), metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, oedema and hyperuricaemia. These substances are also suitable for preventing beta-cell degeneration such as e.g. Apoptosis or necrosis of pancreatic beta cells. The substances are also suitable for improving or restoring the functionality of pancreatic cells, and also for increasing the number and size of pancreatic beta cells. The compounds according to the invention may also be used as diuretics or antihypertensives and are suitable for the prevention and treatment of acute renal failure.

In particular, the compounds according to the invention, including the physiologically acceptable salts thereof, are suitable for the prevention or treatment of diabetes, particularly type 1 and type 2 diabetes mellitus, and/or diabetic complications.

The dosage required to achieve the corresponding activity for treatment or prevention usually depends on the compound which is to be administered, the patient, the nature and gravity of the illness or condition and the method and frequency of administration and is for the patient's doctor to decide. Expediently, the dosage may be from 0.1 to 1000 mg, preferably 0.5 to 500 mg, by intravenous route, and 1 to 1000 mg, preferably 10 to 500 mg, by oral route, in each case administered 1 to 4 times a day. For this purpose, the compounds of formula I prepared according to the invention may be formulated, optionally together with other active substances, together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof, to produce conventional galenic preparations such as plain or coated tablets, capsules, powders, suspensions or suppositories.

The compounds according to the invention may also be used in conjunction with other active substances, particularly for the treatment and/or prevention of the diseases and conditions mentioned above. Other active substances which are suitable for such combinations include in particular those which potentiate the therapeutic effect of an inhibitor of the interaction of glycogen phosphorylase a with the G_{L} subunit of glycogen-associated protein phosphatase 1 (PP1) according to the invention with respect to one of the indications mentioned and/or which allow the dosage of an an inhibitor of the interaction of glycogen phosphorylase a with the GL subunit of glycogen-associated protein phosphatase 1 (PP1) according to the invention to be reduced. Therapeutic agents which are suitable for such a combination include, for example, antidiabetic agents such as metformin, sulphonylureas (e.g. glibenclamide, tolbutamide, glimepiride), nateglinide, repaglinide, thiazolidinediones (e.g. rosiglitazone, pioglitazone), PPAR-gamma-agonists (e.g. GI 262570) and antagonists, PPAR-gamma/alpha modulators (e.g. KRP 297), alpha-glucosidase inhibitors (e.g. miglitol, acarbose, voglibose), DPPIV inhibitors (e.g. sitagliptine, vildagliptine), SGLT2-inhibitors, alpha2-antagonists, insulin and insulin analogues, GLP-1 and GLP-1 analogues (e.g. exendin-4) or amylin. Other active substances suitable as combination partners are inhibitors of protein tyrosinephosphatase 1, substances that affect deregulated glucose production in the liver, such as e.g. inhibitors of glucose-6-phosphatase, or fructose-1,6-bisphosphatase, glycogen phosphorylase, glucagon receptor antagonists and inhibitors of phosphoenol pyruvate carboxykinase, glycogen synthase kinase or pyruvate dehydrokinase, lipid lowering agents such as for example HMG-CoA-reductase inhibitors (e.g. simvastatin, atorvastatin), fibrates (e.g. bezafibrate, fenofibrate), nicotinic acid and the derivatives thereof, PPAR-alpha agonists, PPAR-delta agonists, ACAT inhibitors (e.g. avasimibe) or cholesterol absorption inhibitors such as, for example, ezetimibe, bile acid-binding substances such as, for example, cholestyramine, inhibitors of ileac bile acid transport, HDL-raising compounds such as CETP inhibitors or ABC1 regulators or active substances for treating obesity, such as sibutramine or tetrahydrolipostatin, dexfenfluramine, axokine, antagonists of the cannabinoid1 receptor, MCH-1 receptor antagonists, MC4 receptor agonists, NPY5 or NPY2 antagonists or ß3-agonists such as SB-418790 or AD-9677 and agonists of the 5HT2c receptor.

Moreover, combinations with drugs for influencing high blood pressure, chronic heart failure or atherosclerosis such as e.g. A-II antagonists or ACE inhibitors, ECE inhibitors, diuretics, ß-blockers, Ca-antagonists, centrally acting antihypertensives, antagonists of the alpha-2-adrenergic receptor, inhibitors of neutral endopeptidase, thrombocyte aggregation inhibitors and others or combinations thereof are suitable. Examples of angiotensin II receptor antagonists are candesartan cilexetil, potassium losartan, eprosartan mesylate, valsartan, telmisartan, irbesartan, EXP-3174, L-158809, EXP-3312, olmesartan, medoxomil, tasosartan, KT-3-671, GA-0113, RU-64276, EMD-90423, BR-9701, etc. Angiotensin II receptor antagonists are preferably used for the treatment or prevention of high blood pressure and complications of diabetes, often combined with a diuretic such as hydrochlorothiazide.

A combination with uric acid synthesis inhibitors or uricosurics is suitable for the treatment or prevention of gout.

A combination with GABA-receptor antagonists, Na-channel blockers, topiramat, protein-kinase C inhibitors, advanced glycation end product inhibitors or aldose reductase inhibitors may be used for the treatment or prevention of complications of diabetes.

The dosage for the combination partners mentioned above is usefully 1/5 of the lowest dose normally recommended up to 1/1 of the normally recommended dose.

Therefore, in another aspect, this invention relates to the use of a compound according to the invention or a physiologically acceptable salt of such a compound combined with at least one of the active substances described above as a combination partner, for preparing a pharmaceutical composition which is suitable for the treatment or prevention of diseases or conditions which can be affected by inhibiting the interaction of glycogen phosphorylase a with the G_{L} subunit of glycogen-associated protein phosphatase 1 (PP1). These are preferably metabolic diseases, particularly one of the diseases or conditions listed above, most particularly diabetes or diabetic complications.

The use of the compound according to the invention, or a physiologically acceptable salt thereof, in combination with another active substance may take place simultaneously or at staggered times, but particularly within a short space of time. If they are administered simultaneously, the two active substances are given to the patient together; if they are used at staggered times the two active substances are given to the patient within a period of less than or equal to 12 hours, but particularly less than or equal to 6 hours.

Consequently, in another aspect, this invention relates to a pharmaceutical composition which comprises a compound according to the invention or a physiologically acceptable salt of such a compound and at least one of the active substances described above as combination partners, optionally together with one or more inert carriers and/or diluents.

Thus, for example, a pharmaceutical composition according to the invention comprises a combination of a compound of formula I according to the invention or a physiologically acceptable salt of such a compound and at least one angiotensin II receptor antagonist optionally together with one or more inert carriers and/or diluents.

The compound according to the invention, or a physiologically acceptable salt thereof, and the additional active substance to be combined therewith may both be present together in one formulation, for example a tablet or capsule, or separately in two identical or different formulations, for example as a so-called kit-of-parts.

In the foregoing and following text, H atoms of hydroxyl groups are not explicitly shown in every case in structural formulae. The Examples that follow are intended to illustrate the present invention :

### Preparation of the starting compounds:

### Example I

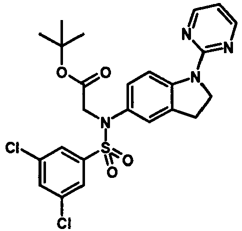

### tert.butyl [(3,5-dichloro-phenylsulphonyl)-(1-pyrimidin-2-yl-2,3-dihydro-1H-indol-5-yl)-amino]-acetate

50 mg 3,5-dichloro-N-(1-pyrimidin-2-yl-2,3-dihydro-1H-indol-5-yl)-phenylsulphonamide are dissolved in 2 ml dimethylformamide. To this are added 50 mg potassium carbonate and 50 µl tert.butyl bromoacetate. The mixture is stirred for 3 hours at ambient temperature, diluted with ethyl acetate and washed once with 10 % citric acid solution and once with saturated sodium sulphate solution. The organic phase is dried on magnesium sulphate. The solvents are eliminated in vacuo and the residue is purified by chromatography on silica gel (cyclohexane/ethyl acetate 95:5 to 50:50).
Yield: 50 mg (79 % of theory)
R_{f} value: 0.67 (silica gel, petroleum ether/ethyl acetate 1:1)

The following compounds are obtained analogously to Example I:
(1) tert.butyl {(3,5-dichloro-phenylsulphonyl)-[1-(1-methyl-1H-imidazol-2-yl)-2,3-dihydro-1H-indol-5-yl]-amino}-acetate 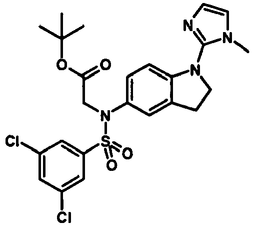 Mass spectrum (ESI⁺): m/z = 537 [M+H]⁺
(2) tert-butyl 2-{5-[tert-butoxycarbonylmethyl-(3,5-dichloro-phenylsulphonyl)-amino]-2,3-dihydro-indol-1-yl}-imidazole-1-carboxylate 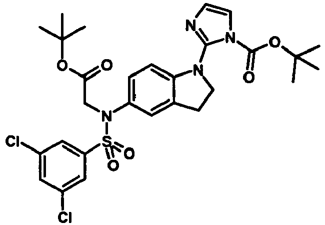 The crude product is further reacted directly in Example 3.
(3) tert.butyl [(3,5-dichloro-phenylsulphonyl)-(1-pyridin-4-yl-2,3-dihydro-1H-indol-5-yl)-amino]-acetate 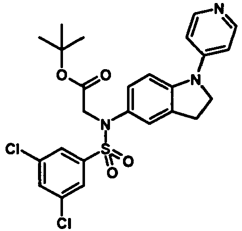 Mass spectrum (ESI⁺): m/z = 534 [M+H]⁺
(4) tert-butyl 4-(2-{5-[tert-butoxycarbonylmethyl-(3,5-dichloro-phenylsulphonyl)-amino]-2,3-dihydro-indol-1-yl}-pyrimidin-4-yl)-piperazine-1-carboxylate 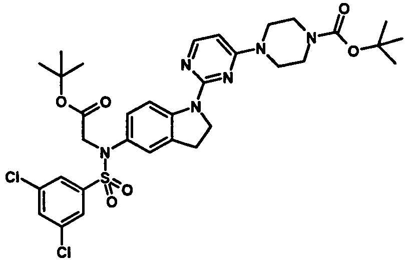 Mass spectrum (ESI⁺): m/z = 719 [M+H]+
(5) tert-butyl 4-(2-{5-[(3,5-dichloro-phenylsulphonyl)-ethoxycarbonylmethylamino]-2,3-dihydro-indol-1-yl}-pyrimidin-4-yl)-piperazine-1-carboxylate 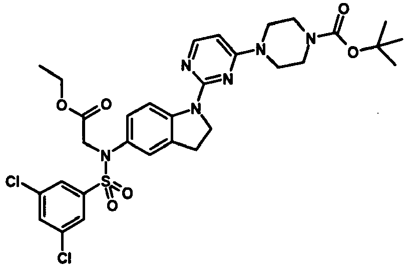 Instead of tert.butyl bromoacetate ethyl bromoacetate is used.
   Mass spectrum (ESI⁺): m/z = 691 [M+H]⁺
(6) tert-butyl 4-(2-{5-[tert-butoxycarbonylmethyl-(3,5-dichloro-phenylsulphonyl)-amino]-indol-1-yl}-pyrimidin-4-yl)-piperazine-1-carboxylate 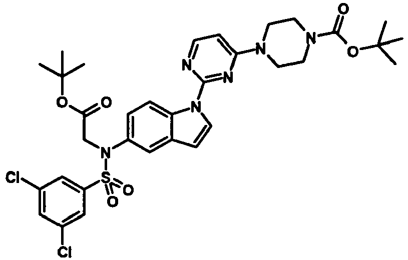 Mass spectrum (ESI⁺): m/z = 717 [M+H]⁺
(7) tert-butyl 4-(2-{5-[(3,5-dichloro-phenylsulphonyl)-ethoxycarbonylmethylamino]-indol-1-yl}-pyrimidin-4-yl)-piperazine-1-carboxylate 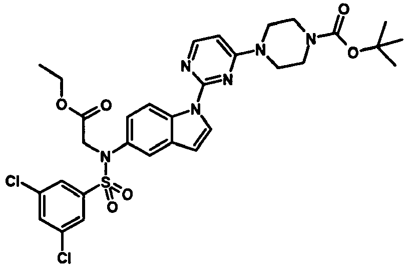 Instead of tert.butyl bromoacetate ethyl bromoacetate is used.
   Mass spectrum (ESI⁺): m/z = 689 [M+H]⁺
(8) tert-butyl 4-(5-{5-[tert-butoxycarbonylmethyl-(3,5-dichloro-phenylsulphonyl)-amino]-2,3-dihydro-indol-1-yl}-pyrazine-2-carbonyl)-piperazine-1-carboxylate 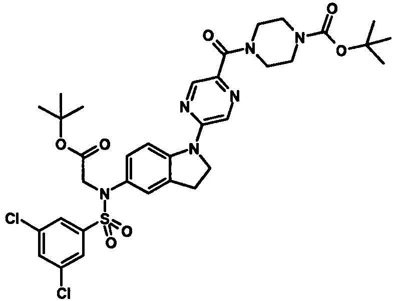 Mass spectrum (ESI⁺): m/z = 747 [M+H]⁺
(9) tert-butyl 4-(5-{5-[tert-butoxycarbonylmethyl-(3,5-dichloro-phenylsulphonyl)-amino]-indol-1-yl}-pyrazine-2-carbonyl)-piperazine-1-carboxylate 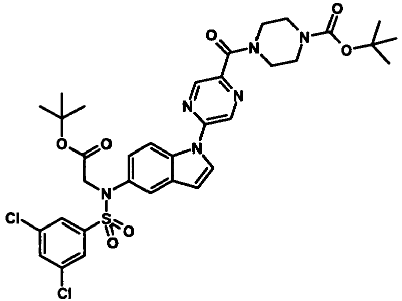 Mass spectrum (ESI⁺): m/z = 745 [M+H]⁺
(10) tert.butyl [(3,5-dichloro-phenylsulphonyl)-(1-pyrimidin-2-yl-1H-indol-4-yl)-amino]-acetate 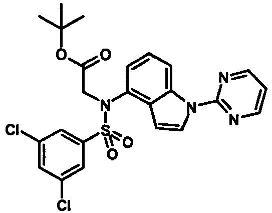 Mass spectrum (ESI⁺): m/z = 533 [M+H]⁺
(11) tert-butyl 6'-{5-[tert-butoxycarbonylmethyl-(3,5-dichloro-phenylsulphonyl)-amino]-indol-1-yl}-2,3,5,6-tetrahydro-[1,2']bipyrazinyl-4-carboxylate 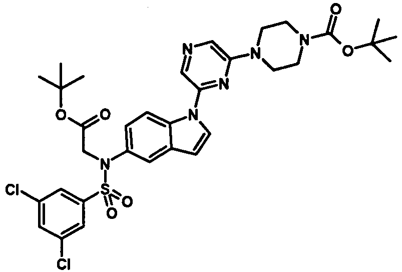 Mass spectrum (ESI⁺): m/z = 717 [M+H]⁺
(12) tert-butyl 6'-{5-[tert-butoxycarbonylmethyl-(3,5-dichloro-phenylsulphonyl)-amino]-2,3-dihydro-indol-1-yl}-2,3,5,6-tetrahydro-[1,2']bipyrazinyl-4-carboxylate 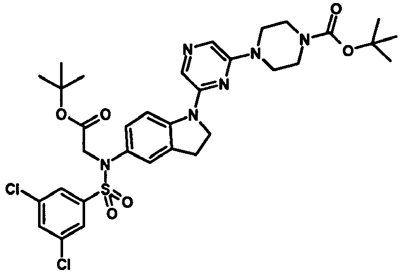 Mass spectrum (ESI⁺): m/z = 719 [M+H]⁺
(13) tert.butyl [(3,5-dichloro-phenylsulphonyl)-(1-pyrimidin-2-yl-2,3-dihydro-1H-indol-4-yl)-amino]-acetate 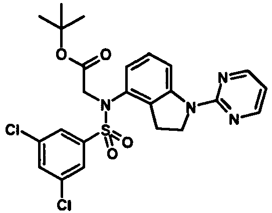 R_{f} value: 0.68 (silica gel, petroleum ether/ethyl acetate 1:1)
(14) tert.butyl [(1-{4-[tert-butoxycarbonylmethyl-(2-dimethylamino-ethyl)-amino]-pyrimidin-2-yl}-2,3-dihydro-1H-indol-5-yl)-(3,5-dichloro-phenylsulphonyl)-amino]-acetate 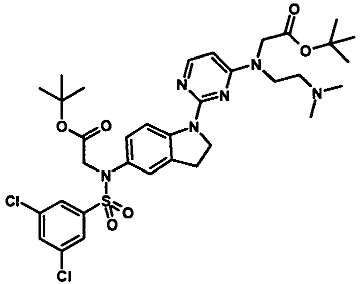 Starting from 3,5-dichloro-N-{1-[4-(2-dimethylamino-ethylamino)-pyrimidin-2-yl]-2,3-dihydro-1H-indol-5-yl}-phenylsulphonamide.
   Mass spectrum (ESI⁺): m/z = 735 [M+H]⁺
(15) tert-butyl 4-(6-{5-[tert-butoxycarbonylmethyl-(3,5-dichloro-phenylsulphonyl)-amino]-2,3-dihydro-indol-1-yl}-pyridazin-3-yl)-piperazine-1-carboxylate 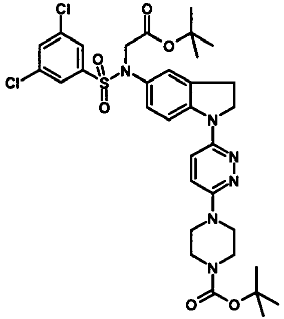 Mass spectrum (ESI⁺): m/z = 719 [M+H]⁺
(16) tert.butyl [(1-{4-[tert-butoxycarbonyl-(2-dimethylamino-ethyl)-amino]-pyrimidin-2-yl}-2,3-dihydro-1H-indol-5-yl)-(3,5-dichloro-phenylsulphonyl)-amino]-acetate 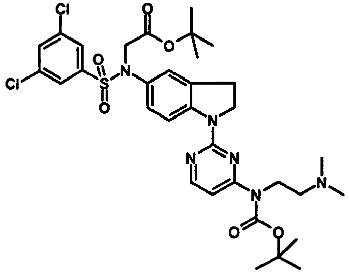 Mass spectrum (ESI⁺): m/z = 721 [M+H]⁺
(17) {2-[tert-butoxycarbonyl-(6-{5-[tert-butoxycarbonylmethyl-(3,5-dichlorophenylsulphonyl)-amino]-2,3-dihydro-indol-1-yl}-pyridazin-3-yl)-amino]-ethyl}-tert-butoxycarbonylmethyl-dimethyl-ammonium-bromide 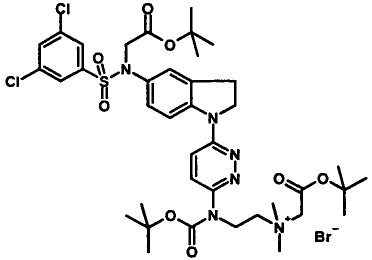 Mass spectrum (ESI⁺): m/z = 835 [M]⁺
(18) {2-[tert-butoxycarbonyl-(2-{5-[tert-butoxycarbonylmethyl-(3,5-dichlorophenylsulphonyl)-amino]-2,3-dihydro-indol-1-yl}-pyrimidin-4-yl)-amino]-ethyl}-tert-butoxycarbonylmethyl-dimethyl-ammonium-bromide 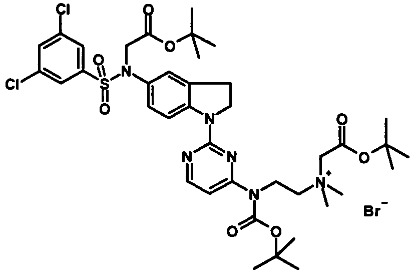 Mass spectrum (ESI⁺): m/z = 835 [M]⁺
(19) tert.butyl [(3,5-dimethyl-phenylsulphonyl)-(1H-indol-5-yl)-amino]-acetate 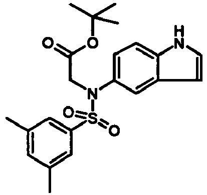 Mass spectrum (ESI⁺): m/z = 415 [M+H]⁺
(20) tert.butyl [(1-{6-[tert-butoxycarbonyl-(2-(bis-tert-butoxycarbonyl)-aminoethyl)-amino]-pyridazin-3-yl}-2,3-dihydro-1H-indol-5-yl)-(3,5-dichlorophenylsulphonyl)-amino]-acetate 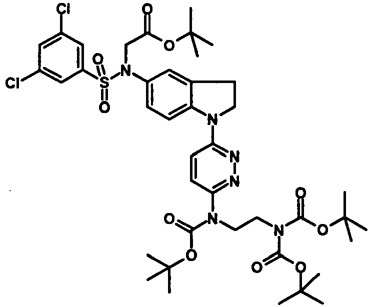 Mass spectrum (ESI⁺): m/z = 893 [M+H]⁺
(21) tert.butyl [(3,5-dichloro-phenylsulphonyl)-(1H-indol-5-yl)-amino]-acetate 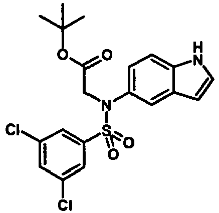 Mass spectrum (ESI⁺): m/z = 472 [M+NH₄]⁺
(22) ethyl [(3,5-dichloro-phenylsulphonyl)-(1H-indol-5-yl)-amino]-acetate 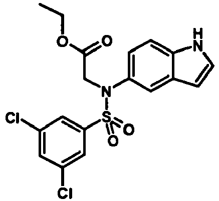 Mass spectrum (ESI⁺): m/z = 427 [M+H]⁺
(23) tert.butyl [(1-{6-[tert-butoxycarbonyl-(2-(bis-tert-butoxycarbonyl)-aminoethyl)-amino]-pyridazin-3-yl}-1H-indol-5-yl)-(3,5-dichloro-phenylsulphonyl)-amino]-acetate 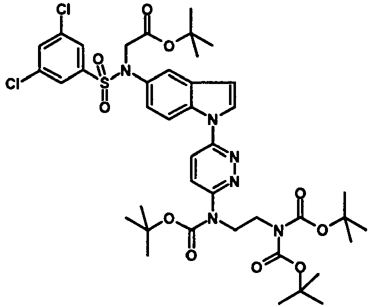 Mass spectrum (ESI⁺): m/z = 891 [M+H]⁺
(24) tert-butyl 3-(6-{5-[tert-butoxycarbonylmethyl-(3,5-dichloro-phenylsulphonyl)-amino]-2,3-dihydro-indol-1-yl}-pyridazin-3-yl)-2-oxo-imidazolidine-1-carboxylate 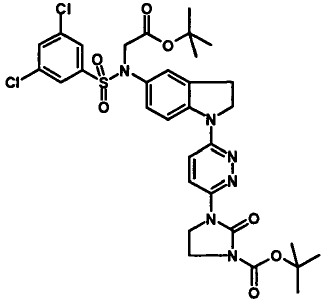 Mass spectrum (ESI⁺): m/z = 719 [M+H]⁺
(25) tert.butyl [(3,5-dichloro-phenylsulphonyl)-(2,3-dimethyl-1H-indol-5-yl)-amino]-acetate 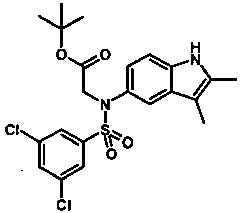 Mass spectrum (ESI⁺): m/z = 500 [M+NH₄]⁺
(26) tert.butyl [(3,5-dichloro-phenylsulphonyl)-(2-methyl-1H-indol-5-yl)-amino]-acetate 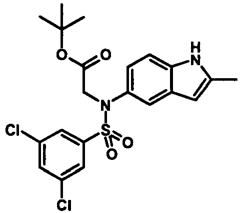 Mass spectrum (ESI⁻): m/z = 467 [M-H^{]-}
(27) tert.butyl [(3-bromo-5-methyl-phenylsulphonyl)-(1H-indol-5-yl)-amino]-acetate 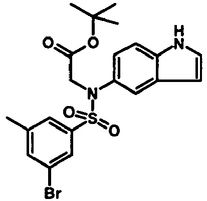 Mass spectrum (ESI⁺): m/z = 498 [M+NH₄]⁺
(28) tert.butyl [(3,5-dichloro-phenylsulphonyl)-(7-methyl-1H-indol-5-yl)-amino]-acetate 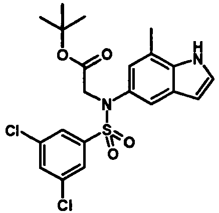 Mass spectrum (ESI⁺): m/z = 486 [M+NH₄]⁺
(29) tert.butyl [(3,5-dichloro-phenylsulphonyl)-(6-methyl-1H-indol-5-yl)-amino]-acetate 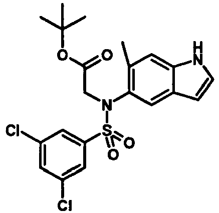 Mass spectrum (ESI⁺): m/z = 486 [M+NH₄]⁺
(30) tert-butyl 5'-{5-[tert-butoxycarbonylmethyl-(3,5-dichloro-phenylsulphonyl)-amino]-indol-1-yl}-2,3,5,6-tetrahydro-[1,2']bipyrazinyl-4-carboxylate 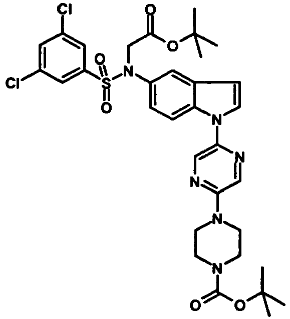 Mass spectrum (ESI⁺): m/z = 734 [M+NH₄]⁺
(31) tert.butyl [(3-chloro-5-methyl-phenylsulphonyl)-(1H-indol-5-yl)-amino]-acetate 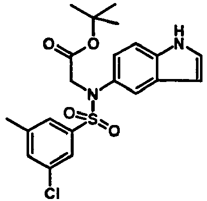 Mass spectrum (ESI⁺): m/z = 435 [M+H]⁺
(32) tert.butyl [(3,5-dibromo-phenylsulphonyl)-(1H-indol-5-yl)-amino]-acetate 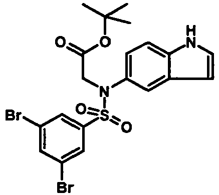 Mass spectrum (ESI⁺): m/z = 560 [M+NH₄]⁺
(33) tert.butyl [(1-{4-[tert-butoxycarbonyl-(2-(bis-tert-butoxycarbonyl)-aminoethyl)-amino]-pyrimidin-2-yl}-1H-indol-5-yl)-(3,5-dichloro-phenylsulphonyl)-amino]-acetate 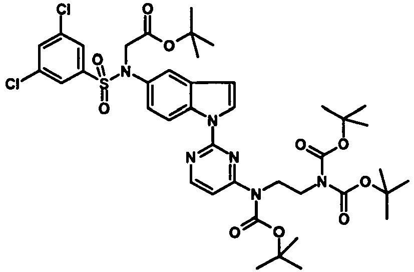 Mass spectrum (ESI⁺): m/z = 908 [M+NH₄]⁺
(34) tert-butyl 4-(6-{5-[tert-butoxycarbonylmethyl-(3,5-dichloro-phenylsulphonyl)-amino]-indol-1-yl}-pyridazin-3-yl)-piperazine-1-carboxylate 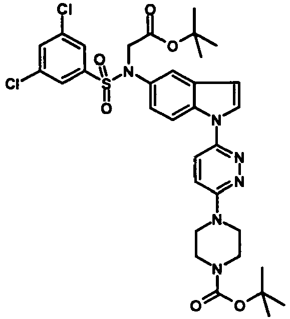 Mass spectrum (ESI⁺): m/z = 717 [M+H]⁺
(35) tert.butyl {(3,5-dichloro-phenylsulphonyl)-[1-(2-methylamino-pyrimidin-4-yl)-1H-indol-5-yl]-amino}-acetate 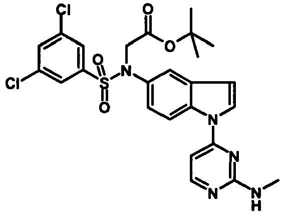 Mass spectrum (ESI⁺): m/z = 562 [M+H]⁺
(36) tert.butyl [(3-bromo-5-chloro-phenylsulphonyl)-(1H-indol-5-yl)-amino]-acetate 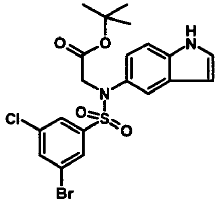 Mass spectrum (ESI⁻): m/z = 497 [M-H]⁻
(37) tert.butyl [(1-{2-[tert-butoxycarbonyl-(2-(bis-tert-butoxycarbonyl)-aminoethyl)-amino]-pyrimidin-4-yl}-1H-indol-5-yl)-(3,5-dichloro-phenylsulphonyl)-amino]-acetate 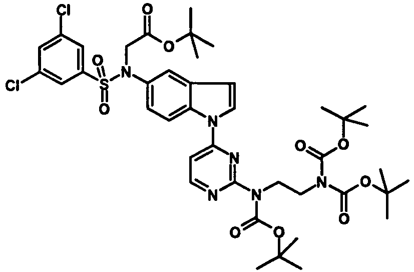 Mass spectrum (ESI⁺): m/z = 891 [M+H]⁺
(38) tert-butyl 4-(6-{5-[tert-butoxycarbonylmethyl-(3,5-dichloro-phenylsulphonyl)-amino]-indol-1-yl}-5-cyano-pyridazin-3-yl)-piperazine-1-carboxylate 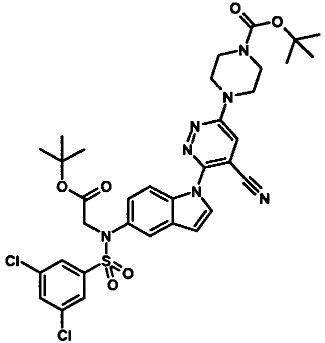 Mass spectrum (ESI⁺): m/z = 891 [M+H-tert.-butyl]⁺
(39) tert.butyl [(3,5-dichloro-phenylsulphonyl)-(3-methyl-1H-indol-5-yl)-amino]-acetate 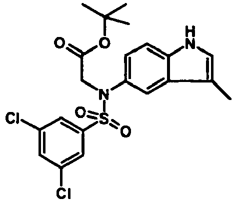 Mass spectrum (ESI⁺): m/z = 486 [M+NH₄]⁺
(40) tert.butyl [(3-cyano-1H-indol-5-yl)-(3,5-dichloro-phenylsulphonyl)-amino]-acetate 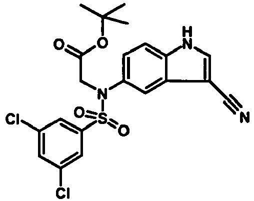 Mass spectrum (ESI⁺): m/z = 497 [M+NH4]⁺
(41) tert.butyl [[1-(6-chloro-pyridazin-3-yl)-2,3-dihydro-1H-indol-5-yl]-(3,5-dichloro-phenylsulphonyl)-amino]-acetate 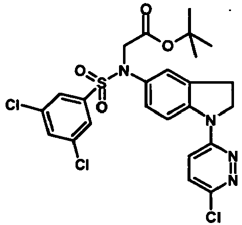 Mass spectrum (ESI⁺): m/z = 569 [M+H]⁺
(42) tert.butyl [(3-chloro-1H-indol-5-yl)-(3,5-dichloro-phenylsulphonyl)-amino]-acetate 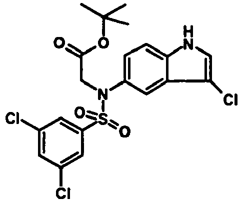 Mass spectrum (ESI⁻): m/z = 487 [M-H]⁻
(43) tert.butyl [(3,5-dichloro-phenylsulphonyl)-(3-trifluoromethyl-1H-indol-5-yl)-amino]-acetate 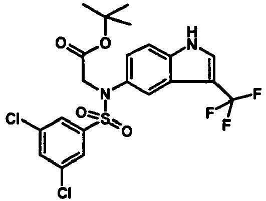 Mass spectrum (ESI⁺): m/z = 521 [M+H]⁺
(44) tert.butyl {(3,5-dichloro-phenylsulphonyl)-[1-(4,6-dimethoxy-[1,3,5]triazin-2-yl)-2,3-dihydro-1H-indol-5-yl]-amino}-acetate 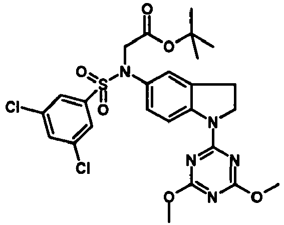 Mass spectrum (ESI⁺): m/z = 596 [M+H]⁺
(45) tert.butyl [(3,5-dichloro-phenylsulphonyl)-(1H-indazol-5-yl)-amino]-acetate 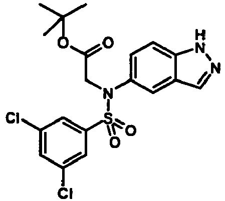 Mass spectrum (ESI⁺): m/z = 294 [M+H]⁺
   R_{f} value: 0.53 (silica gel: ethyl acetate/petroleum ether 1:1)
(46) tert.butyl [[6-chloro-1H-indol-5-yl]-(3,5-dichloro-phenylsulphonyl)-amino]-acetate 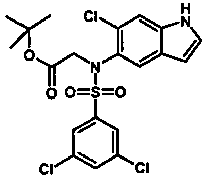 Mass spectrum (ESI⁻): m/z = 487 [M-H]⁻
   R_{f} value: 0.35 (silica gel: petroleum ether/ethyl acetate/conc. Aqueous ammonia solution 70:30:0.1)
(47) tert.butyl [(3,5-dichloro-phenylsulphonyl)-(3-methyl-1H-indazol-5-yl)-amino]-acetate 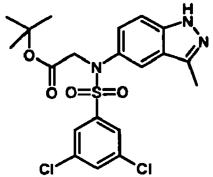 Mass spectrum (ESI⁺): m/z = 470 [M+H]⁺
   R_{f} value: 0.32 (silica gel: petroleum ether/ethyl acetate 2:1)
(48) tert.butyl [(3,5-dichloro-phenylsulphonyl)-(6-ethyl-1H-indol-5-yl)-amino]-acetate 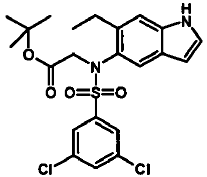 Mass spectrum (ESI⁺): m/z = 500 [M+NH₄]⁺
   R_{f} value: 0.75 (silica gel: cyclohexane/ethyl acetate 1:1)
(49) tert.butyl [(3,5-dichloro-phenylsulphonyl)-(4-methyl-1H-indol-5-yl)-amino]-acetate 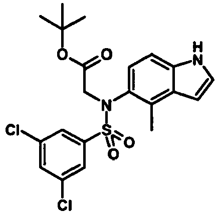 Mass spectrum (ESI⁺): m/z = 486 [M+NH₄]⁺
(50) tert.butyl [[1-(2-chloro-9H-purin-6-yl)-1H-indol-5-yl]-(3,5-dichlorophenylsulphonyl)-amino]-acetate 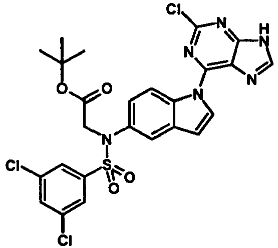 Mass spectrum (ESI⁺): m/z = 607 [M+H]⁺
(51) tert.butyl [(3,5-dichloro-phenylsulphonyl)-(3-ethyl-1H-indol-5-yl)-amino]-acetate 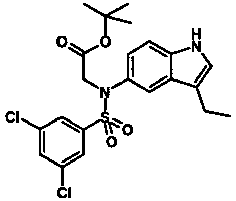 Mass spectrum (ESI⁺): m/z = 500 [M+NH₄]⁺
(52) tert.butyl [[1-(5-carbamoyl-pyrazin-2-yl)-1H-indol-5-yl]-(3,5-dichlorophenylsulphonyl)-amino]-acetate 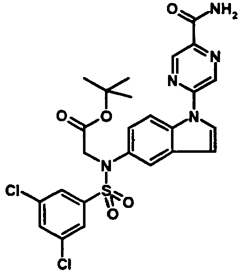 R_{f} value: 0.75 (silica gel: ethyl acetate)
(53) tert.butyl {(3,5-dichloro-phenylsulphonyl)-[1-(5-methylcarbamoyl-pyrazin-2-yl)-1H-indol-5-yl]-amino}-acetate 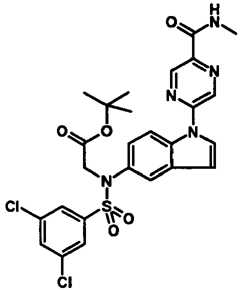 Mass spectrum (ESI⁺): m/z = 590 [M+H]⁺
(54) tert.butyl ((3,5-dichloro-phenylsulphonyl)-{1-[5-(morpholine-4-carbonyl)-pyrazin-2-yl]-1H-indol-5-yl}-amino)-acetate 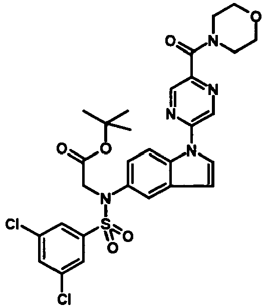 Mass spectrum (ESI⁺): m/z = 646 [M+H]⁺
(55) tert.butyl [[1-(5-cyano-pyrazin-2-yl)-1H-indol-5-yl]-(3,5-dichlorophenylsulphonyl)-amino]-acetate 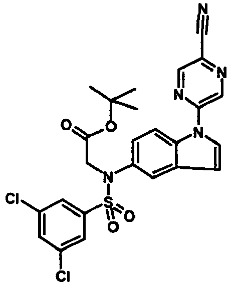 Mass spectrum (ESI⁺): m/z = 575 [M+NH₄]⁺
(56) tert.butyl ((3,5-dichloro-phenylsulphonyl)-{1-[6-(morpholine-4-carbonyl)-pyridazin-3-yl]-1H-indol-5-yl}-amino)-acetate 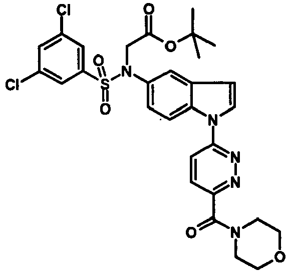 Mass spectrum (ESI⁺): m/z = 646 [M+H]⁺
(57) tert.butyl {(3,5-dichloro-phenylsulphonyl)-[1-(6-dimethylcarbamoyl-pyridazin-3-yl)-1H-indol-5-yl]-amino}-acetate 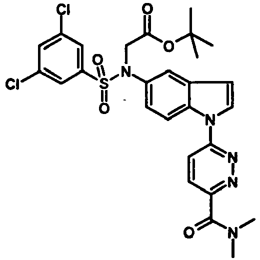 The product is reacted further directly (1 (190)).
(58) tert.butyl [(3-bromo-1H-indol-5-yl)-(3,5-dichloro-phenylsulphonyl)-amino]-acetate 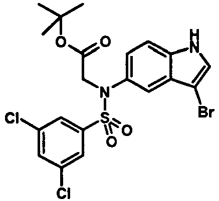 Mass spectrum (ESI⁻) m/z = 533 [M-H]⁻
   R_{f} value: 0.65 (silica gel: cyclohexane/ethyl acetate 2:1)
(59) tert.butyl [(3,5-dichloro-phenylsulphonyl)-(3-pyrimidin-2-yl-3H-benzoimidazol-5-yl)-amino]-acetate 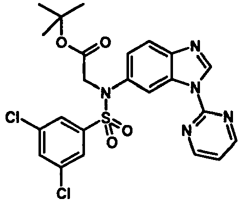 The product is reacted directly without any further purification.
(60) tert.butyl [(3,5-dichloro-phenylsulphonyl)-(1-pyrimidin-2-yl-1H-benzoimidazol-5-yl)-amino]-acetate 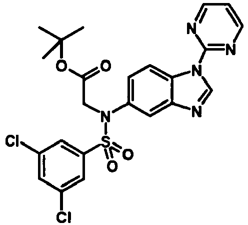 The product is reacted directly without any further purification.
(61) tert.butyl [(2,6-dimethyl-pyridin-4-sulphonyl)-(1H-indol-5-yl)-amino]-acetate 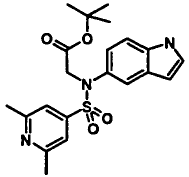 Mass spectrum (ESI⁺): m/z = 416 [M+H]⁺
(62) tert.butyl [(2,6-dichloro-pyridin-4-sulphonyl)-(1H-indol-5-yl)-amino]-acetate 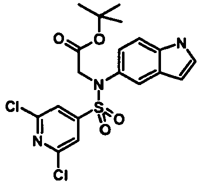 Mass spectrum (ESI⁺): m/z = 456 [M+H]⁺

### Example II

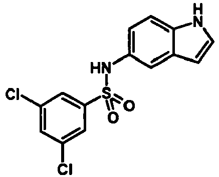

### 3,5-dichloro-N-(1H-indol-5-yl)-phenylsulphonamide

100 mg 5-aminoindole are dissolved in 10 ml of pyridine. To this are added 186 mg 3,5-dichlorophenylsulphonyl chloride and the mixture is left for 4 hours at ambient temperature with stirring. The solvent is eliminated in vacuo and the residue is divided between water and ethyl acetate. The aqueous phase is extracted with ethyl acetate and the combined organic phases are washed with saturated sodium chloride solution. After drying with magnesium sulphate the solvents are eliminated in vacuo and the residue is chromatographed on silica gel (cyclohexane/ethyl acetate 10:1 to 1:5).
Yield: 240 mg (93 % of theory)
Mass spectrum (ESI⁺): m/z = 341 [M+H]⁺

The following compounds are obtained analogously to Example II:
(1) 3,5-dichloro-N-(1-pyrimidin-2-yl-2,3-dihydro-1H-indol-5-yl)-phenylsulphonamide 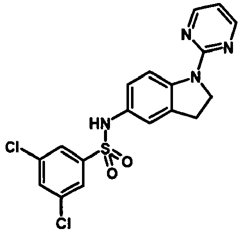 Mass spectrum (ESI⁺): m/z = 421 [M+H]⁺
(2) 3,5-dichloro-N-[1-(1-methyl-1H-imidazol-2-yl)-2,3-dihydro-1H-indol-5-yl]-phenylsulphonamide 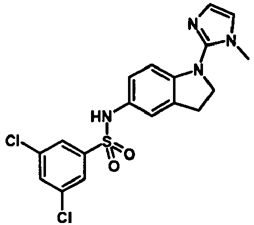 The crude product is extracted from dichloromethane/diisopropylether. Mass spectrum (ESI⁺): m/z = 423 [M+H]⁺
(3) tert-butyl 2-[5-(3,5-dichloro-phenylsulphonylamino)-2,3-dihydro-indol-1-yl]-imidazole-1-carboxylate 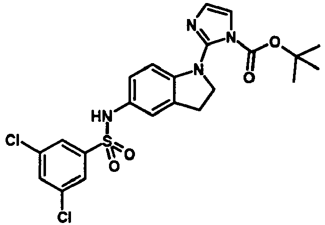 Mass spectrum (ESI⁺): m/z = 509 [M+H]⁺
(4) 3,5-dichloro-N-(1-pyridin-4-yl-2,3-dihydro-1H-indol-5-yl)-phenylsulphonamide 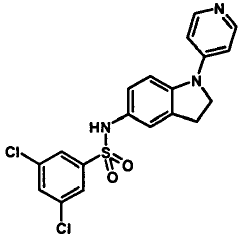 The crude product is extracted from dichloromethane.
   Mass spectrum (ESI⁺): m/z = 420 [M+H]⁺
(5) 3,5-dichloro-N-(1-pyrimidin-2-yl-1H-indol-4-yl)-phenylsulphonamide 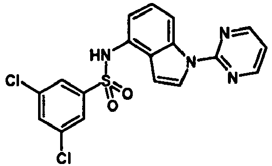 Mass spectrum (ESI⁺): m/z = 436 [M+NH₄]⁺
(6) tert-butyl 6'-[5-(3,5-dichloro-phenylsulphonylamino)-indol-1-yl]-2,3,5,6-tetrahydro-[1,2']bipyrazinyl-4-carboxylate 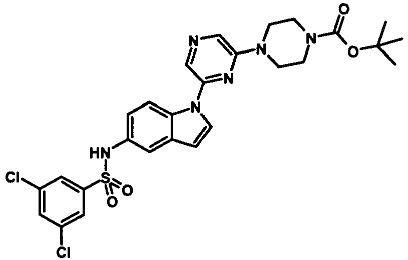 Mass spectrum (ESI⁺): m/z = 603 [M+H]⁺
(7) tert-butyl 6'-[5-(3,5-dichloro-phenylsulphonylamino)-2,3-dihydro-indol-1-yl]-2,3,5,6-tetrahydro-[1,2']bipyrazinyl-4-carboxylate 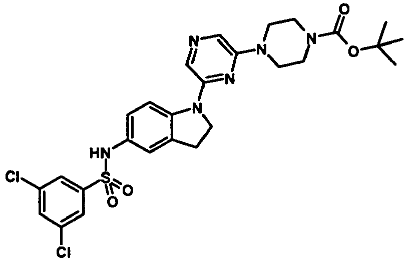 Mass spectrum (ESI⁺): m/z = 605 [M+H]⁺
(8) 3,5-dichloro-N-(1-pyrimidin-2-yl-2,3-dihydro-1H-indol-4-yl)-phenylsulphonamide 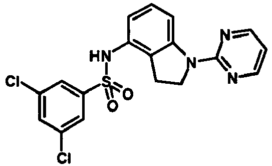 The reaction is carried out in dichloromethane/pyridine 2:1. The crude product is further reacted directly in Example VI (24).
(9) 3,5-dichloro-N-{1-[4-(2-dimethylamino-ethylamino)-pyrimidin-2-yl]-2,3-dihydro-1H-indol-5-yl}-phenylsulphonamide 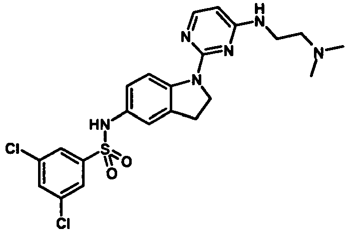 The reaction is carried out in dichloromethane/pyridine 2:1.
   Mass spectrum (ESI⁺): m/z = 507 [M+H]⁺
(10) tert-butyl 4-{6-[5-(3,5-dichloro-phenylsulphonylamino)-2,3-dihydro-indol-1-yl]-pyridazin-3-yl}-piperazine-1-carboxylate 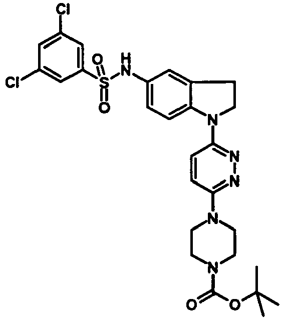 The reaction is carried out in dichloromethane/pyridine 3:1.
   Mass spectrum (ESI⁺): m/z = 605 [M+H]⁺
(11) tert-butyl {2-[5-(3,5-dichloro-phenylsulphonylamino)-2,3-dihydro-indol-1-yl]-pyrimid in-4-yl}-(2-dimethylamino-ethyl)-carbamate 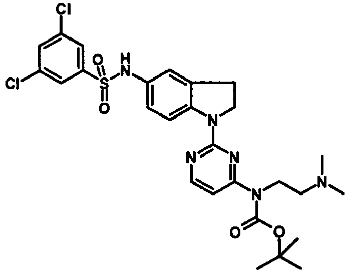 The reaction is carried out in dichloromethane/pyridine 3:1.
   Mass spectrum (ESI⁺): m/z = 607 [M+H]⁺
(12) tert-butyl 6-[5-(3,5-dichloro-phenylsulphonylamino)-2,3-dihydro-indol-1-yl]-pyridazin-3-yl}-(2-dimethylamino-ethyl)-carbamate 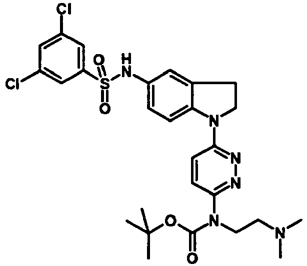 The reaction is carried out in dichloromethane/pyridine 3:1. Mass spectrum (ESI⁺): m/z = 607 [M+H]⁺
(13) N-(1H-indole-5-yl)-3,5-dimethyl-phenylsulphonamide 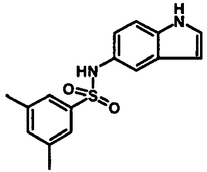 The reaction is is carried out in dichloromethane/pyridine 5:1.
   Mass spectrum (ESI⁺): m/z = 301 [M+H]⁺
(14) tert-butyl (2-(bis-tert-butoxycarbonyl)-amino-ethyl)-{6-[5-(3,5-dichlorophenylsulphonylamino)-2,3-dihydro-indol-1-yl]-pyridazin-3-yl}-carbamate 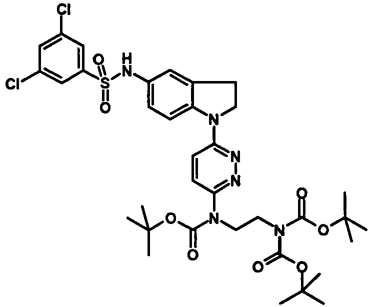 The reaction is carried out in dichloromethane/pyridine 3:1.
   Mass spectrum (ESI⁺): m/z = 779 [M+H]⁺
(15) tert-butyl (2-(bis-tert-butoxycarbonyl)-amino-ethyl)-{6-[5-(3,5-dichlorophenylsulphonylamino)-indol-1-yl]-pyridazin-3-yl}-carbamate 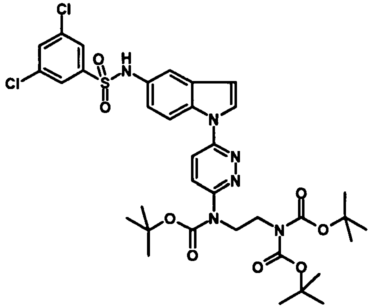 The reaction is carried out in dichloromethane/pyridine 3:1.
   Mass spectrum (ESI⁺): m/z = 777 [M+H]⁺
(16) tert-butyl 3-{6-[5-(3,5-dichloro-phenylsulphonylamino)-2,3-dihydro-indol-1-yl]-pyridazin-3-yl}-2-oxo-imidazolidine-1-carboxylate 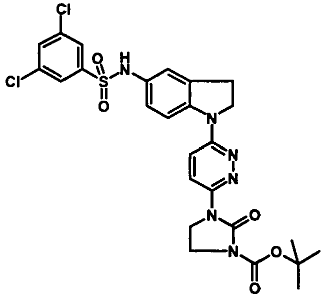 The reaction is carried out in dichloromethane/pyridine 3:1.
   Mass spectrum (ESI⁺): m/z = 605 [M+H]⁺
(17) 3,5-dichloro-N-(2,3-dimethyl-1H-indol-5-yl)-phenylsulphonamide 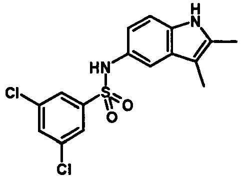 Mass spectrum (ESI⁻): m/z = 367 [M-H]⁻
(18) 3,5-dichloro-N-(2-methyl-1H-indol-5-yl)-phenylsulphonamide 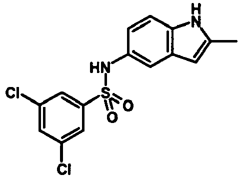 Mass spectrum (ESI⁻): m/z = 353 [M-H]⁻
(19) 3-bromo-N-(1H-indol-5-yl)-5-methyl-phenylsulphonamide 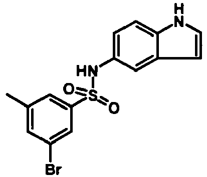 The reaction is carried out in dichloromethane with 3 equivalents of pyridine.
   Mass spectrum (ESI⁺): m/z = 365 [M+H]⁺
(20) tert-butyl 5'-[5-(3,5-dichloro-phenylsulphonylamino)-indol-1-yl]-2,3,5,6-tetrahydro-[1,2']bipyrazinyl-4-carboxylate 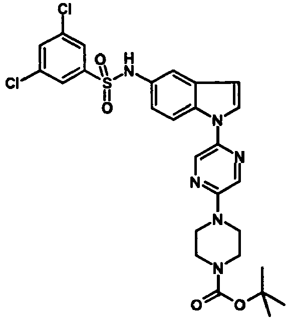 The reaction is carried out in dichloromethane/pyridine 3:1. Mass spectrum (ESI⁺): m/z = 603 [M+H]⁺
(21) 3-chloro-N-(1H-indol-5-yl)-5-methyl-phenylsulphonamide 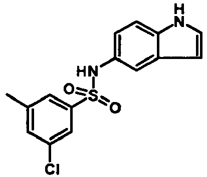 The reaction is carried out in dichloromethane with 3 equivalents of pyridine. Mass spectrum (ESI⁺): m/z = 321 [M+H]⁺
(22) 3,5-dibromo-N-(1H-indol-5-yl)-phenylsulphonamide 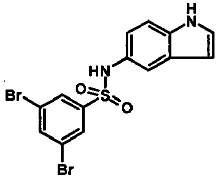 The reaction is carried out in dichloromethane with 3 equivalents of pyridine. Mass spectrum (ESI⁻): m/z = 429 [M-H]⁻
(23) tert-butyl (2-(bis-tert-butoxycarbonyl)-amino-ethyl)-{2-[5-(3,5-dichlorophenylsulphonylamino)-indol-1-yl]-pyrimidin-4-yl}-carbamate 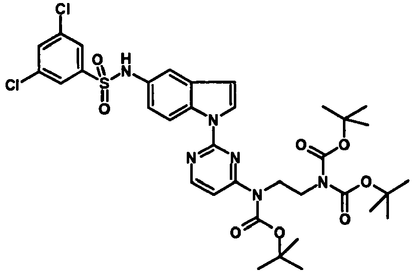 The reaction is carried out in dichloromethane/pyridine 3:1.
   Mass spectrum (ESI⁺): m/z = 777 [M+H]⁺
(24) tert-butyl 4-{6-[5-(3,5-dichloro-phenylsulphonylamino)-indol-1-yl]-pyridazin-3-yl}-piperazine-1-carboxylate 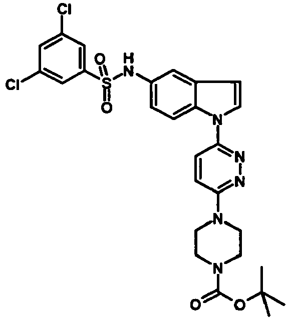 The reaction is carried out in dichloromethane/pyridine 3:1.
   Mass spectrum (ESI⁺): m/z = 603 [M+H]⁺
(25) 3,5-dichloro-N-[1-(2-methylamino-pyrimidin-4-yl)-1H-indol-5-yl]-phenylsulphonamide 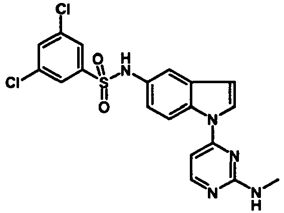 The reaction is carried out in dichloromethane/pyridine 3:1.
   Mass spectrum (ESI⁺): m/z = 448 [M+H]⁺
(26) 3-bromo-5-chloro-N-(1H-indol-5-yl)-phenylsulphonamide 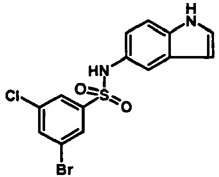 The reaction is carried out in dichloromethane with 3 equivalents of pyridine.
   Mass spectrum (ESI⁺): m/z = 385 [M+H]⁺
(27) tert-butyl (2-(bis-tert-butoxycarbonyl)-amino-ethyl)-{4-[5-(3,5-dichlorophenylsulphonylamino)-indol-1-yl]-pyrimidin-2-yl}-carbamate 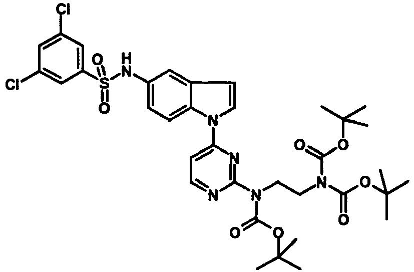 The reaction is carried out in dichloromethane/pyridine 3:1.
   Mass spectrum (ESI⁺): m/z = 777 [M+H]⁺
(28) 3,5-dichloro-N-(3-methyl-1H-indol-5-yl)-phenylsulphonamide 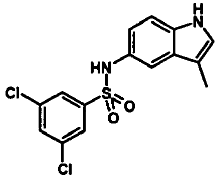 The reaction is is carried out in dichloromethane/pyridine 5:1.
   Mass spectrum (ESI⁺): m/z = 355 [M+H]⁺
(29) 3,5-dichloro-N-(3-cyano-1H-indol-5-yl)-phenylsulphonamide 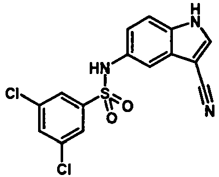 The reaction is is carried out in dichloromethane/pyridine 5:1.
   Mass spectrum (ESI⁺): m/z = 383 [M+NH₄]⁺
(30) 3,5-dichloro-N-[1-(6-chloro-pyridazin-3-yl)-2,3-dihydro-1H-indol-5-yl]-phenylsulphonamide 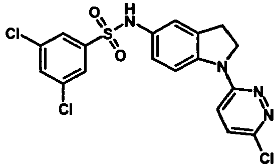 The reaction is is carried out in dichloromethane/pyridine 5:1.
   Mass spectrum (ESI⁺): m/z = 455 [M+H]⁺
(31) 3,5-dichloro-N-(3-chloro-1H-indol-5-yl)-phenylsulphonamide 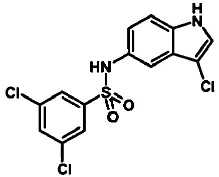 The reaction is is carried out in dichloromethane/pyridine 5:1.
   Mass spectrum (ESI⁺): m/z = 373 [M+H]+
(32) 3,5-dichloro-N-(3-trifluoromethyl-1H-indol-5-yl)-phenylsulphonamide 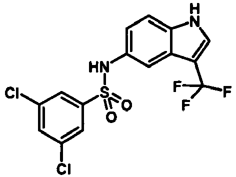 The reaction is is carried out in dichloromethane/pyridine 5:1.
   Mass spectrum (ESI⁺): m/z = 409 [M+H]⁺
(33) 3,5-dichloro-N-[1-(4,6-dimethoxy-[1,3,5]triazin-2-yl)-2,3-dihydro-1H-indol-5-yl]-phenylsulphonamide 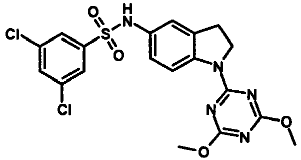 The reaction is is carried out in dichloromethane/pyridine 5:1.
   Mass spectrum (ESI⁺): m/z = 482 [M+H]⁺
(34) 3,5-dichloro-N-(1H-indazol-5-yl)-phenylsulphonamide 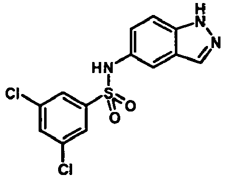 Mass spectrum (ESI⁻): m/z = 340 [M-H]⁻
   R_{f} value: 0.60 (silica gel: ethyl acetate/petroleum ether 2:1)
(35) 3,5-dichloro-N-(6-chloro-1H-indol-5-yl)-phenylsulphonamide 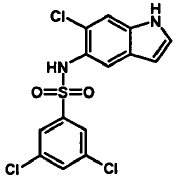 Mass spectrum (ESI⁻): m/z = 373 [M-H]⁻
   R_{f} value: 0.65 (silica gel: cyclohexane/ethyl acetate 1:1)
(36) 3,5-dichloro-N-(3-methyl-1H-indazol-5-yl)-phenylsulphonamide 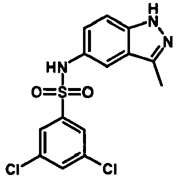 Mass spectrum (ESI⁻): m/z = 354 [M-H]⁻
(37) 3,5-dichloro-N-(6-ethyl-1H-indol-5-yl)-phenylsulphonamide 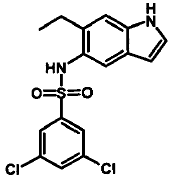 Mass spectrum (ESI⁺): m/z = 368 [M+H]⁺
   R_{f} value: 0.65 (silica gel: cyclohexane/ethyl acetate 1:1)
(38) 3,5-dichloro-N-(3-ethyl-1H-indol-5-yl)-phenylsulphonamide 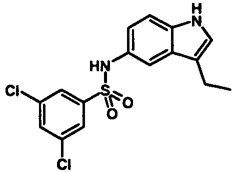 Mass spectrum (ESI⁺): m/z = 369 [M+H]⁺
(39) 5-[5-(3,5-dichloro-phenylsulphonylamino)-indol-1-yl]-pyrazin-2-carboxylic acid-amide 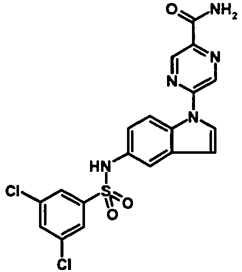 Mass spectrum (ESI⁻): m/z = 460 [M-H]⁻
(40) 5-[5-(3,5-dichloro-phenylsulphonylamino)-indol-1-yl]-pyrazin-2-carboxylic acid-methyl-amide 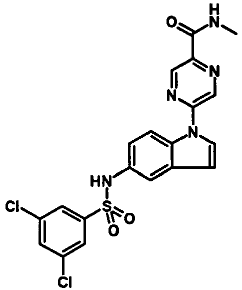 Mass spectrum (ESI⁻): m/z = 474 [M-H]⁻
(41) 3,5-dichloro-N-{1-[5-(morpholine-4-carbonyl)-pyrazin-2-yl]-1H-indol-5-yl}-phenyl-sulphonamide 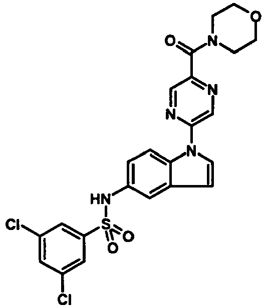 Mass spectrum (ESI⁻): m/z = 530 [M-H]⁻
(42) 3,5-dichloro-N-[1-(5-cyano-pyrazin-2-yl)-1H-indol-5-yl]-phenylsulphonamide 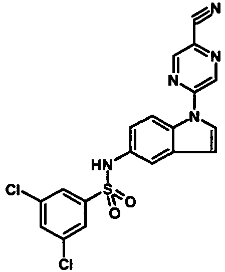 Mass spectrum (ESI⁻): m/z = 442 [M-H]⁻
(43) 3,5-dichloro-N-{1-[6-(morpholine-4-carbonyl)-pyridazin-3-yl]-1H-indol-5-yl}-phenylsulphonamide 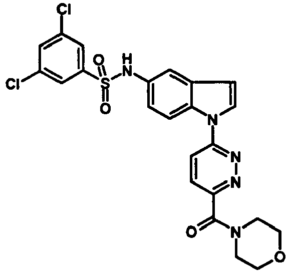 The crude product is further reacted directly (VI (80)).
(44) 6-[5-(3,5-dichloro-phenylsulphonylamino)-indol-1-yl]-pyridazine-3-carboxylic acid-dimethylamide 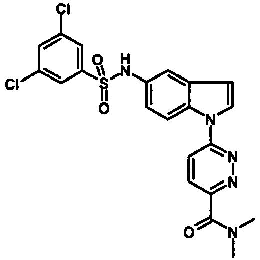 The crude product is further reacted directly (VI (81)).
(45) N-(3-bromo-1H-indol-5-yl)-3,5-dichloro-phenylsulphonamide 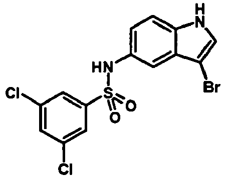 Mass spectrum (ESI⁻): m/z = 419 [M-H]⁻
   R_{f} value: 0.75 (silica gel: cyclohexane/ethyl acetate 1:1)
(46) 3,5-dichloro-N-(3-pyrimidin-2-yl-3H-benzoimidazol-5-yl)-phenylsulphonamide 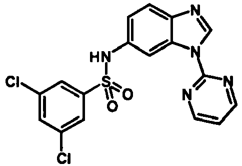 Mass spectrum (ESI⁻): m/z = 418 [M-H]⁻
(47) 3,5-dichloro-N-(1-pyrimidin-2-yl-1H-benzoimidazol-5-yl)-phenylsulphonamide 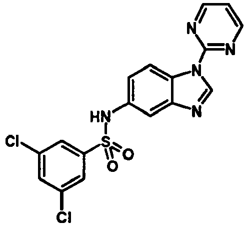 Mass spectrum (ESI⁻): m/z = 418 [M-H]⁻

### Example III

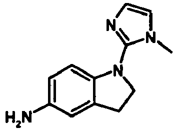

### 1-(1-methyl-1H-imidazol-2-yl)-2,3-dihydro-1H-indol-5-ylamine

170 mg 1-(1-methyl-1H-imidazol-2-yl)-5-nitro-2,3-dihydro-1H-indole are dissolved in 20 ml of tetrahydrofuran. To this are added 20 mg palladium on charcoal (10%) and the mixture is hydrogenated for 1 hour at ambient temperature. Then the catalyst is filtered off and the solvent is eliminated in vacuo. The crude product is extracted from diethyl ether.
Yield: 95 mg (64 % of theory)
Mass spectrum (ESI⁺): m/z = 215 [M+H]⁺

The following compounds are obtained analogously to Example III:
(1) 1-pyrimidin-2-yl-2,3-dihydro-1H-indol-5-ylamine 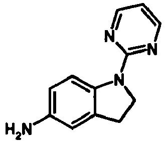 The reaction is carried out in dichloromethane/methanol 1:1. The crude product
   is further reacted directly in Example XXX.
(2) tert-butyl 2-(5-amino-2,3-dihydro-indol-1-yl)-imidazole-1-carboxylate 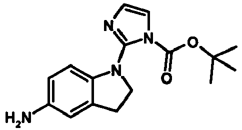 The reaction is carried out in tetrahydrofuran.
   R_{f} value: 0.60 (Aluminiumoxyd, petroleum ether/ethyl acetate 1:4)
(3) 1-pyridin-4-yl-2,3-dihydro-1H-indol-5-ylamine 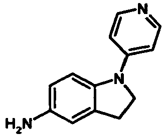 The reaction is carried out in tetrahydrofuran.
   Mass spectrum (ESI⁺): m/z = 212 [M+H]⁺
(4) 1-pyrimidin-2-yl-1H-indol-4-ylamine 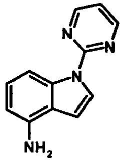 Mass spectrum (ESI⁺): m/z = 211 [M+H]⁺
(5) tert-butyl 6'-(5-amino-indol-1-yl)-2,3,5,6-tetrahydro-[1,2']bipyrazinyl-4-carboxylate 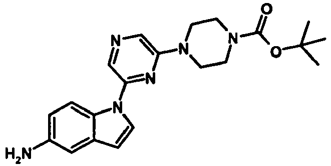 Mass spectrum (ESI⁺): m/z = 395 [M+H]⁺
(6) tert-butyl 6'-(5-amino-2,3-dihydro-indol-1-yl)-2,3,5,6-tetrahydro-[1,2']bipyrazinyl-4-carboxylate 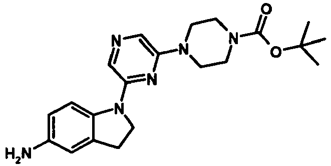 Tetrahydrofuran is used as solvent. The crude product is chromatographed on silica gel.
   Mass spectrum (ESI⁺): m/z = 397 [M+H]⁺
(7) 1-pyrimidin-2-yl-2,3-dihydro-1H-indol-4-ylamine 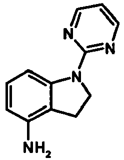 Tetrahydrofuran is used as solvent. The crude product is chromatographed on silica gel and is further reacted directly in Example XI (17).
(8) N-[2-(5-amino-2,3-dihydro-indol-1-yl)-pyrimidin-4-yl]-N',N'-dimethyl-ethan-1,2-diamine 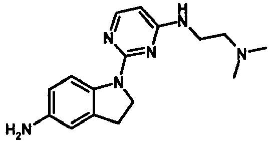 Tetrahydrofuran is used as solvent. The crude product is chromatographed on aluminium oxide.
   Mass spectrum (ESI⁺): m/z = 299 [M+H]⁺
(9) tert-butyl 4-[6-(5-amino-2,3-dihydro-indol-1-yl)-pyridazin-3-yl]-piperazine-1-carboxylate 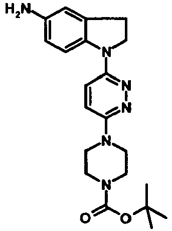 Tetrahydrofuran is used as solvent. The crude product is extracted from diisopropyl ether.
   Mass spectrum (ESI⁺): m/z = 397 [M+H]⁺
(10) tert-butyl [2-(5-amino-2,3-dihydro-indol-1-yl)-pyrimidin-4-yl]-(2-dimethylamino-ethyl)-carbamate 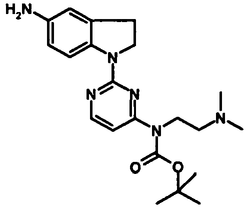 Tetrahydrofuran is used as solvent. The crude product is further reacted directly in Example XI (20).
   R_{f} value: 0.18 (aluminium oxide, petroleum ether/ethyl acetate = 1:4)
(11) tert-butyl [6-(5-amino-2,3-dihydro-indol-1-yl)-pyridazin-3-yl]-(2-dimethylamino-ethyl)-carbamate 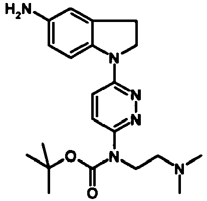 Tetrahydrofuran is used as solvent.
   Mass spectrum (ESI⁺): m/z = 399 [M+H]⁺
(12) tert-butyl [6-(5-amino-2,3-dihydro-indol-1-yl)-pyridazin-3-yl]-(2-(bis-tert-butoxycarbonyl)-amino-ethyl)-carbamate 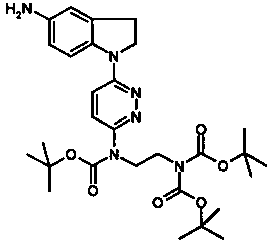 Tetrahydrofuran is used as solvent. The crude product is chromatographed on silica gel.
   Mass spectrum (ESI⁺): m/z = 571 [M+H]⁺
(13) tert-butyl [6-(5-amino-indol-1-yl)-pyridazin-3-yl]-(2-(bis-tert-butoxycarbonyl)-amino-ethyl)-carbamate 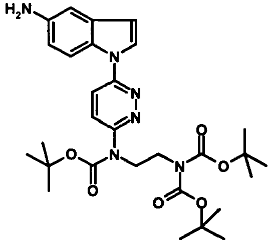 Tetrahydrofuran is used as solvent. The crude product is chromatographed on silica gel.
   Mass spectrum (ESI⁺): m/z = 569 [M+H]⁺
(14) tert-butyl 3-[6-(5-amino-2,3-dihydro-indol-1-yl)-pyridazin-3-yl]-2-oxo-imidazolidine-1-carboxylate 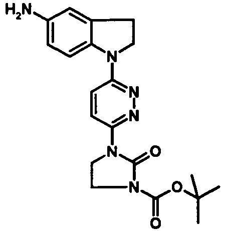 Tetrahydrofuran is used as solvent. The crude product is chromatographed on silica gel.
   Mass spectrum (ESI⁺): m/z = 397 [M+H]⁺
(15) 2,3-dimethyl-1H-indol-5-ylamine 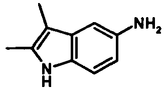 Mass spectrum (ESI⁺): m/z = 161 [M+H]⁺
(16) tert-butyl 5'-(5-amino-indol-1-yl)-2,3,5,6-tetrahydro-[1,2']bipyrazinyl-4-carboxylate 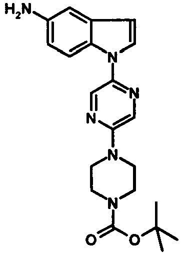 Tetrahydrofuran is used as solvent. The crude product is chromatographed on silica gel.
   Mass spectrum (ESI⁺): m/z = 395 [M+H]⁺
(17) tert-butyl [2-(5-amino-indol-1-yl)-pyrimidin-4-yl]-(2-(bis-tert-butoxycarbonyl)-amino-ethyl)-carbamate 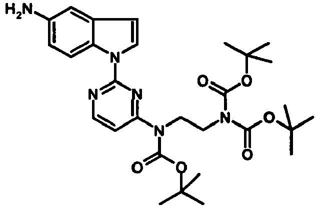 Tetrahydrofuran is used as solvent. The crude product is chromatographed on silica gel.
   Mass spectrum (ESI⁺): m/z = 569 [M+H]⁺
(18) tert-butyl 4-[6-(5-amino-indol-1-yl)-pyridazin-3-yl]-piperazine-1-carboxylate 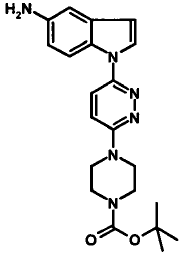 Tetrahydrofuran is used as solvent.
   Mass spectrum (ESI⁺): m/z = 395 [M+H]⁺
(19) 1-(2-methylamino-pyrimidin-4-yl)-1H-indol-5-ylamine 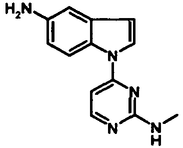 Tetrahydrofuran is used as solvent. The crude product is chromatographed on silica gel.
   Mass spectrum (ESI⁺): m/z = 240 [M+H]⁺
(20) tert-butyl [4-(5-amino-indol-1-yl)-pyrimidin-2-yl]-(2-(bis-tert-butoxycarbonyl)-amino-ethyl)-carbamate 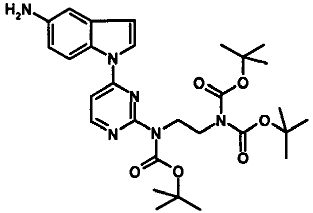 Tetrahydrofuran is used as solvent. The crude product is chromatographed on silica gel.
   Mass spectrum (ESI⁺): m/z = 569 [M+H]⁺
(21) 3-methyl-1H-indol-5-ylamine 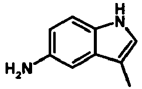 Tetrahydrofuran is used as solvent. The crude product is chromatographed on silica gel.
   Mass spectrum (ESI⁺): m/z = 147 [M+H]⁺
(22) 5-amino-1H-indole-3-carbonitrile 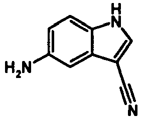 Tetrahydrofuran is used as solvent.
   Mass spectrum (ESI⁺): m/z = 158 [M+H]⁺
(23) 1-(6-chloro-pyridazin-3-yl)-2,3-dihydro-1H-indol-5-ylamine 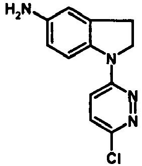 Tetrahydrofuran/methanol 20:15 is used as solvent and Raney nickel is used as catalyst. The mixture is hydrogenated for 24 hours at 2 bar. The crude product is chromatographed on silica gel (dichloromethane/methanol 99:1 to 70:30).
   Mass spectrum (ESI⁺): m/z = 247 [M+H]⁺
(24) 3-trifluoromethyl-1H-indol-5-ylamine 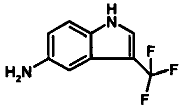 Tetrahydrofuran is used as solvent and the mixture is hydrogenated for 8 hours at 50°C.
   R_{f} value: 0.5 (silica gel; cyclohexane/ethyl acetate 1:1)
(25) 1-(4,6-dimethoxy-[1,3,5]triazin-2-yl)-2,3-dihydro-1H-indol-5-ylamine 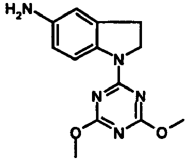 Dichloromethane is used as solvent and the mixture is hydrogenated for 7 hours at 2 bar. The crude product is extracted from diethyl ether.
   Mass spectrum (ESI⁺): m/z = 274 [M+H]⁺
(26) 6-chloro-1H-indol-5-ylamine 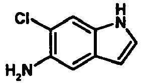 Tetrahydrofuran is used as solvent and Raney nickel as catalyst.
   Mass spectrum (ESI⁺): m/z = 167 [M+H]⁺
   R_{f} value: 0.57 (silica gel; cyclohexane/ethyl acetate 1:1)
(27) 6-ethyl-1H-indol-5-ylamine 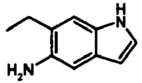 Tetrahydrofuran is used as solvent and Raney nickel as catalyst.
   Mass spectrum (ESI⁺): m/z = 161 [M+H]⁺
   R_{f} value: 0.45 (silica gel; cyclohexane/ethyl acetate 1:1)
(28) 3-ethyl-1H-indol-5-ylamine 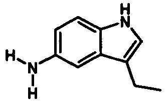 The crude product is chromatographed on silica gel (cyclohexane/ethyl acetate 50:50 to 0:100).
   Mass spectrum (ESI⁺): m/z = 161 [M+H]⁺
(29) 5-(5-amino-indol-1-yl)-pyrazin-2-carboxylic acid-amide 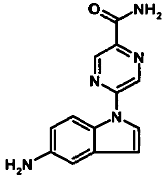 Mass spectrum (ESI⁺): m/z = 254 [M+H]⁺
(30) 5-(5-amino-indol-1-yl)-pyrazin-2-carboxylic acid-methyl-amide 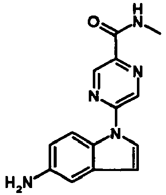 Mass spectrum (ESI⁺): m/z = 268 [M+H]⁺
(31) [5-(5-amino-indol-1-yl)-pyrazin-2-yl]-morpholin-4-yl-methanone 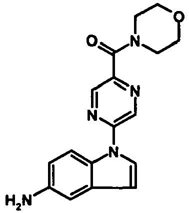 Mass spectrum (ESI⁺): m/z = 324 [M+H]⁺
(32) 5-(5-amino-indol-1-yl)-pyrazine-2-carbonitrile 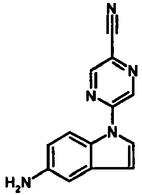 The hydrogenation is carried out in tetrahydrofuran. The crude product is chromatographed on silica gel (cyclohexane/ethyl acetate 50:50 to 0:100). Mass spectrum (ESI⁺): m/z = 236 [M+H]⁺
(33) [6-(5-amino-indol-1-yl)-pyridazin-3-yl]-morpholin-4-yl-methanone 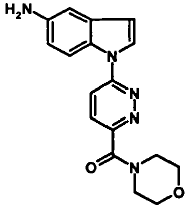 The hydrogenation is carried out in tetrahydrofuran. The crude product is further reacted directly (XI (60)).
(34) 6-(5-amino-indol-1-yl)-pyridazine-3-carboxylic acid-dimethylamide 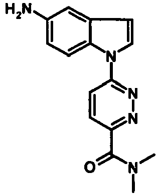 The hydrogenation is carried out in tetrahydrofuran. The crude product is further reacted directly (XI (61)).
(35) 6-amino-1-pyrimidin-2-yl-1H-benzoimidazole 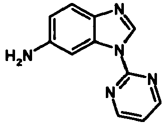 The crude product is further reacted directly.
(36) 5-amino-1-pyrimidin-2-yl-1H-benzoimidazole 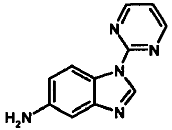 The crude product is further reacted directly.

### Example IV

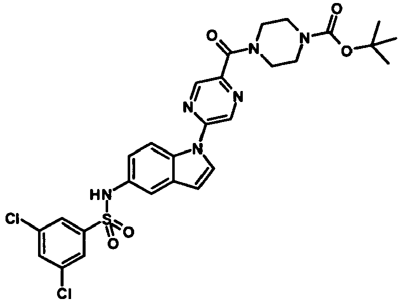

### tert-butyl 4-{5-[5-(3,5-dichloro-phenylsulphonylamino)-indol-1-yl]-pyrazine-2-carbonyl}-piperazine-1-carboxylate

206 mg tert-butyl 4-[5-(5-nitro-indol-1-yl)-pyrazin-2-carbonyl]-piperazine-1-carboxylate are dissolved in 10 ml of methanol. 30 mg palladium on charcoal (10%) are added and the mixture is hydrogenated for 4 hours at ambient temperature. The catalyst is suction filtered and washed with methanol. The solvent is eliminated in vacuo and the residue is taken up in 5 ml of pyridine. 81 mg of 3,5-dichlorophenylsulphonyl chloride are added and the mixture is stirred for 12 hours at ambient temperature. Then the pyridine is eliminated in vacuo and the residue is divided between semisaturated sodium chloride solution and ethyl acetate. The aqueous phase is extracted once with ethyl acetate and the combined organic phases are dried on magnesium sulphate. Then the solvent is eliminated in vacuo.
Yield: 230 mg (80 % of theory)
Mass spectrum (ESI⁻): m/z = 629 [M-H]⁻

The following compounds are obtained analogously to Example IV:
(1) tert-butyl 4-{2-[5-(3,5-dichloro-phenylsulphonylamino)-2,3-dihydro-indol-1-yl]-pyrimidin-4-yl}-piperazine-1-carboxylate 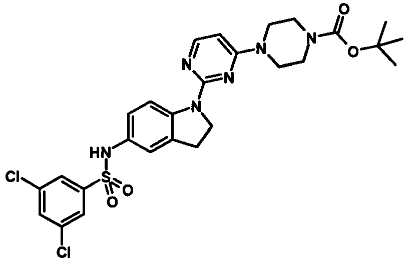 Mass spectrum (ESI⁺): m/z = 605 [M+H]⁺
(2) tert-butyl 4-{2-[5-(3,5-dichloro-phenylsulphonylamino)-indol-1-yl]-pyrimidin-4-yl}-piperazine-1-carboxylate 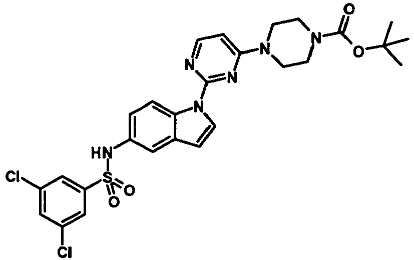
(3) tert-butyl 4-{5-[5-(3,5-dichloro-phenylsulphonylamino)-2,3-dihydro-indol-1-yl]-pyrazin-2-carbonyl}-piperazine-1-carboxylate 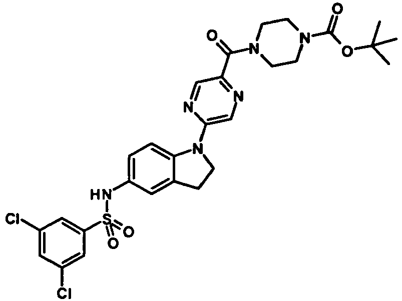 Mass spectrum (ESI⁺): m/z = 633 [M+H]⁺
(4) 3,5-dichloro-N-(7-methyl-1H-indol-5-yl)-phenylsulphonamide 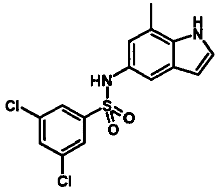 Mass spectrum (ESI⁻): m/z = 353 [M-H]⁻
(5) 3,5-dichloro-N-(6-methyl-1H-indol-5-yl)-phenylsulphonamide 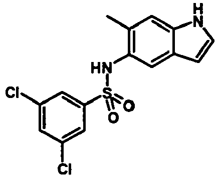 Mass spectrum (ESI⁺): m/z = 355 [M+H]⁺
(6) tert-butyl 4-{5-cyano-6-[5-(3,5-dichloro-phenylsulphonylamino)-indol-1-yl]-pyridazin-3-yl}-piperazine-1-carboxylate 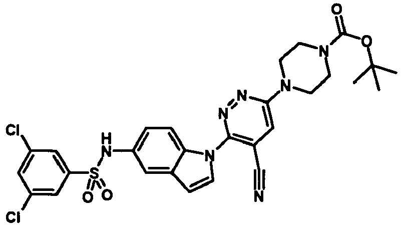 The product is further reacted directly in Example VI (63).
(7) 3,5-dichloro-N-(4-methyl-1H-indol-5-yl)-phenylsulphonamide 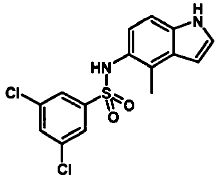 Mass spectrum (ESI⁺): m/z = 355 [M+H]⁺
(8) 3,5-dichloro-N-[1-(2-chloro-9H-purin-6-yl)-1H-indol-5-yl]-phenylsulphonamide 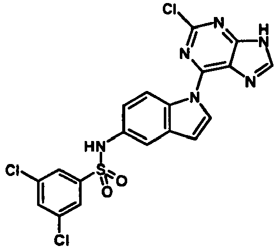 The crude product is further reacted directly (VI (73)).

### Example V

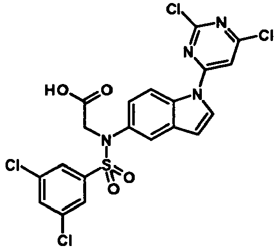

### {(3,5-dichloro-phenylsulphonyl)-[1-(2,6-dichloro-pyrimidin-4-yl)-1H-indol-5-yl]-amino}-acetic acid

162 mg tert.butyl {(3,5-dichloro-phenylsulphonyl)-[1-(2,6-dichloro-pyrimidin-4-yl)-1H-indol-5-yl]-amino}-acetate are dissolved in 3 ml dichloromethane. 0.64 ml trifluoroacetic acid are added thereto and the solution is stirred for 12 hours at ambient temperature. The solvents are eliminated in vacuo, the residue is twice taken up in dichloromethane and this is again eliminated in vacuo. The residue is extracted from diethyl ether.
Yield: 31 mg (18 % of theory)
Mass spectrum (ESI⁺): m/z = 545 [M+H]⁺

### Example VI

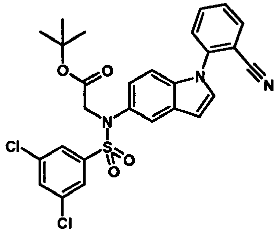

### tert.butyl [[1-(2-cyano-phenyl)-1H-indol-5-yl]-(3,5-dichloro-phenylsulphonyl)-aminol-acetate

300 mg tert.butyl [(3,5-dichloro-phenylsulphonyl)-(1H-indol-5-yl)-amino]-acetate, 12 mg copper iodide and 307 mg potassium phosphate are placed in a flask. It is evacuated twice and filled with argon. Then 1 ml of toluene and 166 mg 2-iodo-benzonitrile are added. After the addition of 21 µl N,N'-dimethyl-trans-cyclohexanediamine the mixture is heated to 110°C for 8 hours. Then it is divided between water and ethyl acetate. The aqueous phase is extracted with ethyl acetate and the combined organic phases are dried on magnesium sulphate. The solvents are eliminated in vacuo and the residue is chromatographed on silica gel (cyclohexane/ethyl acetate 10:1 to 1:2).
Yield: 266 mg (73 % of theory)
Mass spectrum (ESI⁺): m/z = 573 [M+NH₄]⁺

The following compounds are obtained analogously to Example VI:
(1) tert.butyl [(3,5-dichloro-phenylsulphonyl)-(1-pyrimidin-2-yl-1H-indol-5-yl)-amino]-acetate 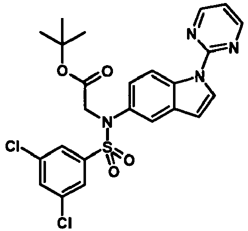 2-lodopyrimidine is used instead of 2-iodo-benzonitrile.
   Mass spectrum (ESI⁺): m/z = 550 [M+NH₄]⁺
(2) tert.butyl [(3,5-dichloro-phenylsulphonyl)-(1-pyrazin-2-yl-1H-indol-5-yl)-amino]-acetate 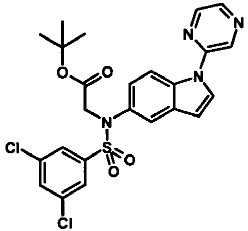 2-lodopyrazine is used instead of 2-iodo-benzonitrile.
   Mass spectrum (ESI⁺): m/z = 550 [M+NH₄]⁺
(3) tert.butyl [(3,5-dichloro-phenylsulphonyl)-(1-pyrimidin-5-yl-1H-indol-5-yl)-amino]-acetate 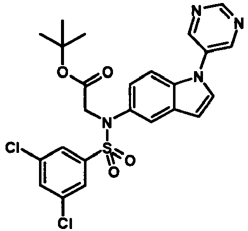 5-lodopyrimidine is used instead of 2-iodo-benzonitrile.
   Mass spectrum (ESI⁺): m/z = 533 [M+H]⁺
(4) benzyl 3-{5-[tert-butoxycarbonylmethyl-(3,5-dichloro-phenylsulphonyl)-amino]-indol-1-yl}-benzoate 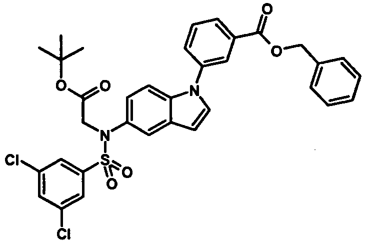 Benzyl 5-iodobenzoate is used instead of 2-iodo-benzonitrile.
   Mass spectrum (ESI⁺): m/z = 682 [M+NH₄]⁺
(5) tert.butyl {(3,5-dichloro-phenylsulphonyl)-[1-(4-morpholin-4-yl-phenyl)-1H-indol-5-yl]-amino}-acetate 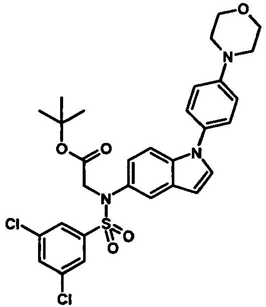 4-Morpholino-iodobenzene is used instead of 2-iodo-benzonitrile.
   Mass spectrum (ESI⁺): m/z = 616 [M+H]⁺
(6) tert.butyl {(3,5-dichloro-phenylsulphonyl)-[1-(4-morpholin-4-ylmethyl-phenyl)-1H-indol-5-yl]-amino}-acetate 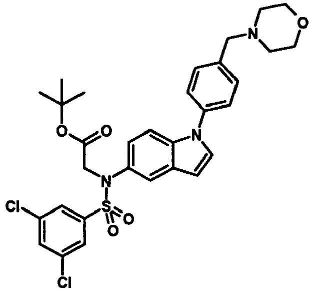 4-Morpholinomethyl-iodobenzene is used instead of 2-iodo-benzonitrile.
   Mass spectrum (ESI⁺): m/z = 630 [M+H]⁺
(7) tert.butyl [(3,5-dichloro-phenylsulphonyl)-(1-pyridin-4-yl-1H-indol-5-yl)-amino]-acetate 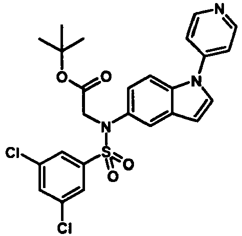 4-iodopyridine is used instead of 2-iodo-benzonitrile.
   Mass spectrum (ESI⁺): m/z = 532 [M+H]⁺
(8) tert.butyl ((3,5-dichloro-phenylsulphonyl)-{1-[5-(2-dimethylamino-ethylamino)-pyrazin-2-yl]-1H-indol-5-yl}-amino)-acetate 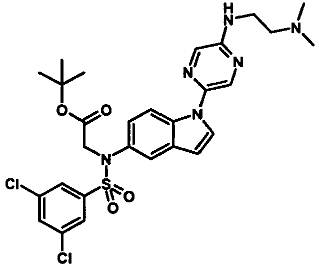 5-dimethylaminoethylamino-2-bromo-pyrazine is used instead of 2-iodo-benzonitrile.
   Mass spectrum (ESI⁺): m/z = 619 [M+H]⁺
(9) tert-butyl 4-(3-{5-[tert-butoxycarbonylmethyl-(3,5-dichloro-phenylsulphonyl)-amino]-indol-1-yl}-benzoyl)-piperazine-1-carboxylate 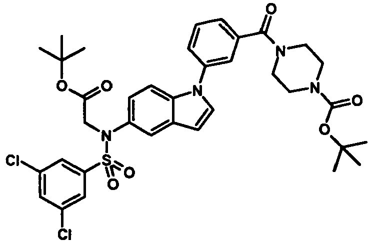 Tert-butyl 4-(3-iodo-benzoyl)-piperazine-1-carboxylate is used instead of 2-iodo-benzonitrile.
   Mass spectrum (ESI⁺): m/z = 760 [M+NH₄]⁺
(10) tert-butyl 4-(4-{5-[tert-butoxycarbonylmethyl-(3,5-dichloro-phenylsulphonyl)-amino]-indol-1-yl}-benzoyl)-piperazine-1-carboxylate 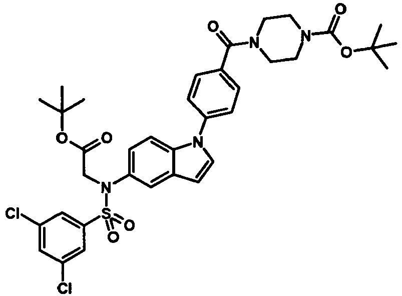 Tert-butyl 4-(4-iodo-benzoyl)-piperazine-1-carboxylate is used instead of 2-iodo-benzonitrile.
   Mass spectrum (ESI⁺): m/z = 760 [M+NH₄]⁺
(11) tert.butyl [(3,5-dichloro-phenylsulphonyl)-(1-pyridin-2-yl-1H-indol-5-yl)-amino]-acetate 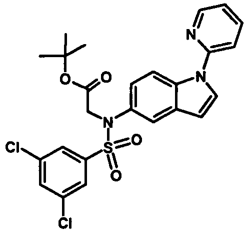 2-iodo-pyridine is used instead of 2-iodo-benzonitrile. The reaction lasts 12 hours at 90°C.
   Mass spectrum (ESI⁺): m/z = 532 [M+H]⁺
(12) tert.butyl {(3,5-dichloro-phenylsulphonyl)-[1-(3-trifluoromethyl-pyridin-2-yl)-1H-indol-5-yl]-amino}-acetate 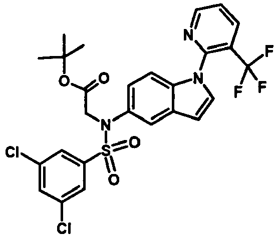 2-bromo-3-trifluoromethyl-pyridine is used instead of 2-iodo-benzonitrile. The reaction lasts 12 hours at 90°C.
   Mass spectrum (ESI⁺): m/z = 617 [M+NH₄]⁺
(13) tert-butyl 4-(6-{5-[tert-butoxycarbonylmethyl-(3,5-dichloro-phenylsulphonyl)-amino]-indol-1-yl}-5-methyl-pyridazin-3-yl)-piperazine-1-carboxylate 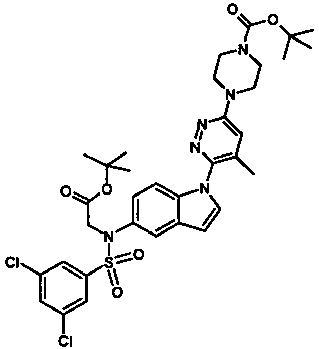 Tert-butyl 4-(6-iodo-5-methyl-pyridazin-3-yl)-piperazine-1-carboxylate is used instead of 2-iodo-benzonitrile. The reaction lasts 5 hours at 100°C.
   Mass spectrum (ESI⁺): m/z = 731 [M+H]⁺
(14) tert.butyl ((3,5-dichloro-phenylsutphonyl)-{1-[6-(2-dimethylamino-ethylamino)-pyridazin-3-yl]-1H-indol-5-yl}-amino)-acetate 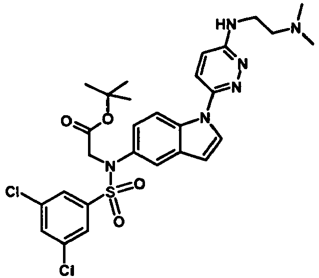 N'-(6-bromo-pyridazin-3-yl)-N,N-dimethyl-ethan-1,2-diamine is used instead of 2-iodo-benzonitrile. The reaction lasts 5 hours at 100°C.
   Mass spectrum (ESI⁺): m/z = 619 [M+H]⁺
(15) tert.butyl [{1-[6-(2-dimethylamino-ethylamino)-pyridazin-3-yl]-1H-indol-5-yl}-(3,5-dimethyl-phenylsulphonyl)-amino]-acetate 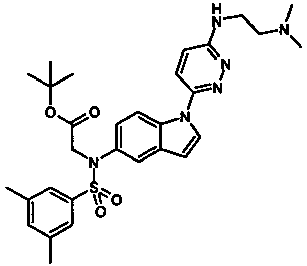 N'-(6-bromo-pyridazin-3-yl)-N,N-dimethyl-ethan-1,2-diamine is used instead of 2-iodo-benzonitrile. The reaction lasts 5 hours at 100°C.
   Mass spectrum (ESI⁺): m/z = 579 [M+H]⁺
(16) tert.butyl {(3,5-dichloro-phenylsulphonyl)-[1-(2-piperazin-1-yl-quinolin-8-yl)-1H-indol-5-yl]-amino}-acetate 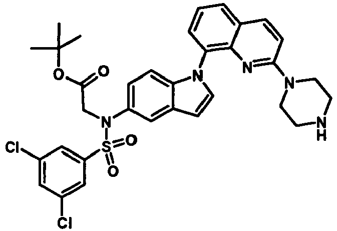 8-bromo-2-piperazin-1-yl-quinoline is used instead of 2-iodo-benzonitrile. The reaction lasts 12 hours at 90°C.
   Mass spectrum (ESI⁺): m/z = 666 [M+H]⁺
(17) tert.butyl {(3,5-dichloro-phenylsulphonyl)-[1-(6-methyl-pyridazin-3-yl)-1H-indol-5-yl]-amino}-acetate 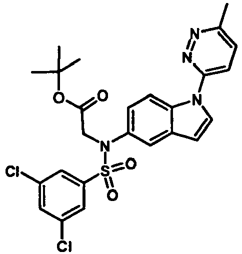 and
   tert.butyl {(3,5-dichloro-phenylsulphonyl)-[3-iodo-1-(6-methyl-pyridazin-3-yl)-1H-indol-5-yl]-amino}-acetate 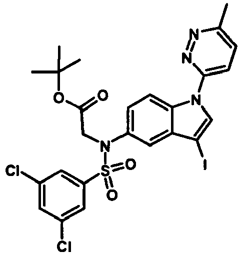 3-lodo-6-methyl-pyridazine is used instead of 2-iodo-benzonitrile. The reaction lasts 12 hours at 90°C.
   Mass spectrum (ESI⁺): m/z = 547 [M+H]⁺ (tert.butyl {(3,5-dichlorophenylsulphonyl)-[1-(6-methyl-pyridazin-3-yl)-1H-indol-5-yl]-amino}-acetate) and
   Mass spectrum (ESI⁺): m/z = 673 [M+H]⁺ (tert.butyl {(3,5-dichlorophenylsulphonyl)-[3-iodo-1-(6-methyl-pyridazin-3-yl)-1H-indol-5-yl]-amino}-acetate)
(18) tert.butyl ((3,5-dichloro-phenylsulphonyl)-{1-[6-(2-dimethylamino-ethylamino)-pyridazin-3-yl]-2,3-dimethyl-1H-indol-5-yl}-amino)-acetate 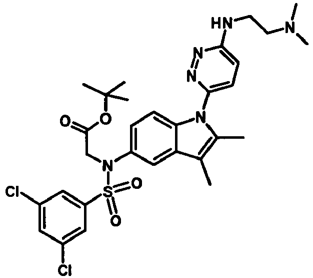 N'-(6-bromo-pyridazin-3-yl)-N,N-dimethyl-ethan-1 ,2-diamine is used instead of 2-iodo-benzonitrile. The reaction lasts 5 hours at 100°C.
   Mass spectrum (ESI⁺): m/z = 647 [M+H]⁺
(19) tert.butyl ((3,5-dichloro-phenylsulphonyl)-{1-[6-(2-dimethylamino-ethylamino)-pyridazin-3-yl]-2-methyl-1H-indol-5-yl}-amino)-acetate 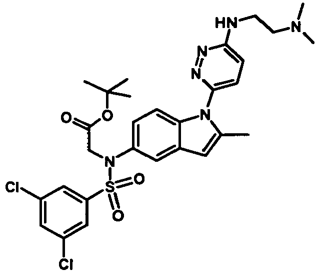 N'-(6-bromo-pyridazin-3-yl)-N,N-dimethyl-ethan-1 ,2-diamine is used instead of 2-iodo-benzonitrile. The reaction lasts 5 hours at 100°C.
   Mass spectrum (ESI⁺): m/z = 633 [M+H]⁺
(20) tert.butyl ((3-bromo-5-methyl-phenylsulphonyl)-{1-[6-(2-dimethylamino-ethylamino)-pyridazin-3-yl]-1H-indol-5-yl}-amino)-acetate 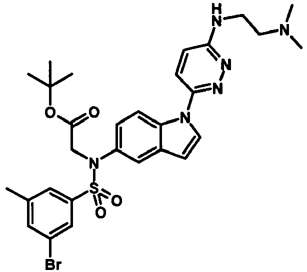 N'-(6-bromo-pyridazin-3-yl)-N,N-dimethyl-ethan-1,2-diamine is used instead of 2-iodo-benzonitrile. The reaction lasts 5 hours at 100°C.
   Mass spectrum (ESI⁺): m/z = 643 [M+H]⁺
(21) tert.butyl ((3,5-dichloro-phenylsulphonyl)-{1-[4-(2-dimethylamino-ethylcarbamoyl)-pyridin-2-yl]-1H-indol-5-yl}-amino)-acetate 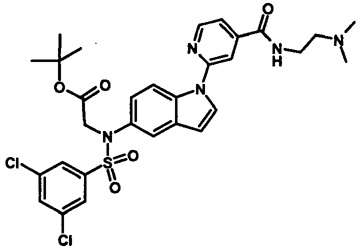 2-bromo-N-(2-dimethylamino-ethyl)-isonicotinamid is used instead of 2-iodo-benzonitrile. The reaction lasts 12 hours at 90°C.
   Mass spectrum (ESI⁺): m/z = 646 [M+H]⁺
(22) tert-butyl 4-(2-{5-[tert-butoxycarbonylmethyl-(3,5-dichloro-phenylsulphonyl)-amino]-indol-1-yl}-pyridin-4-carbonyl)-piperazine-1-carboxylate 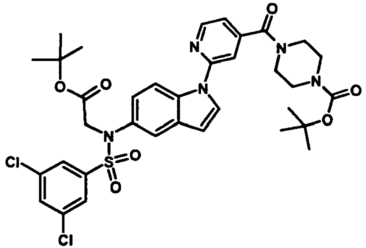 4-(2-bromo-pyridin-4-carbonyl)-piperazine-1-carboxylate tert-butyl is used instead of 2-iodo-benzonitrile. The reaction lasts 12 hours at 90°C.
   Mass spectrum (ESI⁺): m/z = 744 [M+H]⁺
(23) tert.butyl ((3-chloro-5-methyl-phenylsulphonyl)-{1-[6-(2-dimethylamino-ethylamino)-pyridazin-3-yl]-1H-indol-5-yl}-amino)-acetate 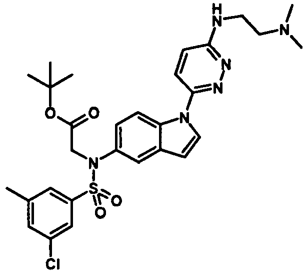 N'-(6-bromo-pyridazin-3-yl)-N,N-dimethyl-ethan-1,2-diamine is used instead of 2-iodo-benzonitrile. The reaction lasts 5 hours at 100°C.
   Mass spectrum (ESI⁺): m/z = 599 [M+H]⁺
(24) tert.butyl [{1-[5-(2-dimethylamino-ethylamino)-pyrazin-2-yl]-1H-indol-5-yl}-(3,5-dimethyl-phenylsulphonyl)-amino]-acetate 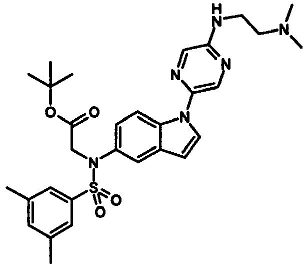 N'-(5-bromo-pyrazin-2-yl)-N,N-dimethyl-ethan-1,2-diamine is used instead of 2-iodo-benzonitrile. The reaction lasts 5 hours at 100°C.
   Mass spectrum (ESI⁺): m/z = 579 [M+H]⁺
(25) tert.butyl ((3-bromo-5-methyl-phenylsulphonyl)-{1-[5-(2-dimethylamino-ethylamino)-pyrazin-2-yl]-1H-indol-5-yl}-amino)-acetate 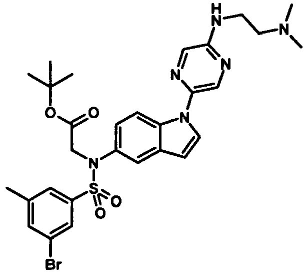 N'-(5-bromo-pyrazin-2-yl)-N,N-dimethyl-ethan-1,2-diamine is used instead of 2-iodo-benzonitrile. The reaction lasts 5 hours at 100°C.
   Mass spectrum (ESI⁺): m/z = 643 [M+H]⁺
(26) tert.butyl ((3,5-dibromo-phenylsulphonyl)-{1-[5-(2-dimethylamino-ethylamino)-pyrazin-2-yl]-1H-indol-5-yl}-amino)-acetate 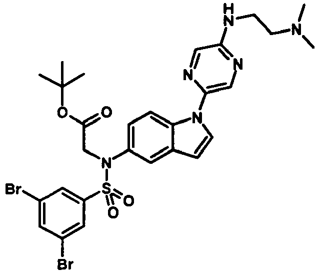 N'-(5-bromo-pyrazin-2-yl)-N,N-dimethyl-ethan-1,2-diamine is used instead of 2-iodo-benzonitrile. The reaction lasts 5 hours at 100°C.
   Mass spectrum (ESI⁻): m/z = 707 [M-H]⁻
(27) tert.butyl ((3-chloro-5-methyl-phenylsulphonyl)-{1-[5-(2-dimethylamino-ethylamino)-pyrazin-2-yl]-1H-indol-5-yl}-amino)-acetate 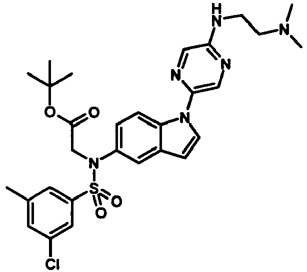 N'-(5-bromo-pyrazin-2-yl)-N,N-dimethyl-ethan-1,2-diamine is used instead of 2-iodo-benzonitrile. The reaction lasts 5 hours at 100°C.
   Mass spectrum (ESI⁺): m/z = 599 [M+H]⁺
(28) tert.butyl ((3,5-dibromo-phenylsulphonyl)-{1-[6-(2-dimethylamino-ethylamino)-pyridazin-3-yl]-1H-indol-5-yl}-amino)-acetate 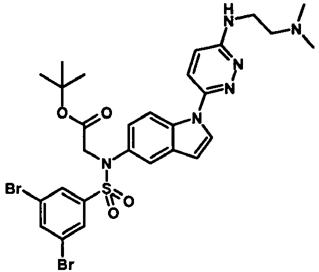 N'-(6-bromo-pyridazin-3-yl)-N,N-dimethyl-ethan-1,2-diamine is used instead of 2-iodo-benzonitrile. The reaction lasts 5 hours at 100°C.
   Mass spectrum (ESI⁻): m/z = 707 [M-H]⁻
(29) tert.butyl [[1-(6-amino-pyridazin-3-yl)-1H-indol-5-yl]-(3,5-dichlorophenylsulphonyl)-amino]-acetate 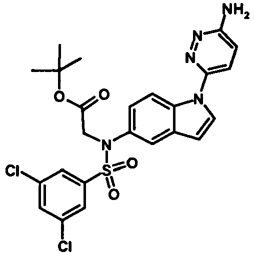 3-bromo-6-amino-pyridazine is used instead of 2-iodo-benzonitrile. The reaction lasts 12 hours at 90°C.
   Mass spectrum (ESI⁺): m/z = 548 [M+H]⁺
(30) tert.butyl [(3,5-dichloro-phenylsulphonyl)-(1-naphthalin-2-yl-1H-indol-5-yl)-amino]-acetate 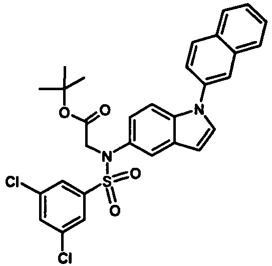 2-lodo-naphthalene is used instead of 2-iodo-benzonitrile. The reaction lasts 12 hours at 90°C.
   Mass spectrum (ESI⁺): m/z = 598 [M+NH₄]⁺
(31) tert.butyl ((3-bromo-5-chloro-phenylsulphonyl)-{1-[6-(2-dimethylamino-ethylamino)-pyridazin-3-yl]-1H-indol-5-yl}-amino)-acetate 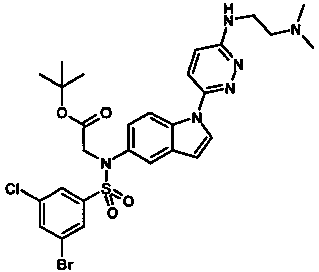 N'-(6-bromo-pyridazin-3-yl)-N,N-dimethyl-ethan-1,2-diamine is used instead of 2-iodo-benzonitrile. The reaction lasts 12 hours at 100°C.
   R_{f} value: 0.4 (dichloromethane/(methanol/water/acetic acid 8:1:1) 4:1)
(32) tert.butyl ((3-bromo-5-chloro-phenylsulphonyl)-{1-[5-(2-dimethylamino-ethylamino)-pyrazin-2-yl]-1H-indol-5-yl}-amino)-acetate 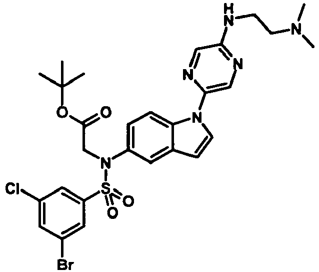 N'-(5-bromo-pyrazin-2-yl)-N,N-dimethyl-ethan-1,2-diamine is used instead of 2-iodo-benzonitrile. The reaction lasts 12 hours at 100°C.
   R_{f} value: 0.5 (dichloromethane/(methanol/water/acetic acid 8:1:1) 4:1)
(33) tert-butyl [[1-(5-amino-pyridin-2-yl)-1H-indol-5-yl]-(3,5-dichlorophenylsulphonyl)-amino]-acetate 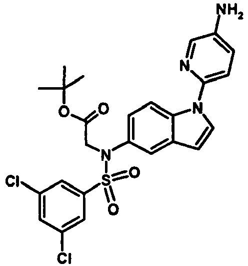 2-iodo-5-amino-pyridine is used instead of 2-iodo-benzonitrile. The reaction lasts 12 hours at 90°C.
   Mass spectrum (ESI⁺): m/z = 547 [M+H]⁺
(34) tert.butyl {(3,5-dichloro-phenylsulphonyl)-[1-(2-methyl-quinolin-6-yl)-1H-indol-5-yl]-amino}-acetate 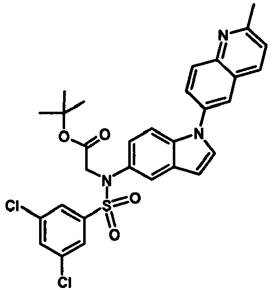 6-bromo-2-metyhl-quinoline is used instead of 2-iodo-benzonitrile. The reaction lasts 12 hours at 90°C.
   Mass spectrum (ESI⁺): m/z = 596 [M+H]⁺
(35) tert.butyl [(3,5-dichloro-phenylsulphonyl)-(1-naphthalin-1-yl-1H-indol-5-yl)-amino]-acetate 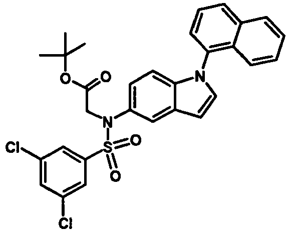 1-iodo-naphthalene is used instead of 2-iodo-benzonitrile. The reaction lasts 12 hours at 90°C.
   Mass spectrum (ESI⁺): m/z = 598 [M+NH₄]⁺
(36) tert.butyl {(3,5-dichloro-phenylsulphonyl)-[1-(3-methyl-pyridin-2-yl)-1H-indol-5-yl]-amino}-acetate 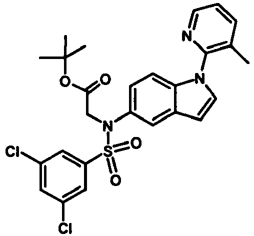 2-bromo-3-methyl-pyridine is used instead of 2-iodo-benzonitrile. The reaction lasts 12 hours at 90°C.
   Mass spectrum (ESI⁺): m/z = 546 [M+H]⁺
(37) tert-butyl 4-(6-{5-[tert-butoxycarbonylmethyl-(3,5-dichloro-phenylsulphonyl)-amino]-indol-1-yl}-pyridin-2-carbonyl)-piperazine-1-carboxylate 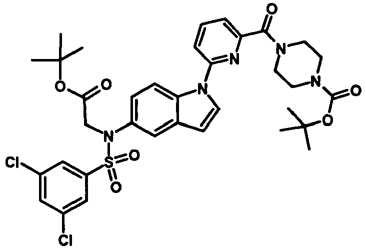 Tert-butyl 4-(6-bromo-pyridine-2-carbonyl)-piperazine-1-carboxylate is used instead of 2-iodo-benzonitrile. The reaction lasts 12 hours at 90°C.
   Mass spectrum (ESI⁺): m/z = 761 [M+NH₄]⁺
(38) tert.butyl ((3,5-dichloro-phenylsulphonyl)-{1-[6-(2-dimethylamino-ethylcarbamoyl)-pyridin-2-yl]-1H-indol-5-yl}-amino)-acetate 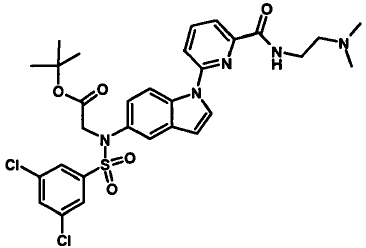 6-bromo-pyridin-2-carboxylic acid-(2-dimethylamino-ethyl)-amide is used instead of 2-iodo-benzonitrile. The reaction lasts 12 hours at 90°C.
   Mass spectrum (ESI⁺): m/z = 646 [M+H]⁺
(39) tert-butyl 4-(6-{5-[tert-butoxycarbonylmethyl-(3,5-dichloro-phenylsulphonyl)-amino]-indol-1-yl}-pyridazin-3-yl)-[1,4]diazepan-1-carboxylate 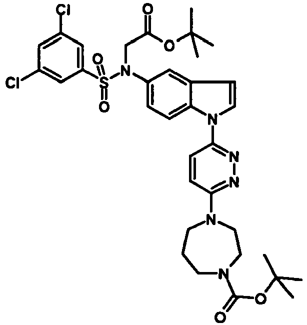 Tert-butyl 4-(6-iodo-pyridazin-3-yl)-[1,4]diazepan-1-carboxylate is used instead of 2-iodo-benzonitrile. The reaction lasts 5 hours at 100°C.
   Mass spectrum (ESI⁺): m/z = 731 [M+H]⁺
(40) tert.butyl {(3,5-dichloro-phenylsulphonyl)-[1-(6-methyl-pyridin-2-yl)-1H-indol-5-yl]-amino}-acetate 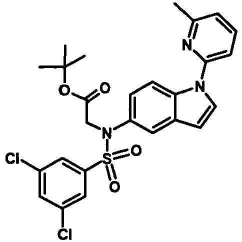 2-bromo-6-methyl-pyridine is used instead of 2-iodo-benzonitrile. The reaction lasts 12 hours at 90°C.
   Mass spectrum (ESI⁺): m/z = 546 [M+H]⁺
(41) tert.butyl [(1-{6-[tert-butoxycarbonyl-(3-(bis-tert-butoxycarbonyl)-aminopropyl)-amino]-pyridazin-3-yl}-1H-indol-5-yl)-(3,5-dichloro-phenylsulphonyl)-amino]-acetate 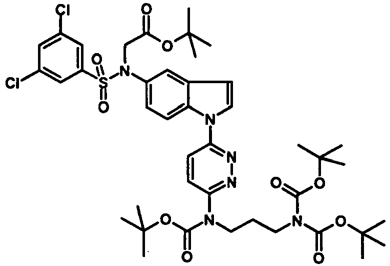 Tert-butyl (3-(bis-tert-butoxycarbonyl)-amino-propyl)-(6-iodo-pyridazin-3-yl)-carbamate is used instead of 2-iodo-benzonitrile. The reaction lasts 5 hours at 100°C.
   Mass spectrum (ESI⁺): m/z = 922 [M+NH₄]⁺
(42) tert.butyl {(3,5-dichloro-phenylsulphonyl)-[1-(4-methyl-pyridin-2-yl)-1H-indol-5-yl]-amino}-acetate 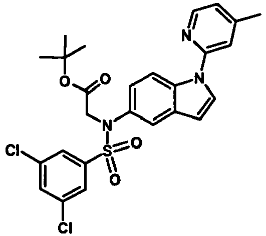 2-bromo-4-methyl-pyridine is used instead of 2-iodo-benzonitrile. The reaction lasts 12 hours at 90°C.
   Mass spectrum (ESI⁺): m/z = 546 [M+H]⁺
(43) tert.butyl [[1-(6-cyclopropylamino-pyridazin-3-yl)-1H-indol-5-yl]-(3,5-dichlorophenylsulphonyl)-amino]-acetate 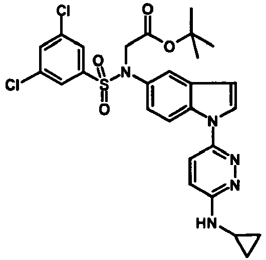 3-iodo-6-cyclopropylamino-pyridazine is used instead of 2-iodo-benzonitrile. The reaction lasts 5 hours at 100°C.
   Mass spectrum (ESI⁺): m/z = 588 [M+H]⁺
(44) tert.butyl {(3,5-dichloro-phenylsulphonyl)-[1-(5-methyl-pyridin-2-yl)-1H-indol-5-yl]-amino}-acetate 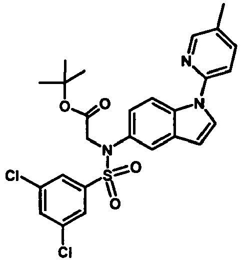 3-iodo-6-cyclopropylamino-pyridazine is used instead of 2-iodo-benzonitrile. The reaction lasts 12 hours at 90°C.
   Mass spectrum (ESI⁺): m/z = 546 [M+H]⁺
(45) tert.butyl [(3,5-dichloro-phenylsulphonyl)-(1-isoquinolin-1-yl-1H-indol-5-yl)-amino]-acetate 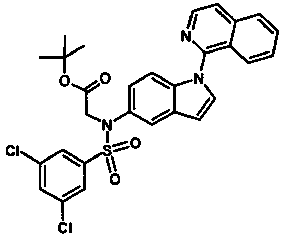 1-bromo-isoquinoline is used instead of 2-iodo-benzonitrile. The reaction lasts 12 hours at 90°C.
   Mass spectrum (ESI⁺): m/z = 582 [M+H]⁺
(46) tert.butyl [[1-(3-amino-pyridin-2-yl)-1H-indol-5-yl]-(3,5-dichlorophenylsulphonyl)-amino]-acetate 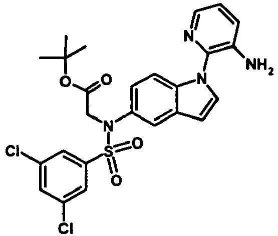 1-bromo-isoquinoline is used instead of 2-iodo-benzonitrile. The reaction lasts 12 hours at 90°C.
   Mass spectrum (ESI⁺): m/z = 547 [M+H]⁺
(47) tert.butyl ((3,5-dichloro-phenylsulphonyl)-{1-[6-(2-dimethylamino-ethylamino)-pyridazin-3-yl]-3-methyl-1H-indol-5-yl}-amino)-acetate 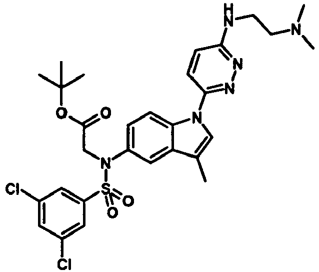 N'-(6-bromo-pyridazin-3-yl)-N,N-dimethyl-ethan-1,2-diamine is used instead of 2-iodo-benzonitrile. The reaction lasts 12 hours at 100°C.
   Mass spectrum (ESI⁺): m/z = 633 [M+H]⁺
(48) tert.butyl [(1-{6-[(2-(bis-tert-butoxycarbonyl)-amino-ethyl)-methyl-amino]-pyridazin-3-yl}-1H-indol-5-yl)-(3,5-dichloro-phenylsulphonyl)-amino]-acetate 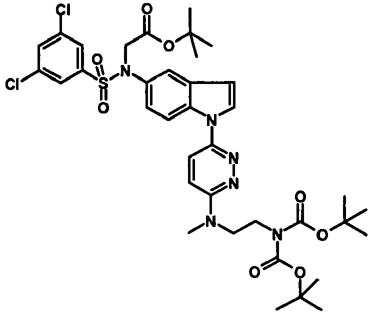 {3-[(2-(bis-tert-butoxycarbonyl)-amino-ethyl)-methyl-amino]}-6-iodo-pyridazine is used instead of 2-iodo-benzonitrile. The reaction lasts 12 hours at 100°C.
   Mass spectrum (ESI⁺): m/z = 805 [M+H]⁺
(49) tert.butyl [[1-(6-{tert-butoxycarbonyl-[2-(tert-butoxycarbonyl-methyl-amino)-ethyl]-amino}-pyridazin-3-yl)-1H-indol-5-yl]-(3,5-dichloro-phenylsulphonyl)-amino]-acetate 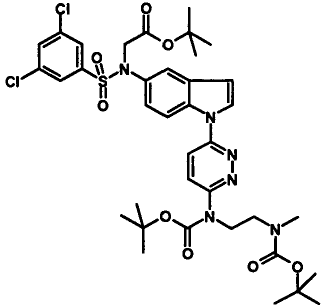 Tert-butyl [2-(tert-butoxycarbonyl-methyl-amino)-ethyl]-(6-iodo-pyridazin-3-yl)-carbamate is used instead of 2-iodo-benzonitrile. The reaction lasts 12 hours at 100°C.
   Mass spectrum (ESI⁺): m/z = 805 [M+H]⁺
(50) tert.butyl ((3,5-dichloro-phenylsulphonyl)-{1-[3-(2-dimethylamino-ethylamino)-pyridin-2-yl]-1H-indol-5-yl}-amino)-acetate 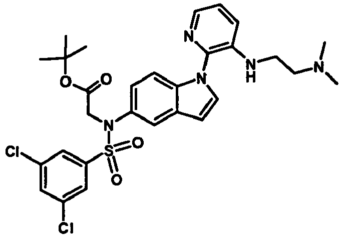 N*3*-(2-dimethylamino-ethyl)-pyridin-2,3-diamine is used instead of 2-iodo-benzonitrile. The reaction lasts 12 hours at 90°C.
   Mass spectrum (ESI⁺): m/z = 618 [M+H]⁺
(51) tert.butyl [{1-[6-(2-amino-2-methyl-propylamino)-pyridazin-3-yl]-1H-indol-5-yl}-(3,5-dichloro-phenylsulphonyl)-amino]-acetate 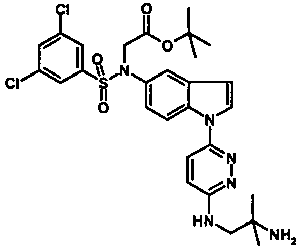 N*1*-(6-iodo-pyridazin-3-yl)-2-methyl-propane-1,2-diamine is used instead of 2-iodo-benzonitrile. The reaction lasts 24 hours at 100°C.
   Mass spectrum (ESI⁺): m/z = 619 [M+H]⁺
(52) tert.butyl [{1-[6-(3-tert-butoxycarbonylamino-piperidin-1-yl)-pyridazin-3-yl]-1H-indol-5-yl}-(3,5-dichloro-phenylsulphonyl)-amino]-acetate 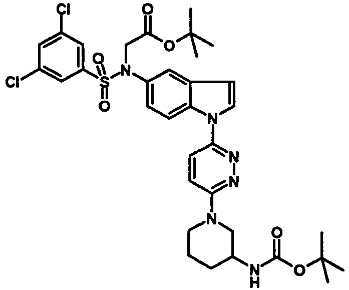 Tert-butyl [1-(6-iodo-pyridazin-3-yl)-piperidin-3-yl]-carbamate is used instead of 2-iodo-benzonitrile. The reaction lasts 12 hours at 100°C.
   Mass spectrum (ESI⁺): m/z = 731 [M+H]⁺
(53) ethyl {(3,5-dichloro-phenylsulphonyl)-[1-(6-methyl-pyridazin-3-yl)-1H-indol-5-yl]-amino}-acetate 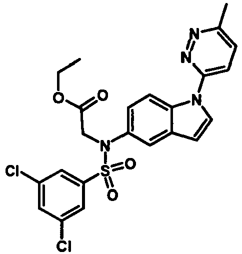 3-iodo-6-methyl-pyridazine is used instead of 2-iodo-benzonitrile. The reaction lasts 12 hours at 90°C.
   Mass spectrum (ESI⁺): m/z = 519 [M+H]⁺
(54) tert.butyl [{3-cyano-1-[6-(2-dimethylamino-ethylamino)-pyridazin-3-yl]-1H-indol-5-yl}-(3,5-dichloro-phenylsulphonyl)-amino]-acetate 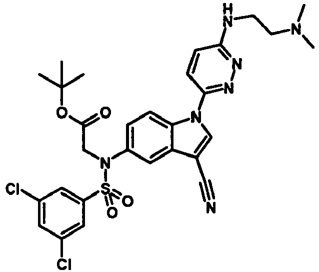 N'-(6-bromo-pyridazin-3-yl)-N,N-dimethyl-ethan-1,2-diamine is used instead of 2-iodo-benzonitrile. The reaction lasts 12 hours at 100°C.
   R_{f} value: 0.5 (dichloromethane/(methanol/33% ammonia in water 9:1) 9:1)
(55) tert.butyl {(3,5-dichloro-phenylsulphonyl)-[1-(6-morpholin-4-yl-pyridazin-3-yl)-1H-indol-5-yl]-amino}-acetate 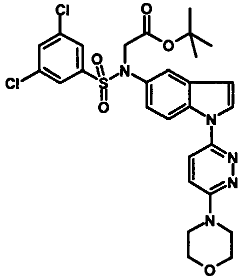 4-(6-iodo-pyridazin-3-yl)-morpholine is used instead of 2-iodo-benzonitrile. The reaction lasts 12 hours at 100°C.
   Mass spectrum (ESI⁺): m/z = 618 [M+H]⁺
(56) tert.butyl [[1-(5-amino-pyrazin-2-yl)-1H-indol-5-yl]-(3,5-dichlorophenylsulphonyl)-amino]-acetate 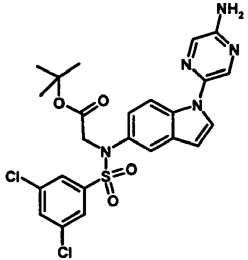 2-amino-5-bromo-pyrazine is used instead of 2-iodo-benzonitrile. The reaction lasts 12 hours at 90°C.
   Mass spectrum (ESI⁺): m/z = 565 [M+NH₄]⁺
(57) tert.butyl [{3-chloro-1-[6-(2-dimethylamino-ethylamino)-pyridazin-3-yl]-1H-indol-5-yl}-(3,5-dichloro-phenylsulphonyl)-amino]-acetate 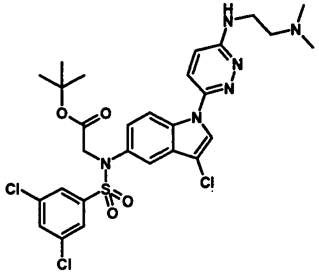 N'-(6-bromo-pyridazin-3-yl)-N,N-dimethyl-ethan-1,2-diamine is used instead of 2-iodo-benzonitrile. The reaction lasts 12 hours at 100°C.
   Mass spectrum (ESI⁺): m/z = 655 [M+H]⁺
(58) tert.butyl ((3,5-dichloro-phenylsulphonyl)-{1-[6-(2-dimethylamino-ethylamino)-pyridazin-3-yl]-3-trifluoromethyl-1H-indol-5-yl}-amino)-acetate 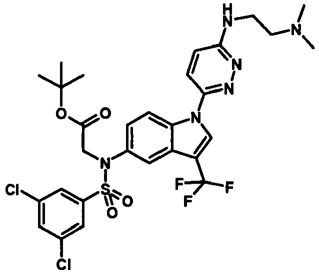 N'-(6-bromo-pyridazin-3-yl)-N,N-dimethyl-ethan-1,2-diamine is used instead of 2-iodo-benzonitrile. The reaction lasts 12 hours at 100°C.
   Mass spectrum (ESI⁺): m/z = 687 [M+H]⁺
(59) tert-butyl 4-(6-{5-[tert-butoxycarbonylmethyl-(3,5-dichloro-phenylsulphonyl)-amino]-indol-1-yl}-pyridin-3-carbonyl)-piperazine-1-carboxylate 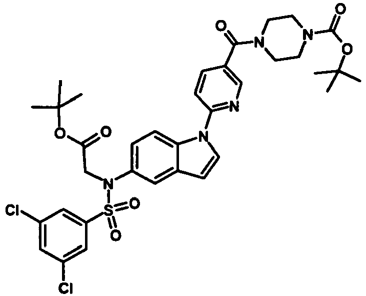 Tert-butyl 4-(6-bromo-pyridin-3-carbonyl)-piperazine-1-carboxylate is used instead of 2-iodo-benzonitrile. The reaction lasts 12 hours at 90°C. Mass spectrum (ESI⁺): m/z = 744 [M+H]⁺
(60) tert.butyl ((3,5-dichloro-phenylsulphonyl)-{1-[5-(2-dimethylamino-ethylcarbamoyl)-pyridin-2-yl]-1H-indol-5-yl}-amino)-acetate 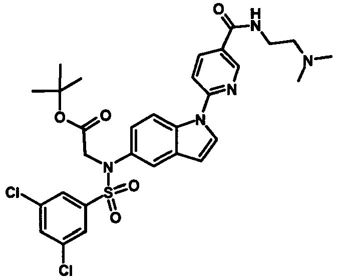 6-bromo-N-(2-dimethylamino-ethyl)-nicotinamide is used instead of 2-iodo-benzonitrile. The reaction lasts 12 hours at 90°C.
   Mass spectrum (ESI⁺): m/z = 646 [M+H]⁺
(61) tert.butyl {(3,5-dichloro-phenylsulphonyl)-[1-(6-dimethylamino-pyridazin-3-yl)-1H-indol-5-yl]-amino}-acetate 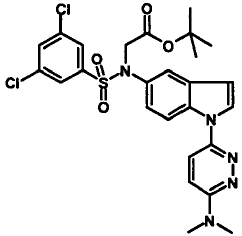 3-dimethylamino-6-iodo-pyridazine is used instead of 2-iodo-benzonitrile. The reaction lasts 12 hours at 100°C.
   Mass spectrum (ESI⁺): m/z = 576 [M+H]⁺
(62) tert.butyl ((3,5-dichloro-phenylsulphonyl)-{1-[6-(2-hydroxy-ethylamino)-pyridazin-3-yl]-1H-indol-5-yl}-amino)-acetate 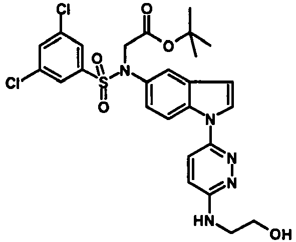 3-(2-hydroxyethyl)-amino-6-iodo-pyridazine is used instead of 2-iodo-benzonitrile. The reaction lasts 5 hours at 100°C.
   Mass spectrum (ESI⁺): m/z = 592 [M+H]⁺
(63) tert.butyl [(1-{6-[tert-butoxycarbonyl-(2-piperidin-1-yl-ethyl)-amino]-pyridazin-3-yl}-1H-indol-5-yl)-(3,5-dichloro-phenylsulphonyl)-amino]-acetate 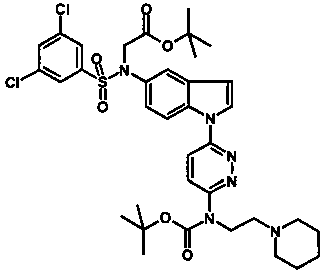 Tert-butyl (6-iodo-pyridazin-3-yl)-(2-piperidin-1-yl-ethyl)-carbamate is used instead of 2-iodo-benzonitrile. The reaction lasts 12 hours at 100°C.
   Mass spectrum (ESI⁺): m/z = 759 [M+H]⁺
(64) tert.butyl {(3,5-dichloro-phenylsulphonyl)-[1-(4-morpholin-4-yl-pyridin-2-yl)-1H-indol-5-yl]-amino}-acetate 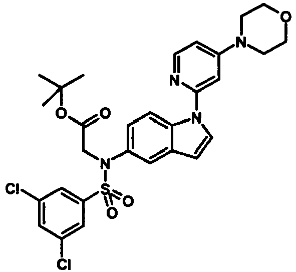 4-(2-bromo-pyridin-4-yl)-morpholine is used instead of 2-iodo-benzonitrile. Mass spectrum (ESI⁺): m/z = 617 [M+H]⁺
   R_{f} value: 0.40 (silica gel: ethyl acetate/petroleum ether 1:1)
(65) tert.butyl {(3,5-dichloro-phenylsulphonyl)-[1-(5-morpholin-4-yl-pyridin-2-yl)-1H-indol-5-yl]-amino}-acetate 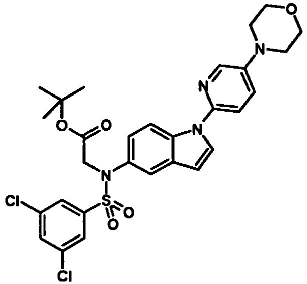 4-(6-bromo-pyridin-3-yl)-morpholine is used instead of 2-iodo-benzonitrile. Mass spectrum (ESI⁺): m/z = 617 [M+H]⁺
   R_{f} value: 0.35 (silica gel: dichloromethane/ethyl acetate 9:1)
(66) tert.butyl ((3,5-dichloro-phenylsulphonyl)-{1-[5-(3-oxo-morpholin-4-yl)-pyrazin-2-yl]-1H-indol-5-yl}-amino)-acetate 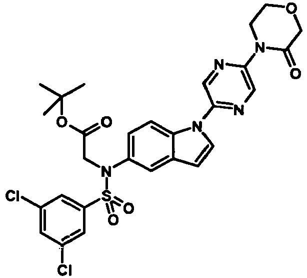 4-(5-bromo-pyrazin-2-yl)-morpholin-3-one is used instead of 2-iodo-benzonitrile. Mass spectrum (ESI⁺): m/z = 632 [M+H]⁺
   R_{f} value: 0.38 (silica gel: ethyl acetate/petroleum ether 1:1)
(67) tert.butyl ((3,5-dichloro-phenylsulphonyl)-{1-[5-(tetrahydro-pyran-4-yloxy)-pyrazin-2-yl]-1H-indol-5-yl}-amino)-acetate 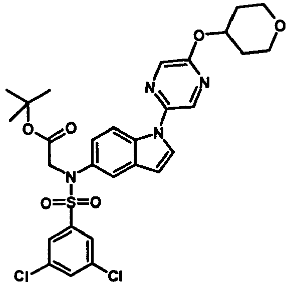 2-bromo-5-(tetrahydro-pyran-4-yloxy)-pyrazine is used instead of 2-iodo-benzonitrile.
   Mass spectrum (ESI⁺): m/z = 650 [M+NH₄]⁺
   R_{f} value: 0.20 (silica gel: petroleum ether/ethyl acetate 8:2)
(68) tert.butyl (S)-((3,5-dichloro-phenylsulphonyl)-{1-[5-(tetrahydro-furan-3-yloxy)-pyrazin-2-yl]-1H-indol-5-yl}-amino)-acetate 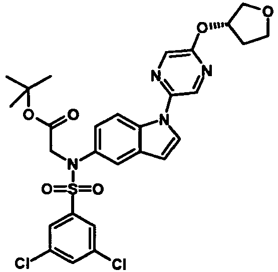 (S)-2-bromo-5-(tetrahydro-furan-3-yloxy)-pyrazine is used instead of 2-iodo-benzonitrile.
   Mass spectrum (ESI⁺): m/z = 636 [M+NH₄]⁺
   R_{f} value: 0.20 (silica gel: petroleum ether/ethyl acetate 8:2)
(69) tert.butyl (R)-((3,5-dichloro-phenylsulphony)-{1-[6-(tetrahydro-furan-3-ylamino)-pyridazin-3-yl]-1H-indol-5-yl}-amino)-acetate 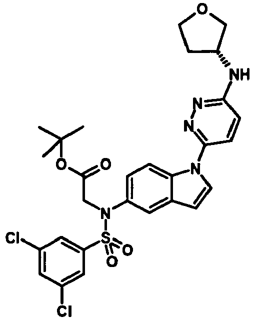 (R)-(6-iodo-pyridazin-3-yl)-(tetrahydro-furan-3-yl)-amine is used instead of 2-iodo-benzonitrile.
   Mass spectrum (ESI⁺): m/z = 618 [M+H]⁺
   R_{f} value: 0.24 (silica gel: dichloromethane/ethyl acetate 7:3)
(70) tert.butyl ((3,5-dichloro-phenylsulphony)-{1-[6-(3-oxo-morpholin-4-yl)-pyridazin-3-yl]-1H-indol-5-yl}-amino)-acetate 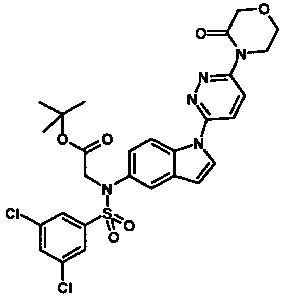 4-(6-bromo-pyridazin-3-yl)-morpholin-3-one is used instead of 2-iodo-benzonitrile.
   Mass spectrum (ESI⁺): m/z = 632 [M+H]⁺
   R_{f} value: 0.22 (silica gel: petroleum ether/ethyl acetate 1:1)
(71) tert.butyl (R)-((3,5-dichloro-phenylsulphonyl)-{1-[5-(tetrahydro-furan-3-yloxy)-pyrazin-2-yl]-1H-indol-5-yl}-amino)-acetate 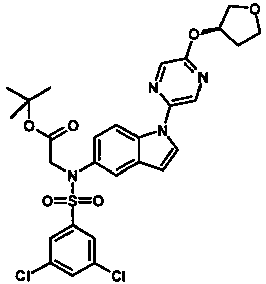 (R)-2-bromo-5-(tetrahydro-furan-3-yloxy)-pyrazine is used instead of 2-iodo-benzonitrile.
   Mass spectrum (ESI⁺): m/z = 636 [M+NH₄]⁺
   R_{f} value: 0.25 (silica gel: petroleum ether/ethyl acetate 7:3)
(72) tert.butyl (S)-((3,5-dichloro-phenylsulphony)-{1-[6-(tetrahydro-furan-3-yloxy)-pyridazin-3-yl]-1H-indol-5-yl}-amino)-acetate 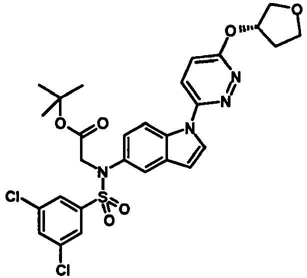 (S)-3-iodo-6-(tetrahydro-furan-3-yloxy)-pyridazine is used instead of 2-iodo-benzonitrile.
   Mass spectrum (ESI⁺): m/z = 619 [M+H]⁺
   R_{f} value: 0.15 (silica gel: petroleum ether/ethyl acetate 7:3)
(73) tert.butyl ((3,5-dichloro-phenylsulphonyl)-{1-[6-(tetrahydro-pyran-4-yloxy)-pyridazin-3-yl]-1H-indol-5-yl)-amino)-acetate 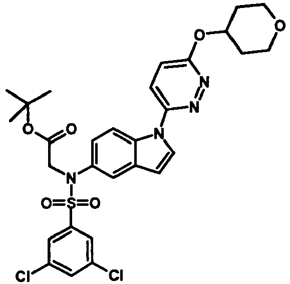 3-iodo-6-(tetrahydro-pyran-4-yloxy)-pyridazine is used instead of 2-iodo-benzonitrile.
   Mass spectrum (ESI⁺): m/z = 633 [M+H]⁺
   R_{f} value: 0.20 (silica gel: petroleum ether/ethyl acetate 7:3)
(74) tert.butyl (R)-((3,5-dichloro-phenylsulphonyl)-{1-[6-(tetrahydro-furan-3-yloxy)-pyridazin-3-yl]-1H-indol-5-yl}-amino)-acetate 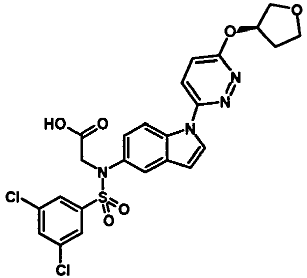 (R)-3-iodo-6-(tetrahydro-furan-3-yloxy)-pyridazine is used instead of 2-iodo-benzonitrile.
   Mass spectrum (ESI⁺): m/z = 619 [M+H]⁺
   R_{f} value: 0.45 (silica gel: cyclohexane/ethyl acetate 1:1)
(75) tert.butyl ((3,5-dichloro-phenylsulphonyl)-{1-[5-(3-oxo-morpholin-4-yl)-pyridin-2-yl]-1H-indol-5-yl}-amino)-acetate 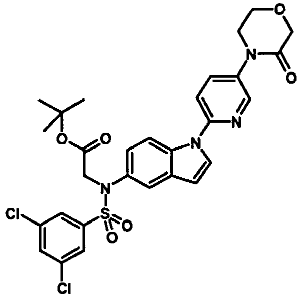 4-(6-bromo-pyridin-3-yl)-morpholin-3-one is used instead of 2-iodo-benzonitrile. Mass spectrum (ESI⁺): m/z = 631 [M+H]⁺
   R_{f} value: 0.45 (silica gel: dichloromethane/ethyl acetate 7:3)
(76) tert.butyl (R)-((3,5-dichloro-phenylsulphonyl)-{1-[5-(tetrahydro-furan-3-ylamino)-pyrazin-2-yl]-1H-indol-5-yl}-amino)-acetate 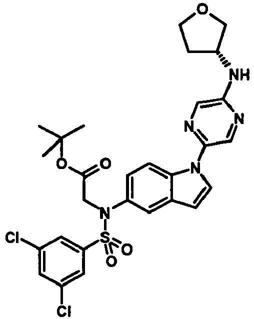 (R)-(5-bromo-pyrazin-2-yl)-(tetrahydro-furan-3-yl)-amine is used instead of 2-iodo-benzonitrile.
   Mass spectrum (ESI⁺): m/z = 618 [M+H]⁺
   R_{f} value: 0.48 (silica gel: dichloromethane/ethyl acetate 7:3)
(77) tert.butyl ((3,5-dichloro-phenylsulphonyl)-{1-[4-(3-oxo-morpholin-4-yl)-pyridin-2-yl]-1H-indol-5-yl}-amino)-acetate 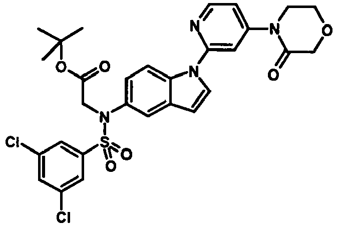 4-(2-bromo-pyridin-4-yl)-morpholin-3-one is used instead of 2-iodo-benzonitrile. Mass spectrum (ESI⁺): m/z = 631 [M+H]⁺
   R_{f} value: 0.57 (silica gel: dichloromethane/ethyl acetate 7:3)
(78) tert.butyl [(3,5-dichloro-phenylsulphonyl)-(1-{6-[methyl-(tetrahydro-pyran-4-yl)-amino]-pyridazin-3-yl}-1H-indol-5-yl)-amino]-acetate 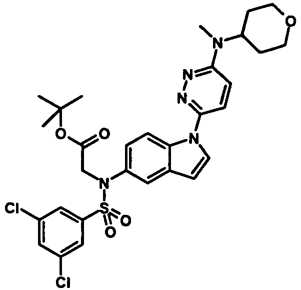 (6-iodo-pyridazin-3-yl)-methyl-(tetrahydro-pyran-4-yl)-amine is used instead of 2-iodo-benzonitrile.
   Mass spectrum (ESI⁺): m/z = 646 [M+H]⁺
   R_{f} value: 0.57 (silica gel: dichloromethane/ethyl acetate 7:3)
(79) tert.butyl ((3,5-dichloro-phenylsulphonyl)-{1-[5-(3-oxo-piperazin-1-yl)-pyrazin-2-yl]-1H-indol-5-yl}-amino)-acetate 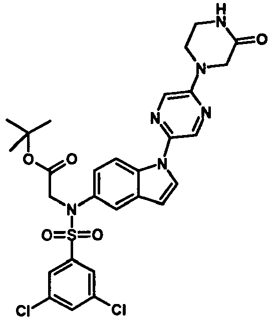 4-(5-bromo-pyrazin-2-yl)-piperazin-2-one is used instead of 2-iodo-benzonitrile. Mass spectrum (ESI⁺): m/z = 631 [M+H]⁺
   R_{f} value: 0.40 (silica gel: cyclohexane/ethyl acetate 1:1)
(80) tert.butyl ((3,5-dichloro-phenylsulphonyl)-{1-[5-(tetrahydro-pyran-4-ylamino)-pyrazin-2-yl]-1H-indol-5-yl}-amino)-acetate 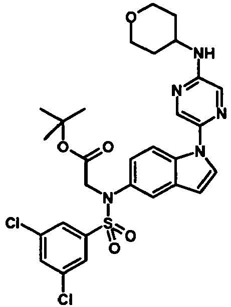 (5-bromo-pyrazin-2-yl)-(tetrahydro-pyran-4-yl)-amine is used instead of 2-iodo-benzonitrile.
   R_{f} value: 0.40 (silica gel: dichloromethane/ethyl acetate 7:3)
(81) tert.butyl ((3,5-dichloro-phenylsulphonyl)-{1-[6-(tetrahydro-pyran-4-ylamino)-pyridazin-3-yl]-1H-indol-5-yl}-amino)-acetate 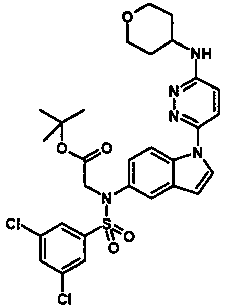 (6-iodo-pyridazin-3-yl)-(tetrahydro-pyran-4-yl)-amine is used instead of 2-iodo-benzonitrile.
   Mass spectrum (ESI⁺): m/z = 632 [M+H]⁺
   R_{f} value: 0.24 (silica gel: dichloromethane/ethyl acetate 7:3)
(82) (114) tert.butyl {(3,5-dichloro-phenylsulphonyl)-[1-(6-methyl-pyridazin-3-yl)-1H-indazol-5-yl]-amino}-acetate 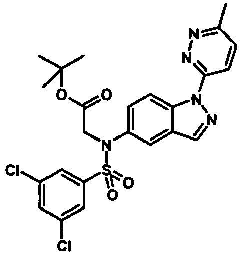 Tert.butyl [(3,5-dichloro-phenylsulphonyl)-(1H-indazol-5-yl)-amino]-acetate and 3-iodo-6-methylpyridazine are used.
   Mass spectrum (ESI⁺): m/z = 548 [M+H]⁺
   R_{f} value: 0.80 (silica gel: dichloromethane/ethyl acetate 7:3)
(83) tert.butyl ((3,5-dichloro-phenylsulphonyl)-{1-[5-(tetrahydro-furan-3-yloxy)-pyridin-2-yl]-1H-indol-5-yl}-amino)-acetate 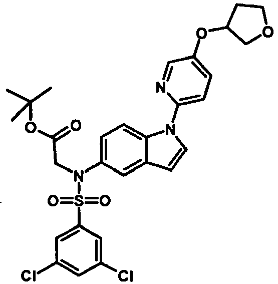 2-bromo-5-(tetrahydro-furan-3-yloxy)-pyridine is used instead of 2-iodo-benzonitrile.
   Mass spectrum (ESI⁺): m/z = 618 [M+H]⁺
   R_{f} value: 0.50 (silica gel: cyclohexane/ethyl acetate 1:1)
(84) tert.butyl ((3,5-dichloro-phenylsulphonyl)-{1-[5-(tetrahydro-pyran-4-yloxy)-pyridin-2-yl]-1H-indol-5-yl}-amino)-acetate 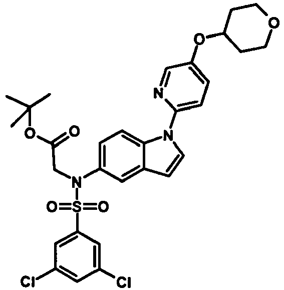 2-bromo-5-(tetrahydro-pyran-4-yloxy)-pyridine is used instead of 2-iodo-benzonitrile.
   Mass spectrum (ESI⁺): m/z = 632 [M+H]⁺
   R_{f} value: 0.60 (silica gel: cyclohexane/ethyl acetate 1:1)
(85) tert.butyl ((3,5-dichloro-phenylsulphonyl)-{1-[4-(tetrahydro-furan-3-yloxy)-pyridin-2-yl]-1H-indol-5-yl}-amino)-acetate 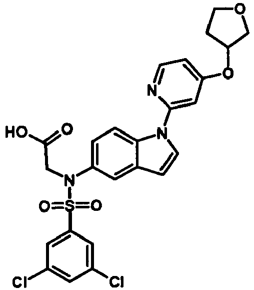 2-bromo-4-(tetrahydro-furan-3-yloxy)-pyridine is used instead of 2-iodo-benzonitrile.
   Mass spectrum (ESI⁺): m/z = 618 [M+H]⁺
   R_{f} value: 0.45 (silica gel: cyclohexane/ethyl acetate 1:1)
(86) tert.butyl [(3,5-dichloro-phenylsulphonyl)-(1-{5-[methyl-(tetrahydro-pyran-4-yl)-amino]-pyrazin-2-yl)-1H-indol-5-yl)-amino]-acetate 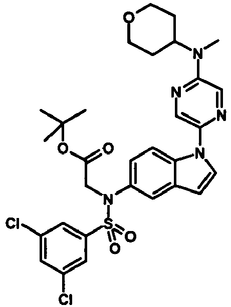 (5-bromo-pyrazin-2-yl)-methyl-(tetrahydro-pyran-4-yl)-amine is used instead of 2-iodo-benzonitrile.
   Mass spectrum (ESI⁺): m/z = 646 [M+H]⁺
   R_{f} value: 0.75 (silica gel: dichloromethane/ethyl acetate 7:3)
(87) tert.butyl ((3,5-dichloro-phenylsulphonyl)-{3-iodo-1-[6-(3-oxo-piperazin-1-yl)-pyridazin-3-yl]-1H-indol-5-yl}-amino)-acetate 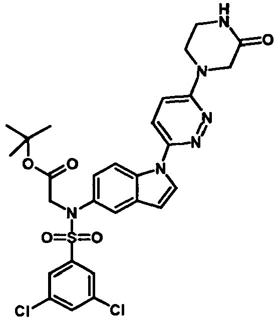 4-(6-iodo-pyridazin-3-yl)-piperazin-2-one is used instead of 2-iodo-benzonitrile.
   Mass spectrum (ESI⁺): m/z = 631 [M+H]⁺
(88) tert.butyl ((3,5-dichloro-phenylsulphonyl)-{1-[6-(3-oxo-piperazin-1-yl)-pyridazin-3-yl]-1H-indol-5-yl}-amino)-acetate 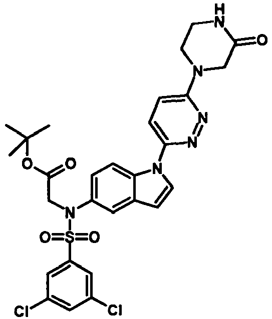 4-(6-iodo-pyridazin-3-yl)-piperazin-2-one is used instead of 2-iodo-benzonitrile. Mass spectrum (ESI⁺): m/z = 757 [M+H]⁺
(89) tert.butyl ((3,5-dichloro-phenylsulphonyl)-{1-[4-(tetrahydro-pyran-4-yloxy)-pyridin-2-yl]-1H-indol-5-yl}-amino)-acetate 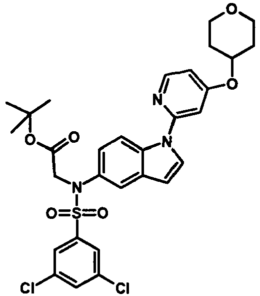 2-bromo-4-(tetrahydro-pyran-4-yloxy)-pyridine is used instead of 2-iodo-benzonitrile.
   Mass spectrum (ESI⁺): m/z = 632 [M+H]⁺
   R_{f} value: 0.45 (silica gel: cyclohexane/ethyl acetate 1:1)
(90) tert.butyl [[6-chloro-1-(6-methyl-pyridazin-3-yl)-1H-indol-5-yl]-(3,5-dichlorophenylsulphonyl)-amino]-acetate 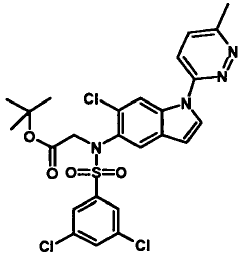 3-iodo-6-methyl-pyridazine is used instead of 2-iodo-benzonitrile.
   Mass spectrum (ESI⁺): m/z = 581 [M+H]⁺
   R_{f} value: 0.75 (silica gel: dichloromethane/methanol 98:2)
(91) tert.butyl [[3-methyl-1-(6-methyl-pyridazin-3-yl)-1H-indazol-5-yl]-(3,5-dichloro-phenylsulphonyl)-amino]-acetate 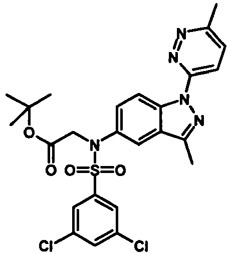 3-iodo-6-methyl-pyridazine is used instead of 2-iodo-benzonitrile.
   Mass spectrum (ESI⁺): m/z = 562 [M+H]⁺
   R_{f} value: 0.58 (silica gel: petroleum ether/ethyl acetate 1:1)
(92) tert.butyl {(3,5-dichloro-phenylsulphonyl)-[1-(5-trifluoromethyl-pyridazin-3-yl)-1H-indol-5-yl]-amino)-acetate 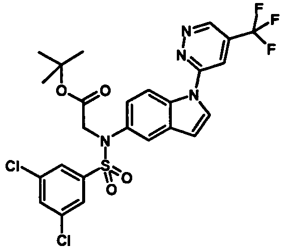 3-Iodo-5-trifluoromethyl-pyridazine is used instead of 2-iodo-benzonitrile. Mass spectrum (ESI⁺): m/z = 601 [M+H]⁺
   R_{f} value: 0.31 (silica gel: petroleum ether/ethyl acetate 5:1)
(93) tert.butyl {(3,5-dichloro-phenylsulphonyl)-[3-iodo-1-(5-trifluoromethylpyridazin-3-yl)-1H-indol-5-yl]-amino}-acetate 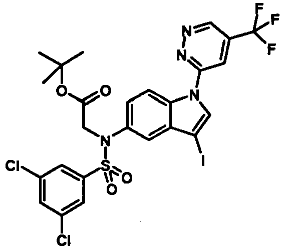 3-lodo-5-trifluoromethyl-pyridazine is used instead of 2-iodo-benzonitrile.
   Mass spectrum (ESI⁺): m/z = 744 [M+NH₄]⁺
   R_{f} value: 0.38 (silica gel: petroleum ether/ethyl acetate 5:1)
(94) tert.butyl {(3,5-dichloro-phenylsulphonyl)-[1-(6-iodo-pyrimidin-4-yl)-1H-indol-5-yl]-amino}-acetate 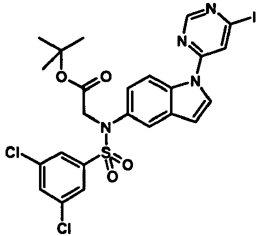 4,6-diiodo-pyrimidine is used instead of 2-iodo-benzonitrile.
   Mass spectrum (ESI⁺): m/z = 676 [M+NH₄]⁺
   R_{f} value: 0.67 (silica gel: petroleum ether/ethyl acetate 2:1)
(95) (128) tert.butyl [[6-ethyl-1-(6-methyl-pyridazin-3-yl)-1H-indol-5-yl]-(3,5-dichloro-phenylsulphonyl)-amino]-acetate 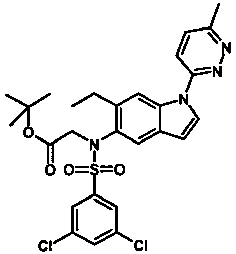 3-iodo-6-methyl-pyridazine is used instead of 2-iodo-benzonitrile.
   Mass spectrum (ESI⁺): m/z = 575 [M+H]⁺
   R_{f} value: 0.40 (silica gel: cyclohexane/ethyl acetate 1:1)
(96) tert.butyl {(3,5-dichloro-phenylsulphonyl)-[1-(6-methylamino-pyridazin-3-yl)-1H-indol-5-yl]-amino}-acetate 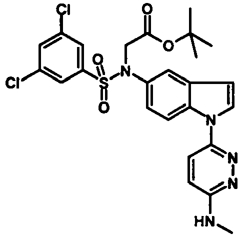 and
(97) tert.butyl {(3,5-dichloro-phenylsulphonyl)-[3-iodo-1-(6-methylaminopyridazin-3-yl)-1H-indol-5-yl]-amino}-acetate 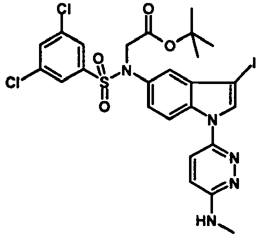 are obtained by reacting tert.butyl [(3,5-dichloro-phenylsulphonyl)-(1H-indol-5-yl)-amino]-acetate with 3-iodo-6-methylamino-pyridazine. The products are separated by preparative HPLC.
   Mass spectrum (ESI⁺): m/z = 562 [M+H]⁺ tert.butyl {(3,5-dichlorophenylsulphonyl)-[1-(6-methylamino-pyridazin-3-yl)-1H-indol-5-yl]-amino}-acetate and
   Mass spectrum (ESI⁺): m/z = 688 [M+H]⁺tert.butyl {(3,5-dichlorophenylsulphonyl)-[3-iodo-1-(6-methylamino-pyridazin-3-yl)-1H-indol-5-yl]-amino}acetate
(98) tert.butyl ((3,5-dichloro-phenylsulphonyl)-{3-iodo-1-[6-(2-oxo-imidazolidin-1-yl)-pyridazin-3-yl]-1H-indol-5-yl}-amino)-acetate 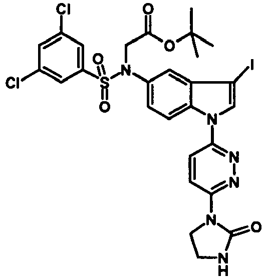 1-(6-iodo-pyridazin-3-yl)-imidazolidin-2-one is used instead of 2-iodo-benzonitrile. The reaction lasts 4 hours at 100°C. The product is purified by preparative HPLC.
   Mass spectrum (ESI⁺): m/z = 743 [M+H]⁺
(99) tert.butyl [[1-(5-cyano-pyridin-2-yl)-1H-indol-5-yl]-(3,5-dichlorophenylsulphonyl)-amino]-acetate 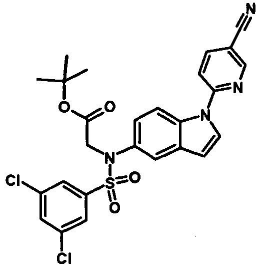 2-bromo-5-cyano-pyridine is used instead of 2-iodo-benzonitrile. Mass spectrum (ESI⁺): m/z = 574 [M+NH₄]⁺
(100) tert.butyl [[1-(4-amino-pyridin-2-yl)-1H-indol-5-yl]-(3,5-dichlorophenylsulphonyl)-amino]-acetate 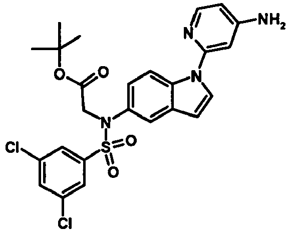 2-bromo-4-amino-pyridine is used instead of 2-iodo-benzonitrile. Mass spectrum (ESI⁺): m/z = 547 [M+H]⁺
(101) tert.butyl [[1-(6-cyano-pyridin-2-yl)-1H-indol-5-yl]-(3,5-dichlorophenylsulphonyl)-amino]-acetate 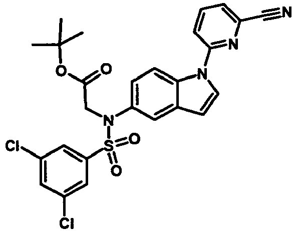 2-bromo-6-cyano-pyridine is used instead of 2-iodo-benzonitrile.
   Mass spectrum (ESI⁺): m/z = 574 [M+NH₄]⁺
(102) tert.butyl ((3,5-dichloro-phenylsulphonyl)-{1-[6-(2-dimethylamino-ethoxy)-pyridazin-3-yl]-1H-indol-5-yl}-amino)-acetate 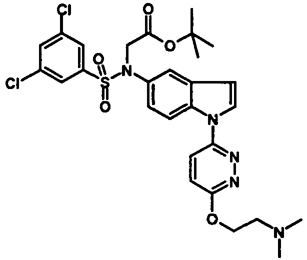 3-iodo-6-(2-dimethylamino)-ethoxy-pyridine is used instead of 2-iodo-benzonitrile.
   Mass spectrum (ESI⁺): m/z = 620 [M+H]⁺
(103) tert-butyl 4-(6-{5-[(3,5-dichloro-phenylsulphonyl)-ethoxycarbonylmethylamino]-indol-1-yl}-pyridazin-3-yl)-[1,4]diazepan-1-carboxylate 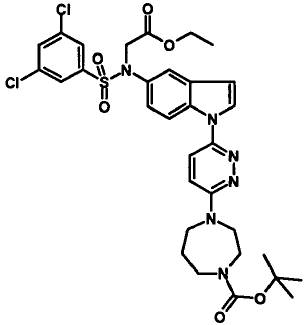 Tert-butyl 4-(6-iodo-pyridazin-3-yl)-[1,4]diazepan-1-carboxylate is used instead of 2-iodo-benzonitrile.
   Mass spectrum (ESI⁺): m/z = 703 [M+H]⁺
(104) tert.butyl {(3,5-dichloro-phenylsulphonyl)-[1-(2,4-dimethoxy-pyrimidin-5-yl)-1H-indol-5-yl]-amino}-acetate 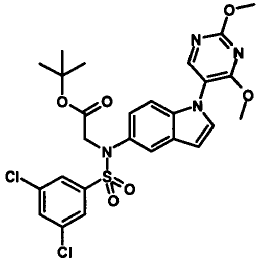 2,4-dimethoxy-5-iodo-pyrimidine is used instead of 2-iodo-benzonitrile.
   Mass spectrum (ESI⁺): m/z = 593 [M+H]⁺
(105) tert.butyl [[1-(6-{tert-butoxycarbonyl-[3-(tert-butoxycarbonyl-methyl-amino)-propyl]-amino}-pyridazin-3-yl)-1H-indol-5-yl]-(3,5-dichloro-phenylsulphonyl)-amino]-acetate 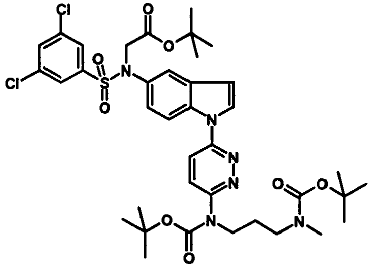 tert-butyl 3-(tert-butoxycarbonyl-methyl-amino)-propyl]-(6-iodo-pyridazin-3-yl)-carbamate is used instead of 2-iodo-benzonitrile.
   Mass spectrum (ESI⁺): m/z = 819 [M+H]⁺
(106) tert.butyl [(1-{6-[(3-(bis-tert-butoxycarbonyl)-amino-propyl)-methyl-amino]-pyridazin-3-yl}-1H-indol-5-yl)-(3,5-dichloro-phenylsulphonyl)-amino]-acetate 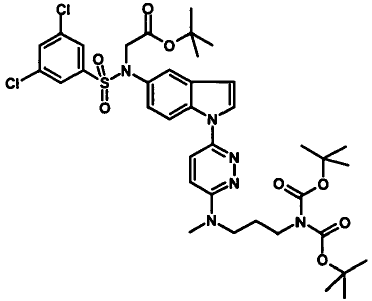 {3-[(3-(bis-tert-butoxycarbonyl)-amino-propyl)-methyl-amino]}-6-iodo-pyridazine is used instead of 2-iodo-benzonitrile. The reaction lasts 12 hours at 100°C.
   Mass spectrum (ESI⁺): m/z = 805 [M+H]⁺
(107) tert.butyl {(3,5-dichloro-phenylsulphonyl)-[1-(1,3-dimethyl-2,4-dioxo-1,2,3,4-tetrahydro-pyrimidin-5-yl)-1H-indol-5-yl]-amino}-acetate 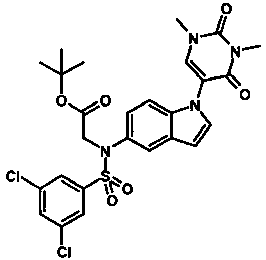 5-bromo-1,3-dimethyl-uracil is used instead of 2-iodo-benzonitrile. Mass spectrum (ESI⁺): m/z = 610 [M+NH₄]⁺
(108) tert.butyl [[1-(6-benzyloxy-pyridazin-3-yl)-1H-indol-5-yl]-(3,5-dichlorophenylsulphonyl)-amino]-acetate 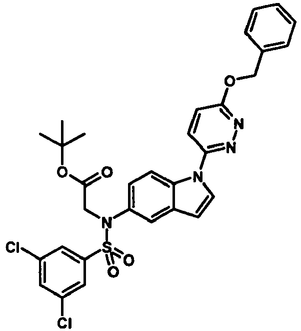 3-iodo-5-benzyloxy-pyridazine is used instead of 2-iodo-benzonitrile. Mass spectrum (ESI⁺): m/z = 639 [M+H]⁺
(109) tert.butyl ((3,5-dichloro-phenylsulphonyl)-{1-[4-(morpholine-4-carbonyl)-pyridin-2-yl]-1H-indol-5-yl}-amino)-acetate 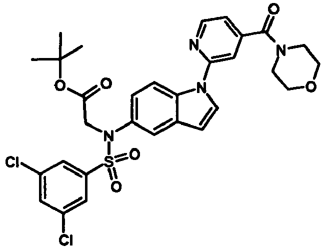 (2-Bromo-pyridin-4-yl)-morpholin-4-yl-methanone is used instead of 2-iodo-benzonitrile.
   Mass spectrum (ESI⁺): m/z = 645 [M+H]⁺
(110) tert.butyl [[1-(3-cyano-pyridin-2-yl)-1H-indol-5-yl]-(3,5-dichlorophenylsulphonyl)-amino]-acetate 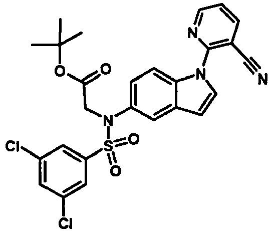 2-iodo-3-cyano-pyridine is used instead of 2-iodo-benzonitrile.
   Mass spectrum (ESI⁺): m/z = 574 [M+NH₄]⁺
(111) tert.butyl {(3,5-dichloro-phenylsulphonyl)-[4-methyl-1-(6-morpholin-4-yl-pyridazin-3-yl)-1H-indol-5-yl]-amino}-acetate 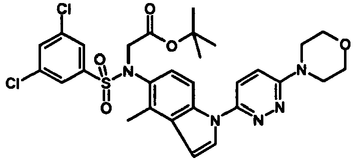 3-iodo-6-morpholin-4-yl-pyridazine is used instead of 2-iodo-benzonitrile and tert.butyl [(3,5-dichloro-phenylsulphonyl)-(4-methyl-1H-indol-5-yl)-amino]-acetate is used instead of tert.butyl [(3,5-dichloro-phenylsulphonyl)-(1H-indol-5-yl)-amino]-acetate.
   Mass spectrum (ESI⁺): m/z = 632 [M+H]⁺
(112) tert.butyl ((3,5-dichloro-phenylsulphonyl)-{1-[4-(2-hydroxy-ethylcarbamoyl)-pyridin-2-yl]-1H-indol-5-yl}-amino)-acetate 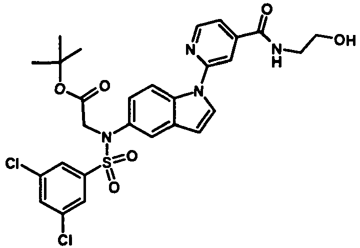 2-bromo-N-(2-hydroxy-ethyl)-isonicotinamide is used instead of 2-iodo-benzonitrile.
   Mass spectrum (ESI⁺): m/z = 619 [M+H]⁺
(113) tert.butyl [(3,5-dichloro-phenylsulphonyl)-(1-{6-[2-(tetrahydro-pyran-2-yloxy)-ethoxy]-pyridazin-3-yl}-1H-indol-5-yl)-amino]-acetate 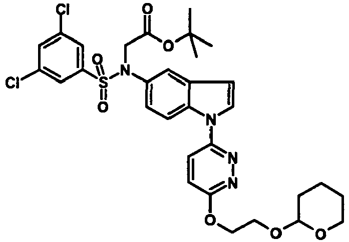 3-iodo-6-[2-(tetrahydro-pyran-2-yloxy)-ethoxy]-pyridazine is used instead of 2-iodo-benzonitrile.
   Mass spectrum (ESI⁺): m/z = 677 [M+H]⁺
(114) tert.butyl [(1-{5-[2-(tert-butyl-dimethyl-silanyloxy)-ethylamino]-pyridin-2-yl}-1H-indol-5-yl)-(3,5-dichloro-phenylsulphonyl)-amino]-acetate 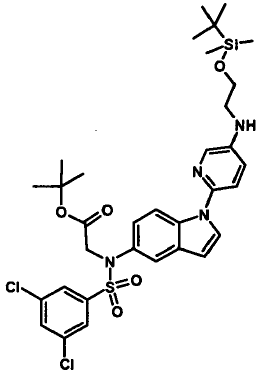 (6-bromo-pyridin-3-yl)-[2-(tert-butyl-dimethyl-silanyloxy)-ethyl]-amine is used instead of 2-iodo-benzonitrile.
   Mass spectrum (ESI⁺): m/z = 705 [M+H]⁺
(115) tert.butyl {(3,5-dichloro-phenylsulphonyl)-[1-(5-hydroxymethyl-pyridin-2-yl)-1H-indol-5-yl]-amino}-acetate 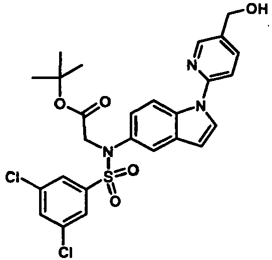 (6-bromo-pyrimidin-3-yl)-methanol is used instead of 2-iodo-benzonitrile.
   Mass spectrum (ESI⁺): m/z = 562 [M+H]⁺
(116) tert.butyl {(3,5-dichloro-phenylsulphonyl)-[1-(6-iodo-pyridazin-3-y1)-1H-indol-5-yl]-amino}-acetate 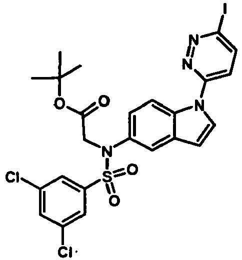 3,6-diido-pyridazine is used instead of 2-iodo-benzonitrile.
   Mass spectrum (ESI⁺): m/z = 659 [M+H]⁺
(117) tert.butyl ((3,5-dichloro-phenylsulphonyl)-{1-[6-(2-pyrrolidin-1-yl-ethylamino)-pyridazin-3-yl]-1H-indol-5-yl}-amino)-acetate 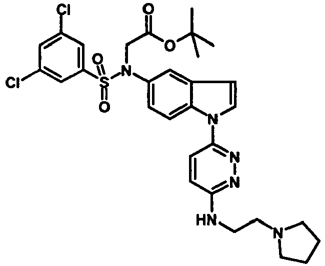 (6-iodo-pyridazin-3-yl)-(2-pyrrolidin-1-yl-ethyl)-amine is used instead of 2-iodo-benzonitrile.
   Mass spectrum (ESI⁺): m/z = 645 [M+H]⁺
(118) tert-butyl 3-(6-{5-[tert-butoxycarbonylmethyl-(3,5-dichlorophenylsulphonyl)-amino]-indol-1-yl}-pyridazin-3-yl)-2-oxo-imidazolidine-1-carboxylate 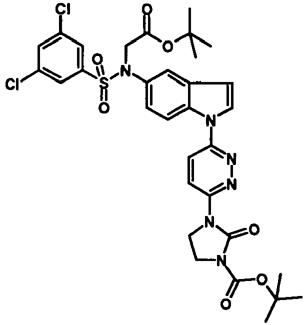 Tert-butyl 3-(6-iodo-pyridazin-3-yl)-2-oxo-imidazolidine-1-carboxylate is used instead of 2-iodo-benzonitrile.
   Mass spectrum (ESI⁺): m/z = 717 [M+H]⁺
(119) tert.butyl {(3,5-dichloro-phenylsulphonyl)-[1-(6-methylsulphanyl-pyridazin-3-yl)-1H-indol-5-yl]-amino}-acetate 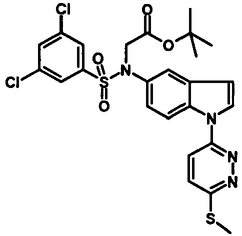 3-iodo-6-methylsulphanyl-pyridazine is used instead of 2-iodo-benzonitrile.
   Mass spectrum (ESI⁺): m/z = 579 [M+H]⁺
(120) tert.butyl ((3,5-dichloro-phenylsulphonyl)-{1-[5-(2.2-dimethyl-[1.3]dioxan-5-ylamino)-pyridin-2-yl]-1H-indol-5-yl}-amino)-acetate 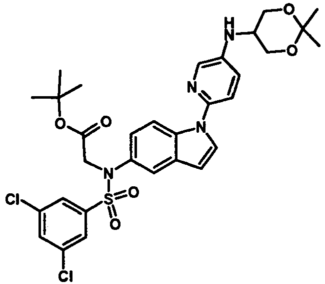 (6-bromo-pyridin-3-yl)-(2.2-dimethyl-[1.3]dioxan-5-yl)-amine is used instead of 2-iodo-benzonitrile.
   Mass spectrum (ESI⁺): m/z = 661 [M+H]⁺
(121) tert.butyl {(3,5-dichloro-phenylsulphonyl)-[1-(4-methylcarbamoyl-pyridin-2-yl)-1H-indol-5-yl]-amino}-acetate 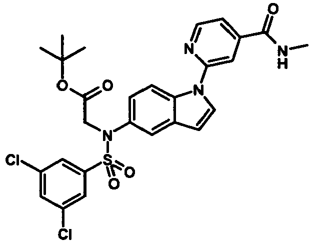 2-bromo-N-methyl-isonicotinamid is used instead of 2-iodo-benzonitrile.
   Mass spectrum (ESI⁺): m/z = 589 [M+H]⁺
(122) tert.butyl ((3,5-dichloro-phenylsulphonyl)-{1-[4-(2-methoxy-ethylcarbamoyl)-pyridin-2-yl]-1H-indol-5-yl}-amino)-acetate 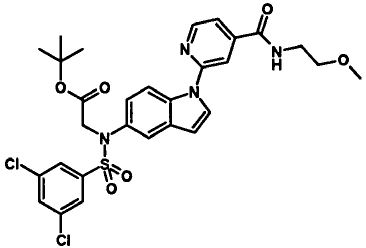 2-bromo-N-(2-methoxyethyl)-isonicotinamid is used instead of 2-iodo-benzonitrile.
   Mass spectrum (ESI⁺): m/z = 633 [M+H]⁺
(123) tert.butyl [[1-(4-cyano-pyridin-2-yl)-1H-indol-5-yl]-(3,5-dichlorophenylsulphonyl)-amino]-acetate 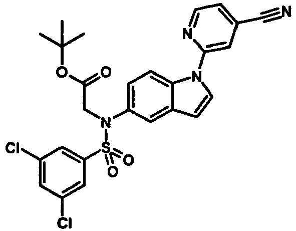 2-bromo-4-cyano-pyridine is used instead of 2-iodo-benzonitrile.
   Mass spectrum (ESI⁺): m/z = 574 [M+NH4]⁺
(124) tert.butyl {(3,5-dichloro-phenylsulphonyl)-[3-methyl-1-(6-methyl-pyridazin-3-yl)-1H-indol-5-yl]-amino}-acetate 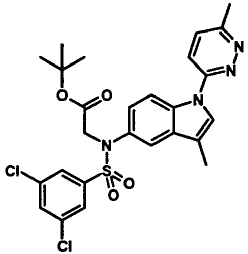 3-lodo-6-methyl-pyridazine is used instead of 2-iodo-benzonitrile.
   Mass spectrum (ESI⁺): m/z = 561 [M+H]⁺
(125) tert.butyl {(3,5-dichloro-phenylsulphonyl)-[1-(6-thiomorpholin-4-yl-pyridazin-3-yl)-1H-indol-5-yl]-amino}-acetate 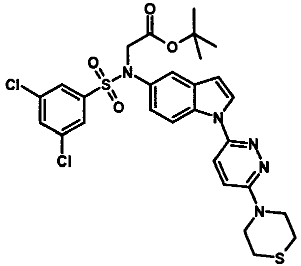 4-(6-iodo-pyridazin-3-yl)-thiomorpholine is used instead of 2-iodo-benzonitrile.
   Mass spectrum (ESI⁺): m/z = 634 [M+H]⁺
(126) tert.butyl {(3,5-dichloro-phenylsulphonyl)-[1-(6-methanesulphinyl-pyridazin-3-yl)-1H-indol-5-yl]-aminol-acetate 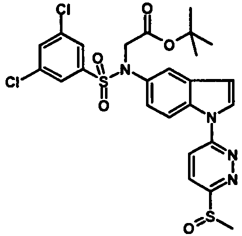 3-iodo-6-methanesulphinyl-pyridazine is used instead of 2-iodo-benzonitrile.
   Mass spectrum (ESI⁺): m/z = 595 [M+H]⁺
(127) tert.butyl [[3-chloro-1-(6-methyl-pyridazin-3-yl)-1H-indol-5-yl]-(3,5-dichlorophenylsulphonyl)-amino]-acetate 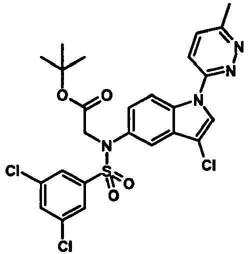 3-iodo-6-methyl-pyridazine is used instead of 2-iodo-benzonitrile.
   Mass spectrum (ESI⁺): m/z = 581 [M+H]⁺
(128) tert.butyl ((3,5-dichloro-phenylsulphonyl)-{1-[6-(2-phenyl-[1.3]dioxan-5-yloxy)-pyridazin-3-yl]-1H-indol-5-yl}-amino)-acetate 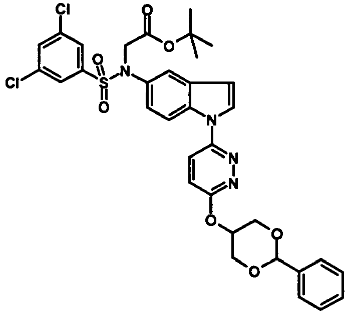 3-iodo-6-(2-phenyl-[1.3]dioxan-5-yloxy)-pyridazine is used instead of 2-iodo-benzonitrile.
   Mass spectrum (ESI⁺): m/z = 711 [M+H]⁺
(129) tert.butyl {(3,5-dichloro-phenylsulphonyl)-[1-(5-methylsulphanyl-pyrazin-2-yl)-1H-indol-5-yl]-amino}-acetate 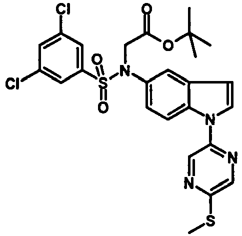 2-iodo-5-methylsulphanyl-pyrazine is used instead of 2-iodo-benzonitrile.
   Mass spectrum (ESI⁺): m/z = 596 [M+NH₄]⁺
(130) tert.butyl {(3,5-dichloro-phenylsulphonyl)-[1-(5-methylsulphanyl-pyridin-2-yl)-1H-indol-5-yl]-amino}-acetate 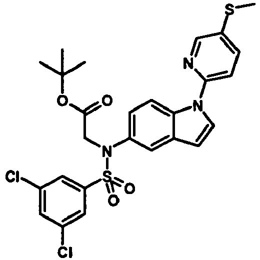 2-bromo-5-methylsulphanyl-pyridine is used instead of 2-iodo-benzonitrile.
   Mass spectrum (ESI⁺): m/z = 578 [M+H]⁺
(131) tert.butyl {(3,5-dichloro-phenylsulphonyl)-[1-(6-methanesulphonylpyridazin-3-yl)-1H-indol-5-yl]-amino}-acetate 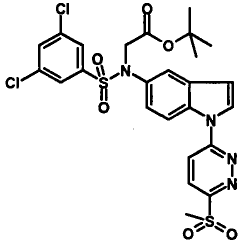 3-iodo-6-methanesulphonyl-pyridazine is used instead of 2-iodo-benzonitrile.
   Mass spectrum (ESI⁺): m/z = 628 [M+NH₄]⁺
(132) tert.butyl ((3,5-dichloro-phenylsulphonyl)-{1-[5-(2-phenyl-[1.3]dioxan-5-yloxy)-pyrazin-2-yl]-1H-indol-5-yl}-amino)-acetate 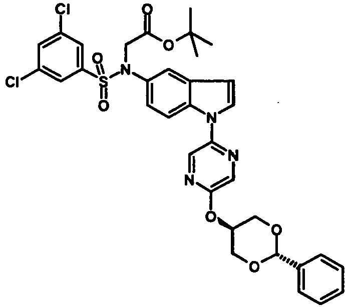 2-bromo-5-(2-phenyl-[1.3]dioxan-5-yloxy)-pyrazine is used instead of 2-iodo-benzonitrile.
   Mass spectrum (ESI⁺): m/z = 728 [M+NH₄]⁺
(133) tert.butyl [(3,5-dichloro-phenylsulphonyl)-(1-{5-[2-(tetrahydro-pyran-2-yloxy)-ethylamino]-pyrazin-2-yl}-1H-indol-5-yl)-amino]-acetate 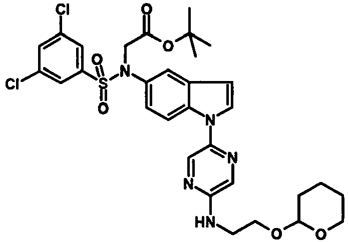 (5-bromo-pyrazin-2-yl)-[2-(tetrahydro-pyran-2-yloxy)-ethyl]-amine is used instead of 2-iodo-benzonitrile.
   Mass spectrum (ESI⁺): m/z = 676 [M+H]⁺
(134) tert.butyl ((3,5-dichloro-phenylsulphonyl)-{1-[5-(2-dimethylamino-ethoxy)-pyrazin-2-yl]-1H-indol-5-yl}-amino)-acetate 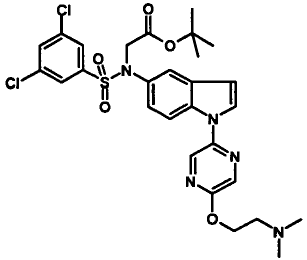 [2-(5-bromo-pyrazin-2-yloxy)-ethyl]-dimethyl-amine is used instead of 2-iodo-benzonitrile.
   Mass spectrum (ESI⁺): m/z = 620 [M+H]⁺
(135) tert.butyl {(3,5-dichloro-phenylsulphonyl)-[1-(4-methylsulphanyl-pyridin-2-yl)-1H-indol-5-yl)-amino}-acetate 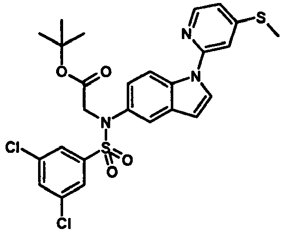 2-iodo-4-methylsulphanyl-pyridine is used instead of 2-iodo-benzonitrile.
   Mass spectrum (ESI⁺): m/z = 578 [M+H]⁺
(136) tert.butyl [{1-[5-(2-tert-butoxycarbonylamino-ethoxy)-pyrazin-2-yl]-1H-indol-5-yl}-(3,5-dichloro-phenylsulphonyl)-amino]-acetate 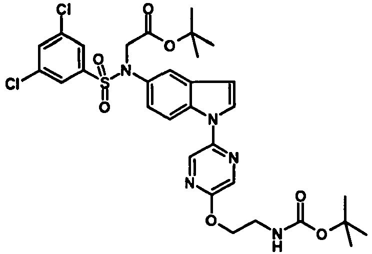 Tert-butyl [2-(5-bromo-pyrazin-2-yloxy)-ethyl]-carbamate is used instead of 2-iodo-benzonitrile.
   Mass spectrum (ESI⁺): m/z = 692 [M+H]⁺
(137) tert.butyl ((3,5-dichloro-phenylsulphonyl)-{1-[6-(1-oxo-1λ⁴-thiomorpholin-4-yl)-pyridazin-3-yl]-1H-indol-5-yl}-amino)-acetate 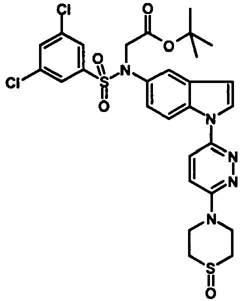 4-(6-iodo-pyridazin-3-yl)-thiomorpholine-1-oxide is used instead of 2-iodo-benzonitrile.
   Mass spectrum (ESI⁺): m/z = 650 [M+H]⁺
(138) tert.butyl {(3,5-dichloro-phenylsulphonyl)-[3-ethyl-1-(6-methyl-pyridazin-3-yl)-1H-indol-5-yl]-amino}-acetate 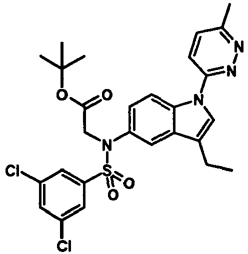 3-iodo-6-methyl-pyridazine is used instead of 2-iodo-benzonitrile.
   Mass spectrum (ESI⁺): m/z = 575 [M+H]⁺
(139) tert.butyl ((3,5-dichloro-phenylsulphonyl)-{1-[5-(1-oxo-1λ⁴-thiomorpholin-4-yl)-pyrazin-2-yl]-1H-indol-5-yl}-amino)-acetate 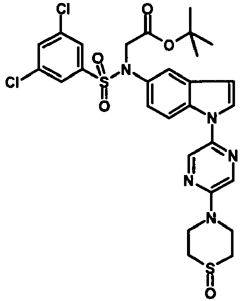 4-(5-bromo-pyrazin-2-yl)-thiomorpholin-1-oxide is used instead of 2-iodo-benzonitrile.
   Mass spectrum (ESI⁺): m/z = 650 [M+H]⁺
(140) tert.butyl ((3,5-dichloro-phenylsulphonyl)-{1-[5-(2-hydroxy-ethylamino)-pyrazin-2-yl]-1H-indol-5-yl}-amino)-acetate 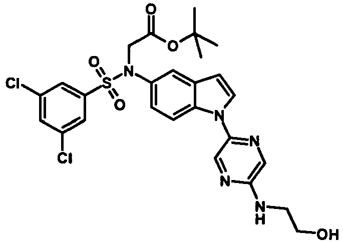 2-(5-bromo-pyrazin-2-ylamino)-ethanol is used instead of 2-iodo-benzonitrile.
   Mass spectrum (ESI⁺): m/z = 592 [M+H]⁺
(141) 2-{5-[tert-butoxycarbonylmethyl-(3,5-dichloro-phenylsulphonyl)-amino]-indol-1-yl}-isonicotinic acid 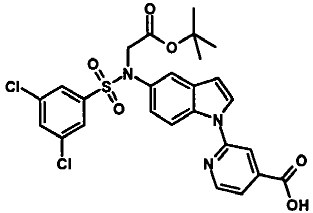 2-bromo-isonicotinic acid is used instead of 2-iodo-benzonitrile.
   R_{f} value: 0.18 (silica gel: dichloromethane/methanol 9:1)
(142) tert.butyl {(3,5-dichloro-phenylsulphonyl)-[1-(4-iodo-pyridin-2-yl)-1H-indol-5-yl]-amino}-acetate 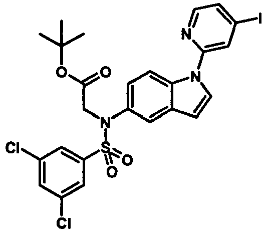 2,4-diiodo-pyridine is used instead of 2-iodo-benzonitrile.
   Mass spectrum (ESI⁺): m/z = 658 [M+H]⁺
(143) tert.butyl [{1-[5-(2-tert-butoxycarbonylamino-ethylamino)-pyrazin-2-yl]-1H-indol-5-yl}-(3,5-dichloro-phenylsulphonyl)-amino]-acetate 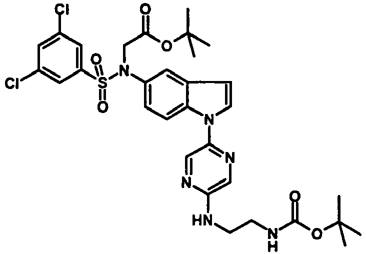 Tert-butyl [2-(5-bromo-pyrazin-2-ylamino)-ethyl]-carbamate is used instead of 2-iodo-benzonitrile.
   Mass spectrum (ESI⁺): m/z = 691 [M+H]⁺
(144) tert.butyl [[1-(4-cyclopropylcarbamoyl-pyridin-2-yl)-1H-indol-5-yl]-(3,5-dichloro-phenylsulphonyl)-amino]-acetate 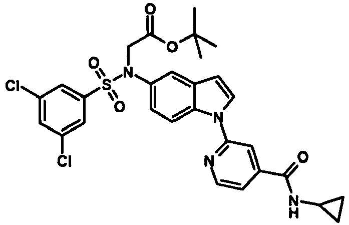 [2-Bromo-N-cyclopropyl-isonicotinamide is used instead of 2-iodo-benzonitrile.
   R_{f} value: 0.39 (aluminium oxide: petroleum ether/ethyl acetate 6:4)
(145) methyl 2-{5-[tert-butoxycarbonylmethyl-(3,5-dichloro-phenylsulphonyl)-amino]-indol-1-yl}-isonicotinate 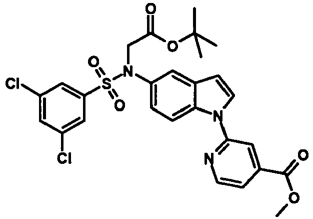 Methyl 2-bromo-isonicotinate is used instead of 2-iodo-benzonitrile.
   Mass spectrum (ESI⁺): m/z = 590 [M+H]⁺
(146) tert.butyl ((3,5-dichloro-phenylsulphonyl)-{1-[4-(methoxy-methylcarbamoyl)-pyridin-2-yl]-1H-indol-5-yl}-amino)-acetate 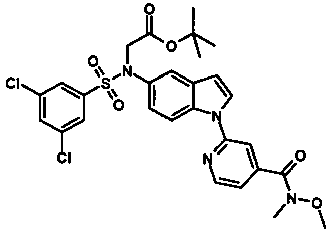 2-bromo-N-methoxy-N-methyl-isonicotinamide is used instead of 2-iodo-benzonitrile.
   Mass spectrum (ESI⁺): m/z = 619 [M+H]⁺
(147) tert-butyl 5'-{5-[tert-butoxycarbonylmethyl-(3,5-dichloro-phenylsulphonyl)-amino]-indol-1-yl}-2,3,5,6-tetrahydro-[1,2']bipyrazinyl-4-carboxylate 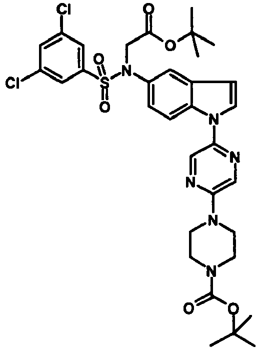 Tert-butyl 5'-bromo-2,3,5,6-tetrahydro-[1,2']bipyrazinyl-4-carboxylate is used instead of 2-iodo-benzonitrile.
   Mass spectrum (ESI⁺): m/z = 717 [M+H]⁺
(148) tert.butyl {(3,5-dichloro-phenylsulphonyl)-[1-(4-ethansulphinyl-pyridin-2-yl)-1H-indol-5-yl]-amino}-acetate 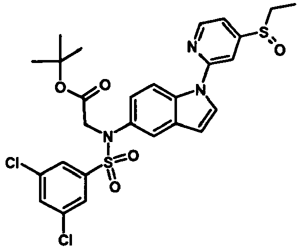 2-bromo-4-ethansulphinyl-pyridine is used instead of 2-iodo-benzonitrile.
   Mass spectrum (ESI⁺): m/z = 608 [M+H]⁺
(149) tert.butyl [(3,5-dichloro-phenylsulphonyl)-(1-imidazo[1,2-a]pyrazin-6-yl-1H-indol-5-yl)-amino]-acetate 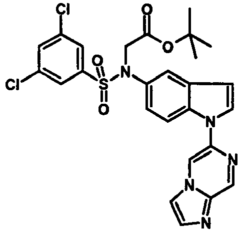 6-bromo-imidazo[1,2-a]pyrazine is used instead of 2-iodo-benzonitrile.
   Mass spectrum (ESI⁺): m/z = 572 [M+H]⁺
(150) tert.butyl {(3,5-dichloro-phenylsulphonyl)-[1-(4-oxazol-2-yl-pyridin-2-yl)-1H-indol-5-yl]-amino}-acetate 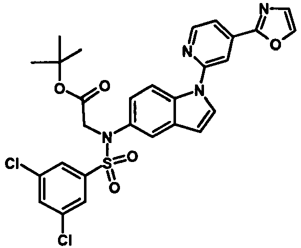 2-bromo-4-oxazol-2-yl-pyridine is used instead of 2-iodo-benzonitrile.
   Mass spectrum (ESI⁺): m/z = 599 [M+H]⁺
(151) tert.butyl [[1-(4-acetyl-pyridin-2-yl)-1H-indol-5-yl]-(3,5-dichlorophenylsulphonyl)-amino]-acetate 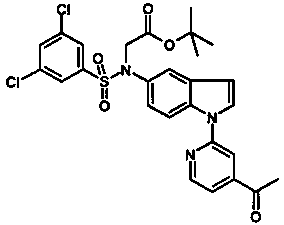 1-(2-bromo-pyridin-4-yl)-ethanone is used instead of 2-iodo-benzonitrile.
   Mass spectrum (ESI⁺): m/z = 574 [M+H]⁺
(152) tert.butyl {(3,5-dichloro-phenylsulphonyl)-[1-(4-propionyl-pyridin-2-yl)-1H-indol-5-yl]-amino}-acetate 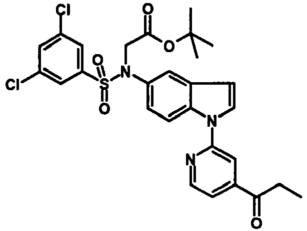 1-(2-bromo-pyridin-4-yl)-propanone is used instead of 2-iodo-benzonitrile.
   The crude product is further reacted directly (1 (188)).
(153) tert.butyl [[1-(4-cyclopropylcarbamoyl-pyridin-2-yl)-3-methyl-1H-indol-5-yl]-(3,5-dichloro-phenylsulphonyl)-amino]-acetate 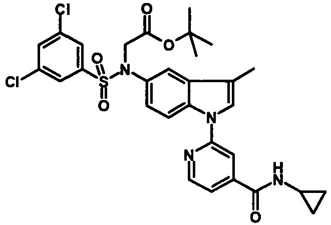 2-bromo-N-cyclopropyl-isonicotinamide is used instead of 2-iodo-benzonitrile.
   Mass spectrum (ESI⁺): m/z = 629 [M+H]⁺
(154) tert.butyl [{1-[4-(tert-butoxycarbonylamino-methyl)-pyridin-2-yl]-1H-indol-5-yl}-(3,5-dichloro-phenylsulphonyl)-amino]-acetate 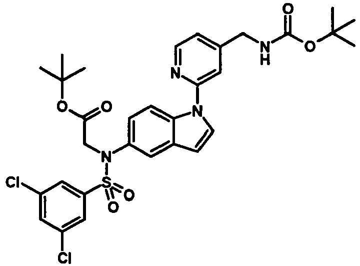 Tert-butyl (2-bromo-pyridin-4-ylmethyl)-carbamate is used instead of 2-iodo-benzonitrile.
   Mass spectrum (ESI⁺): m/z = 661 [M+H]⁺
(155) tert.butyl [(1-{5-[tert-butoxycarbonyl-(2-dimethylamino-ethyl)-amino]-pyrazin-2-yl}-3-methyl-1H-indol-5-yl)-(3,5-dichloro-phenylsulphonyl)-amino]-acetate 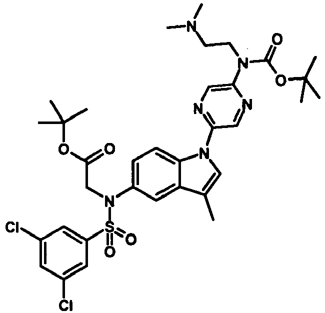 Tert. butyl (5-bromo-pyrazin-2-yl)-(2-dimethylamino-ethyl)-carbamate
   is used instead of 2-iodo-benzonitrile.
   Mass spectrum (ESI⁺): m/z = 733 [M+H]⁺
(156) tert.butyl {(3,5-dichloro-phenylsulphonyl)-[1-(5-morpholin-4-yl-pyrazin-2-yl)-1H-indol-5-yl]-amino}-acetate 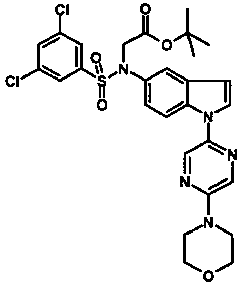 4-(5-bromo-pyrazin-2-yl)-morpholine is used instead of 2-iodo-benzonitrile.
   Mass spectrum (ESI⁺): m/z = 618 [M+H]⁺
(157) tert.butyl {(3,5-dichloro-phenylsulphonyl)-[1-(1-methyl-1H-pyrazol-4-yl)-l H-indol-5-yl]-amino}-acetate 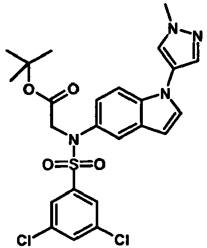 Mass spectrum (ESI⁺): m/z = 535 [M+H]⁺
(158) tert.butyl {(3,5-dichloro-phenylsulphonyl)-[1-(5-thiazol-2-yl-pyridazin-3-yl)-1H-indol-5-yl]-amino}-acetate 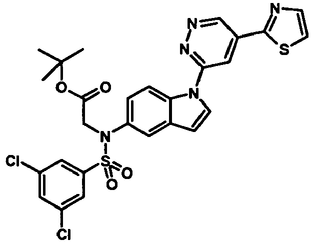 Mass spectrum (ESI⁺): m/z = 616 [M⁺H]⁺
   R_{f} value: 0.38 (silica gel petroleum ether/ethyl acetate 1:1)
(159) tert.butyl {(3,5-dichloro-phenylsulphonyl)-[1-(1-methyl-1H-imidazol-4-yl)-1H-indol-5-yl]-amino}-acetate 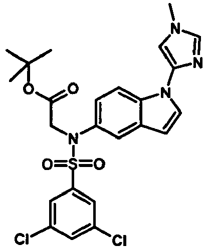 Mass spectrum (ESI⁺): m/z = 535 [M+H]⁺
   R_{f} value: 0.15 (silica gel: cyclohexane/ethyl acetate 1:1)
(160) tert.butyl [(3,5-dichloro-phenylsulphonyl)-(1-thiazol-2-yl-1H-indol-5-yl)-amino]-acetate 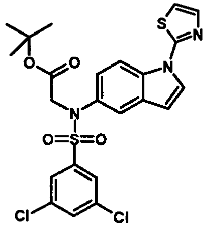 Mass spectrum (ESI⁺): m/z = 538 [M+H]⁺
   R_{f} value: 0.75 (silica gel: cyclohexane/ethyl acetate 1:1)
(161) tert.butyl [[1-(2-cyano-thiophen-3-yl)-1H-indol-5-yl]-(3,5-dichlorophenylsulphonyl)-amino]-acetate 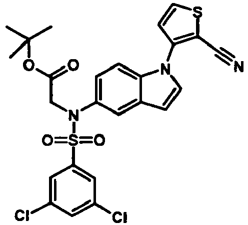 Mass spectrum (ESI⁺): m/z = 579 [M+NH₄]⁺
   R_{f} value: 0.75 (silica gel: dichloromethane/methanol 99:1)
(162) tert.butyl [[1-(5-acetyl-thiophen-2-yl)-1H-indol-5-yl]-(3,5-dichlorophenylsulphonyl)-amino]-acetate 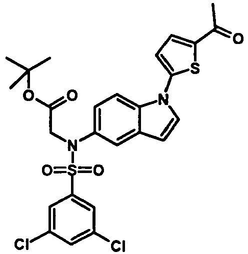 Mass spectrum (ESI⁺): m/z = 579 [M+H]⁺
   R_{f} value: 0.35 (silica gel: petroleum ether/ethyl acetate 7:3)
(163) tert.butyl {(3,5-dichloro-phenylsulphonyl)-[1-(2,6-dimethyl-pyrimidin-4-yl)-1H-indol-5-yl]-amino}-acetate 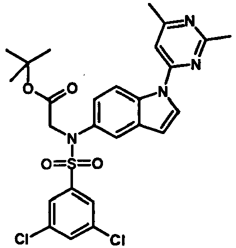 Mass spectrum (ESI⁺): m/z = 561 [M+H]⁺
   R_{f} value: 0.35 (silica gel: cyclohexane/ethyl acetate 1:1)
(164) tert.butyl {(3,5-dichloro-phenylsulphonyl)-[1-(5-methyl-[1.3.4]thiadiazol-2-yl)-1H-indol-5-yl]-amino}-acetate 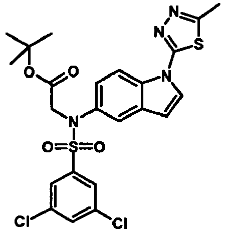 Mass spectrum (ESI⁺): m/z = 553 [M+H]⁺
   R_{f} value: 0.50 (silica gel: cyclohexane/ethyl acetate 1:1)
(165) tert.butyl {(3,5-dichloro-phenylsulphonyl)-[1-(6-methoxy-pyrimidin-4-yl)-1H-indol-5-yl]-amino}-acetate 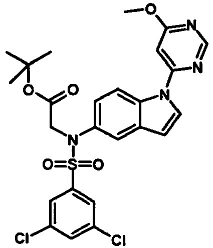 Mass spectrum (ESI⁺): m/z = 563 [M+H]⁺
   R_{f} value: 0.73 (silica gel: cyclohexane/ethyl acetate 1:1)
(166) ethyl 2-(5-{5-[tert-butoxycarbonylmethyl-(3,5-dichloro-phenylsulphonyl)-amino]-indol-1-yl}-thiophen-2-yl)-thiazole-4-carboxylate 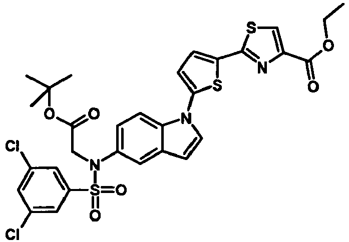 Mass spectrum (ESI⁺): m/z = 692 [M+H]⁺
   R_{f} value: 0.13 (silica gel: petroleum ether/ethyl acetate 5:1)
(167)tert.butyl {(3,S-dichloro-phenylsulphonyl)-[1-(6-iodo-pyrimidin-4-yl)-1H-indol-5-yl]-amino}-acetate 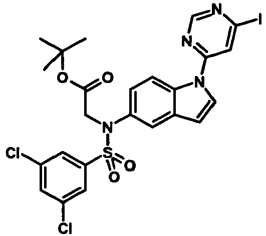 Mass spectrum (ESI⁺): m/z = 676 [M+NH₄]⁺
   R_{f} value: 0.34 (silica gel: petroleum ether/ethyl acetate 5:1)
(168) ethyl 5-(5-{5-[tert-butoxycarbonylmethyl-(3,5-dichloro-phenylsulphonyl)-amino]-indol-1-yl}-thiophen-2-yl)-isoxazole-3-carboxylate 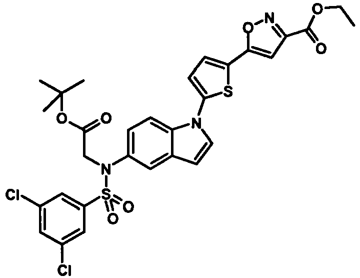 Mass spectrum (ESI⁺): m/z = 693 [M+NH₄]⁺
   R_{f} value: 0.58 (silica gel: petroleum ether/ethyl acetate 2:1)
(169) tert.butyl {(3,5-dichloro-phenylsulphonyl)-[3-methyl-1-(4-methyl-pyridin-2-yl)-1H-indol-5-yl]-amino}-acetate 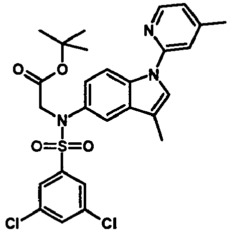 Mass spectrum (ESI⁺): m/z = 560 [M+H]⁺
   R_{f} value: 0.53 (silica gel: petroleum ether/ethyl acetate 3:1)
(170) tert.butyl [{3-bromo-1-[6-(2-dimethylamino-ethylamino)-pyridazin-3-y)]-1H-indol-5-yl}-(3,5-dichloro-phenylsulphonyl)-amino]-acetate 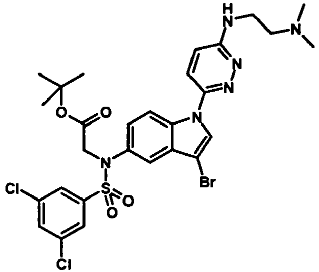 R_{f} value: 0.30 (silica gel: dichloromethane/methanol/water/acetic acid 40:8:1:1)
(171) tert.butyl {(2,6-dimethyl-pyridin-4-sulphonyl)-[1-(6-methyl-pyridazin-3-yl)-1H-indol-5-yl]-amino}-acetate 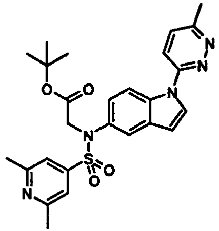 Mass spectrum (ESI⁺): m/z = 508 [M+H]⁺
(172) tert.butyl {(2,6-dichloro-pyridin-4-sulphonyl)-[1-(6-methyl-pyridazin-3-yl)-1H-indol-5-yl]-amino}-acetate 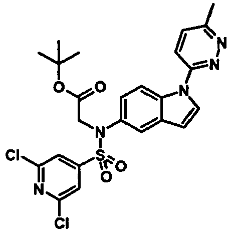 The product is further reacted directly in 1(181).
(173) tert.butyl {(2,6-dichloro-pyridin-4-sulphonyl)-[1-(4-methylcarbamoyl-pyridin-2-yl)-1H-indol-5-yl]-amino}-acetate 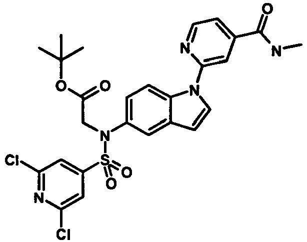 Mass spectrum (ESI⁺): m/z = 590 [M+H]⁺

### Example VII

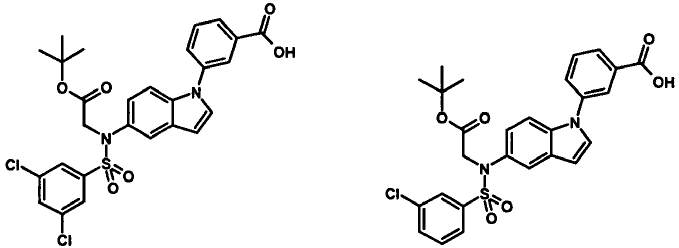

### 3-{5-[tert-butoxycarbonylmethyl-(3,5-dichloro-phenylsulphonil)-amino]-indol-1-yl}-benzoic acid and 3-{5-[tert-butoxycarbonylmethyl-(3-chloro-phenylsulphonyl)-amino]-indol-1-yl}-benzoic acid

120 mg benzyl 3-{5-[tert-butoxycarbonylmethyl-(3,5-dichloro-phenylsulphonyl)-amino]-indol-1-yl}-benzoate are dissolved in 5 ml of tetrahydrofuran and 4 ml of methanol. 10 mg palladium on charcoal (10%) are added and the mixture is hydrogenated for 2 hours at ambient temperature and 1 bar pressure. Then the catalyst is filtered off and the remainder is washed twice with 10 ml of methanol. The solvents are eliminated in vacuo. The crude product is a mixture of 3-{5-[tert-butoxycarbonylmethyl-(3,5-dichloro-phenylsulphonyl)-amino]-indol-1-yl}-benzoic acid and 3-{5-[tert-butoxycarbonylmethyl-(3-chloro-phenylsulphonyl)-amino]-indol-1-yl}-benzoic acid and is further reacted directly in Example XVIII.

The following compounds are obtained analogously to Example VII:
(1) tert.butyl {(3,5-dichloro-phenylsulphonyl)-[1-(6-oxo-1.6-dihydro-pyridazin-3-yl)-1H-indol-5-yl]-amino}-acetate 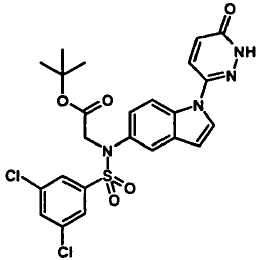 Tert.butyl [[1-(6-benzyloxy-pyridazin-3-yl)-1H-indol-5-yl]-(3,5-dichlorophenylsulphonyl)-amino]-acetate is used instead of benzyl 3-{5-[tert-butoxycarbonylmethyl-(3,5-dichloro-phenylsulphonyl)-amino]-indol-1-yl}-benzoate. The reaction is carried out in methanol. The crude product is chromatographed on silica gel (cyclohexane/ethyl acetate 50:50 to 0:100).
   Mass spectrum (ESI⁻): m/z = 547 [M-H]⁻
(2) tert.butyl ((3,5-dichloro-phenylsulphonyl)-{1-[6-(3-hydroxy-propyl)-pyridazin-3-yl]-1H-indol-5-yl}-amino)-acetate 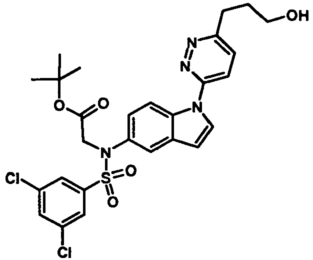 Obtained by hydrogenation of tert.butyl ((3,5-dichloro-phenylsulphonyl)-{1-[6-(3-hydroxy-prop-1-ynyl)-pyridazin-3-yl]-1H-indol-5-yl}-amino)-acetate in tetrahydrofuran. The crude product is chromatographed on silica gel (ethyl acetate).
   Mass spectrum (ESI⁺): m/z = 591 [M+H]⁺
(3) tert.butyl ((3,5-dichloro-phenylsulphonyl)-{1-[4-(3-hydroxy-propyl)-pyridin-2-yl]-1H-indol-5-yl}-amino)-acetate 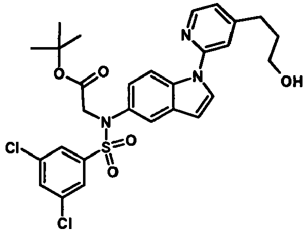 Obtained by hydrogenation of tert.butyl ((3,5-dichloro-phenylsulphonyl)-{1-[4-(3-hydroxy-prop-1-ynyl)-pyridin-2-yl]-1H-indol-5-yl}-amino)-acetate in tetrahydrofuran. The crude product is chromatographed on silica gel (ethyl acetate).
   Mass spectrum (ESI⁺): m/z = 590 [M+H]⁺

### Example VIII

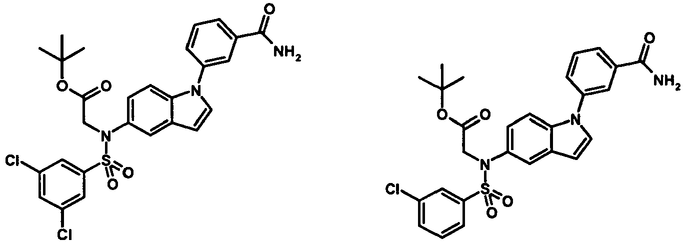

### tert.butyl [[1-(3-carbamoyl-phenyl)-1H-indol-5-yl]-(3,5-dichlorophenylesulphonyl)-amino]-acetate and tert.butyl [1-(3-carbamoyl-phenyl)-1H-indol-5-yl]-(3-chloro-phenylsulphonyl)-amino]-acetate

52 mg of a mixture of 3-{5-[tert-butoxycarbonylmethyl-(3,5-dichloro-phenylsulphonyl)-amino]-indol-1-yl}-benzoic acid and 3-{5-[tert-butoxycarbonylmethyl-(3-chloro-phenylsulphonyl)-amino]-indol-1-yl}-benzoic acid from Example VII are dissolved in 8 ml of tetrahydrofuran. 16 mg carbonyldiimidazole are added and the mixture is heated to 60°C for 2 hours. Ammonia gas is piped into this solution first of all at ambient temperature and then at 50°C over a total of 2 hours. Then 100 ml of water are added, the tetrahydrofuran is largely eliminated in vacuo and then the remainder is adjusted to pH 2with 2 N HCl. The mixture is extracted 3 times with ethyl acetate, dried with sodium sulphate and the solvents are eliminated in vacuo. The residue is purified by preparative HPLC and further reacted directly.
Yield: 13 mg (25 % of theory) (tert.butyl [[1-(3-carbamoyl-phenyl)-1H-indol-5-yl]-(3,5-dichloro-phenylsulphonyl)-amino]-acetate)
Mass spectrum (ESI⁺): m/z = 518 [M+H-tert-butyl]⁺
and
9 mg (19 % of theory) (tert.butyl [[1-(3-carbamoyl-phenyl)-1H-indol-5-yl]-(3-chloro-phenylsulphonyl)-amino]-acetate)
Mass spectrum (ESI⁺): m/z = 484 [M+H-tert-butyl]⁺

The following compounds are obtained analogously to Example VIII:
(1) tert.butyl {(3,5-dichloro-phenylsulphonyl)-[1-(3-methylcarbamoyl-phenyl)-1H-indol-5-yl]-amino}-acetate 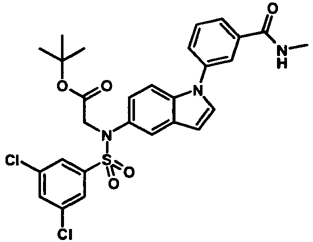 and tert.butyl {(3-chloro-phenylsulphonyl)-[1-(3-methylcarbamoyl-phenyl)-1H-indol-5-yl]-amino}-acetate 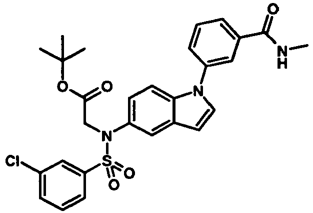 Instead of ammonia gas methylamine gas is used.
   Mass spectrum (ESI⁺): m/z = 532 [M+H-tert-butyl]⁺ (tert.butyl {(3,5-dichlorophenylsulphonyl)-[1-(3-methylcarbamoyl-phenyl)-1H-indol-5-yl]-amino}-acetate) and
   Mass spectrum (ESI⁺): m/z = 498 [M+H-tert-butyl]⁺ (tert.butyl {(3-chlorophenylsulphonyl)-[1-(3-methylcarbamoyl-phenyl)-1H-indol-5-yl]-amino}-acetate)
(2) 3.6-dichloro-pyridazin-4-carboxylic acid-amide 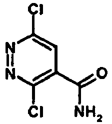 After elimination of the tetrahydrofuran in vacuo the mixture is adjusted to pH 1 with 2 N HCl. It is extracted twice with ethyl acetate and the combined organic phases are washed with saturated sodium chloride solution. After drying with magnesium sulphate the solvent is eliminated in vacuo.
   Mass spectrum (ESI⁻): m/z = 190 [M-H]⁻

### Example IX

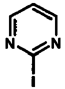

### 2-iodopyrimidine

500 mg of 2-bromopyrimidine are dissolved in 5 ml dioxane under argon. 943 mg sodium iodide, 60 mg copper iodide and 45 mg N,N'-dimethyl-trans-cyclohexanediamine are added. Then the mixture is refluxed for 8 hours. It is divided between water and ethyl acetate, the aqueous phase is extracted with ethyl acetate and the combined organic phases are dried on sodium sulphate. The solvents are eliminated in vacuo and the residue is chromatographed on silica gel (cyclohexane/ethyl acetate 10:1 to 1:2). The product (235 mg, 36 % of theory) is further reacted directly in Example XVI (1).

### Example X

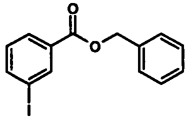

### benzyl 3-iodo-benzoate

1.5 g 3-iodo-benzoic acid are dissolved in 10 ml acetonitrile. 919 mg potassium carbonate are added and then 647 µl benzylbromide are added dropwise thereto. Then the mixture is heated to 50°C for 4 hours. The solvent is eliminated in vacuo and the residue is divided between ethyl acetate and saturated sodium hydrogen carbonate solution. The aqueous phase is extracted with ethyl acetate and the combined organic phases are dried on sodium sulphate. The solvents are eliminated in vacuo.
Yield: 2 g (98% of theory)
Mass spectrum (ESI⁺): m/z = 356 [M+NH₄]⁺

### Example XI

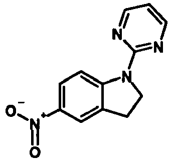

### 5-nitro-1-pyrimidin-2-yl-2,3-dihydro-1H-indole

300 mg 5-nitro-2,3-dihydro-1H-indole are dissolved in 4 ml N-methyl-pyrrolidine. 80 mg NaH (60 % suspension in mineral oil) are added and the mixture is stirred for another 15 minutes at ambient temperature. Then 380 mg of 2-bromopyrimidine are added and the mixture is then heated to 60°C for 3 hours. It is then diluted with diethyl ether and washed with dilute citric acid solution and saturated sodium chloride solution. After drying with magnesium sulphate the solvents are eliminated in vacuo.
Yield: 410 mg (95 % of theory)
Rf value: 0.61 (silica gel: ethyl acetate/petroleum ether 1:1)

### Example XII

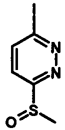

### 3-iodo-6-methanesulphinyl-pyridazine

400 mg 3-iodo-6-methylsulphanyl-pyridazine are dissolved in 5 ml dichloromethane and 310 mg 3-chloro-perbenzoic acid are added. The mixture is stirred for 1 hour at ambient temperature, diluted with ethyl acetate and washed with dilute sodium hydroxide solution. After drying with magnesium sulphate the solvents are eliminated in vacuo and the residue is chromatographed on silica gel (cyclohexane/ethyl acetate 50:50 to 0:100).
Yield: 140 mg (52 % of theory)
Mass spectrum (ESI⁺): m/z = 269 [M+H]⁺

The following compounds are obtained analogously to Example XII:
(1) tert.butyl ((3,5-dichloro-phenylsulphonyl)-{1-[6-(1,1-dioxo-1λ⁶-thiomorpholin-4-yl)-pyridazin-3-yl]-1H-indol-5-yl}-amino)-acetate 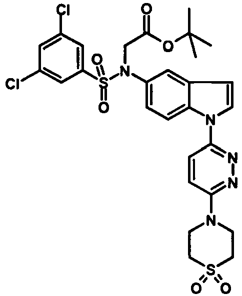 Obtained from the oxidation of tert.butyl {(3,5-dichloro-phenylsulphonyl)-[1-(6-thiomorpholin-4-yl-pyridazin-3-yl)-1H-indol-5-yl]-amino}-acetate.
   Mass spectrum (ESI⁺): m/z = 666 [M+H]⁺
(2) tert.butyl {(3,5-dichloro-phenylsulphonyl)-[1-(5-methanesulphinyl-pyrazin-2-yl)-1H-indol-5-yl]-amino}-acetate 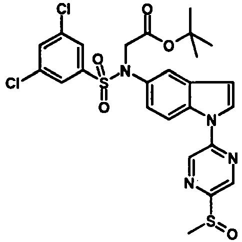 and
   tert.butyl {(3,5-dichloro-phenylsulphonyl)-[1-(5-methanesulphonyl-pyrazin-2-yl)-1H-indol-5-yl]-amino}-acetate 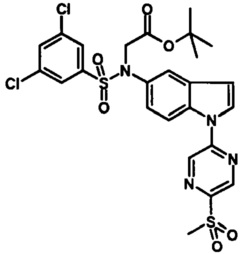 The products are separated by chromatography on silica gel (cyclohexane/ethyl acetate 60:40 to 0:100).
   Mass spectrum (ESI⁺): m/z = 595 [M+H]⁺ tert.butyl {(3,5-dichlorophenylsulphonyl)-[1-(5-methanesulphinyl-pyrazin-2-yl)-1H-indol-5-yl]-amino}-acetate and
   Mass spectrum (ESI⁺): m/z = 628 [M+NH₄]⁺ tert.butyl {(3,5-dichlorophenylsulphonyl)-[1-(5-methanesulphonyl-pyrazin-2-yl)-1H-indol-5-yl]-amino}-acetate
(3) 3-iodo-6-methanesulphonyl-pyridazine 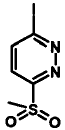 Obtained from the oxidation of 3-iodo-6-methanesulphinyl-pyridazine.
   Mass spectrum (ESI⁺): m/z = 285 [M+H]⁺
(4) tert.butyl {(3,5-dichloro-phenylsulphonyl)-[1-(5-methanesulphinyl-pyridin-2-yl)-1H-indol-5-yl]-amino}-acetate 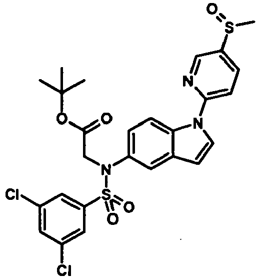 Obtained from the oxidation of tert.butyl {(3,5-dichloro-phenylsulphonyl)-[1-(5-methylsulphanyl-pyridin-2-yl)-1H-indol-5-yl]-amino}-acetate with 1 equivalent of metachloro-perbenzoic acid.
   Mass spectrum (ESI⁺): m/z = 594 [M+H]⁺
(5) tert.butyl {(3,5-dichloro-phenylsulphonyl)-[1-(5-methanesulphonyl-pyridin-2-yl)-1H-indol-5-yl]-amino}-acetate 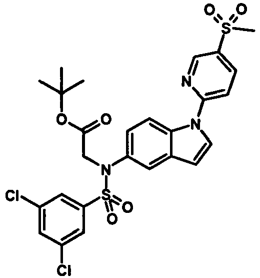 Obtained from the oxidation of tert.butyl {(3,5-dichloro-phenylsulphonyl)-[1-(5-methylsulphanyl-pyridin-2-yl)-1H-indol-5-yl]-amino}-acetate with 2.1 equivalents of metachloro-perbenzoic acid.
   Mass spectrum (ESI⁺): m/z = 610 [M+H]⁺
(6) 4-(6-iodo-pyridazin-3-yl)-thiomorpholin-1-oxide 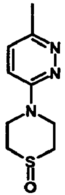 Obtained from the oxidation of 4-(6-iodo-pyridazin-3-yl)-thiomorpholine in admixture with the sulphone.
   Mass spectrum (ESI⁺): m/z = 324 [M+H]⁺
(7) 4-(5-bromo-pyrazin-2-yl)-thiomorpholin-1-oxide 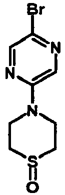 Obtained from the oxidation of 4-(5-bromo-pyrazin-2-yl)-thiomorpholine.
   Mass spectrum (ESI⁺): m/z = 276 [M+H]⁺
(8) 2-bromo-4-ethanesulphinyl-pyridine 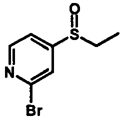 Obtained from the oxidation of 2-bromo-4-ethylsulphanyl-pyridine.
   Mass spectrum (ESI⁺): m/z = 234 [M+H]⁺

### Example XIII

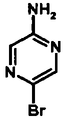

### 5-bromo-pyrazin-2-ylamine

24.5 g of 2-aminopyrazine are dissolved in 750 ml dichloromethane and cooled to 0°C. 45.8 g N-bromosuccinimide are added and the mixture is stirred for 24 hours at 0°C. The solid is suction filtered and the mother liquor is washed 3 times with semisaturated sodium carbonate solution and 3 times with water. The organic phase is dried on magnesium sulphate, filtered through activated charcoal and evaporated down in vacuo. The residue is extracted from diisopropyl ether. Further product is obtained by evaporating the mother liquor down in vacuo and chromatographing the residue on silica gel (petroleum ether/ethyl acetate 1:1 to 1:2).
Yield: 20.8 g (47 % of theory)
R_{f} value: 0.5 (silica gel, petroleum ether/ethyl acetate 1:1)

### Example XIV

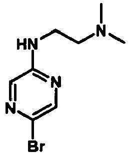

### N'-(5-bromo-pyrazin-2-yl)-N,N-dimethyl-ethan-1,2-diamine

24 g 5-bromo-pyrazin-2-ylamine are dissolved in 300 ml dimethylformamide. 20 g of 2-chloroethyl-dimethylamine hydrochloride are added and the mixture is cooled to 0°C. Then 13.8 g NaH (60 % suspension in mineral oil) are added in batches. After the end of the addition the mixture is stirred for 10 minutes at 0°C and then heated for 2 hours to 80°C. Then it is divided between semisaturated sodium chloride solution and ethyl acetate, the aqueous phase is extracted twice with ethyl acetate and the combined organic phases are dried on magnesium sulphate. The solvents are eliminated in vacuo and the residue is chromatographed on silica gel (dichloromethane/methanol 9:1 to 1:1). The product thus obtained is taken up in ethyl acetate and combined with activated charcoal. It is stirred for 1 hour and then the activated charcoal is filtered off. The mother liquor is evaporated down in vacuo and petroleum ether is added to the residue. This is incubated for 1 hour in the deep-freeze, the petroleum ether is decanted off and the solid is dried under a high vacuum.
Yield: 11 g (33 % of theory)
Mass spectrum (ESI⁺): m/z = 245 [M+H]⁺

The following compounds are obtained analogously to Example XXIV:
(1) N'-(2-bromo-pyridin-3-yl)-N,N-dimethyl-ethan-1,2-diamine 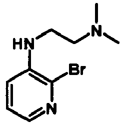 Potassium-hexamethyldisilazide is used as base. The reaction is heated to 70°C for 6 hours.
   Mass spectrum (ESI⁺): m/z = 244 [M+H]⁺

### Example XV

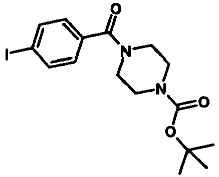

### tert-butyl 4-(4-iodo-benzoyl)-piperazine-1-carboxylate

1 g 4-iodo-benzoic acid is dissolved in 10 ml of tetrahydrofuran, 726 mg carbonyldiimidazole are added and the mixture is stirred for 3 hours at ambient temperature. To this are added 820 mg tert-butyl piperazine-1-carboxylate and the mixture is stirred overnight at ambient temperature. The solvent is eliminated in vacuo and the residue is taken up in ethyl acetate. It is washed with water, 1 N HCl, saturated sodium hydrogen carbonate solution and saturated sodium chloride solution. After drying with magnesium sulphate the solvent is eliminated in vacuo.
Yield: 1.65 g (98 % of theory)
Mass spectrum (ESI⁺): m/z = 417 [M+H]⁺

The following compounds are obtained analogously to Example XV:
(1) tert-butyl 4-(3-iodo-benzoyl)-piperazine-1-carboxylate 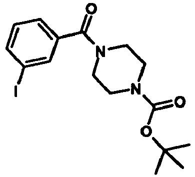 Mass spectrum (ESI⁺): m/z = 417 [M+H]⁺
(2) 6-bromo-pyridin-2-carboxylic acid-(2-dimethylamino-ethyl)-amide 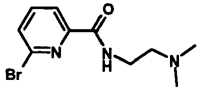 After stirring overnight in tetrahydrofuran the mixture is diluted with toluene and heated to 90°C for 5 hours. Then it is evaporated to dryness, the residue is divided between water and diethyl ether and the aqueous phase is extracted twice with diethyl ether. The combined organic phases are washed with saturated sodium chloride solution and dried on magnesium sulphate. The solvent is eliminated in vacuo and the residue is chromatographed on aluminium oxide with ethyl acetate.
   Mass spectrum (ESI⁺): m/z = 272 [M+H]⁺
(3) tert-butyl 4-(6-bromo-pyridin-3-carbonyl)-piperazine-1-carboxylate 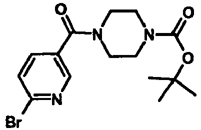 After stirring overnight in tetrahydrofuran the mixture is diluted with toluene and heated to 90°C for 5 hours. Then it is evaporated to dryness, the residue is triturated with water and the solid is suction filtered. Then it is taken up in ethyl acetate, dried on magnesium sulphate and the solvent is eliminated in vacuo. The residue is extracted from diethyl ether.
   Mass spectrum (ESI⁺): m/z = 370 [M+H]⁺
(4) 6-bromo-N-(2-dimethylamino-ethyl)-nicotinamide 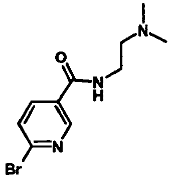 After stirring overnight in tetrahydrofuran the mixture is diluted with toluene and heated to 90°C for 5 hours. Then it is evaporated to dryness, the residue is divided between water and diethyl ether and the aqueous phase is extracted twice with diethyl ether. The aqueous phase is saturated with potassium carbonate and extracted another 3 times with diethyl ether. The combined organic phases are dried on magnesium sulphate. The solvent is eliminated in vacuo and the residue is chromatographed on aluminium oxide with ethyl acetate.
   Mass spectrum (ESI⁺): m/z = 272 [M+H]⁺

### Example XVI

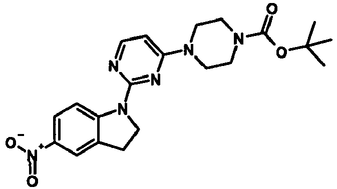

### tert-butyl 4-[2-(5-nitro-2,3-dihydro-indol-1-yl)-pyrimidin-4-yl]-piperazine-1-carboxylate

500 mg tert-butyl 4-(2-chloro-pyrimidin-4-yl)-piperazine-1-carboxylate and 302 mg 5-nitro-2,3-dihydro-1H-indole are mixed and heated to 150°C for 1 hour. The mixture is divided between water and ethyl acetate, the aqueous phase is extracted twice with ethyl acetate and the combined organic phases are dried with magnesium sulphate. The solvent is eliminated in vacuo and the residue is chromatographed on silica gel (cyclohexane/ethyl acetate 10:1 to 1:2).
Yield: 143 mg (20 % of theory)
Mass spectrum (ESI⁺): m/z = 427 [M+H]⁺

The following compounds are obtained analogously to Example XVI:
(1) 5-nitro-1-pyridin-4-yl-2,3-dihydro-1H-indole 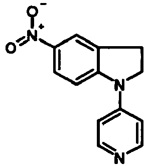 The reaction lasts 10 minutes at 180°C. Then the mixture is dissolved in methanol and this is then eliminated again in vacuo. The residue is chromatographed on silica gel.
   Mass spectrum (ESI⁺): m/z = 242 [M+H]⁺
(2) tert.butyl {(3,5-dichloro-phenylsulphonyl)-[1-(6-morpholin-4-yl-pyridazin-3-yl)-2,3-dihydro-1H-indol-5-yl]-amino}-acetate 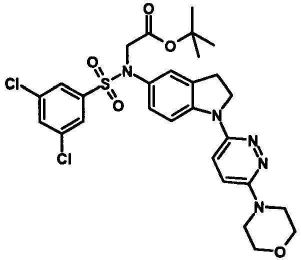 Obtained from the reaction of tert.butyl [[1-(6-chloro-pyridazin-3-yl)-2,3-dihydro-1H-indol-5-yl]-(3,5-dichloro-phenylsulphonyl)-amino]-acetate and morpholine. The reaction is carried out in the presence of 1 equivalent of N-methylpyrrolidine at 150°C for 12 hours. The crude product thus obtained is chromatographed on silica gel with dichloromethane/methanol (99:1 to 80:20).
   Mass spectrum (ESI⁺): m/z = 620 [M+H]⁺

### Example XVII

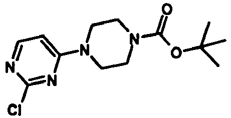

### tert-butyl 4-(2-chloro-pyrimidin-4-yl)-piperazine-1-carboxylate

1 g 2,4-dichloropyrimidine is dissolved in 30 ml of tetrahydrofuran and cooled to 0°C. A solution of 1.25 g tert-butyl piperazine-1-carboxylate and 1.2 ml triethylamine in 30 ml of tetrahydrofuran is added dropwise thereto. The mixture is stirred for 1 hour at 0°C and 2 hours at ambient temperature. Then the solvent is eliminated in vacuo, the residue is divided between water and dichloromethane and the aqueous phase is extracted twice with dichloromethane. The combined organic phases are dried on magnesium sulphate, the solvent is eliminated in vacuo and the residue is extracted from diisopropyl ether.
Yield: 1.5 g (75 % of theory)
Mass spectrum (ESI⁺): m/z = 299 [M+H]⁺

### Example XVIII

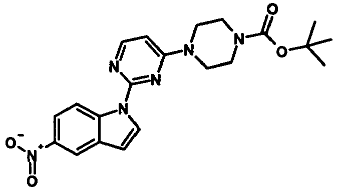

### tert-butyl 4-[2-(5-nitro-indol-1-yl)-pyrimidin-4-yl]-piperazine-1-carboxylate

298 mg 5-nitro-1H-indole are dissolved in 10 ml dimethylformamide and 74 mg NaH (60 % suspension in mineral oil) are added. The mixture is stirred for 1 h at ambient temperature and then 500 mg tert-butyl 4-(2-chloro-pyrimidin-4-yl)-piperazine-1-carboxylate are added. The solution is heated to 70°C for 5 hours, divided between water and ethyl acetate and the aqueous phase is extracted twice with ethyl acetate. The combined organic phases are dried on magnesium sulphate and the solvent is eliminated in vacuo. The residue is chromatographed on silica gel (cyclohexane/ethyl acetate 10:1 to 1:2).
Yield: 390 mg (55 % of theory)
Mass spectrum (ESI⁺): m/z = 425 [M+H]⁺

The following compounds are obtained analogously to Example XVIII:
(1) tert-butyl 4-[5-(5-nitro-2,3-dihydro-indol-1-yl)-pyrazin-2-carbonyl]-piperazine-1-carboxylate 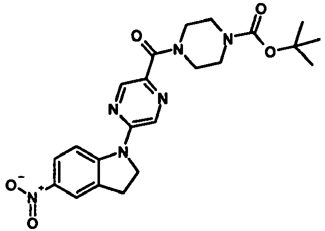 After heating to 70°C the solvent is eliminated in vacuo and the residue is extracted from water.
   Mass spectrum (ESI⁺): m/z = 455 [M+H]⁺
(2) tert-butyl 4-[5-(5-nitro-indol-1-yl)-pyrazin-2-carbonyl]-piperazine-1-carboxylate 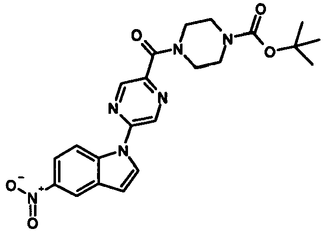 After heating to 70°C the solvent is eliminated in vacuo and the residue is extracted from water.
   Mass spectrum (ESI⁺): m/z = 453 [M+H]⁺
(3) 4-nitro-1-pyrimidin-2-yl-1H-indole 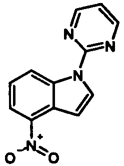 The reaction is carried out for 3 hours at 70°C. Then it is diluted with dichloromethane and washed with semisaturated sodium chloride solution. After drying with magnesium sulphate the solvent is eliminated in vacuo and the residue is extracted from ethyl acetate/diisopropylether.
   Mass spectrum (ESI⁺): m/z = 241 [M+H]⁺
(4) tert-butyl 6'-(5-nitro-2,3-dihydro-indol-1-yl)-2,3,5,6-tetrahydro-[1,2']bipyrazinyl-4-carboxylate 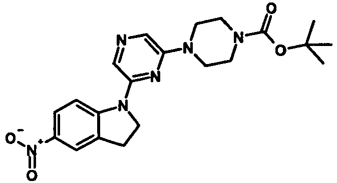 and
   tert-butyl 6'-(5-nitro-indol-1-yl)-2,3,5,6-tetrahydro-[1,2']bipyrazinyl-4-carboxylate 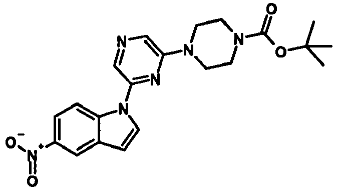 The reaction is carried out for 48 hours at 100°C.
   Mass spectrum (ESI⁺): m/z = 427 [M+H]⁺ (tert-butyl 6'-(5-nitro-2,3-dihydro-indol-1-yl)-2,3,5,6-tetrahydro-[1,2']bipyrazinyl-4-carboxylate) and
   Mass spectrum (ESI⁺): m/z = 425 [M+H]⁺ (tert. butyl 6'-(5-nitro-indol-1-yl)-2,3,5,6-tetrahydro-[1,2']bipyrazinyl-4-carboxylate)
(5) N,N-dimethyl-N'-[2-(5-nitro-2,3-dihydro-indol-1-yl)-pyrimidin-4-yl]-ethan-1,2-diamine 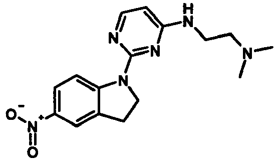 The reaction is carried out for 48 hours at 120°C. Then it is diluted with dichloromethane and washed with semisaturated sodium chloride solution. After drying with magnesium sulphate the solvent is eliminated in vacuo and the residue is chromatographed on silica gel.
   Mass spectrum (ESI⁺): m/z = 329 [M+H]⁺
(6) 1-(6-chloro-pyridazin-3-yl)-5-nitro-2,3-dihydro-1H-indole 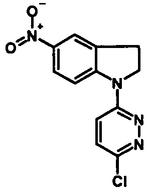 The reaction lasts 2 hours. The crude product is extracted from diethyl ether.
   R_{f} value: 0.55 (silica gel, petroleum ether/ethyl acetate 1:4)
(7) 1-(2-chloro-pyrimidin-4-yl)-5-nitro-2,3-dihydro-1H-indole 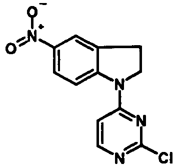 and
   1-(4-chloro-pyrimidin-2-yl)-5-nitro-2,3-dihydro-1H-indole 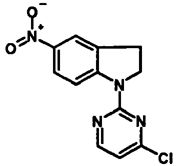 The reaction lasts 2 hours. The crude product is extracted from ethyl acetate/diisopropylether, the products being obtained as a mixture of isomers. The isomers can be separated in the next step (Example XXXIX (1)).
   Mass spectrum (ESI⁺): m/z = 277 [M+H]⁺
(8) 1-(6-chloro-pyridazin-3-yl)-5-nitro-2,3-dihydro-1H-indole 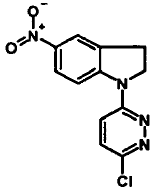 The reaction lasts 2 hours. The crude product is extracted from diethyl ether.
   Mass spectrum (ESI⁺): m/z = 277 [M+H]⁺
(9) 1-(6-chloro-pyridazin-3-yl)-5-nitro-1H-indole 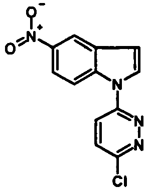 The reaction lasts 12 hours at ambient temperature. The reaction mixture is added to water. The solid is suction filtered, washed with water, ethyl acetate and diethyl ether and then dried.
   Mass spectrum (ESI⁺): m/z = 275 [M+H]⁺
(10) 5-nitro-1-(4-oxy-pyrazin-2-yl)-1H-indole 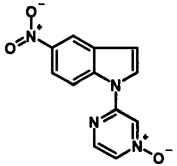 The reaction lasts 12 hours at ambient temperature. The reaction mixture is added to water. The solid is suction filtered, washed with water, ethyl acetate and diethyl ether and then dried.
   Mass spectrum (ESI⁺): m/z = 257 [M+H]⁺
(11) 1-(4-chloro-pyrimidin-2-yl)-5-nitro-1H-indole 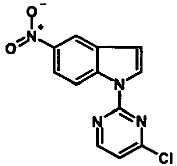 and
   1-(2-chloro-pyrimidin-4-yl)-5-nitro-1H-indole 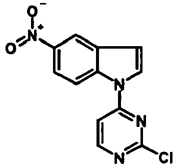 The reaction lasts 48 hours at ambient temperature. The reaction mixture is added to water. The solid is suction filtered and separated into the regioisomers by chromatography on silica gel.
   Mass spectrum (ESI⁺): m/z = 292 [M+NH₄]⁺ (1-(4-chloro-pyrimidin-2-yl)-5-nitro-1H-indole)
   and
   Mass spectrum (ESI⁻): m/z = 319 [M+HCOO]⁻ (1-(2-chloro-pyrimidin-4-yl)-5-nitro-1H-indole)
(12) 1-(4,6-dimethoxy-[1,3,5]triazin-2-yl)-5-nitro-2,3-dihydro-1H-indole 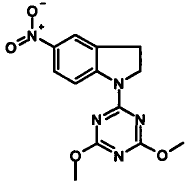 Mass spectrum (ESI⁺): m/z = 304 [M+H]⁺
(13) 2-chloro-6-(5-nitro-indol-1-yl)-9H-purine 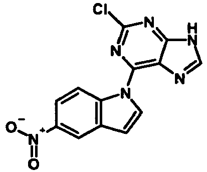 The reaction is carried out for 3 hours at 100°C. Then the mixture is added to water, the solid is suction filtered and dried.
   Mass spectrum (ESI⁻): m/z = 313 [M-H]⁻
(14) 5-(5-nitro-indol-1-yl)-pyrazin-2-carboxylic acid-amide 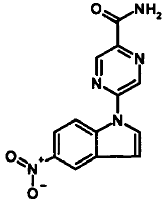 The reaction is carried out for 12 hours at ambient temperature. Then the mixture is added to water, the solid is suction filtered and dried.
   Mass spectrum (ESI⁺): m/z = 284 [M+H]⁺
(15) 5-(5-nitro-indol-1-yl)-pyrazin-2-carboxylic acid-methyl-amide 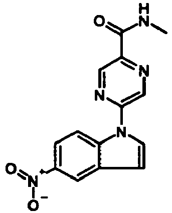 The reaction is carried out for 12 hours at ambient temperature. Then the mixture is added to water, the solid is suction filtered and dried.
   Mass spectrum (ESI⁺): m/z = 298 [M+H]⁺
(16) morpholin-4-yl-[5-(5-nitro-indol-1-yl)-pyrazin-2-yl]-methanone 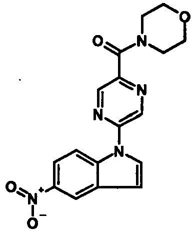 The reaction is carried out for 12 hours at ambient temperature. Then the mixture is added to water, the solid is suction filtered and dried.
   Mass spectrum (ESI⁺): m/z = 354 [M+H]⁺
(17) 5-(5-nitro-indol-1-yl)-pyrazin-2-carbonitrile 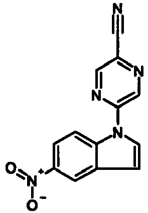 The reaction is carried out for 12 hours at ambient temperature. Then the mixture is added to water, the solid is suction filtered and dried.
   Mass spectrum (ESI⁻): m/z = 310 [M+HCOO]⁻
(18) 6-(5-nitro-indol-1-yl)-pyridazine-3-carboxylic acid 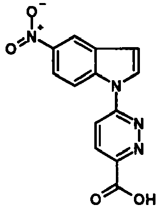 The reaction is carried out for 4 days at 120°C. Then the mixture is added to 2 N hydrochloric acid, the solid is suction filtered and dried.
   Mass spectrum (ESI⁺): m/z = 285 [M+H]⁺
(19) tert.butyl {(3,5-dichloro-phenylsulphonyl)-[1-(6-trifluoromethyl-pyrimidin-4-yl)-1H-indol-5-yl]-amino}-acetate 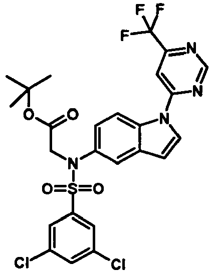 The reaction is carried out with 4-bromo-6-trifluoromethyl-pyrimidine in the presence of caesium carbonate in N,N-dimethylformamide at 80°C.
   Mass spectrum (ESI⁺): m/z = 618 [M+NH₄]⁺
   R_{f} value: 0.85 (silica gel: cyclohexane/ethyl acetate 1:1)
(20) tert.butyl [[1-(2-bromo-pyrimidin-4-yl)-1H-indol-5-yl]-(3,5-dichlorophenylsulphonyl)-amino]-acetate 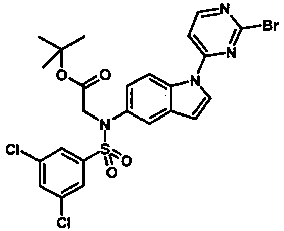 The reaction is carried out with 2,4-dibromo-pyridine in the presence of potassium carbonate in N,N-dimethylformamide at 70°C.
   Mass spectrum (ESI⁺): m/z = 628 [M+NH₄]⁺
   R_{f} value: 0.33 (silica gel: petroleum ether/ethyl acetate 2:1)
(21) 6-nitro-1-pyrimidin-2-yl-1H-benzimidazole 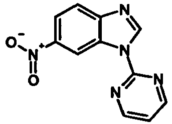 Mass spectrum (ESI⁺): m/z = 242 [M+H]⁺
(22) 5-nitro-1-pyrimidin-2-yl-1H-benzimidazole 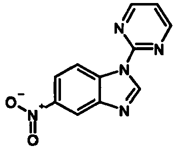 Mass spectrum (ESI⁺): m/z = 242 [M+H]⁺

### Example IXX

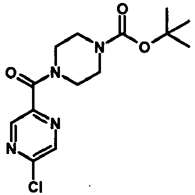

### tert-butyl 4-(5-chloro-pyrazin-2-carbonyl)-piperazine-1-carboxylate

1 g 5-oxo-4,5-dihydro-pyrazine-2-carboxylic acid is dissolved in 10 ml phosphorus oxychloride, heated to 90°C for 3 hours and then freed from the solvent in vacuo. The residue is taken up in 10 ml dichloromethane and a solution of 1.3 g tert-butyl piperazine-1-carboxylate and 3 ml triethylamine in 10 ml dichloromethane is added dropwise thereto. The mixture is stirred for 3 hours at ambient temperature, divided between 1 N HCl and dichloromethane and the aqueous phase is extracted with dichloromethane. The combined organic phases are washed with saturated sodium hydrogen carbonate solution and saturated sodium chloride solution. After drying with magnesium sulphate the solvent is eliminated in vacuo and the residue is taken up in 20 ml diisopropylether. The mixture is decanted from the solid, left to cool to ambient temperature and the precipitated solid is suction filtered. The product thus obtained is purified by chromatography on silica gel (cyclohexane/ethyl acetate 10:1 to 1:2).
Yield: 380 mg (16 % of theory)
Mass spectrum (ESI⁺): m/z = 327 [M+H]⁺

The following compounds are obtained analogously to Example IXX:
(1) 5-chloro-pyrazine-2-carboxylic acid-amide 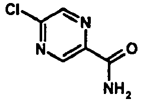 To prepare the acid chloride thionyl chloride with 1 drop of dimethylformamide is used. The amide is prepared in tetrahydrofuran with 10 equivalents of a 32% solution of ammonia in water.
   Mass spectrum (ESI⁺): m/z = 158 [M+H]⁺
(2) 5-chloro-pyrazin-2-carboxylic acid-methyl-amide 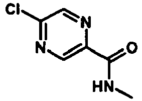 To prepare the acid chloride thionyl chloride with 1 drop of dimethylformamide is used. The amide is prepared in tetrahydrofuran.
   Mass spectrum (ESI⁺): m/z = 172 [M+H]⁺
(3) (5-chloro-pyrazin-2-yl)-morpholin-4-yl-methanone 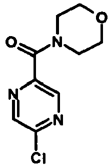 To prepare the acid chloride thionyl chloride with 1 drop of dimethylformamide is used. The amide is prepared in tetrahydrofuran.
   Mass spectrum (ESI⁺): m/z = 228 [M+H]⁺
(4) morpholin-4-yl-[6-(5-nitro-indol-1-yl)-pyridazin-3-yl]-methanone 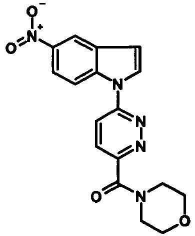 To prepare the acid chloride thionyl chloride in toluene with 1 drop of dimethylformamide is used. To prepare the amide pyridine is used instead of triethylamine. The crude product is taken up in dichloromethane/methanol 10:1, combined with activated charcoal, filtered through kieselgur, freed from the solvents in vacuo and extracted from diethyl ether.
   Mass spectrum (ESI⁺): m/z = 354 [M+H]⁺
(5) methyl 6-chloro-pyridazine-3-carboxylate 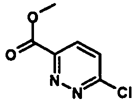 This is obtained by reacting methyl 6-oxo-1,6-dihydro-pyridazine-3-carboxylate with thionyl chloride in toluene in the presence of 1 drop of dimethylformamide. After heating for 12 hours to 100°C this mixture is added to water and the solid is suction filtered. After the solid has been taken up in dichloromethane/ methanol 10:1 activated charcoal is added, the mixture is filtered through kieselgur, the solvents are eliminated in vacuo and the residue is extracted from diethyl ether.
   Mass spectrum (ESI⁺): m/z = 155 [M+H]⁺
(6) 6-(5-nitro-indol-1-yl)-pyridazine-3-carboxylic acid-dimethylamide 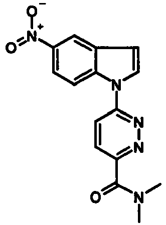 The acid chloride is prepared using thionyl chloride in toluene with 1 drop of dimethylformamide. To prepare the amide pyridine is used instead of triethylamine. The crude product is taken up in dichloromethane/methanol 10:1, combined with activated charcoal, filtered through kieselguhr, freed from the solvents in vacuo and extracted from diethyl ether.
   Mass spectrum (ESI⁺): m/z = 312 [M+H]⁺

### Example XX

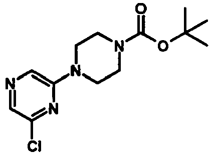

### tert-butyl 6'-chloro-2,3,5,6-tetrahydro-[1,2']bipyrazinyl-4-carboxylate

630 mg tert-butyl piperazine-1-carboxylate are dissolved in 12 ml dimethylformamide. 500 mg potassium carbonate and 500 mg 2,6-dichloropyrazine are added. Then the mixture is stirred overnight at ambient temperature. It is diluted with dichloromethane, the solid is filtered off and the mother liquor is evaporated down in vacuo. The residue is chromatographed on silica gel (cyclohexane/ethyl acetate 10:1 to 1:2).
Yield: 525 mg (53 % of theory)
Mass spectrum (ESI⁺: m/z = 299 [M+H]⁺

The following compounds are obtained analogously to Example XX:
(1) N'-(2-chloro-pyrimidin-4-yl)-N,N-dimethyl-ethan-1,2-diamine 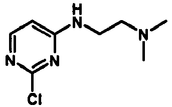 Mass spectrum (ESI⁺): m/z = 201 [M+H]⁺
(2) tert-butyl 4-(6-iodo-5-methyl-pyridazin-3-yl)-piperazine-1-carboxylate 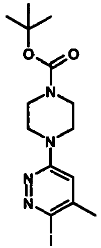 The reaction lasts 5 hours at 60°C. The crude product is further reacted directly in Example XVI (13).
(3) N'-(6-chloro-pyridazin-3-yl)-N,N-dimethyl-ethan-1,2-diamine 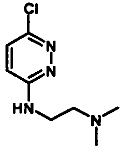 The reaction lasts 12 hours at 60°C.
   Mass spectrum (ESI⁺): m/z = 201 [M+H]⁺
(4) tert-butyl 4-(6-iodo-pyridazin-3-yl)-[1,4]diazepan-1-carboxylate 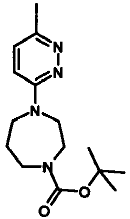 The reaction is carried out in dioxane for 24 hours at 120°C in a pressurised vessel.
   Mass spectrum (ESI⁺): m/z = 405 [M+H]⁺
(5) N*1*-(6-iodo-pyridazin-3-yl)-propane-1,3-diamine 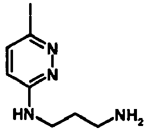 The reaction is carried out in dioxane for 24 hours at 120°C in a pressurised vessel. The crude product is further reacted directly.
   R_{f} value: 0.08 (aluminium oxide; dichloromethane/methanol 9:1)
(6) cyclopropyl-(6-iodo-pyridazin-3-yl)-amine 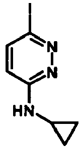 The reaction is carried out in dioxane for 24 hours at 120°C in a pressurised vessel. Then another 3 equivalents cyclopropylamine are added and the mixture is heated for 2 hours in the microwave.
   Mass spectrum (ESI⁺): m/z = 262 [M+H]⁺
(7) N*1*-(6-iodo-pyridazin-3-yl)-N*1*-methyl-ethan-1,2-diamine 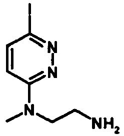 and
   N-(6-iodo-pyridazin-3-yl)-N'-methyl-ethan-1,2-diamine 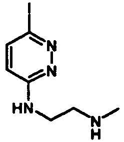 The reaction is carried out in dioxane for 24 hours at 120°C in a pressurised vessel. The two regiosiomers may be separated from one another in the next step (cf Example XXXVII (12)).
   Mass spectrum (ESI⁺): m/z = 279 [M+H]⁺
(8) N*1*-(6-iodo-pyridazin-3-yl)-2-methyl-propane-1,2-diamine 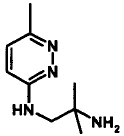 The reaction is carried out in dioxane for 10 minutes at 180°C in a pressurised vessel in the microwave.
   Mass spectrum (ESI⁺): m/z = 293 [M+H]⁺
(9) tert-butyl [1-(6-iodo-pyridazin-3-yl)-piperidin-3-yl]-carbamate 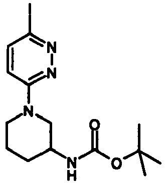 Caesium carbonate is used instead of potassium carbonate. The reaction is carried out in dioxane for 48 hours at 120°C in a pressurised vessel.
   Mass spectrum (ESI⁺): m/z = 405 [M+H]⁺
(10) 4-(6-iodo-pyridazin-3-yl)-morpholine 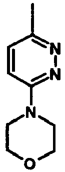 The reaction is carried out in dioxane for 12 hours at 120°C in a pressurised vessel.
   Mass spectrum (ESI⁺): m/z = 292 [M+H]⁺
(11) (6-iodo-pyridazin-3-yl)-dimethyl-amine 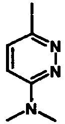 The reaction is carried out in 2 M solution of dimethylamine in tetrahydrofuran. It is heated for 10 minutes to 160°C in a pressurised vessel in the microwave. Then it is diluted with ethyl acetate and washed with saturated sodium chloride solution. After drying with magnesium sulphate the mixture is chromatographed on silica gel with cyclohexane/ethyl acetate (80:20 to 0:100).
   Mass spectrum (ESI⁺): m/z = 250 [M+H]⁺
(12) 2-(6-iodo-pyridazin-3-ylamino)-ethanol 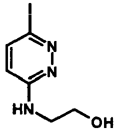 The reaction is carried out in dioxane for 12 hours at 120°C in a pressurised vessel.
   Mass spectrum (ESI⁺): m/z = 266 [M+H]⁺
(13) (6-iodo-pyridazin-3-yl)-(2-piperidin-1-yl-ethyl)-amine 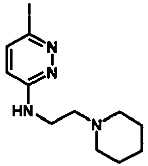 The reaction is carried out in dioxane for 12 hours at 120°C in a pressurised vessel. The crude product is chromatographed on aluminium oxide with cyclohexane/ethyl acetate (70:30 to 0:100).
   Mass spectrum (ESI⁺): m/z = 333 [M+H]⁺
(14) (R)-(6-iodo-pyridazin-3-yl)-(tetrahydro-furan-3-yl)-amine 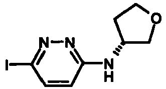 The reaction is carried out in dioxane at 180°C in a pressurised vessel in the microwave.
   Mass spectrum (ESI⁺): m/z = 292 [M+H]⁺
(15) (R)-(5-bromo-pyrazin-2-yl)-(tetrahydro-furan-3-yl)-amine 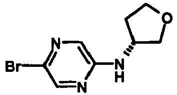 The reaction is carried out with triethylamine in dioxane at 175°C in a pressurised vessel in the microwave.
   R_{f} value: 0.32 (silica gel: petroleum ether/ethyl acetate 1:1)
(16) (6-iodo-pyridazin-3-yl)-methyl-(tetrahydro-pyran-4-yl)-amine 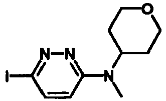 The reaction is carried out with triethylamine in dioxane at 170°C in a pressurised vessel in the microwave.
   Mass spectrum (ESI⁺): m/z = 320 [M+H]⁺
   R_{f} value: 0.20 (silica gel: petroleum ether/ethyl acetate 1:1)
(17) 4-(5-bromo-pyrazin-2-yl)-piperazin-2-one 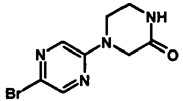 The reaction lasts 15 minutes at 140°C.
   Mass spectrum (ESI⁺): m/z = 257 [M+H]⁺
   R_{f} value: 0.25 (silica gel: dichloromethane/methanol 95:5)
(18) (5-bromo-pyrazin-2-yl)-(tetrahydro-pyran-4-yl)-amine 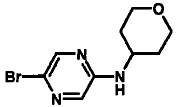 The reaction is carried out with triethylamine in dioxane at 120°C in a pressurised vessel.
   Mass spectrum (ESI⁻): m/z = 256 [M-H]⁻
   R_{f} value: 0.35 (silica gel: petroleum ether/ethyl acetate 1:1)
(19) (6-iodo-pyridazin-3-yl)-(tetrahydro-pyran-4-yl)-amine 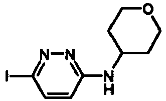 The reaction is carried out with triethylamine in dioxane at 170°C in a pressurised vessel in the microwave.
   R_{f} value: 0.12 (silica gel: petroleum ether/ethyl acetate 1:1)
(20) 2-bromo-4-(tetrahydro-furan-3-yloxy)-pyridine 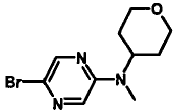 The reaction is carried out with triethylamine in dioxane at 140°C in a pressurised vessel in the microwave.
   Mass spectrum (ESI⁺): m/z = 272 [M+H]⁺
   R_{f} value: 0.35 (silica gel: petroleum ether/ethyl acetate 2:1)
(21) 4-(6-iodo-pyridazin-3-yl)-piperazin-2-one 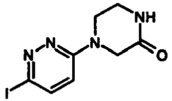 The reaction is carried out at 140°C.
   Mass spectrum (ESI⁺): m/z = 305 [M+H]⁺
   R_{f} value: 0.18 (silica gel: methylene chloride/methanol 95:5)
(22) 3-bromo-6-morpholin-4-yl-pyrazine 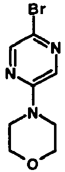 The reaction is carried out for 12 hours at 100°C.
   Mass spectrum (ESI⁻): m/z = 243 [M-H]⁻
(23) N-(6-iodo-pyridazin-3-yl)-N'-methyl-propane-1,3-diamine 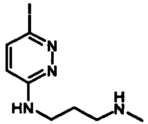 and
   N*1*-(6-iodo-pyridazin-3-yl)-N*1*-methyl-propane-1,3-diamine 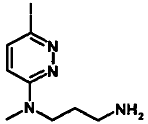 The reaction is carried out in dioxane for 5 hours at 120°C in a pressurised vessel. The two regioisomers may be separated from one another in the next step (cf. Example XXXVII (14)).
   Mass spectrum (ESI⁺): m/z = 293 [M+H]⁺
(24) tert.butyl {(3,5-dichloro-phenylsulphonyl)-[1-(2,6-dichloro-pyrimidin-4-yl)-1H-indol-5-yl]-amino}-acetate 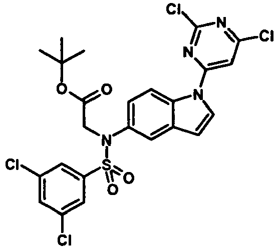 Obtained by reacting tert.butyl [(3,5-dichloro-phenylsulphonyl)-(1H-indol-5-yl)-amino]-acetate with 2,4,6-trichloro-pyrimidine. The reaction is carried out for 4 hours at 80°C. Then the solvent is eliminated in vacuo, the residue is taken up in ethyl acetate and washed once with water. After drying with magnesium sulphate the solvent is eliminated in vacuo and the residue is chromatographed on silica gel (cyclohexane/ethyl acetate 100:0 to 66:34).
   Mass spectrum (ESI⁺): m/z = 618 [M+NH₄]⁺
(25) (6-iodo-pyridazin-3-yl)-(2-pyrrolidin-1-yl-ethyl)-amine 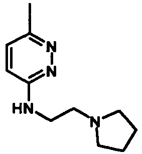 The reaction is carried out in dioxane for 24 hours at 140°C in a pressurised vessel.
   Mass spectrum (ESI⁺): m/z = 319 [M+H]⁺
(26) 4-(6-iodo-pyridazin-3-yl)-thiomorpholine 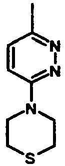 The reaction is carried out in dioxane for 12 hours at 140°C in a pressurised vessel.
   Mass spectrum (ESI⁺): m/z = 308 [M+H]⁺
(27) 2-(5-bromo-pyrazin-2-ylamino)-ethanol 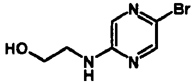 The reaction is carried out in dioxane for 12 hours at 120°C in a pressurised vessel.
   Mass spectrum (ESI⁻): m/z = 216 [M-H]⁻
(28) 4-(5-bromo-pyrazin-2-yl)-thiomorpholin 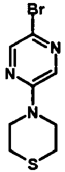 The reaction is carried out in dioxane for 24 hours at 150°C in a pressurised vessel.
   Mass spectrum (ESI⁺): m/z = 260 [M+H]⁺
(29) 2-(5-bromo-pyrazin-2-ylamino)-ethanol 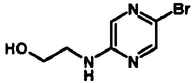 The reaction is carried out in dioxane for 12 hours at 150°C in a pressurised vessel.
   Mass spectrum (ESI⁺): m/z = 218 [M+H]⁺
(30) N*1*-(5-bromo-pyrazin-2-yl)-ethan-1,2-diamine 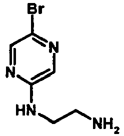 The reaction is carried out in dioxane for 12 hours at 120°C in a pressurised vessel. The crude product is further reacted directly (XXXVII (16)).
(31) tert-butyl 5'-bromo-2,3,5,6-tetrahydro-[1,2']bipyrazinyl-4-carboxylate 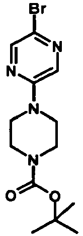 The reaction is carried out in dioxane for 12 hours at 120°C in a pressurised vessel. The crude product is further reacted directly (XVI (147)).

### Example XXI

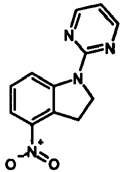

### 4-nitro-1-pyrimidin-2-yl-2,3-dihydro-1H-indole

280 mg 4-nitro-1-pyrimidin-2-yl-1H-indole are dissolved in 1 ml acetic acid and 4 ml trifluoroacetic acid. To this are added 160 mg sodium cyanoborohydride and the solution is stirred for 48 hours at ambient temperature. Then the solvents are eliminated in vacuo and the residue is divided between ethyl acetate and saturated sodium hydrogen carbonate solution. The organic phase is washed with saturated sodium chloride solution and dried with magnesium sulphate. After elimination of the solvents in vacuo the residue is chromatographed on silica gel (cyclohexane/ethyl acetate 10:1 to 1:3).
Yield: 30 mg (11 % of theory)
Mass spectrum (ESI⁺): m/z = 243 [M+H]⁺

### Example XXII

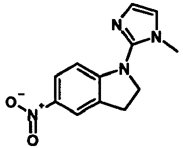

### 1-(1-methyl-1H-imidazol-2-yl)-5-nitro-2,3-dihydro-1H-indole

230 mg 1-(1H-imidazol-2-yl)-5-nitro-2,3-dihydro-1H-indole are dissolved in 6 ml DMF. 200 mg potassium carbonate and 75 µl methyl iodide are added and the mixture is stirred overnight at ambient temperature. Then it is diluted with ethyl acetate and extracted twice with 2 N HCl. The combined aqueous phases are washed with dichloromethane, adjusted to pH 12 with 40 % sodium hydroxide solution and extracted with dichloromethane. This organic phase is washed with saturated sodium chloride solution and dried on magnesium sulphate. The solvent is eliminated in vacuo and the residue is chromatographed on silica gel (dichloromethane/methanol 99:1 to 9:1).
Yield: 170 mg (70 % of theory)
Mass spectrum (ESI⁺): m/z = 245 [M+H]⁺

### Example XXIII

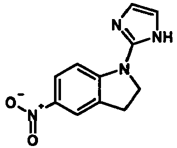

### 1-(1H-imidazol-2-yl)-5-nitro-2,3-dihydro-1H-indole

150 mg N-(2,2-diethoxy-ethyl)-5-nitro-2,3-dihydro-indol-1-carboxamidine are heated to 60°C in 8 ml trifluoroacetic acid for 3 hours. The solvent is then eliminated in vacuo and the residue is chromatographed on aluminium oxide (dichloromethane/methanol 99:1 to 7:3).
Yield: 230 mg (69 % of theory)
Mass spectrum (ESI⁻): m/z = 229 [M-H]⁻

### Example XXIV

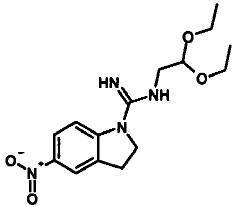

### N-(2,2-diethoxy-ethyl)-5-nitro-2,3-dihydro-indol-1-carboxamidine

410 mg methyl 5-nitro-2,3-dihydro-indol-1-carboximidothioate are dissolved in 4 ml dimethylformamide and 350 µl aminoacetaldehyde-diethylacetal are added thereto. The mixture is heated for 4 hours to 100°C. Then the solvent is eliminated in vacuo and the residue is chromatographed on aluminium oxide (dichloromethane/methanol 99:1 to 7:3).
Yield: 330 mg (91 % of theory)
Mass spectrum (ESI⁺): m/z = 323 [M+H]⁺

### Example XXV

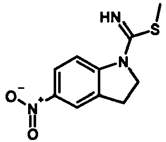

### methyl 5-nitro-2,3-dihydro-indol-1-carboximidothioate

3.5 g 5-nitro-2,3-dihydro-indol-1-carbothioic acidamide are dissolved in 50 ml dimethylformamide and 1.2 ml methyl iodide are added. The mixture is stirred overnight at ambient temperature, the solvent is eliminated in vacuo and the residue is extracted from diethyl ether.
Yield: 4.85 g (85 % of theory)
Mass spectrum (ESI⁺): m/z = 238 [M+H]⁺

### Example XXVI

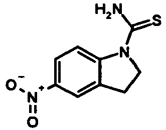

### 5-nitro-2,3-dihydro-indol-1-carbothioic acid amide

5 g 5-nitro-2,3-dihydro-1H-indole are dissolved in 100 ml dichloromethane under argon and cooled to 0°C. 298 µl N,N-diisopropyl-ethylamine and 2.4 ml thiophosgene are added and the mixture is stirred for another 1.5 hours. Then 300 ml of tetrahydrofuran are added, then ammonia gas is piped in for 2 hours and the mixture is stirred overnight at ambient temperature. The solvents are eliminated in vacuo and the residue is extracted from 1 N HCl.
Yield: 6.35 g (96 % of theory)
Mass spectrum (ESI⁺): m/z = 224 [M+H]⁺

### Example XXVII

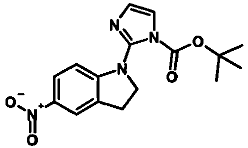

### tert-butyl 2-(5-nitro-2,3-dihydro-indol-1-yl)-imidazole-1-carboxylate

500 mg 1-(1H-imidazol-2-yl)-5-nitro-2,3-dihydro-1H-indole are dissolved in 10 ml of tetrahydrofuran, 10 mg N,N-dimethylaminopyridine and 500 mg di-tert-butyl-dicarbonate are added and the mixture is stirred for 1 hour at ambient temperature. Then the solvent is eliminated in vacuo and the residue is chromatographed on aluminium oxide with dichloromethane.
Yield: 590 mg (82 % of theory)
Mass spectrum (ESI⁺): m/z = 331 [M+H]⁺

The following compounds are obtained analogously to Example XXXVII:
(1) tert-butyl 4-[6-(5-nitro-2,3-dihydro-indol-1-yl)-pyridazin-3-yl]-piperazine-1-carboxylate 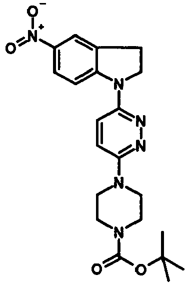 The crude product is extracted from diisopropylether.
   Mass spectrum (ESI⁺): m/z = 427 [M+H]⁺
(2) tert-butyl (2-dimethylamino-ethyl)-[2-(5-nitro-2,3-dihydro-indol-1-yl)-pyrimidin-4-yl]-carbamate 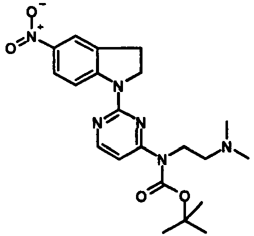 The crude product is extracted from diisopropylether/petroleum ether.
   Mass spectrum (ESI⁺): m/z = 429 [M+H]⁺
(3) tert-butyl (2-dimethylamino-ethyl)-[6-(5-nitro-2,3-dihydro-indol-1-yl)-pyridazin-3-yl]-carbamate 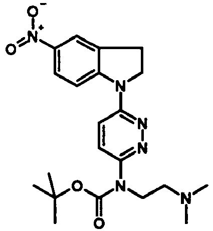 The crude product is chromatographed on silica gel.
   Mass spectrum (ESI⁺): m/z = 429 [M+H]⁺
(4) tert-butyl (2-(bis-tert-butoxycarbonyl)-amino-ethyl)-[6-(5-nitro-2,3-dihydro-indol-1-yl)-pyridazin-3-yl]-carbamate 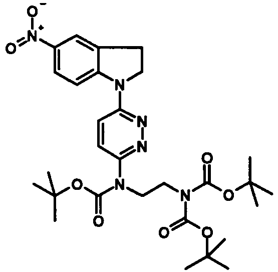 and
   tert-butyl 3-[6-(5-nitro-2,3-dihydro-indol-1-yl)-pyridazin-3-yl]-2-oxo-imidazolidine-1-carboxylate 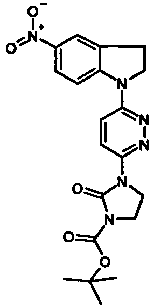 The crude product is extracted from diethyl ether, to obtain tert-butyl 3-[6-(5-nitro-2,3-dihydro-indol-1-yl)-pyridazin-3-yl]-2-oxo-imidazolidine-1-carboxylate. The mother liquor is evaporated down in vacuo and the residue is chromatographed on aluminium oxide, to obtain tert-butyl (2-(bis-tert-butoxycarbonyl)-amino-ethyl)-[6-(5-nitro-2,3-dihydro-indol-1-yl)-pyridazin-3-yl]-carbamate.
   Mass spectrum (ESI⁺): m/z = 601 [M+H]⁺ (tert-butyl (2-(bis-tert-butoxycarbonyl)-amino-ethyl)-[6-(5-nitro-2,3-dihydro-indol-1-yl)-pyridazin-3-yl]-carbamate)
   and
   Mass spectrum (ESI⁺): m/z = 427 [M+H]⁺ (tert-butyl 3-[6-(5-nitro-2,3-dihydro-indol-1-yl)-pyridazin-3-yl]-2-oxo-imidazolidine-1-carboxylate)
(5) tert-butyl (2-(bis-tert-butoxycarbonyl)-amino-ethyl)-[6-(5-nitro-indol-1-yl)-pyridazin-3-yl]-carbamate 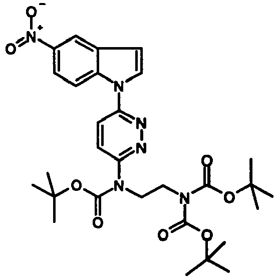 The crude product is chromatographed on silica gel.
   Mass spectrum (ESI⁺): m/z = 599 [M+H]⁺
(6) tert-butyl 5'-(5-nitro-indol-1-yl)-2,3,5,6-tetrahydro-[1,2']bipyrazinyl-4-carboxylate 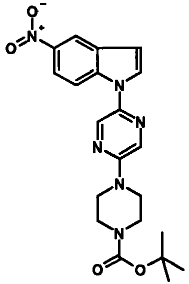 The crude product is extracted from diethyl ether.
   Mass spectrum (ESI⁺): m/z = 425 [M+H]⁺
(7) tert-butyl (2-tert-butoxycarbonylamino-ethyl)-[2-(5-nitro-indol-1-yl)-pyrimidin-4-yl]-carbamate 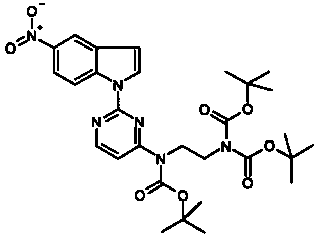 The crude product is chromatographed on silica gel.
   Mass spectrum (ESI⁺): m/z = 599 [M+H]⁺
(8) tert-butyl 4-[6-(5-nitro-indol-1-yl)-pyridazin-3-yl]-piperazine-1-carboxylate 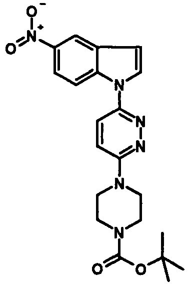 The crude product is extracted from diethyl ether.
   Mass spectrum (ESI⁺): m/z = 425 [M+H]⁺
(9) tert-butyl (2-(bis-tert-butoxycarbonyl)-amino-ethyl)-[4-(5-nitro-indol-1-yl)-pyrimidin-2-yl]-carbamate 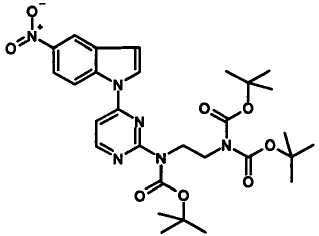 The crude product is chromatographed on silica gel.
   Mass spectrum (ESI⁺): m/z = 599 [M+H]⁺
(10) tert-butyl (3-(bis-tert-butoxycarbonyl)-amino-propyl)-(6-iodo-pyridazin-3-yl)-carbamate 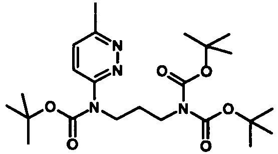 The crude product is chromatographed on silica gel.
   Mass spectrum (ESI⁺): m/z = 579 [M+H]⁺
(11) tert-butyl [2-(tert-butoxycarbonyl-methyl-amino)-ethyl]-(6-iodo-pyridazin-3-yl)-carbamate 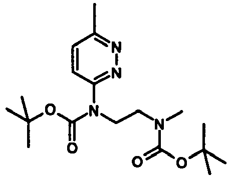 and
   3-{[2-(bis-tert.-butoxycarbonyl)-amino)-ethyl]-methyl}-amino-6-iodo-pyridazine 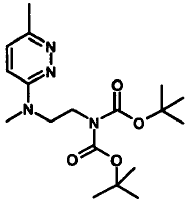 are obtained by reacting a mixture of N-(6-iodo-pyridazin-3-yl)-N'-methyl-ethan-1,2-diamine and N*1*-(6-iodo-pyridazin-3-yl)-N*1*-methyl-ethan-1,2-diamine (cf. Example XXX (7)). The products may be separated by chromatography on silica gel.
   Mass spectrum (ESI⁺): m/z = 479 [M+H]⁺ (tert-butyl [2-(tert-butoxycarbonylmethyl-amino)-ethyl]-(6-iodo-pyridazin-3-yl)-carbamate)
   and
   Mass spectrum (ESI⁺): m/z = 479 [M+H]⁺ (3-{[2-(bis-tert.-butoxycarbonyl)-amino)-ethyl]-methyl}-amino-6-iodo-pyridazine)
(12) tert-butyl (6-iodo-pyridazin-3-yl)-(2-piperidin-1-yl-ethyl)-carbamate 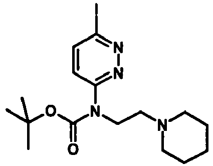 The crude product is chromatographed on silica gel.
   Mass spectrum (ESI⁺): m/z = 433 [M+H]⁺
(13) tert-butyl [3-(tert-butoxycarbonyl-methyl-amino)-propyl]-(6-iodo-pyridazin-3-yl)-carbamate 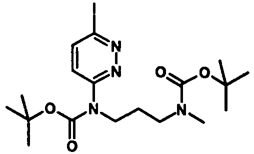 and
   3-{[3-(bis-tert.-butoxycarbonyl)-amino)-propyl)-methyl}-amino-6-iodo-pyridazine 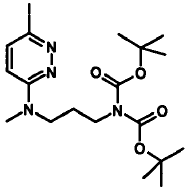 are obtained by reacting a mixture of N-(6-iodo-pyridazin-3-yl)-N'-methylpropane-1,3-diamine and N*1*-(6-iodo-pyridazin-3-yl)-N*1*-methyl-propane-1,3-diamine (cf. Example XXX (23)). The products may be separated by chromatography on silica gel.
   Mass spectrum (ESI⁺): m/z = 493 [M+H]⁺ tert-butyl [3-(tert-butoxycarbonylmethyl-amino)-propyl]-(6-iodo-pyridazin-3-yl)-carbamate
   and
   Mass spectrum (ESI⁺): m/z = 493 [M+H]⁺ 3-{[3-(bis-tert.-butoxycarbonyl)-amino)-propyl]-methyl}-amino-6-iodo-pyridazine
(14) tert-butyl [2-(6-iodo-pyridazin-3-ylamino)-ethyl]-carbamate 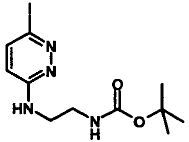 The reaction is carried out in the absence of 4-dimethylamino-pyridine at 60°C. After the reaction has ended the solvent is eliminated in vacuo and the residue is extracted from diethyl ether.
   Mass spectrum (ESI⁺): m/z = 365 [M+H]⁺
(15) tert-butyl [2-(5-bromo-pyrazin-2-ylamino)-ethyl]-carbamate 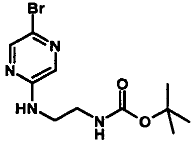 The reaction is carried out in the absence of 4-dimethylamino-pyridine at 60°C. After the reaction has ended the solvent is eliminated in vacuo and the residue is chromatographed on silica gel.
   Mass spectrum (ESI⁺): m/z = 317 [M+H]⁺
(16) tert-butyl (5-bromo-pyrazin-2-yl)-(2-dimethylamino-ethyl)-carbamate 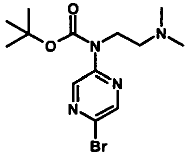 The reaction is carried out in the absence of 4-dimethylamino-pyridin at 60°C. After the reaction has ended the solvent is eliminated in vacuo and the residue is chromatographed on silica gel.
   Mass spectrum (ESI⁺): m/z = 345 [M+H]⁺

### Example XXVIII

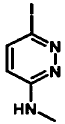

### 3-iodo-6-metyhlamino-pyridazine

500 mg 3,6-diiodo-pyridazine are dissolved in 2 ml dioxane, 1.5 ml methylamine (2 N in tetrahydrofuran are added and the mixture is heated for 48 hours to 120°C in a pressurised vessel. Then it is diluted with ethyl acetate and washed once with semisaturated sodium chloride solution. The organic phase is dried on magnesium sulphate, the solvents are eliminated in vacuo and the residue is chromatographed on silica gel (dichloromethane/methanol 99:1 to 90:10).
Yield: 35 mg (10 % of theory)
Mass spectrum (ESI⁺): m/z = 236 [M+H]⁺

### Example XXIX

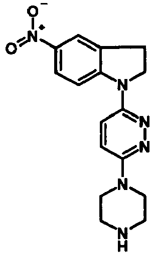

### 5-nitro-1-(6-piperazin-1-yl-pyridazin-3-yl)-2,3-dihydro-1H-indole

250 mg 1-(6-chloro-pyridazin-3-yl)-5-nitro-2,3-dihydro-1H-indole and 500 mg piperazine are mixed in a microwave vessel and heated to 180°C for 2 hours in a microwave oven. After cooling to ambient temperature the mixture is stirred with dichloromethane and methanol. The solvents are eliminated in vacuo and the residue is divided between water and dichloromethane. The organic phase is washed with saturated sodium chloride solution and dried on magnesium sulphate. The solvent is eliminated in vacuo and the residue is extracted from diethyl ether/diisopropylether.
Yield: 245 mg (83 % of theory)
Mass spectrum (ESI⁺): m/z = 327 [M+H]⁺

The following compounds are obtained analogously to Example XXIX:
(1) N,N-dimethyl-N'-[2-(5-nitro-2,3-dihydro-indol-1-yl)-pyrimidin-4-yl]-ethan-1,2-diamine 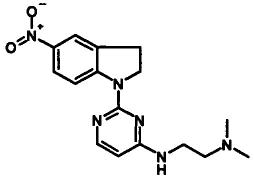 and
   N,N-dimethyl-N'-[4-(5-nitro-2,3-dihydro-indol-1-yl)-pyrimidin-2-yl]-ethan-1,2-diamine 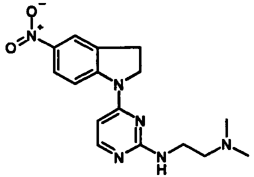 Obtained by reacting a mixture of 1-(4-chloro-pyrimidin-2-yl)-5-nitro-2,3-dihydro-1H-indole and 1-(2-chloro-pyrimidin-4-yl)-5-nitro-2,3-dihydro-1H-indole (Example XXVIII (7)). The reaction lasts 30 minutes. The crude product is separated into the two products by chromatography on aluminium oxide.
   Mass spectrum (ESI⁺): m/z = 329 [M+H]⁺ (N,N-dimethyl-N'-[2-(5-nitro-2,3-dihydro-indol-1-yl)-pyrimidin-4-yl]-ethan-1,2-diamine)
   and
   Mass spectrum (ESI⁺): m/z = 329 [M+H]⁺ (N,N-dimethyl-N'-[4-(5-nitro-2,3-dihydro-indol-1-yl)-pyrimidin-2-yl]-ethan-1,2-diamine)
(2) N,N-dimethyl-N'-[6-(5-nitro-2,3-dihydro-indol-1-yl)-pyridazin-3-yl]-ethan-1,2-diamine 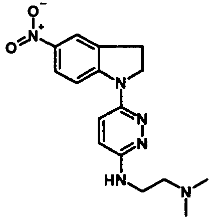 The reaction lasts 30 minutes. The crude product is purified by chromatography on aluminium oxide.
   Mass spectrum (ESI⁺): m/z = 329 [M+H]⁺
(3) N*1*-[6-(5-nitro-2,3-dihydro-indol-1-yl)-pyridazin-3-yl]-ethan-1,2-diamine 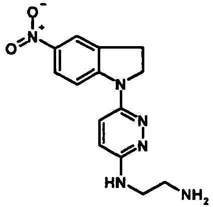 The reaction lasts 30 minutes. The crude product is added to water. The solid is suction filtered, dissolved in methanol and diluted with dichloromethane. After drying with magnesium sulphate the solvents are eliminated in vacuo.
   Mass spectrum (ESI⁺): m/z = 301 [M+H]⁺
(4) N*1*-[6-(5-nitro-indol-1-yl)-pyridazin-3-yl]-ethan-1,2-diamine 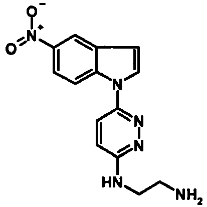 The reaction lasts 30 minutes. The crude product is added to water. The solid is suction filtered, dissolved in methanol and diluted with dichloromethane. After drying with magnesium sulphate the solvents are eliminated in vacuo.
   Mass spectrum (ESI⁺): m/z = 299 [M+H]⁺
(5) 5'-(5-nitro-indol-1-yl)-3,4,5,6-tetrahydro-2H-[1,2']bipyrazinyl 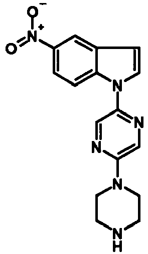 Mass spectrum (ESI⁺): m/z = 325 [M+H]⁺
(6) N*1*-[2-(5-nitro-indol-1-yl)-pyrimidin-4-yl]-ethan-1,2-diamine 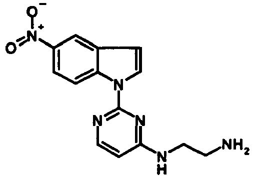 The reaction lasts 30 minutes. The crude product is added to water. The solid is suction filtered, dissolved in methanol and diluted with dichloromethane. After drying with magnesium sulphate the solvents are eliminated in vacuo.
   Mass spectrum (ESI⁺): m/z = 299 [M+H]⁺
(7) 5-nitro-1-(6-piperazin-1-yl-pyridazin-3-yl)-1H-indole 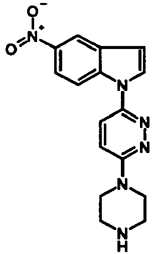 Mass spectrum (ESI⁺): m/z = 325 [M+H]⁺
(8) methyl-[4-(5-nitro-indol-1-yl)-pyrimidin-2-yl]-amine 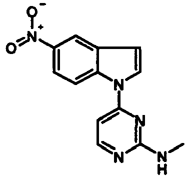 The reaction lasts 20 minutes at 120°C. The crude product is extracted from dichloromethane.
   Mass spectrum (ESI⁺): m/z = 270 [M+H]⁺
(9) N*1*-[4-(5-nitro-indol-1-yl)-pyrimidin-2-yl]-ethan-1,2-diamine 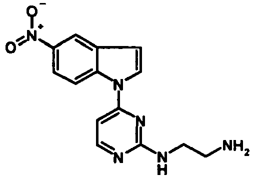 The reaction lasts 10 minutes at 80°C. The crude product is added to water. The solid is suction filtered and dried.
   Mass spectrum (ESI⁺): m/z = 299 [M+H]⁺

### Example XXX

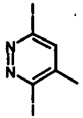

### 3,6-diiodo-4-methyl-pyridazine

5 g 3,6-dichloro-4-methyl-pyridazine are dissolved in 60 ml of a 67% solution of HI in water. The mixture is heated to 150°C for 45 minutes, cooled to ambient temperature and then the solution is added to 600 ml of 4 N NaOH. Then it is decanted from the insoluble oil. The oil is taken up in 100 ml of ethyl acetate and 100 ml petroleum ether are added thereto. After brief treatment in the ultrasound bath the solid formed is suction filtered (1.5 g). The mother liquor is evaporated down in vacuo and the residue is chromatographed on silica gel (cyclohexane/ ethyl acetate 10:1 to 1:3), to obtain another 220 mg product.
Yield: 1.72 g (16 % of theory)
Mass spectrum (ESI⁺): m/z = 347 [M+H]⁺

### Example XXXI

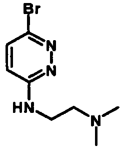

### N'-(6-bromo-pyridazin-3-yl)-N,N-dimethyl-ethan-1,2-diamine

483 mg N'-(6-chloro-pyridazin-3-yl)-N,N-dimethyl-ethan-1,2-diamine are dissolved in 5.5 ml HBr solution (48 % in water). The mixture is heated for 12 hours to 100°C. It is carefully diluted with water and then adjusted to pH 9 with 1 N NaOH. Then it is extracted 3 times with ethyl acetate and the combined organic phases are then washed with saturated sodium chloride solution. After drying with magnesium sulphate the solvents are eliminated in vacuo.
Yield: 314 mg (53 % of theory)
Mass spectrum (ESI⁺): m/z = 245 [M+H]⁺

### Example XXXII

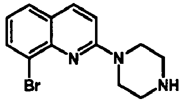

### 8-bromo-2-piperazin-1-yl-quinoline

670 mg 8-bromo-2-chloro-quinoline and 920 mg piperazine are dissolved in 9 ml of n-butanol and refluxed for 4 hours. Then the solvent is eliminated in vacuo, the residue is dissolved in ethyl acetate and the organic phase is washed with saturated sodium hydrogen carbonate solution and saturated sodium chloride solution. After drying with magnesium sulphate the solvent is eliminated in vacuo and the residue is chromatographed on silica gel with dichloromethane/methanol 5:1.
Yield: 625 mg (77 % of theory)
Mass spectrum (ESI⁺): m/z = 292 [M+H]⁺

### Example XXXIII

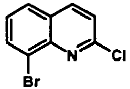

### 8-bromo-2-chloro-quinoline

2.8 g 8-bromo-1H-quinolin-2-one are dissolved in 20 ml phosphorus oxychloride and refluxed for 90 minutes. Sufficient concentrated ammonia solution is added to give an alkaline pH. The solid is suction filtered, washed with water and dried.
Yield: 2.8 g (92 % of theory)
melting point: 115°C

The following compounds are obtained analogously to Example XLIII:
(1) 3-chloro-5-trifluoromethyl-pyridazine 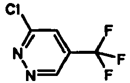 Mass spectrum (ESI⁺): m/z = 183 [M+H]⁺
   R_{f} value: 0.75 (silica gel; petroleum ether/ethyl acetate 2:1)

### Example XXXIV

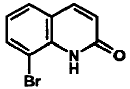

### 8-bromo-1H-quinolin-2-one

6 g N-(2-bromo-phenyl)-3-ethoxy-acrylamide are added batchwise to 30 ml concentrated sulphuric acid. After the addition has ended the mixture is stirred for 1 hour and then poured onto ice water. The solid is suction filtered and dried. The product thus obtained is chromatographed on silica gel (dichloromethane/ethyl acetate 3:1 to 1:1).
Yield: 2.95 g (59 % of theory)
melting point: 186°C

### Example XXXV

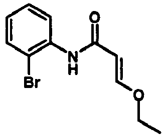

### N-(2-bromo-phenyl)-3-ethoxy-acrylamide

20 g ethyl 3-ethoxy-acrylate are dissolved in 160 ml of 2 N sodium hydroxide solution and refluxed for 4 hours. The mixture is cooled to ambient temperature and then adjusted to pH 3 with 4 N HCl. The precipitated solid is filtered off and dried. The mother liquor is extracted with ethyl acetate. The organic phase is treated with activated charcoal and sodium sulphate, filtered and evaporated down in vacuo. A total of 9.2 g product are thus obtained, which are dissolved in 120 ml of toluene. 6.9 ml of thionyl chloride are added and the mixture is refluxed for 3 hours. The solvents are eliminated in vacuo, the crude product is taken up in 60 ml of pyridine and cooled to 0°C. 11.6 g of 2-bromoaniline are added, the mixture is stirred for 1 hour at 0°C and for 2 hours at ambient temperature. Then it is diluted with water and the aqueous phase is extracted with ethyl acetate. The organic phase is washed twice with water and twice with 1 N hydrochloric acid. Activated charcoal and sodium sulphate are added, the mixture is filtered and the solvents are eliminated in vacuo. The residue is recrystallised from isopropanol/water.
Yield: 6.5 g (36 % of theory)
melting point: 98°C

### Example XXXVI

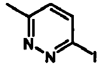

### 3-iodo-6-methyl-pyridazine

2.5 g 3-chloro-6-methyl-pyridazine are suspended in 10 ml of 67% HI solution. The mixture is heated to 120°C for 2 hours, cooled to ambient temperature and neutralised with 1 N NaOH solution. Then it is extracted with ethyl acetate and the organic phase is washed twice with water and once with saturated sodium chloride solution. After drying with magnesium sulphate the solvent is eliminated in vacuo.
Yield: 3.6 g (84 % of theory)
Mass spectrum (ESI⁺): m/z = 221 [M+H]⁺

The following compounds are obtained analogously to Example XXXVI:
(1) 3,6-diiodo-pyridazine 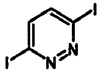 The reaction is carried out for 30 minutes at 150°C. Then the mixture is added to ice water, neutralised with 40% sodium hydroxide solution and the precipitated solid is suction filtered. The solid is dissolved in dichloromethane, dried on magnesium sulphate and the solvent is eliminated in vacuo. The crude product thus obtained is extracted from diethyl ether.
   Mass spectrum (ESI⁺): m/z = 333 [M+H]⁺
(2) 3-iodo-5-trifluoromethyl-pyridazine 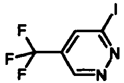 3-chloro-5-trifluoromethyl-pyridazine is refluxed in acetone in the presence of 2.9 equivalents of sodium iodide and two drops of conc. hydriodic acid for approx. 20 minutes.
   R_{f} value: 0.53 (silica gel; petroleum ether/ethyl acetate 5:1)
(3) 3-iodo-5-(thiazol-2-yl)-pyridazine 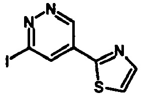 3-chloro-5-(thiazol-2-yl)-pyridazine is refluxed in acetone in the presence of 2.6 equivalents of sodium iodide and one drop of conc. hydriodic acid for approx. 20 minutes.

### Example XXXVII

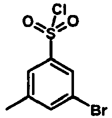

### 3-bromo-5-methyl-phenylsulphonyl-chloride

1.3 g 3-bromo-5-methyl-phenylamine are dissolved in 3 ml concentrated HCl and cooled to 0°C. To this a solution of 560 mg sodiumnitrite in 1 ml of water is added dropwise. The solution thus prepared is added dropwise at 0°C to a solution of 280 mg of copper-II-chloride, 0.6 ml of water and 7 ml of sulphur dioxide in glacial acetic acid (30%). The cooling bath is removed and the mixture is stirred for 15 minutes at ambient temperature. Then it is diluted with ice water, the solid is suction filtered and dried in the desiccator. The product is further reacted directly in Example XI without further purification.
Yield: 1.354 g (72 % of theory)

The following compounds are obtained analogously to Example XXXVII:
(1) 3-chloro-5-methyl-phenylsulphonyl-chloride 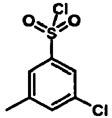 The product is further reacted directly in Example XI without further purification.
(2) 3,5-dibromo-phenylsulphonyl-chloride 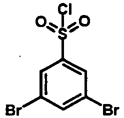 The product is further reacted directly in Example XI without further purification.
(3) 3-bromo-5-chloro-phenylsulphonyl-chloride 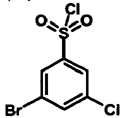 The aqueous phase is extracted with ethyl acetate. The organic phase is washed with saturated sodium chloride solution. After drying with magnesium sulphate the solvent is eliminated in vacuo, the residue is taken up in toluene and this is in turn eliminated in vacuo.
   The product is further reacted directly in Example XI without further purification.

### Example XXXVIII

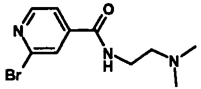

### 2-bromo-N-(2-dimethylamino-ethyl)-isonicotinamide

5 g 2-bromopyridine-4-carboxylic acid are dissolved in 40 ml of tetrahydrofuran. 4.4 g of carbonyldiimidazole are added thereto and the mixture is stirred for 3 hours at ambient temperature and for 1 hour at 40°C. Then 20 ml of this solution are added dropwise to a solution of 1.2 ml N,N-dimethylethylenediamine in 10 ml of tetrahydrofuran. After stirring overnight the mixture is evaporated down in vacuo and the residue is divided between ethyl acetate and saturated sodium hydrogen carbonate solution. The organic phase is washed with saturated sodium chloride solution and dried on magnesium sulphate. After elimination of the solvents in vacuo the residue is chromatographed on silica gel with dichloromethane/methanol 5:1. The product thus obtained is chromatographed on aluminium oxide with ethyl acetate.
Yield: 1.7 g (62 % of theory)
Mass spectrum (ESI⁺): m/z = 272 [M+H]⁺

The following compounds are obtained analogously to Example XXXVIII:
(1) tert-butyl 4-(2-bromo-pyridin-4-carbonyl)-piperazine-1-carboxylate 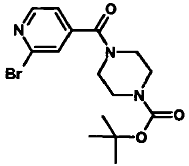 The crude product is extracted from a little diethyl ether. The product thus obtained is chromatographed on silica gel.
   Mass spectrum (ESI⁺): m/z = 370 [M+H]⁺
(2) (2-bromo-pyridin-4-yl)-morpholin-4-yl-methanone 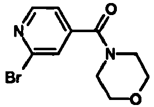 Mass spectrum (ESI⁺): m/z = 271 [M+H]⁺
(3) 2-bromo-N-(2-hydroxy-ethyl)-isonicotinamide 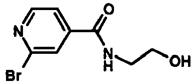 Mass spectrum (ESI⁺): m/z = 245 [M+H]⁺
(4) 2-bromo-N-methyl-isonicotinamide 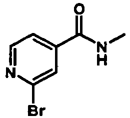 The reaction is carried out at ambient temperature.
   Mass spectrum (ESI⁺): m/z = 215 [M+H]⁺
(5) 2-bromo-N-(2-methoxy-ethyl)-isonicotinamide 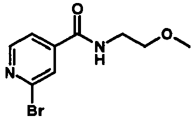 The reaction is carried out at ambient temperature.
   Mass spectrum (ESI⁻): m/z = 257 [M-H]⁻
(6) 2-bromo-isonicotinamide 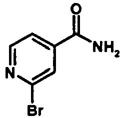 The reaction is carried out at ambient temperature.
   Mass spectrum (ESI⁻): m/z = 199 [M-H]⁻
(7) 2-bromo-N-cyclopropyl-isonicotinamide 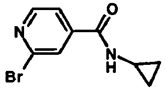 The reaction is carried out at ambient temperature. After the reaction has ended the mixture is diluted with ethyl acetate, washed once with 1 N hydrochloric acid, dried on magnesium sulphate and the solvent is eliminated in vacuo.
   Mass spectrum (ESI⁺): m/z = 241 [M+H]⁺
(8) 2-bromo-N-methoxy-N-methyl-isonicotinamide 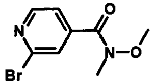 N,O-dimethylhydroxylamine-hydrochloride is used. The reaction is carried out at ambient temperature in the presence of 0.9 equivalents triethylamine. After the reaction has ended the mixture is diluted with ethyl acetate, washed once with 1 N hydrochloric acid, dried on magnesium sulphate and the solvent is eliminated in vacuo.
   Mass spectrum (ESI⁺): m/z = 245 [M+H]⁺
(9) 2-bromo-N-(2,2-dimethoxy-ethyl)-isonicotinamide 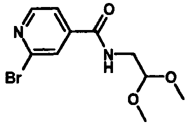 The coupling with the amine is carried out in tetrahydrofuran/toluene 2:1 at 100°C for 4 hours.
   Mass spectrum (ESI⁺): m/z = 289 [M+H]⁺

### Example XXXIX

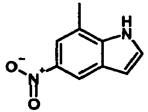

### 7-methyl-5-nitro-1H-indole

113 mg 2-ethynyl-6-methyl-4-nitro-phenylamine are dissolved in 5 ml N-methylpyrrolidone. To this are added 180 mg potassium-tert.-butoxide and the mixture is stirred for 8 hours at ambient temperature. Then it is divided between water and ethyl acetate, the aqueous phase is extracted once with ethyl acetate and the combined organic phases are dried on magnesium sulphate. The solvents are eliminated in vacuo and the residue is chromatographed on silica gel (cyclohexane/ ethyl acetate 10:1 to 1:2).
Yield: 65 mg (58 % of theory)
Mass spectrum (ESI⁻): m/z = 175 [M-H]⁻

The following compounds are obtained analogously to Example XXXIX:
(1) 6-methyl-5-nitro-1H-indole 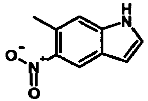 Mass spectrum (ESI⁻): m/z = 175 [M-H]⁻

### Example XL

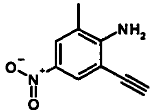

### 2-ethynyl-6-methyl-4-nitro-phenylamine

160 mg 2-methyl-4-nitro-6-trimethylsilanylethynyl-phenylamine are dissolved in 3 ml of methanol. 142 mg potassium carbonate are added to this and the mixture is stirred for 5 hours at ambient temperature. Then it is divided between water and ethyl acetate, the aqueous phase is extracted once with ethyl acetate and the combined organic phases are dried on magnesium sulphate. The solvents are eliminated in vacuo.
Yield: 113 mg (100 % of theory)
Mass spectrum (ESI⁺): m/z = 177 [M+H]⁺

The following compounds are obtained analogously to Example XL:
(1) 2-ethynyl-5-methyl-4-nitro-phenylamine 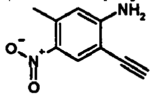 Mass spectrum (ESI⁺): m/z = 177 [M+H]⁺

### Example XLI

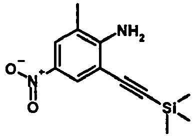

### 2-methyl-4-nitro-6-trimethylsilanylethynyl-phenylamine

250 mg 2-iodo-6-methyl-4-nitro-phenylamine are dissolved in 5 ml of tetrahydrofuran. 17 mg copper-I-iodide and 63 mg bis-triphenylphosphine-palladium dichloride are added under argon. Then 635 µl trimethylsilylacetylene and 626 µl triethylamine are added. The mixture is stirred for 2 hours at ambient temperature, diluted with diethyl ether and the insoluble constituents are filtered off. Then the solvents are eliminated in vacuo and the residue is chromatographed on silica gel (cyclohexane/ethyl acetate 10:1 to 1:1).
Yield: 172 mg (73 % of theory)
Mass spectrum (ESI⁺): m/z = 249 [M+H]⁺

The following compounds are obtained analogously to Example XLI:
(1) 5-methyl-4-nitro-2-trimethylsilanylethynyl-phenylamine 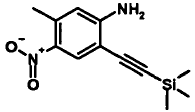 Mass spectrum (ESI⁺): m/z = 249 [M+H]⁺

### Example XLII

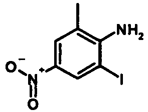

### 2-iodo-6-methyl-4-nitro-phenylamine

2.3 g iodine are dissolved in 20 ml of ethanol, 2 g silver sulphate and 1 g 2-methyl-4-nitro-phenylamine are added. The mixture is stirred for 4 hours at ambient temperature, the solvent is eliminated in vacuo and divided between saturated sodium thiosulphate solution and dichloromethane. The organic phase is washed once with saturated sodium chloride solution and dried on magnesium sulphate. Then the solvents are eliminated in vacuo and the residue is extracted from diisopropylether/petroleum ether.
Yield: 1.62 g (89 % of theory)
Mass spectrum (ESI⁺): m/z = 279 [M+H]⁺

The following compounds are obtained analogously to Example XLII:
(1) 2-iodo-5-methyl-4-nitro-phenylamine 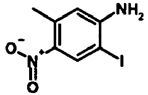 The crude product is purified by chromatography on silica gel.
   Mass spectrum (ESI⁺): m/z = 279 [M+H]⁺

### Example XLIII

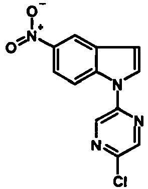

### 1-(5-chloro-pyrazin-2-yl)-5-nitro-1H-indole

600 mg 5-nitro-1-(4-oxy-pyrazin-2-yl)-1H-indole are added batchwise to 4 ml of phosphorus oxychloride warmed to 60°C. After the addition has ended the mixture is heated to 100°C for 3 hours. After cooling to ambient temperature it is added to ice water and the aqueous phase is extracted once with dichloromethane. The organic phase is washed with saturated sodium chloride solution and then dried on magnesium sulphate. After elimination of the solvent in vacuo the residue is chromatographed on silica gel (cyclohexane/ethyl acetate 10:1 to 1:1).
Yield: 140 mg (22 % of theory)
Mass spectrum (ESI⁺): m/z = 275 [M+H]⁺

### Example XLIV

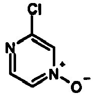

### 2-chloro-pyrazin-4-oxide

10 ml chloropyrazine are dissolved in 10 ml of peracetic acid (39% in glacial acetic acid) and heated to 80°C for 24 hours. Then 10 ml of 35% hydrogen peroxide solution are added and the mixture is heated to 80°C for another 24 hours. After cooling to ambient temperature, sufficient sodium sulphide is carefully added until there is no further development of gas. Then the mixture is diluted with 500 ml dichloromethane. After drying with magnesium sulphate the solvent is eliminated in vacuo and the residue is chromatographed on silica gel with dichloromethane/methanol 99:1. The product thus obtained is extracted from petroleum ether.
Yield: 5.8 g (40 % of theory)
Mass spectrum (ESI⁺): m/z = 131 [M+H]⁺

### Example XLV

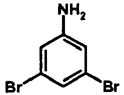

### 3,5-dibromo-phenylamine

5.95 g 1,3-dibromo-5-nitrobenzene are dissolved in 40 ml of ethanol. 23.9 g of tin-II-chloride dihydrate are added and the mixture is slowly heated to reflux temperature. Once reflux temperature has been reached it is maintained for another 30 minutes. Then the solvent is eliminated in vacuo and the residue is made alkaline with 4 N NaOH. Ethyl acetate is added and the mixture is filtered through kieselguhr. The filter cake is washed thoroughly with ethyl acetate. Then the aqueous phase is extracted 3 times with ethyl acetate and the combined organic phases are washed with saturated sodium chloride solution. After drying with magnesium sulphate the solvent is eliminated in vacuo and the residue is chromatographed on silica gel (cyclohexane/ethyl acetate 85:15 to 70:30).
Yield: 4.53 g (85 % of theory)
R_{f} value: 0.35 (silica gel, petroleum ether/ethyl acetate 5:1)

### Example XLVI

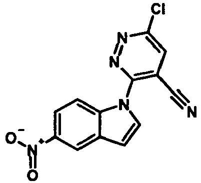

### 6-chloro-3-(5-nitro-indol-1-yl)-pyridazin-4-carbonitrile

200 mg 5-nitro-1H-indole are dissolved in 3 ml N-methylpyrrolidone and combined with 131 mg of potassium-tert.-butoxide. The mixture is stirred for 10 minutes at ambient temperature and then 214 mg 3,6-dichloro-pyridazine-4-carbonitrile in 2 ml N-methylpyrrolidone are added dropwise. The reaction mixture is heated to 65°C for 2 hours, divided between water and ethyl acetate and the aqueous phase is extracted twice with ethyl acetate. The combined organic phases are washed with saturated sodium chloride solution and dried on magnesium sulphate. Then the solvents are eliminated in vacuo and the residue is chromatographed on silica gel (cyclohexane/ethyl acetate 10:1 to 1:3). The product thus obtained is extracted from ethyl acetate.
Yield: 105 mg (28 % of theory)
Mass spectrum (ESI⁺): m/z = 317 [M+NH₄]⁺

The following compounds are obtained analogously to Example XLVI:
(1) tert-butyl 4-[5-cyano-6-(5-nitro-indol-1-yl)-pyridazin-3-yl]-piperazine-1-carboxylate 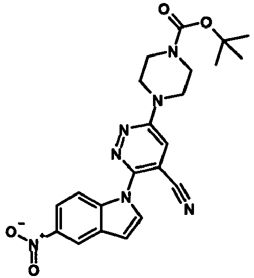 Obtained by reacting 6-chloro-3-(5-nitro-indol-1-yl)-pyridazin-4-carbonitrile and tert-butyl piperazine-1-carboxylate. The reaction lasts 2 hours at 70°C.
   Mass spectrum (ESI⁺): m/z = 450 [M+H]⁺

### Example XLVII

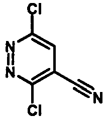

### 3,6-dichloro-pyridazin-4-carbonitrile

2.6 g 3,6-dichloro-pyridazine-4-carboxylic acid-amide are dissolved in 40 ml dichloromethane and cooled to 0°C. 7.6 ml triethylamine and then 3.9 ml trifluoroacetic anhydride are added. Then the mixture is stirred for 1 hour at 0°C and for 1 hour at ambient temperature. It is then divided between dichloromethane and saturated sodium hydrogen carbonate solution. The aqueous phase is extracted once with dichloromethane and the combined organic phases are washed once with saturated sodium chloride solution. After drying with magnesium sulphate the solvents are eliminated in vacuo and the residue is chromatographed on silica gel (cyclohexane/ethyl acetate 10:1 to 1:2).
Yield: 1.9 g (81 % of theory)
Mass spectrum (EI): m/z = 173 [M]⁺

The following compounds are obtained analogously to Example XLVII:
(1) 2-bromo-4-cyano-pyridine 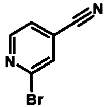 R_{f} value: 0.7 (silica gel: cyclohexane/ethyl acetate 2:1)

### Example XLVIII

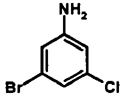

### 3-bromo-5-chloro-phenylamine

10.4 g 1-bromo-3-chloro-5-nitro-benzene are dissolved in 200 ml of tetrahydrofuran and 50 ml of methanol. 1 g Raney nickel is added and the mixture is hydrogenated for 32 hours at 50 psi and ambient temperature. Then the catalyst is suction filtered and the solvent is eliminated in vacuo.
Yield: 9.25 g (102 % of theory)
R_{f} value: 0.40 (silica gel, petroleum ether/ethyl acetate 5:1)

### Example XLIX

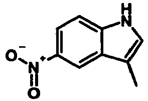

### 3-methyl-5-nitro-1H-indole

1.75 g N-(4-nitro-phenyl)-N'-propylidene-hydrazine are dissolved in 50 ml of toluene and to this are added 10 ml of an 85 % phosphoric acid solution. The mixture is heated to 100°C for 3 hours and then the toluene phase is decanted off. Then another 50 ml of toluene are added and the mixture is heated to 100°C for 12 hours. The toluene phase is decanted off again and combined with the first toluene phase. The combined toluene phases are washed successively with water and saturated sodium chloride solution. After drying with magnesium sulphate the solvent is eliminated in vacuo and the residue is chromatographed on silica gel with cyclohexane/ethyl acetate (100:0 to 50:50).
Yield: 509 mg (32 % of theory)
Mass spectrum (ESI⁺): m/z = 177 [M+H]⁺

The following compounds are obtained analogously to Example XLIX:
(1) 3-ethyl-5-nitro-1H-indole 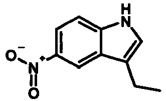 Mass spectrum (ESI⁺): m/z = 191 [M+H]⁺

### Example L

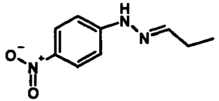

### N-(4-nitro-phenyl)-N'-propylidene-hydrazine

2 g 4 nitrophenylhydrazine are suspended in 8 ml of ethanol and 1 ml of propionaldehyde is added. The mixture is stirred for 12 hours at ambient temperature, the solvent is eliminated in vacuo and the residue is extracted from diisopropylether.
Yield: 1.75 g (69 % of theory)
Mass spectrum (ESI⁺): m/z = 194 [M+H]⁺

The following compounds are obtained analogously to Example L:
(1) N-butyliden-N'-(4-nitro-phenyl)-hydrazine 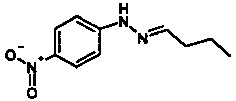 Mass spectrum (ESI⁺): m/z = 208 [M+H]⁺

### Example LI

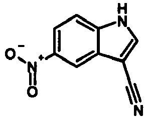

### 5-nitro-1H-indol-3-carbonitrile

1 g 5-nitroindole-3-carboxaldehyde is suspended in 40 ml formic acid and to this are added 510 mg hydroxylamine hydrochloride. The mixture is refluxed for 12 hours, allowed to cool to ambient temperature and adjusted to pH 5 with 4 N sodium hydroxide solution. Then the mixture is extracted with ethyl acetate and the organic phase is washed successively with water and saturated sodium chloride solution. After drying with magnesium sulphate the solvent is eliminated in vacuo and the residue is chromatographed on silica gel with cyclohexane/ethyl acetate (50:50 to 0:100).
Yield: 226 mg (23 % of theory)
Mass spectrum (ESI⁻): m/z = 186 [M-H]⁻

### Example LII

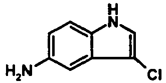

### 3-chloro-1H-indol-5-ylamine

2.17 g 3-chloro-5-nitro-1H-indole are dissolved in 120 ml acetone and 20 ml of water. 17 g of ammonium acetate are added and then within 0.5 hours 60 ml of a 20 % solution of titanium-(III)-chloride in 3 % hydrochloric acid are added dropwise. Then the mixture is stirred for another hour, added to 300 ml of water and neutralised with 4 N sodium hydroxide solution. Then it is extracted 3 times with ethyl acetate and the combined organic phases are washed with saturated sodium chloride solution. After drying with magnesium sulphate the solvent is eliminated in vacuo.
Yield: 2.12 g (115 % of theory)
Mass spectrum (ESI⁺): m/z = 167 [M+H]⁺

### Example LIII

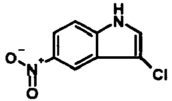

### 3-chloro-5-nitro-1H-indole

2 g 5-nitroindole are dissolved in 20 ml dimethylformamide. 2 g N-bromosuccinimide are added batchwise thereto and the mixture is stirred for 12 hours at ambient temperature. Then it is added to 200 ml of water, the precipitate is suction filtered and washed with a little diethyl ether. The solid is dried in vacuo.
Yield: 2.17 g (90 % of theory)
Mass spectrum (ESI⁺): m/z = 197 [M+H]⁺

The following compounds are obtained analogously to Example LIII:
(1) tert.butyl [(3-chloro-1H-indol-5-yl)-(3,5-dichloro-phenylsulphonyl)-amino]-acetate 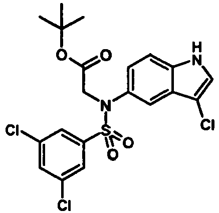 Mass spectrum (ESI⁺): m/z = 506 [M+NH₄]⁺
(2) 3-iodo-5-nitro-1H-indole 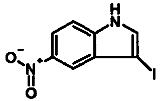 Mass spectrum (ESI⁺): m/z = 289 [M+H]⁺

### Example LIV

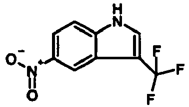

### 5-nitro-3-trifluoromethyl-1H-indole

1.7 g 3-iodo-5-nitro-1H-indole are dissolved in 20 ml dimethylformamide. To this are added 1.5 ml of methyl fluorosulphonyl-2,2-difluoroacetate and 150 mg copper-I-iodide. Then the mixture is heated to 80°C for 4 hours. After cooling to ambient temperature it is divided between water and ethyl acetate. The aqueous phase is extracted with ethyl acetate and the combined organic phases are dried on magnesium sulphate. The solvents are eliminated in vacuo and the residue is chromatographed on silica gel with cyclohexane/ethyl acetate (100:0 to 50:50).
Yield: 612 mg (45 % of theory)
Mass spectrum (ESI⁻): m/z = 229 [M-H]⁻

### Example LV

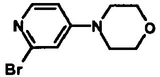

### 4-(2-bromo-pyridin-4-yl)-morpholine

1 g of 4-amino-2-bromo-pyridine is combined with 0.59 g sodium hydride (approx. 60% in mineral oil) under an argon atmosphere in dry N,N-dimethylformamide while cooling with an ice bath. After five minutes 0.70 ml of bis(2-chloroethyl)ether are pipetted in. The reaction mixture is heated to ambient temperature overnight, combined with ethyl acetate and washed with water. The organic phase is dried on magnesium sulphate and evaporated down. The resin-like flask residue is stirred with a little ethyl acetate and suction filtered. The crude product is chromatographed through a silica gel column with cyclohexane/ ethyl acetate (60:40 to 40:60).
Yield: 870 mg (62% of theory)
Mass spectrum (ESI⁺): m/z = 243 [M+H]⁺
R_{f} value: 0.32 (silica gel: ethyl acetate/petroleum ether 1:1)

The following compounds are obtained analogously to Example LV:
(1) 4-(6-bromo-pyridin-3-yl)-morpholine 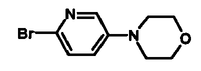 Mass spectrum (ESI⁺): m/z = 243 [M+H]⁺
   R_{f} value: 0.47 (silica gel: ethyl acetate/petroleum ether 1:1)

### Example LVI

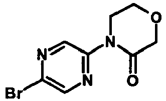

### 4-(5-bromo-pyrazin-2-yl)-morpholin-3-one

160 mg N-(5-bromo-pyrazin-2-yl)-2-(2-chloro-ethoxy)-acetamide and 323 mg caesium carbonate are stirred in 5 ml acetonitrile overnight at ambient temperature. Then the reaction mixture is evaporated down, combined with ethyl acetate and washed with semisaturated sodium chloride solution. The organic phase is dried on magnesium sulphate and evaporated down. The flask residue is stirred with a little tert-butylmethylether, suction filtered and dried.
Yield: 110 mg (79% of theory)
Mass spectrum (ESI⁺): m/z = 258 [M+H]⁺
R_{f} value: 0.52 (silica gel: ethyl acetate/petroleum ether 1:1)

The following compounds are obtained analogously to Example LVI:
(1) 4-(6-bromo-pyridazin-3-yl)-morpholin-3-one 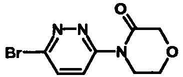 Mass spectrum (ESI⁺): m/z = 258 [M+H]⁺
   R_{f} value: 0.35 (silica gel: ethyl acetate/petroleum ether 1:1)
(2) 4-(6-bromo-pyridin-3-yl)-morpholin-3-one 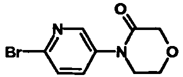 Mass spectrum (ESI⁺): m/z = 257 [M+H]⁺
   R_{f} value: 0.17 (silica gel: ethyl acetate/petroleum ether 1:1)
(3) 4-(2-bromo-pyridin-4-yl)-morpholin-3-one 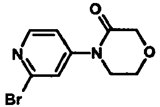
Mass spectrum (ESI⁺): m/z = 257 [M+H]⁺
R_{f} value: 0.24 (silica gel: ethyl acetate/petroleum ether 1:1)

### Example LVII

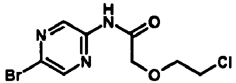

### N-(5-bromo-pyrazin-2-yl)-2-(2-chloro-ethoxy)-acetamide

5 ml of a 0.5 M solution of (2-chloroethoxy)-acetyl chloride in tetrahydrofuran are added dropwise to 500 mg 2-amino-5-bromo-pyrazine and 1 ml triethylamine in 5 ml of tetrahydrofuran while cooling with an ice bath. The reaction mixture is stirred overnight at ambient temperature, then a total of 5 ml (2-chloroethoxy)-acetyl chloride (0.5 M in tetrahydrofuran) and 1 ml triethylamine are added again. After another 48 h at ambient temperature the reaction mixture is combined with ethyl acetate and washed with 1 N hydrochloric acid, saturated sodium hydrogen carbonate solution and saturated sodium chloride solution. The organic phase is dried on magnesium sulphate, evaporated down and chromatographed through a silica gel column with cyclohexane/ethyl acetate (70:30 to 60:40).
Yield: 165 mg (20% of theory)
R_{f} value: 0.58 (silica gel: ethyl acetate/petroleum ether 1:1)

The following compounds are obtained analogously to Example LVII:
(1) N-(6-bromo-pyridazin-3-yl)-2-(2-chloro-ethoxy)-acetamide 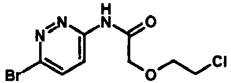 Mass spectrum (ESI⁺): m/z = 294 [M+H]⁺
   R_{f} value: 0.46 (silica gel: ethyl acetate/petroleum ether 1:1)
(2) N-(6-bromo-pyridin-3-yl)-2-(2-chloro-ethoxy)-acetamide 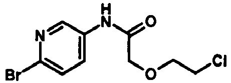 R_{f} value: 0.70 (silica gel: dichloromethane/methanol 95:5)
(3) N-(2-bromo-pyridin-4-yl)-2-(2-chloro-ethoxy)-acetamide 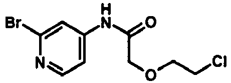 R_{f} value: 0.68 (silica gel: dichloromethane/methanol 95:5)

### Example LVIII

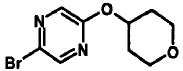

### 2-bromo-5-(tetrahydro-pyran-4-yloxy)-pyrazine

66 mg sodium hydride (55% in mineral oil) are added at ambient temperature to 300 mg 2,5-dibromo-pyrazine and 135 mg 4-hydroxy-tetrahydro-pyran in 6 ml of tetrahydrofuran and the reaction mixture is stirred for five hours at ambient temperature. Then it is diluted with copious amounts of tert-butylmethylether, washed with saturated sodium chloride solution, dried on magnesium sulphate and evaporated down. The solid flask residue is gently heated with petroleum ether, cooled in the ice bath cooled, suction filtered and washed with a little petroleum ether. The filtrate is evaporated down, the residue is stirred with a little n-hexane, cooled in the ice bath, suction filtered and washed with a little n-hexane. The combined filter cakes are dried.
Yield: 246 mg (75% of theory)
Mass spectrum (EI): m/z = 258 [M]⁺
R_{f} value: 0.50 (silica gel: petroleum ether/ethyl acetate 8:2)

The following compounds are obtained analogously to Example LVIII:
(1) (S)-2-bromo-5-(tetrahydro-furan-3-yloxy)-pyrazine 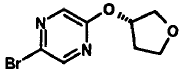 R_{f} value: 0.40 (silica gel: petroleum ether/ethyl acetate 8:2)
(2) (R)-2-bromo-5-(tetrahydro-furan-3-yloxy)-pyrazine 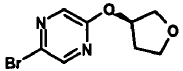 Mass spectrum (ESI⁺): m/z = 245 [M+H]⁺
   R_{f} value: 0.40 (silica gel: petroleum ether/ethyl acetate 8:2)
(3) (S)-3-iodo-6-(tetrahydro-furan-3-yloxy)-pyridazine 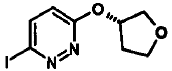 Mass spectrum (ESI⁺): m/z = 293 [M+H]⁺
   R_{f} value: 0.35 (silica gel: cyclohexane/ethyl acetate 7:3)
(4) 3-iodo-6-(tetrahydro-pyran-4-yloxy)-pyridazine 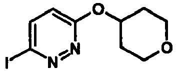 Mass spectrum (ESI⁺): m/z = 307 [M+H]⁺
   R_{f} value: 0.40 (silica gel: cyclohexane/ethyl acetate 7:3)
(5) (R)-3-iodo-6-(tetrahydro-furan-3-yloxy)-pyridazine 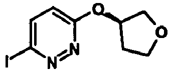 Mass spectrum (ESI⁺): m/z = 293 [M+H]⁺
   R_{f} value: 0.30 (silica gel: cyclohexane/ethyl acetate 7:3)
(6) 3-iodo-6-(2-dimethylamino)-ethoxy-pyridazine 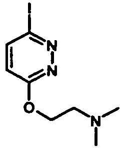 Once the reaction has ended the solvent is eliminated in vacuo and the residue is chromatographed on aluminium oxide.
   Mass spectrum (ESI⁺): m/z = 294 [M+H]⁺
(7) 3-iodo-6-benzyloxy-pyridazine 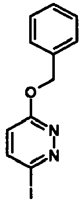 Mass spectrum (ESI⁺): m/z = 313 [M+H]⁺
(8) 3-iodo-6-[2-(tetrahydro-pyran-2-yloxy)-ethoxy]-pyridazine 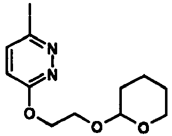 The reaction is carried out for 12 hours at ambient temperature and 3 hours at 60°C. After the reaction has ended the solvent is eliminated in vacuo and the residue is chromatographed on silica gel.
   Mass spectrum (ESI⁺): m/z = 351 [M+H]⁺
(9) 3-iodo-6-methylsulphanyl-pyridazine 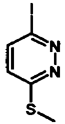 This is obtained analogously by reacting 3,6-diiodo-pyridazine with sodium thiomethoxide.
   Mass spectrum (ESI⁺): m/z = 253 [M+H]⁺
(10) 3-iodo-6-(2-phenyl-[1.3]dioxan-5-yloxy)-pyridazine 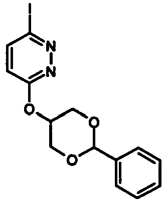 The reaction is carried out for 24 hours at ambient temperature. After the reaction has ended the solvent is eliminated in vacuo and the residue is chromatographed on silica gel.
   Mass spectrum (ESI⁺): m/z = 385 [M+H]⁺
(11) 2-bromo-5-methylsulphanyl-pyrazine 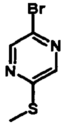 Is obtained analogously by reacting 2,5-dibromo-pyrazine with sodium thiomethoxide.
   R_{f} value: 0.43 (silica gel: cyclohexane/ethyl acetate 20:1)
(12) 2-bromo-5-(2-phenyl-[1.3]dioxan-5-yloxy)-pyrazine 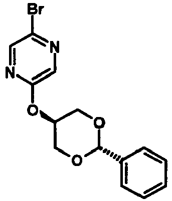 Is obtained in admixture with the cis-isomer by reacting 2,5-dibromopyrazine with 2-phenyl-[1,3]dioxan-5-ol. The stereoisomers are separated from one another by chromatography on silica gel (cyclohexane/ethyl acetate 90:10 to 50:50).
   Mass spectrum (ESI⁺): m/z = 337 [M+H]⁺
(13) [2-(5-bromo-pyrazin-2-yloxy)-ethyl]-dimethyl-amine 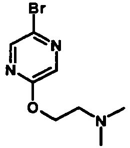 R_{f} value: 0.48 (aluminium oxide: cyclohexane/ethyl acetate 4:1)
(14) 2-iodo-4-methylsulphanyl-pyridine 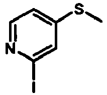 is obtained analogously by reacting 2,4-diiodo-pyridine with sodium thiomethoxide in dimethylformamide at 60°C.
   Mass spectrum (ESI⁺): m/z = 252 [M+H]⁺
(15) tert-butyl [2-(5-bromo-pyrazin-2-yloxy)-ethyl]-carbamate 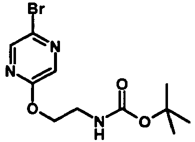 Mass spectrum (ESI⁺): m/z = 318 [M+H]⁺
(16) 2-bromo-4-ethylsulphanyl-pyridine 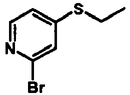 is obtained analogously by reacting 2-bromo-4-iodo-pyridine with sodium thiomethoxide in dimethylformamide at 80°C for 3 days.
   Mass spectrum (ESI⁺): m/z = 218 [M+H]⁺

### Example LIX

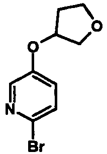

### 2-bromo-5-(tetrahydro-furan-3-yloxy)-pyridine

A mixture of 500 mg 2-bromo-5-hydroxy-pyridine and tetrahydro-furan-3-yl-toluene-4-sulphonate in 5 ml N,N-dimethylformamide is stirred for five hours in an oil bath warmed to 60°C, then another 0.2 ml tetrahydro-furan-3-yl-toluene-4-sulphonate is added and the mixture is stirred for another five hours at 80°C. For working up the reaction mixture is evaporated down and combined with ice water. The precipitate formed is suction filtered, washed with water and dried. The crude product is stirred with a little methanol, suction filtered, washed with a little methanol and dried in the desiccator.
Yield: 496 mg (71 % of theory)
Mass spectrum (ESI⁺): m/z = 244 [M+H]⁺

The following compounds are obtained analogously to Example LIX:
(1) 2-bromo-5-(tetrahydro-pyran-4-yloxy)-pyridine 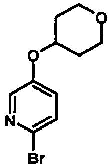 Mass spectrum (ESI⁺): m/z = 258 [M+H]⁺
   R_{f} value: 0.50 (silica gel: cyclohexane/ethyl acetate 1:1)
(2) 2-bromo-4-(tetrahydro-furan-3-yloxy)-pyridine 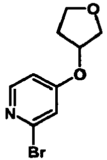 Mass spectrum (ESI⁺): m/z = 244 [M+H]⁺
   R_{f} value: 0.35 (silica gel: cyclohexane/ethyl acetate 1:1)
(3) 2-bromo-4-(tetrahydro-pyran-4-yloxy)-pyridine 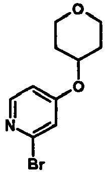 Mass spectrum (ESI⁺): m/z = 258 [M+H]⁺
   R_{f} value: 0.48 (silica gel: cyclohexane/ethyl acetate 1:1)

### Example LX

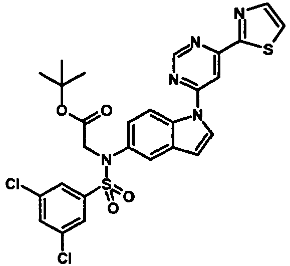

### tert.butyl {(3,5-dichloro-phenylsulphonyl)-[1-(6-thiazol-2-yl-pyrimidin-4-yl)-1H-indol-5-yl]-amino}-acetate

1.30 ml of a 0.5 M solution of 2-thiazolyl-zinc bromide in tetrahydrofuran and 20 mg tetrakis(triphenylphosphine)palladium(0) are added under argon to 150 mg tert.butyl {(3,5-dichloro-phenylsulphonyl)-[1-(6-iodo-pyrimidin-4-yl)-1H-indol-5-yl]-amino}-acetate. The reaction mixture refluxed for 15 minutes at reflux temperature and after cooling evaporated down in vacuo. The flask residue is chromatographed through a silica gel column with cyclohexane/ethyl acetate (80:20 to 60:40) as eluant.
Yield: 110 mg (78 % of theory)
Mass spectrum (ESI⁺): m/z = 616 [M+H]⁺
R_{f} value: 0.55 (silica gel: petroleum ether/ethyl acetate 2:1)

The following compounds are obtained analogously to Example LX:
(1) tert.butyl {(3,5-dichloro-phenylsulphonyl)-[1-(4-thiazol-2-yl-pyridin-2-yl)-1H-indol-5-yl]-amino}-acetate 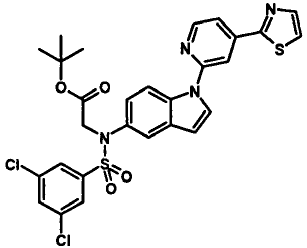 After the reaction has ended the mixture is divided between 1 N hydrochloric acid and ethyl acetate. The aqueous phase is extracted twice with ethyl acetate and the combined organic are washed once with saturated sodium hydrogen carbonate solution and once with saturated sodium chloride solution. After drying with magnesium sulphate the solvents are eliminated in vacuo and the residue is chromatographed on silica gel.
   Mass spectrum (ESI⁺): m/z = 615 [M+H]⁺
(2) tert.butyl ((3,5-dichloro-phenylsulphonyl)-{1-[4-(1-ethoxymethyl-1H-imidazol-2-yl)-pyridin-2-yl]-1H-indol-5-yl}-amino)-acetate 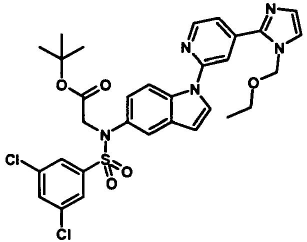 In order to prepare 1-ethoxymethyl-1H-imidazol-2yl-zinc chloride, 1-ethoxymethyl-2-iodo-1H-imidazole in tetrahydrofuran is combined at -78°C with 1 equivalent of n-butyllithium (2.5 N in n-hexane). After 30 minutes, 1 equivalent of a 0.5 N solution of zinc chloride in tetrahydrofuran is added and the mixture is left to come up to 0°C. Then the aryliodine compound and tetrakis(triphenylphosphine)palladium(0) are added and the mixture is refluxed for 5 hours. After the reaction has ended the mixture is divided between semisaturated sodium chloride solution and ethyl acetate. The aqueous phase is extracted twice with ethyl acetate and the combined organic phases are dried on magnesium sulphate. The solvents are eliminated in vacuo and the residue is chromatographed on silica gel.
   The product is further reacted directly (19).
(3) tert.butyl ((3,5-dichloro-phenylsulphonyl)-{1-[4-(1-methyl-1H-imidazol-2-yl)-pyridin-2-yl]-1H-indol-5-yl}-amino)-acetate 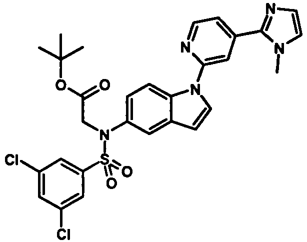 In order to prepare 1-methyl-1H-imidazol-2yl-zinc chloride, 1-methyl-2-iodo-1H-imidazole is combined in tetrahydrofuran at -78°C with 1 equivalent n-butyllithium (2.5 N in n-hexane). After 30 minutes 1 equivalent of a 0.5 N solution of zinc chloride in tetrahydrofuran is added and the mixture is allowed to come up to ambient temperature. Then the aryliodine compound and
   tetrakis(triphenylphosphine)palladium(0) are added and the mixture is refluxed for 2 hours. After the reaction has ended the mixture is divided between water and ethyl acetate. The organic phase is dried on magnesium sulphate. The solvents are eliminated in vacuo and the residue is chromatographed on silica gel.
   Mass spectrum (ESI⁺): m/z = 612 [M+H]⁺
(4) 2-(tetrahydro-pyran-2-yl)-5-(thiazol-2-yl)-2H-pyridazin-3-one 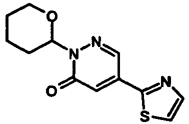 R_{f} value: 0.14 (silica gel: petroleum ether/ethyl acetate 2:1)
(5) tert.butyl {(3,5-dichloro-phenylsulphonyl)-[1-(2-thiazol-2-yl-pyrimidin-4-yl)-1H-indol-5-yl]-amino}-acetate 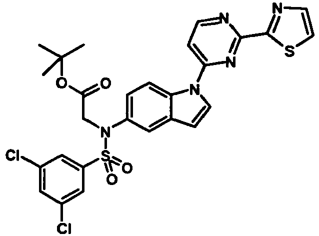 Mass spectrum (ESI⁺): m/z = 616 [M+H]⁺
   R_{f} value: 0.33 (silica gel: petroleum ether/ethyl acetate 1:2)
(6) tert.butyl ((3,5-dichloro-phenylsulphonyl)-{1-[6-(1-methyl-1H-imidazol-2-yl)-pyrimidin-4-yl]-1H-indol-5-yl}-amino)-acetate 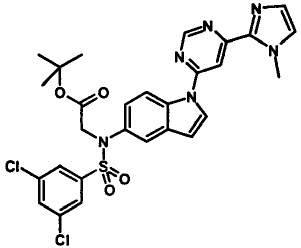 Mass spectrum (ESI⁺): m/z = 613 [M+H]⁺
   R_{f} value: 0.33 (silica gel: petroleum ether/ethyl acetate 1:2)
(7) tert.butyl {(2-chloro-6-methyl-pyridin-4-sulphonyl)-[1-(6-methyl-pyridazin-3-yl)-1H-indol-5-yl]-amino}-acetate 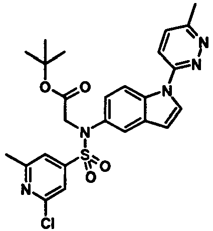 The compound is obtained from tert.butyl {(2,6-dichloro-pyridin-4-sulphonyl)-[1-(6-methyl-pyridazin-3-yl)-1H-indol-5-yl]-amino}-acetate and methylzinc chloride. It is refluxed for 4 hours.
   Mass spectrum (ESI⁺): m/z = 528 [M+H]⁺

### Example LXI

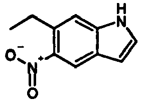

### 6-ethyl-5-nitro-1H-indole

11.49 ml boron trifluoride-diethyletherate are added dropwise to 5.00 g 6-ethyl-5-nitro-1H-indol-2,3-dione in 300 ml of tetrahydrofuran at -25°C under argon. Then 2.58 g sodium borohydride are added batchwise and the reaction mixture is stirred for one hour at -20°C. After being slowly heated to ambient temperature the reaction mixture is poured onto approx. 500 ml ice water and 300 ml tert-butylmethylether with stirring. The aqueous phase is extracted with tert-butylmethylether and the combined extracts are washed with water and saturated sodium chloride solution, dried on magnesium sulphate and evaporated down.
The flask residue is taken up in dichloromethane, filtered through a layer of aluminium oxide (activity stage II) and washed with dichloromethane. The yellow filtrate is evaporated down and the residue is stirred with petroleum ether and a little tert-butylmethylether, suction filtered, washed with petroleum ether and dried.
Yield: 2.43 g (56 % of theory)
Mass spectrum (ESI⁻): m/z = 189 [M-H]⁻
R_{f} value: 0.60 (silica gel: cyclohexane/ethyl acetate 1:1)

### Example LXII

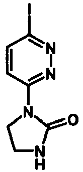

### 1-(6-iodo-pyridazin-3-yl)-imidazolidin-2-one

220 mg 3-iodo-6-(2-aminoethyl)-amino-pyridazine and 290 µl N,N-diisopropyl-N-ethyl-amine are dissolved in 5 ml dichloromethane. 440 µl of a 20% solution of phosgene in toluene is added dropwise thereto. The mixture is stirred for another 1 hour, diluted with dichloromethane and washed once with semisaturated sodium chloride solution. After drying with magnesium sulphate the solvent is eliminated in vacuo and the residue is chromatographed on silica gel.
Yield: 60 mg (25 % of theory)
Mass spectrum (ESI⁺): m/z = 291 [M+H]⁺

The following compounds are obtained analogously to Example LXII:
(1) tert-butyl 3-(6-iodo-pyridazin-3-yl)-2-oxo-imidazolidine-1-carboxylate 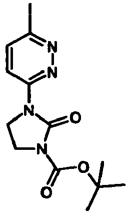 The crude product is extracted from diethyl ether.
   Mass spectrum (ESI⁺): m/z = 391 [M+H]⁺

### Example LXIII

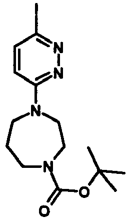

### tert-butyl 4-(6-iodo-pyridazin-3-yl)-[1,4]diazepan-1-carboxylate

500 mg 3,6-diiodo-pyridazine are dissolved in 3 ml dioxane. 220 mg potassium carbonate and 330 µl Boc-homopiperazine are added and the mixture is heated to 120°C for 24 hours. Then it is diluted with dichloromethane and filtered to remove the insoluble constituents. The mother liquor is evaporated down in vacuo and the residue is chromatographed on silica gel (cyclohexane/ethyl acetate 80:20 to 0:100).
Yield: 490 mg (80 % of theory)
Mass spectrum (ESI⁺): m/z = 405 [M+H]⁺

The following compounds are obtained analogously to Example LXIII:
(1) 3-iodo-6-(2-aminoethyl)-amino-pyridazine 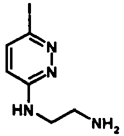 The reaction time is 48 hours. The crude product is further reacted directly.

### Example LXIV

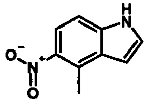

### 4-methyl-5-nitro-1H-indole

1 g 5-nitro-1H-indole is dissolved in 30 ml of tetrahydrofuran and cooled to - 10°C. To this is added dropwise a 3 N solution of methylmagnesium bromide in diethyl ether and the mixture is stirred for 30 minutes at -10°C. Then 1.7 g 2,3-dichloro-5,6-dicyano-p-benzoquinone are added and the mixture is allowed to come up to ambient temperature within 20 minutes. Then it is diluted with diethyl ether and washed once with saturated sodium hydrogen carbonate solution and once with saturated sodium chloride solution. The organic phase is dried on magnesium sulphate, the solvent is eliminated in vacuo and the residue is chromatographed on silica gel.
Yield: 668 mg (61 % of theory)
Mass spectrum (ESI⁺): m/z = 177 [M+H]⁺

### Example LXV

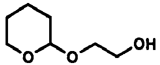

### 2-(tetrahydro-pyran-2-yloxy)-ethanol

37.2 g ethyleneglycol are dissolved in 380 ml diethyl ether. 0.5 ml of concentrated hydrochloric acid are added and then 54 g of dihydro-2H-pyran are added dropwise. Then the mixture is stirred for 1.5 hours, then 8 g potassium carbonate are added and stirring is continued for a further 10 minutes. Then the solids are filtered off, the mother liquor is evaporated down in vacuo and the residue is chromatographed on silica gel (cyclohexane/ethyl acetate 50:50 to 0:100).
Yield: 38.4 g (44 % of theory)
Mass spectrum (ESI⁺): m/z = 146 [M+H]⁺

The following compounds are obtained analogously to Example LXV:
(1) (5-bromo-pyrazin-2-yl)-[2-(tetrahydro-pyran-2-yloxy)-ethyl]-amine 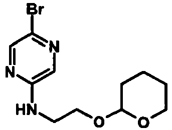 The reaction is carried out in dichloromethane instead of diethyl ether.
   Mass spectrum (ESI⁺): m/z = 302 [M+H]⁺

### Example LXVI

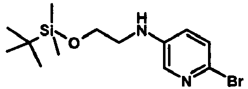

### (6-bromo-pyridin-3-yl)-[2-(tert-butyl-dimethyl-silanyloxy)-ethyl]-amine

1 g 5-amino-2-bromo-pyridine are dissolved in 20 ml dichloromethane. To this are added successively 1.22 ml 2-tert-butyl-dimethylsilyloxy-acetaldehyde and 478 µl acetic acid. After stirring for 1 hour at ambient temperature, 2.2 g sodium triacetoxyborohydride are added in batches. Then the mixture is stirred for 3 days, 8 ml of methanol are added and stirring is continued for a further 12 hours. Then the solvents are eliminated in vacuo, the residue is taken up in ethyl acetate and washed once with 10 % citric acid solution and once with saturated sodium chloride solution. After drying with magnesium sulphate the solvent is eliminated in vacuo and the residue is chromatographed on silica gel (cyclohexane/ethyl acetate 10:1).
Yield: 619 mg (32 % of theory)
Mass spectrum (ESI⁺): m/z = 331 [M+H]⁺

The following compounds are obtained analogously to Example LXVI:
(1) (6-bromo-pyridin-3-yl)-(2,2-dimethyl-[1,3]dioxan-5-yl)-amine 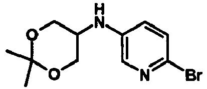 Mass spectrum (ESI⁺): m/z = 287 [M+H]⁺

### Example LXVII

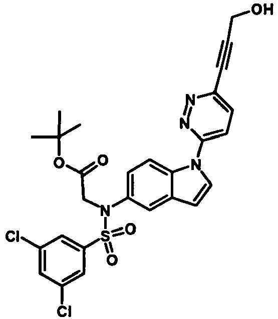

### tert.butyl ((3,5-dichloro-phenylsulphonyl)-{1-[6-(3-hydroxy-prop-1-ynyl)-pyridazin-3-yl]-1H-indol-5-yl}-amino)-acetate

Under argon 380 mg tert.butyl {(3,5-dichloro-phenylsulphonyl)-[1-(6-iodo-pyridazin-3-yl)-1H-indol-5-yl]-amino}-acetate are dissolved in 10 ml of tetrahydrofuran. To this are added successively 38 µl propargylalcohol, 4 mg bistriphenylphosphine-palladium-dichloride, 2.5 mg copper iodide and 162 µl diisopropylamine. The mixture is heated to 65°C for 4 hours, then another 38 µl propargylalcohol, 4 mg bistriphenylphosphine-palladium dichloride, 2.5 mg copper iodide and 162 µl diisopropylamine are added and the mixture is heated for another 12 hours to 65°C. Then it is diluted with ethyl acetate, washed once with water and once with saturated sodium chloride solution and dried on magnesium sulphate. Then the solvents are eliminated in vacuo and the residue is chromatographed on silica gel (cyclohexane/ethyl acetate 34:66).
Yield: 235 mg (69 % of theory)
Mass spectrum (ESI⁺): m/z = 587 [M+H]⁺

The following compounds are obtained analogously to Example LXXXI:
(1) tert.butyl ((3,5-dichloro-phenylsulphonyl)-{1-[4-(3-hydroxy-prop-1-ynyl)-pyridin-2-yl]-1H-indol-5-yl}-amino)-acetate 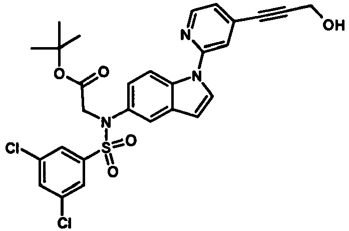 The reaction is carried out for 5 hours at 65°C.
   R_{f} value: 0.6 (silica gel: petroleum ether/ethyl acetate 1:1)

### Example LXVIII

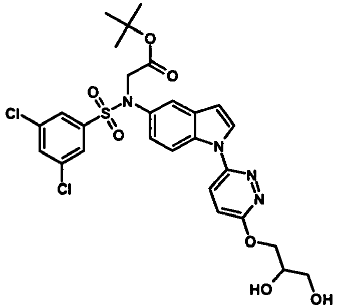

### tert.butyl ((3,5-dichloro-phenylsulphonyl)-{1-[6-(2,3-dihydroxy-propoxy)-pyridazin-3-yl]-1H-indol-5-yl}-amino)-acetate

380 mg tert.butyl ((3,5-dichloro-phenylsulphonyl)-{1-[6-(2-phenyl-[1,3]dioxan-5-yloxy)-pyridazin-3-yl]-1H-indol-5-yl}-amino)-acetate are dissolved in 2 ml acetic acid, 2 ml of tetrahydrofuran and 100 µl of water. The mixture is heated to 100°C for 2 hours in a pressurised vessel, then diluted with ethyl acetate, washed once with saturated sodium chloride solution and dried with magnesium sulphate. The solvents are eliminated in vacuo and the residue is chromatographed on silica gel (dichloromethane/methanol 97:3 to 90:10).
Yield: 160 mg (48 % of theory)
Mass spectrum (ESI⁺): m/z = 623 [M+H]⁺

The following compounds are obtained analogously to Example LXVIII:
(1) tert.butyl ((3,5-dichloro-phenylsulphonyl)-{1-[5-(2,3-dihydroxy-propoxy)-pyrazin--yl]-1H-indol-5-yl}-amino)-acetate 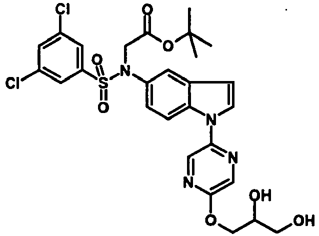 The reaction is carried out for 12 hours at 100°C and 30 minutes at 160°C in a pressurised vessel.
   Mass spectrum (ESI⁺): m/z = 623 [M+H]⁺

### Example LXIX

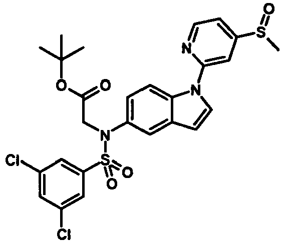

### tert.butyl {(3,5-dichloro-phenylsulphonyl)-[1-(4-methanesulphinyl-pyridin-2-yl)-1H-indol-5-yl]-amino}-acetate

140 mg tert.butyl {(3,5-dichloro-phenylsulphonyl)-[1-(4-methylsulphanyl-pyridin-2-yl)-1H-indol-5-yl]-amino}-acetate are dissolved in 5 ml hexafluorisopropanol and to this are added 51 µl of a 32 % solution of hydrogen peroxide in water. The mixture is stirred for 2 hours, combined with another 51 µl of a 32 % solution of hydrogen peroxide in water and stirred for another 2 hours. Then it is combined with 10 % sodium thiosulphate and 10 % sodium hydrogen carbonate solution, extracted 3 times with ethyl acetate, the organic phase is dried on magnesium sulphate and the solvent is eliminated in vacuo. The residue is chromatographed on silica gel (cyclohexane/ethyl acetate 70:30 to 0:100).
Yield: 145 mg (100 % of theory)
Mass spectrum (ESI⁺): m/z = 594 [M+H]⁺

### Example LXX

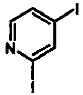

### 2,4-diiodo-pyridine

20 g 2,4-dichloro-pyridine are dissolved in 250 ml acetonitrile. 61 g of sodium iodide and 19.2 ml acetyl chloride are added and the mixture is refluxed for 12 hours. Then it is diluted with dichloromethane and washed once with 10 % potassium carbonate solution and once with 5 % sodium hydrogen sulphite solution. The organic phase is dried on magnesium sulphate and the solvents are eliminated in vacuo. The residue is chromatographed on silica gel (cyclohexane/ethyl acetate 5:1).
Yield: 145 mg (100 % of theory)
R_{f} value: 0.50 (silica gel: cyclohexane/ethyl acetate 5:1)

### Example LXXI

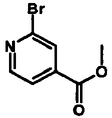

### methyl 2-bromo-isonicotinate

500 mg 2-bromo-isonicotinic acid are dissolved in 4 ml diethyl ether. 230 µl of oxalyl chloride and 10 ul DMF are added successively and the mixture is refluxed for 1 hour. Then the volatile constituents are eliminated in vacuo, the residue is taken up twice in 4 ml dichloromethane and this is then eliminated again in vacuo. The acid chloride thus obtained is taken up in 5 ml diethyl ether and to this are added dropwise 500 µl methanol and 370 µl pyridine. The mixture is stirred for 1 hour and divided between water and ethyl acetate. The organic phase is dried on magnesium sulphate, the solvent is eliminated in vacuo and the residue is chromatographed on silica gel (dichloromethane/methanol 99:1 to 80:20).
Yield: 385 mg (79 % of theory)
Mass spectrum (ESI⁺): m/z = 216 [M+H]⁺

### Example LXXII

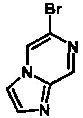

### 6-bromo-imidazo[1,2-a]pyrazine

500 mg 5-bromo-pyrazin-2-ylamine are dissolved in 20 ml of ethanol and 750 µl bromo-acetaldehyde-diethylacetal and 2.5 ml 48 % hydrobromic acid are added. The mixture is heated to 70°C for 12 hours, diluted with water and combined with 20 ml 1 N sodium hydroxide solution. Then it is extracted 3 times with dichloromethanelisopropanol (4:1). The combined organic phases are washed with semisaturated sodium chloride solution, dried on magnesium sulphate and freed from the solvents in vacuo. The residue is extracted from diisopropyl ether.
Yield: 105 mg (18 % of theory)
Mass spectrum (ESI⁺): m/z = 198 [M+H]⁺

### Example LXXIII

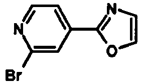

### 2-bromo-4-oxazol-2-yl-pyridine

650 mg 2-bromo-N-(2,2-dimethoxy-ethyl)-isonicotinamide are added to a mixture of 9 ml methanesulphonic acid and 1 g phosphorus pentoxide and heated to 135°C for 6 hours. Then the mixture is added to ice, the aqueous phase is extracted twice with ethyl acetate and the combined organic phases are washed twice with saturated sodium hydrogen carbonate solution and once with saturated sodium chloride solution. After drying with magnesium sulphate the solvents are eliminated in vacuo and the residue is chromatographed on silica gel (petroleum ether/ ethyl acetate 3:1).
Yield: 105 mg (21 % of theory)
Mass spectrum (ESI⁺): m/z = 225 [M+H]⁺

### Example LXXIV

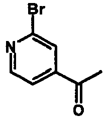

### 1-(2-bromo-pyridin-4-yl)-ethanone

200 mg 2-bromo-N-methoxy-N-methyl-isonicotinamide are dissolved in 3 ml of tetrahydrofuran, cooled to -10°C and to this are added dropwise 275 µl of a 3 N solution of methylmagnesium bromide in tetrahydrofuran. Then the mixture is allowed to come up to ambient temperature, stirred for 12 hours and divided between ethyl acetate and semisaturated sodium chloride solution. The organic phase is dried on magnesium sulphate, freed from the solvents in vacuo and the residue is chromatographed on silica gel (cyclohexane/ ethyl acetate 90:10 to 50:50).
Yield: 110 mg (67 % of theory)
Mass spectrum (ESI⁺): m/z = 200 [M+H]⁺

The following compounds are obtained analogously to Example LXXIV:
(1) 1-(2-bromo-pyridin-4-yl)-propan-1-one 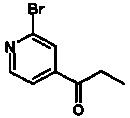 After 12 hours at ambient temperature another 1.5 equivalents of a 1 N solution of ethylmagnesium bromide in tetrahydrofuran are added dropwise. The mixture is stirred for another 2 hours and the reaction is ended by the addition of water. The solvents are eliminated in vacuo, the residue is taken up in acetonitrile, the solid is filtered off and the mother liquor is freed from the solvent in vacuo. The residue is chromatographed on silica gel.
   Yield: 155 mg (46 % of theory)
   R_{f} value: 0.70 (silica gel: petroleum ether/ethyl acetate 2:1)

### Example LXXV

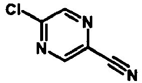

### 5-chloro-pyrazine-2-carbonitrile

374 mg 5-chloro-pyrazine-2-carboxylic acid-amide are dissolved in 5 ml phosphorus oxychloride and refluxed for 2 hours. Then the phosphorus oxychloride is eliminated in vacuo and the residue is twice taken up in toluene and the latter is again eliminated in vacuo. Then the mixture is divided between water and ethyl acetate, the organic phase is washed once with saturated sodium chloride solution, dried on magnesium sulphate and freed from the solvent in vacuo.
Yield: 128 mg (39 % of theory)
R_{f} value: 0.90 (silica gel: cyclohexane/ethyl acetate 1:1)

### Example LXXVI

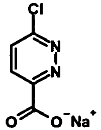

### sodium 6-chloro-pyridazine-3-carboxylate

250 mg methyl 6-chloro-pyridazine-3-carboxylate are dissolved in 5 ml of tetrahydrofuran, 1.5 ml 1 N sodium hydroxide solution are added and the mixture is stirred for 2 hours. Then the solvent is eliminated in vacuo and the residue is extracted from ethyl acetate.
Yield: 255 mg (98 % of theory)
Mass spectrum (ESI⁺): m/z = 157 [M+H]⁺

### Example LXXVII

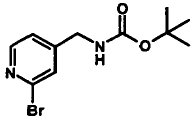

### tert-butyl (2-bromo-pyridin-4-ylmethyl)-carbamate

50 mg C-(2-bromo-pyridin-4-yl)-methylamine are dissolved in 3 ml of tetrahydrofuran. The mixture is cooled to 0°C, 320 µl of 1 N sodium hydroxide solution are added and then 65 mgl of di-tert-butyl-dicarbonate are added. After stirring for 12 hours the mixture is divided between ethyl acetate and water. The organic phase is washed twice with water and once with saturated sodium chloride solution, dried on magnesium sulphate and evaporated down in vacuo.
Yield: 60 mg (78 % of theory)
Mass spectrum (ESI⁺): m/z = 287 [M+H]⁺

### Example LXXVIII

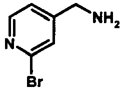

### C-(2-bromo-pyridin-4-yl)-methylamine

148 mg 2-bromo-isonicotinamide are dissolved in 3 ml of tetrahydrofuran, cooled to 0°C and to this 2.2 ml of a 1 N solution of borane-tetrahydrofuran complex in tetrahydrofuran are added dropwise. Then the mixture is heated to 70°C for 4 hours, cooled to ambient temperature, 1.5 ml of methanol and 1.5 ml of 1 N sodium hydroxide solution are added and the mixture is heated to 70°C for 30 minutes. Then it is diluted with dichloromethane, washed once with saturated sodium chloride solution, dried on magnesium sulphate, the solvents are eliminated in vacuo and the residue is chromatographed on silica gel (dichloromethane/methanol 100:0 to 80:20).
Yield: 50 mg (36 % of theory)
Mass spectrum (ESI⁺): m/z = 187 [M+H]⁺

### Example LXXIX

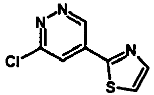

### 3-chloro-5-(thiazol-2-yl)-pyridazine

2 ml phosphorus oxychloride are added to 225 mg 2-(tetrahydro-pyran-2-yl)-5-(thiazol-2-yl)-2H-pyridazin-3-one and the reaction mixture is heated to 90°C for 10 minutes. After cooling to ambient temperature the reaction mixture is evaporated down in vacuo and slowly poured onto ice water with stirring. The mixture is made alkaline with sodium carbonate and extracted with dichloromethane. The organic phase is washed with saturated sodium chloride solution, dried on magnesium sulphate and evaporated down. The flask residue is chromatographed through a silica gel column with cyclohexane/ethyl acetate (80:20 to 40:60) as eluant.
Yield: 62 mg (37 % of theory)
R_{f} value: 0.48 (silica gel: petroleum ether/ethyl acetate 1:1)

### Example LXXX

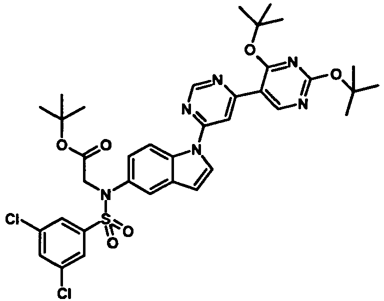

### tert.butyl [[1-(2',4'-di-tert-butoxy-[4.5']bipyrimidinyl-6-yl)-1H-indol-5-yl]-(3,5-dichloro-phenylsulphonyl)-amino]-acetate

89 mg 2,4-di(tert-butoxy)-pyrimidin-5-yl-boric acid and 1ml of a 1 M sodium hydrogen carbonate solution are added to a mixture of 200 mg tert.butyl {(3,5-dichloro-phenylsulphonyl)-[1-(6-iodo-pyrimidin-4-yl)-1H-indol-5-yl]-amino}-acetate and 18 mg tetrakis(triphenylphosphine)palladium(0) in 3 ml 1,2-dimethoxy-ethane. The reaction mixture is refluxed for 2.5 hours. After cooling to ambient temperature the mixture is diluted with ethyl acetate, washed with water and saturated sodium chloride solution, dried on magnesium sulphate and evaporated down. The flask residue is chromatographed through a silica gel column with cyclohexane/ ethyl acetate (90:10 to 75:25) as eluant.
Yield: 150 mg (65 % of theory)
Mass spectrum (ESI⁺): m/z = 755 [M+H]⁺
R_{f} value: 0.42 (silica gel: petroleum ether/ethyl acetate 5:1)

### Example LXXXI

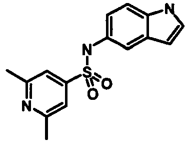

### 2,6-dimethyl-pyridin-4-sulphonic acid-(1H-indol-5-yl)-amide

1 g 4-bromo-2,6-dimethyl-pyridine is dissolved in 10 ml of tetrahydrofuran and cooled to -78°C. 3.53 ml of a 1.6 molar solution of n-butyllithium in hexane are added dropwise thereto. The mixture is stirred for 1 hour and then sulphur dioxide is piped through the solution for 5 minutes. Then within 45 minutes it is allowed to come up to -40°C, 30 ml of hexane are added, the mixture is allowed to come up to ambient temperature and the precipitated solid is suction filtered. The solid is dried in vacuo, then taken up in 15 ml dichloromethane and at 5°C combined with 789 mg of N-chlorosuccinimide. The mixture is stirred for another 1 hour, the solid constituents are filtered through kieselguhr and the mother liquor is added dropwise at 0°C to a solution of 871 mg of 1H-indol-5-yl-amine in 10 ml of pyridine. Then the mixture is stirred for 3 hours at ambient temperature, the solvents are eliminated in vacuo and the residue is divided between water and ethyl acetate. The aqueous phase is extracted twice with ethyl acetate and the combined organic phases are dried on magnesium sulphate. The solvent is eliminated in vacuo and the residue is chromatographed on silica gel (cyclohexane/ethyl acetate 80:20 to 0:100).
Yield: 684 mg (42 % of theory)
Mass spectrum (ESI⁺): m/z = 302 [M+H]⁺

### Example LXXXII

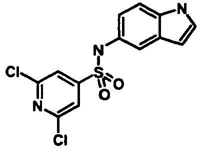

### 2,6-dichloro-pyridin-4-sulphonic acid -(1H-indol-5-yl)-amide

3.76 ml of a 2 molar solution of isopropylmagnesium chloride in tetrahydrofuran are mixed with 315 mg lithium chloride and stirred for 1 hour at ambient temperature. 1.2 ml 2,2,6,6-tetramethylpiperidine are added dropwise thereto and stirring is continued for another 6 hours. Then a solution of 1 g 2,6-dichloropyridine in 5 ml of tetrahydrofuran is added dropwise. The mixture is stirred for 30 minutes and then cooled to -50°C. Then sulphur dioxide is piped through the solution for 5 minutes. Then the mixture is allowed to come up to - 20°C within 45 minutes, 40 ml hexane are added, the mixture is allowed to come up to ambient temperature and the supernatant above the precipitated oil is removed. Another 5 ml of hexane are added to the oil, the mixture is stirred vigorously for 5 minutes and after the oil has settled out the supernatant is removed. The oil is dried in vacuo, then taken up in 20 ml dichloromethane and at 5°C combined with 992 mg N-chlorosuccinimide. The mixture is stirred for another 1 hour, the solid constituents are filtered through kieselguhr and the mother liquor is added dropwise at 0°C to a solution of 893 mg 1H-indol-5-yl-amine in 10 ml of pyridine. Then the mixture is stirred overnight at ambient temperature, the solvents are eliminated in vacuo and the residue is distributed between water and ethyl acetate. The aqueous phase is extracted twice with ethyl acetate and the combined organic phases are dried on magnesium sulphate. The solvent is eliminated in vacuo and the residue is chromatographed on silica gel (cyclohexane/ethyl acetate 80:20 to 0:100).
Yield: 514 mg (22 % of theory)
Mass spectrum (ESI⁻): m/z = 340 [M-H]⁻

### Preparation of the end compounds:

### Example 1

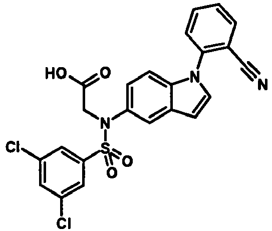

### [[1-(2-cyano-phenyl)-1H-indol-5-yl-(3,5-dichloro-phenylsulphonyl)-amino]-acetic acid

40 mg of tert.butyl [[1-(2-cyano-phenyl)-1H-indol-5-yl]-(3,5-dichlorophenylsulphonyl)-amino]-acetate are dissolved in 3 ml dichloromethane. 1.5 ml trifluoroacetic acid are added, with stirring. The mixture is stirred for 1.5 hours at ambient temperature and then the solvents are eliminated in vacuo. The residue is extracted from diisopropyl ether.
Yield: 20 mg (56 % of theory)
Mass spectrum (ESI⁺): m/z = 517 [M+NH₄]⁺

The following compounds are obtained analogously to Example 1:
(1) [(3,5-dichloro-phenylsulphonyl)-(1-pyrimidin-2-yl-1H-indol-5-yl)-amino]-acetic acid 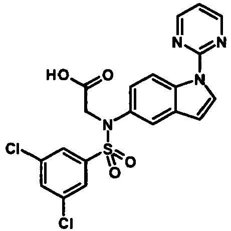 Mass spectrum (ESI⁺): m/z = 477 [M+H]⁺
(2) [(3,5-dichloro-phenylsulphonyl)-(1-pyrazin-2-yl-1H-indol-5-yl)-amino]-acetic acid 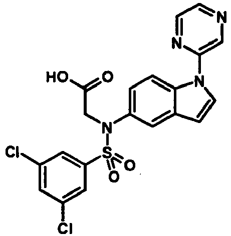 Mass spectrum (ESI⁺): m/z = 477 [M+H]⁺
(3) [(3,5-dichloro-phenylsulphonyl)-(1-pyrimidin-5-yl-1H-indol-5-yl)-amino]-acetic acid 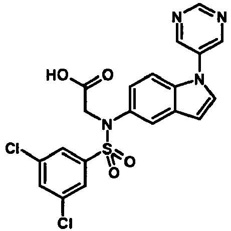 Mass spectrum (ESI⁺): m/z = 477 [M+H]⁺
(4) [[1-(3-carbamoyl-phenyl)-1H-indol-5-yl]-(3-chloro-phenylsulphonyl)-amino]-acetic acid 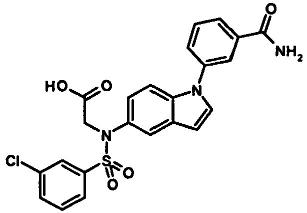 Mass spectrum (ESI⁺): m/z = 484 [M+H]⁺
(5) [[1-(3-carbamoyl-phenyl)-1H-indol-5-yl]-(3,5-dichloro-phenylsulphonyl)-amino]-acetic acid 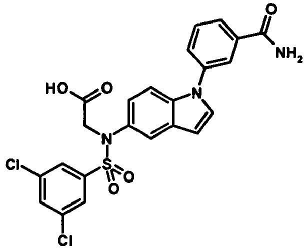 Mass spectrum (ESI⁺): m/z = 518 [M+H]⁺
(6) {(3-chloro-phenylsulphonyl)-[1-(3-methylcarbamoyl-phenyl)-1H-indol-5-yl]-amino}-acetic acid 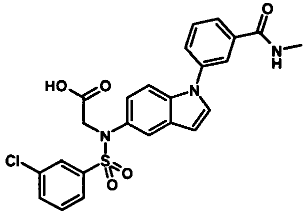 Mass spectrum (ESI⁺): m/z = 498 [M+H]⁺
(7) {(3,5-dichloro-phenylsulphonyl)-[1-(3-methylcarbamoyl-phenyl)-1H-indol-5-yl]-amino}-acetic acid 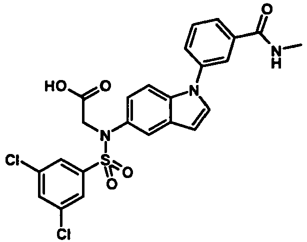 Mass spectrum (ESI⁺): m/z = 532 [M+H]⁺
(8) {(3,5-dichloro-phenylsulphonyl)-[1-(4-morpholin-4-yl-phenyl)-1H-indol-5-yl]-amino}-acetic acid · CF₃CO₂H 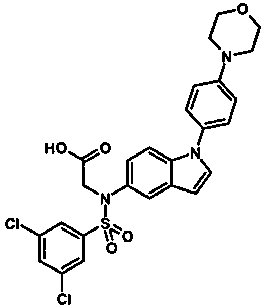 Mass spectrum (ESI⁺): m/z = 560 [M+H]⁺
(9) {(3,5-dichloro-phenylsulphonyl)-[1-(4-morpholin-4-ylmethyl-phenyl)-1H-indol-5-yl]-amino}-acetic acid CF₃CO₂H 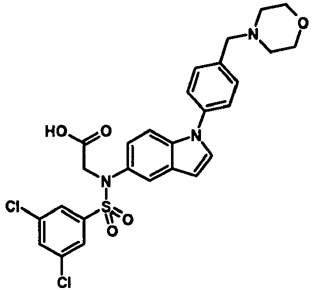 Mass spectrum (ESI⁺): m/z = 574 [M+H]⁺
(10) [(3,5-dichloro-phenylsulphonyl)-(1-pyrimidin-2-yl-2,3-dihydro-1H-indol-5-yl)-amino]-acetic acid 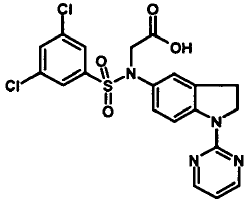 Mass spectrum (ESI⁻): m/z = 477 [M-H]⁻
(11) [(3,5-dichloro-phenylsulphonyl)-(1-pyridin-4-yl-1H-indol-5-yl)-amino]-acetic acid · CF₃CO₂H 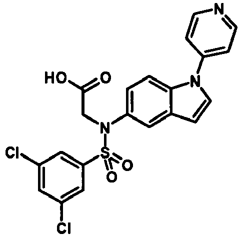 Mass spectrum (ESI⁺): m/z = 476 [M+H]⁺
(12) {(3,5-dichloro-phenylsulphonyl)-[1-(1-methyl-1H-imidazol-2-yl)-2,3-dihydro-1H-indol-5-yl]-amino}-acetic acid · CF₃CO₂H 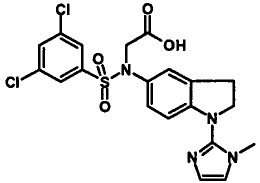 Mass spectrum (ESI⁺): m/z = 481 [M+H]⁺
(13) [(3,5-dichloro-phenylsulphonyl)-(1-pyrimidin-2-yl-1H-indol-4-yl)-amino]-acetic acid 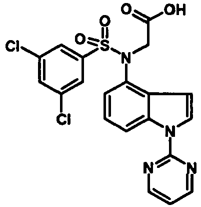 Mass spectrum (ESI⁺): m/z = 494 [M+NH₄]⁺
(14) {(3,5-dichloro-phenylsulphonyl)-[1-(4-methyl-6-piperazin-1-yl-pyridazin-3-yl)-1H-indol-5-yl]-amino}-acetic acid · CF₃CO₂H 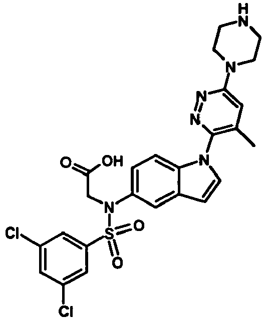 Mass spectrum (ESI⁺): m/z = 575 [M+H]⁺
(15) ((3,5-dichloro-phenylsulphonyl)-{1-[6-(2-dimethylamino-ethylamino)-pyridazin-3-yl]-1H-indol-5-yl}-amino)-acetic acid · CF₃CO₂H 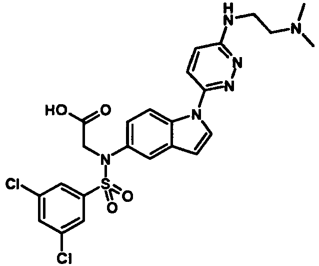 The crude product is purified by preparative HPLC.
   Mass spectrum (ESI⁺): m/z = 563 [M+H]⁺
(16) [{1-[6-(2-dimethylamino-ethylamino)-pyridazin-3-yl]-1H-indol-5-yl}-(3,5-dimethyl-phenylsulphonyl)-amino]-acetic acid CF₃CO₂H 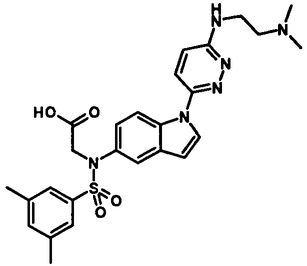 Mass spectrum (ESI⁺): m/z = 523 [M+H]⁺
(17) {(3,5-dichloro-phenylsulphonyl)-[1-(2-piperazin-1-yl-quinolin-8-yl)-1H-indol-5-yl]-amino}-acetic acid · CF₃CO₂H 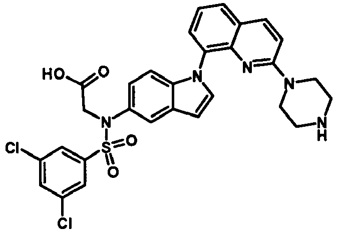 Mass spectrum (ESI⁺): m/z = 610 [M+H]⁺
(18) ((3-bromo-5-methyl-phenylsulphonyl)-{1-[6-(2-dimethylamino-ethylamino)-pyridazin-3-yl]-1H-indol-5-yl}-amino)-acetic acid · CF₃CO₂H 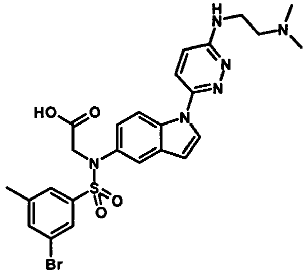 The crude product is purified by preparative HPLC.
   Mass spectrum (ESI⁺): m/z = 587 [M+H]⁺
(19) ((3-chloro-5-methyl-phenylsulphonyl)-{1-[6-(2-dimethylamino-ethylamino)-pyridazin-3-yl]-1H-indol-5-yl}-amino)-acetic acid · CF₃CO₂H 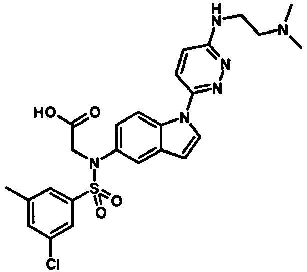 The crude product is purified by preparative HPLC.
   Mass spectrum (ESI⁺): m/z = 543 [M+H]⁺
(20) [{1-[S-(2-dimethylamino-ethylamino)-pyrazin-2-yl]-1H-indol-5-yl}-(3,5-dimethyl-phenylsulphonyl)-amino]-acetic acid · CF₃CO₂H 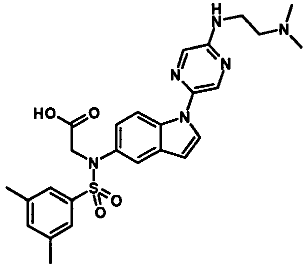 The crude product is purified by preparative HPLC.
   Mass spectrum (ESI⁺): m/z = 523 [M+H]⁺
(21) ((3-bromo-5-methyl-phenylsulphonyl)-{1-[5-(2-dimethylamino-ethylamino)-pyrazin-2-yl]-1H-indol-5-yl}-amino)-acetic acid · CF₃CO₂H 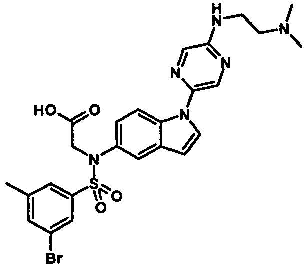 The crude product is purified by preparative HPLC.
   Mass spectrum (ESI⁺): m/z = 587 [M+H]⁺
(22) ((3,5-dibromo-phenylsulphonyl)-{1-[5-(2-dimethylamino-ethylamino)-pyrazin-2-yl]-1H-indol-5-yl}-amino)-acetic acid · CF₃CO₂H 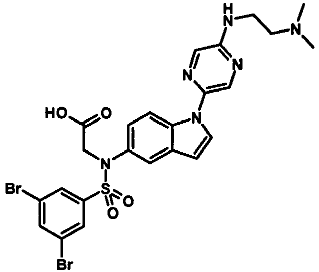 The crude product is purified by preparative HPLC.
   Mass spectrum (ESI⁺): m/z = 651 [M+H]⁺
(23) ((3-chloro-5-methyl-phenylsulphonyl)-{1-[5-(2-dimethylamino-ethylamino)-pyrazin-2-yl]-1H-indol-5-yl}-amino)-acetic acid · CF₃CO₂H 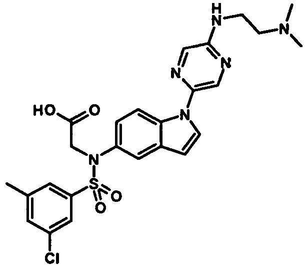 The crude product is purified by preparative HPLC.
   Mass spectrum (ESI⁺): m/z = 543 [M+H]⁺
(24) ((3,5-dibromo-phenylsulphonyl)-{1-[6-(2-dimethylamino-ethylamino)-pyridazin-3-yl]-1H-indol-5-yl}-amino)-acetic acid · CF₃CO₂H 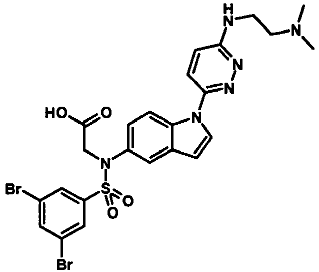 The crude product is purified by preparative HPLC.
   Mass spectrum (ESI⁺): m/z = 651 [M+H]⁺
(25) {(3,5-dichloro-phenylsulphonyl)-[1-(6-piperazin-1-yl-pyridazin-3-yl)-1H-indol-5-yl]-amino}-acetic acid · CF₃CO₂H 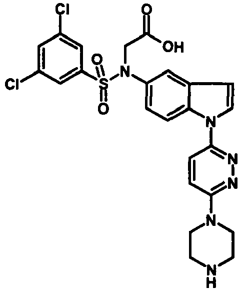 Mass spectrum (ESI⁺): m/z = 561 [M+H]⁺
(26) {(3,5-dichloro-phenylsulphonyl)-[1-(2-methylamino-pyrimidin-4-yl)-1H-indol-5-yl]-amino}-acetic acid · CF₃CO₂H 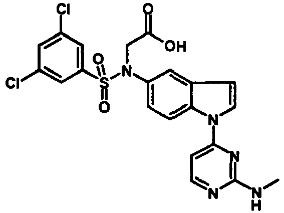 Mass spectrum (ESI⁺): m/z = 506 [M+H]⁺
(27) [(3,5-dichloro-phenylsulphonyl)-(1-naphthalen-2-yl-1H-indol-5-yl)-amino]-acetic acid 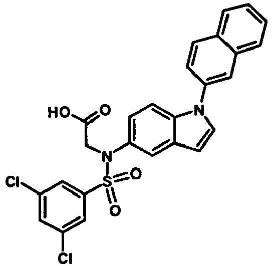 Mass spectrum (ESI⁺): m/z = 525 [M+H]⁺
(28) ((3-bromo-5-chloro-phenylsulphonyl)-{1-[6-(2-dimethylamino-ethylamino)-pyridazin-3-yl]-1H-indol-5-yl}-amino)-acetic acid · CF₃CO₂H 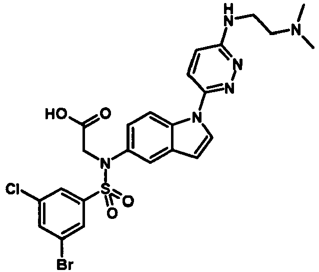 The crude product is purified by preparative HPLC.
   Mass spectrum (ESI⁺): m/z = 607 [M+H]⁺
(29) ((3-bromo-5-chloro-phenylsulphonyl)-{1-[5-(2-dimethylamino-ethylamino)-pyrazin-2-yl]-1H-indol-5-yl}-amino)-acetic acid · CF₃CO₂H 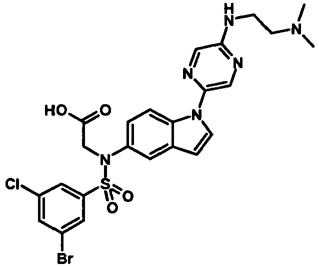 The crude product is purified by preparative HPLC.
   Mass spectrum (ESI⁺): m/z = 607 [M+H]⁺
(30) [[1-(5-amino-pyridin-2-yl)-1H-indol-5-yl]-(3,5-dichloro-phenylsulphonyl)-amino]-acetic acid ·CF₃CO₂H 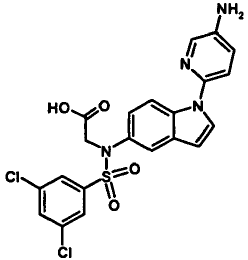 Mass spectrum (ESI⁺): m/z = 491 [M+H]⁺
(31) {(3,5-dichloro-phenylsulphonyl)-[1-(2-methyl-quinolin-6-yl)-1H-indol-5-yl]-amino)-acetic acid · CF₃CO₂H 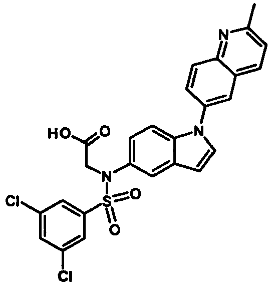 Mass spectrum (ESI⁺): m/z = 540 [M+H]⁺
(32) [(3,5-dichloro-phenylsulphonyl)-(1-naphthalen-1-yl-1H-indol-5-yl)-amino]-acetic acid 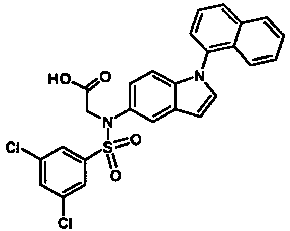 Mass spectrum (ESI⁺): m/z = 542 [M+NH₄]⁺
(33) [{1-[2-(2-amino-ethylamino)-pyrimidin-4-yl]-1H-indol-5-yl}-(3,5-dichlorophenylsulphonyl)-amino]-acetic acid · CF₃CO₂H 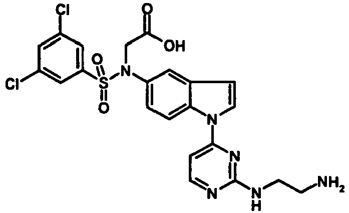 Mass spectrum (ESI⁺): m/z = 535 [M+H]⁺
(34) {(3,5-dichloro-phenylsulphonyl)-[1-(3-methyl-pyridin-2-yl)-1H-indol-5-yl]-amino}-acetic acid · CF₃CO₂H 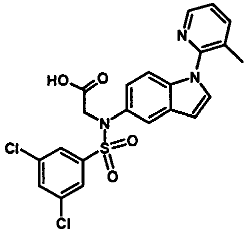 Mass spectrum (ESI⁺): m/z = 490 [M+H]⁺
(35) [[1-(4-cyano-6-piperazin-1-yl-pyridazin-3-yl)-1H-indol-5-yl]-(3,5-dichlorophenylsulphonyl)-amino]-acetic acid · CF₃CO₂H 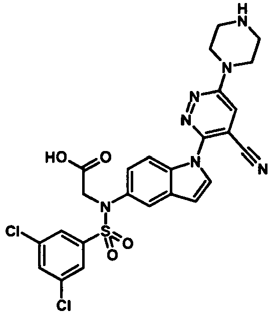 Mass spectrum (ESI⁺): m/z = 586 [M+H]⁺
(36) ((3,5-dichloro-phenylsulphonyl)-{1-[6-(piperazine-1-carbonyl)-pyridin-2-yl]-1H-indol-5-yl}-amino)-acetic acid · CF₃CO₂H 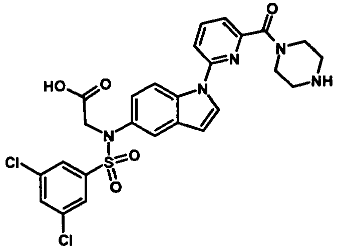 Mass spectrum (ESI⁺): m/z = 588 [M+H]⁺
(37) ((3,5-dichloro-phenylsulphonyl)-{1-[6-(2-dimethylamino-ethylcarbamoyl)-pyridin-2-yl]-1H-indol-5-yl}-amino)-acetic acid · CF₃CO₂H 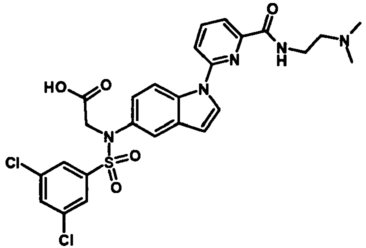 Mass spectrum (ESI⁺): m/z = 590 [M+H]⁺
(38) [[1-(6-[1,4]diazepan-1-yl-pyridazin-3-yl)-1H-indol-5-yl]-(3,5-dichlorophenylsulphonyl)-amino]-acetic acid · CF₃CO₂H 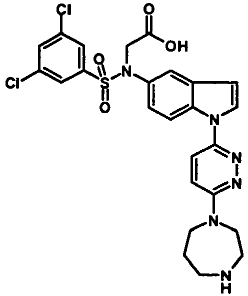 Mass spectrum (ESI⁺): m/z = 575 [M+H]⁺
(39) {(3,5-dichloro-phenylsulphonyl)-[1-(6-methyl-pyridin-2-yl)-1H-indol-5-yl]-amino}-acetic acid · CF₃CO₂H 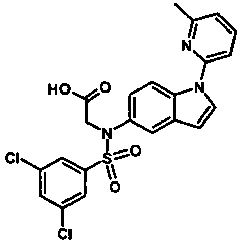 Mass spectrum (ESI⁺): m/z = 490 [M+H]⁺
(40) [{1-[6-(3-amino-propylamino)-pyridazin-3-yl]-1H-indol-5-yl}-(3,5-dichlorophenylsulphonyl)-amino]-acetic acid · CF₃CO₂H 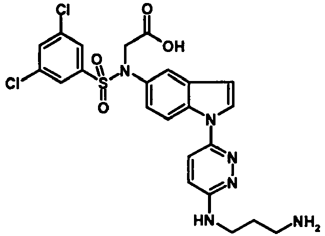 Mass spectrum (ESI⁺): m/z = 549 [M+H]⁺
(41) {(3,5-dichloro-phenylsulphonyl)-[1-(4-methyl-pyridin-2-yl)-1H-indol-5-yl]-amino}-acetic acid · CF₃CO₂H 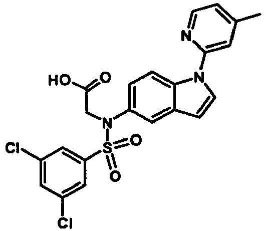 Mass spectrum (ESI⁺): m/z = 490 [M+H]⁺
(42) [[1-(6-cyclopropylamino-pyridazin-3-yl)-1H-indol-5-yl]-(3,5-dichlorophenylsulphonyl)-amino]-acetic acid · CF₃CO₂H 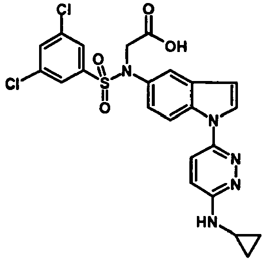 Mass spectrum (ESI⁺): m/z = 532 [M+H]⁺
(43) {(3,5-dichloro-phenylsulphonyl)-[1-(5-methyl-pyridin-2-yl)-1H-indol-5-yl]-amino}-acetic acid · CF₃CO₂H 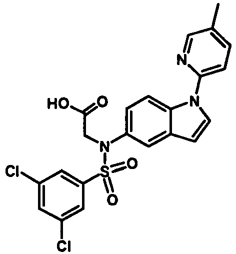 Mass spectrum (ESI⁺): m/z = 490 [M+H]⁺
(44) [(3,5-dichloro-phenylsulphonyl)-(1-isoquinolin-1-yl-1H-indol-5-yl)-amino]-acetic acid · CF₃CO₂H 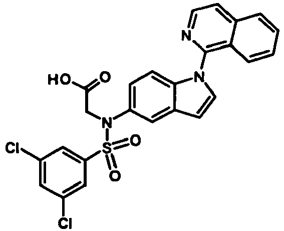 Mass spectrum (ESI⁺): m/z = 526 [M+H]⁺
(45) [[1-(3-amino-pyridin-2-yl)-1H-indol-5-yl]-(3,5-dichloro-phenylsulphonyl)-amino]-acetic acid · CF₃CO₂H 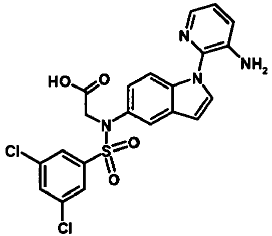 Mass spectrum (ESI⁺): m/z = 491 [M+H]⁺
(46) ((3,5-dichloro-phenylsulphonyl)-{1-[6-(2-dimethylamino-ethylamino)-pyridazin-3-yl]-3-methyl-1H-indol-5-yl}-amino)-acetic acid · CF₃CO₂H 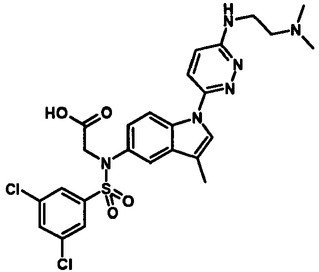 Mass spectrum (ESI⁺): m/z = 577 [M+H]⁺
(47) [(1-{6-[(2-amino-ethyl)-methyl-amino]-pyridazin-3-yl}-1H-indol-5-yl)-(3,5-dichloro-phenylsulphonyl)-amino]-acetic acid · CF₃CO₂H 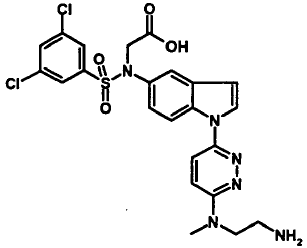 Mass spectrum (ESI⁺): m/z = 549 [M+H]⁺
(48) ((3,5-dichloro-phenylsulphonyl)-{1-[6-(2-methylamino-ethylamino)-pyridazin-3-yl]-1H-indol-5-yl}-amino)-acetic acid · CF₃CO₂H 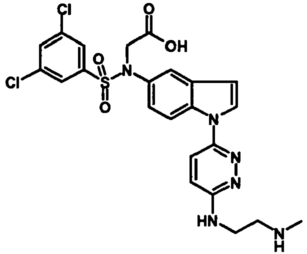 Mass spectrum (ESI⁺): m/z = 549 [M+H]⁺
(49) ((3,5-dichloro-phenylsulphonyl)-{1-[3-(2-dimethylamino-ethylamino)-pyridin-2-yl]-1H-indol-5-yl}-amino)-acetic acid · CF₃CO₂H 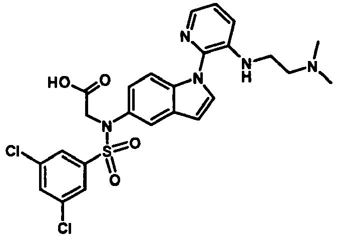 Mass spectrum (ESI⁺): m/z = 562 [M+H]⁺
(50) [{1-[6-(2-amino-2-methyl-propylamino)-pyridazin-3-yl]-1H-indol-5-yl}-(3,5-dichloro-phenylsulphonyl)-amino]-acetic acid · CF₃CO₂H 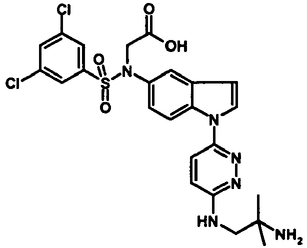 Mass spectrum (ESI⁺): m/z = 563 [M+H]⁺
(51) [{1-[6-(3-amino-piperidin-1-yl)-pyridazin-3-yl]-1H-indol-5-yl}-(3,5-dichlorophenylsulphonyl)-amino]-acetic acid · CF₃CO₂H 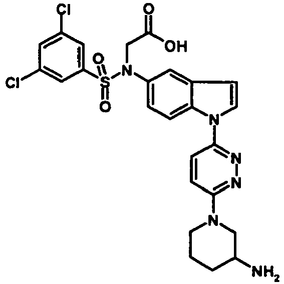 Mass spectrum (ESI⁺): m/z = 575 [M+H]⁺
(52) [{3-cyano-1-[6-(2-dimethylamino-ethylamino)-pyridazin-3-yl]-1H-indol-5-yl}-(3,5-dichloro-phenylsulphonyl)-amino]-acetic acid · CF₃CO₂H 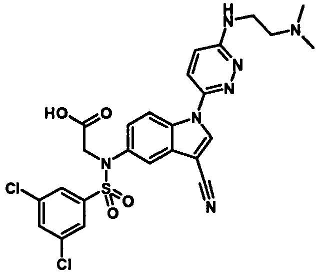 Mass spectrum (ESI⁺): m/z = 588 [M+H]⁺
(53) {(3,5-dichloro-phenylsulphonyl)-[1-(6-morpholin-4-yl-pyridazin-3-yl)-1H-indol-5-yl]-amino}-acetic acid · CF₃CO₂H 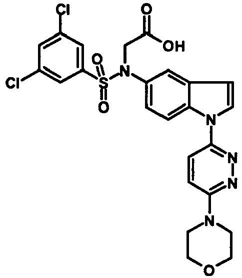 Mass spectrum (ESI⁺): m/z = 562 [M+H]⁺
(54) {(3,5-dichloro-phenylsulphonyl)-[1-(6-morpholin-4-yl-pyridazin-3-yl)-2,3-dihydro-1H-indol-5-yl]-aminol-acetic acid · CF₃CO₂H 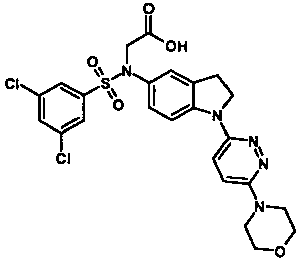 Mass spectrum (ESI⁺): m/z = 564 [M+H]⁺
(55) [[1-(5-amino-pyrazin-2-yl)-1H-indol-5-yl]-(3,5-dichloro-phenylsulphonyl)-amino]-acetic acid · CF₃CO₂H 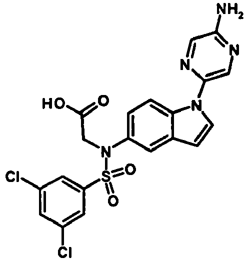 Mass spectrum (ESI⁺): m/z = 492 [M+H]⁺
(56) [{3-chloro-1-[6-(2-dimethylamino-ethylamino)-pyridazin-3-yl]-1H-indol-5-yl}-(3,5-dichloro-phenylsulphonyl)-amino]-acetic acid · CF₃CO₂H 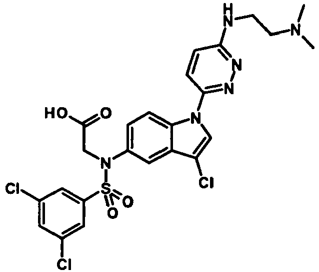 Mass spectrum (ESI⁺): m/z = 597 [M+H]⁺
(57) ((3,5-dichloro-phenylsulphonyl)-{1-[6-(2-dimethylamino-ethylamino)-pyridazin-3-yl]-3-trifluoromethyl-1H-indol-5-yl}-amino)-acetic acid · CF₃CO₂H 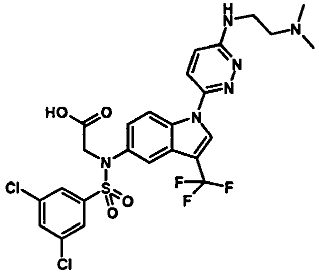 Mass spectrum (ESI⁺): m/z = 631 [M+H]⁺
(58) {(3,5-dichloro-phenylsulphonyl)-[1-(4,6-dimethoxy-[1,3,5]triazin-2-yl)-2,3-dihydro-1H-indol-5-yl]-amino}-acetic acid 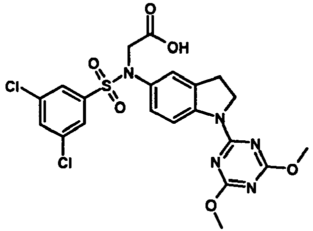 Mass spectrum (ESI⁺): m/z = 540 [M+H]⁺
(59) ((3,5-dichloro-phenylsulphonyl)-{1-[5-(piperazine-1-carbonyl)-pyridin-2-yl]-1H-indol-5-yl}-amino)-acetic acid · CF₃CO₂H 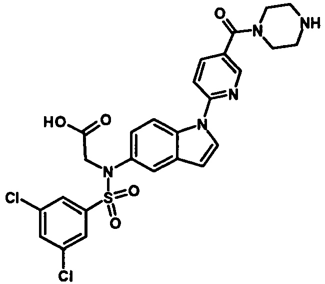 Mass spectrum (ESI⁺): m/z = 588 [M+H]⁺
(60) ((3,5-dichloro-phenylsulphonyl)-{1-[5-(2-dimethylamino-ethylcarbamoyl)-pyridin-2-yl]-1H-indol-5-yl}-amino)-acetic acid · CF₃CO₂H 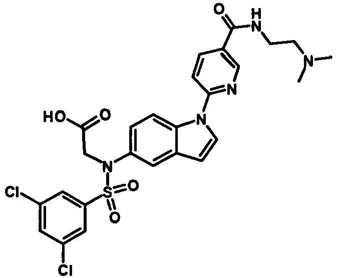 Mass spectrum (ESI⁺): m/z = 590 [M+H]⁺
(61) {(3,5-dichloro-phenylsulphonyl)-[1-(6-dimethylamino-pyridazin-3-yl)-1H-indol-5-yl]-amino}-acetic acid · CF₃CO₂H 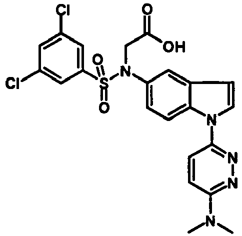 Mass spectrum (ESI⁺): m/z = 520 [M+H]⁺
(62) ((3,5-dichloro-phenylsulphonyl)-{1-[6-(2-hydroxy-ethylamino)-pyridazin-3-yl]-1H-indol-5-yl}-amino)-acetic acid 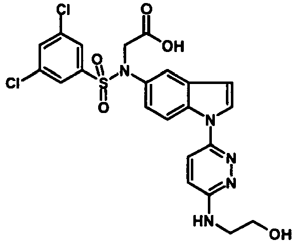 Mass spectrum (ESI⁺): m/z = 536 [M+H]⁺
(63) ((3,5-dichloro-phenylsulphonyl)-{1-[6-(2-piperidin-1-yl-ethylamino)-pyridazin-3-yl]-1H-indol-5-yl}-amino)-acetic acid · CF₃CO₂H 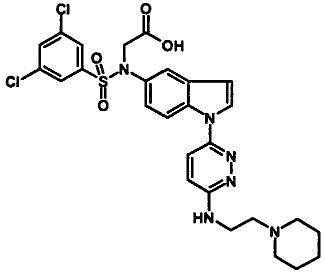 Mass spectrum (ESI⁺): m/z = 603 [M+H]⁺
(64) {(3,5-dichloro-phenylsulphonyl)-[1-(4-morpholin-4-yl-pyridin-2-yl)-1H-indol-5-yl]-amino}-acetic acid 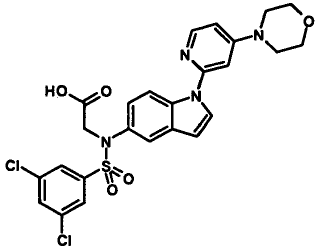 Mass spectrum (ESI⁺): m/z = 561 [M+H]⁺
   R_{f} value: 0.25 (silica gel: dichloromethane/methanol 95:5)
(65) {(3,5-dichloro-phenylsulphonyl)-[1-(5-morpholin-4-yl-pyridin-2-yl)-1H-indol-5-yl]-amino}-acetic acid 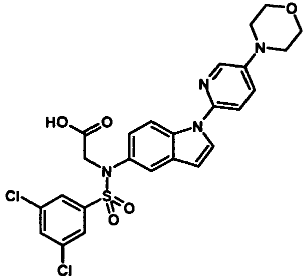 Mass spectrum (ESI⁺): m/z = 561 [M+H]⁺
   R_{f} value: 0.35 (silica gel: dichloromethane/methanol 95:5)
(66) ((3,5-dichloro-phenylsulphonyl)-{1-[5-(3-oxo-morpholin-4-yl)-pyrazin-2-yl]-1H-indol-5-yl}-amino)-acetic acid 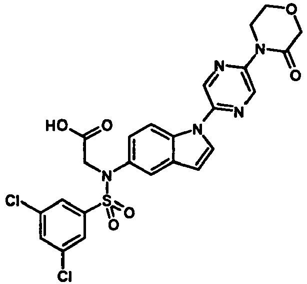 Mass spectrum (ESI⁺): m/z = 576 [M+H]⁺
   R_{f} value: 0.28 (silica gel: dichloromethane/methanol 95:5)
(67) ((3,5-dichloro-phenylsulphonyl)-{1-[5-(tetrahydro-pyran-4-yloxy)-pyrazin-2-yl]-1H-indol-5-yl}-amino)-acetic acid 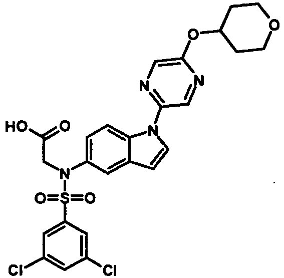 Mass spectrum (ESI⁺): m/z = 577 [M+H]⁺
   R_{f} value: 0.30 (silica gel: dichloromethane/methanol/acetic acid 98:2:0.1)
(68) (S)-((3,5-dichloro-phenylsulphonyl)-{1-[5-(tetrahydro-furan-3-yloxy)-pyrazin-2-yl]-1H-indol-5-yl}-amino)-acetic acid 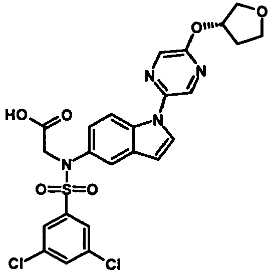 Mass spectrum (ESI⁻): m/z = 561 [M-H]⁻
   R_{f} value: 0.30 (silica gel: dichloromethane/methanol/acetic acid 98:2:0.1)
(69) (R)-((3,5-dichloro-phenylsulphony)-{1-[6-(tetrahydro-furan-3-ylamino)-pyridazin-3-yl]-1H-indol-5-yl}-amino)-acetic acid 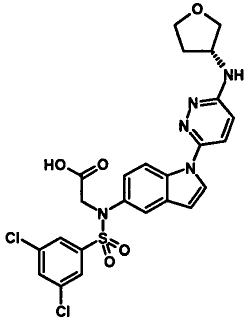 Mass spectrum (ESI⁺): m/z = 562 [M+H]⁺
   R_{f} value: 0.20 (silica gel: toluene/dioxane/ethanol/glacial acetic acid 90:10:10:6)
(70) ((3,5-dichloro-phenylsulphony)-{1-[6-(3-oxo-morpholin-4-yl)-pyridazin-3-yl]-1H-indol-5-yl}-amino)-acetic acid 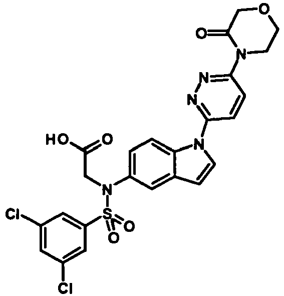 Mass spectrum (ESI⁺): m/z = 576 [M+H]⁺
   R_{f} value: 0.53 (silica gel: toluene/dioxane/ethanol/glacial acetic acid 90:10:10:6)
(71) (R)-((3,5-dichloro-phenylsulphonyl)-{1-[5-(tetrahydro-furan-3-yloxy)-pyrazin-2-yl]-1H-indol-5-yl}-amino)-acetic acid 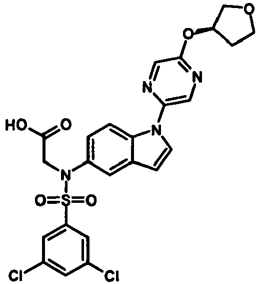 Mass spectrum (ESI⁻): m/z = 561 [M-H]⁻
   R_{f} value: 0.35 (silica gel: dichloromethane/methanol/acetic acid 98:2:0.1)
(72) (S)-((3,5-dichloro-phenylsulphony)-{1-[6-(tetrahydro-furan-3-yloxy)-pyridazin-3-yl]-1H-indol-5-yl}-amino)-acetic acid 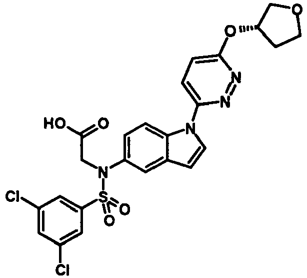 Mass spectrum (ESI⁻): m/z = 561 [M-H]⁻
   R_{f} value: 0.20 (silica gel: dichloromethane/methanol/acetic acid 98:2:0.1)
(73) ((3,5-dichloro-phenylsulphonyl)-{1-[6-(tetrahydro-pyran-4-yloxy)-pyridazin-3-yl]-1H-indol-5-yl}-amino)-acetic acid 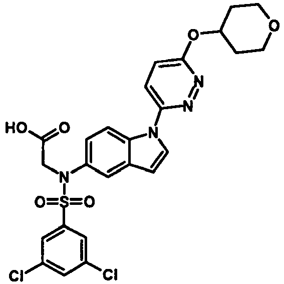 Mass spectrum (ESI⁺): m/z = 577 [M+H]⁺
   R_{f} value: 0.25 (silica gel: dichloromethane/methanol/acetic acid 98:2:0.1)
(74) (R)-((3,5-dichloro-phenylsulphonyl)-{1-[6-(tetrahydro-furan-3-yloxy)-pyridazin-3-yl]-1H-indol-5-yl}-amino)-acetic acid 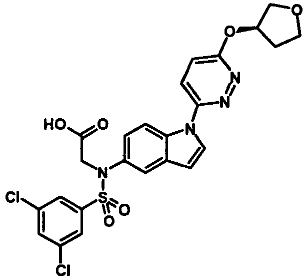 Mass spectrum (ESI⁻): m/z = 561 [M-H]⁻
   R_{f} value: 0.25 (silica gel: dichloromethane/methanol/acetic acid 98:2:0.1)
(75) ((3,5-dichloro-phenylsutphonyl)-{1-[5-(3-oxo-morpholin-4-yl)-pyridin-2-yl]-1H-indol-5-yl}-amino)-acetic acid 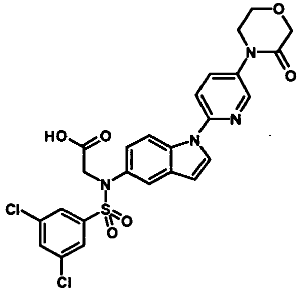 Mass spectrum (ESI⁺): m/z = 575 [M+H]⁺
   R_{f} value: 0.40 (silica gel: toluene/dioxane/ethanol/glacial acetic acid 90:10:10:6)
(76) (R)-((3,5-dichloro-phenylsulphonyl)-{1-[5-(tetrahydro-furan-3-ylamino)-pyrazin-2-yl]-1H-indol-5-yl}-amino)-acetic acid 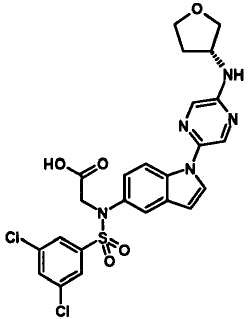 Mass spectrum (ESI⁺): m/z = 562 [M+H]⁺
   R_{f} value: 0.46 (silica gel: toluene/dioxane/ethanol/glacial acetic acid 90:10:10:6)
(77) ((3,5-dichloro-phenylsulphonyl)-{1-[4-(3-oxo-morpholin-4-yl)-pyridin-2-yl]-1H-indol-5-yl}-amino)-acetic acid 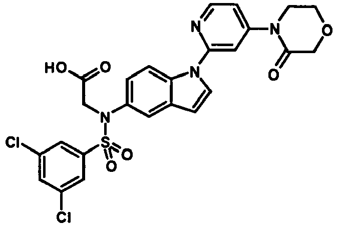 Mass spectrum (ESI⁺): m/z = 575 [M+H]⁺
   R_{f} value: 0.44 (silica gel: toluene/dioxane/ethanol/glacial acetic acid 90:10:10:6)
(78) [(3,5-dichloro-phenylsulphonyl)-(1-{6-[methyl-(tetrahydro-pyran-4-yl)-amino]-pyridazin-3-yl}-1H-indol-5-yl)-amino]-acetic acid 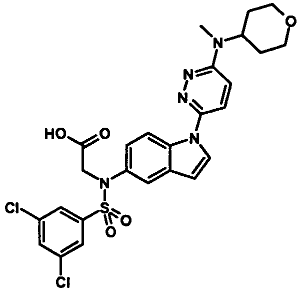 Mass spectrum (ESI⁺): m/z = 590 [M+H]⁺
   R_{f} value: 0.30 (silica gel: toluene/dioxane/ethanol/glacial acetic acid 90:10:10:6)
(79) ((3,5-dichloro-phenylsulphonyl)-{1-[5-(3-oxo-piperazin-1-yl)-pyrazin-2-yl]-1H-indol-5-yl}-amino)-acetic acid 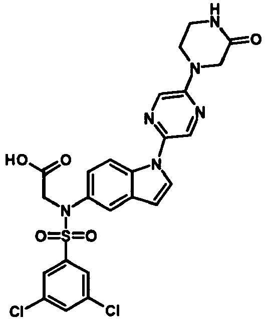 Mass spectrum (ESI⁻): m/z = 573 [M-H]⁻
   R_{f} value: 0.27 (silica gel: dichloromethane/methanol/acetic acid 95:5:0.1)
(80) ((3,5-dichloro-phenylsulphonyl)-{1-[5-(tetrahydro-pyran-4-ylamino)-pyrazin-2-yl]-1H-indol-5-yl}-amino)-acetic acid 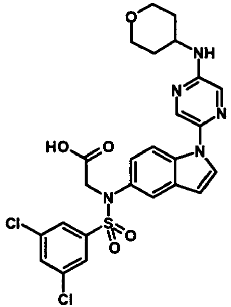 Mass spectrum (ESI⁺): m/z = 576 [M+H]⁺
   R_{f} value: 0.45 (silica gel: toluene/dioxane/ethanol/glacial acetic acid 90:10:10:6)
(81) ((3,5-dichloro-phenylsulphonyl)-{1-[6-(tetrahydro-pyran-4-ylamino)-pyridazin-3-yl]-1H-indol-5-yl}-amino)-acetic acid 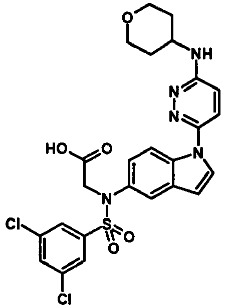 Mass spectrum (ESI⁺): m/z = 576 [M+H]⁺
   R_{f} value: 0.10 (silica gel: toluene/dioxane/ethanol/glacial acetic acid 90:10:10:6)
(82) {(3,5-dichloro-phenylsulphonyl)-[1-(6-methyl-pyridazin-3-yl)-1H-indazol-5-yl]-amino}-acetic acid 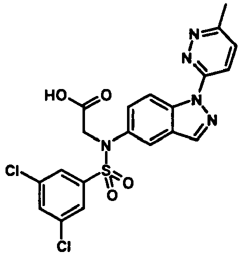 Mass spectrum (ESI⁺): m/z = 492 [M+H]⁺
   R_{f} value: 0.54 (silica gel: toluene/dioxane/ethanol/glacial acetic acid 90:10:10:6)
(83) ((3,5-dichloro-phenylsulphonyl)-{1-[5-(tetrahydro-furan-3-yloxy)-pyridin-2-yl]-1H-indol-5-yl}-amino)-acetic acid 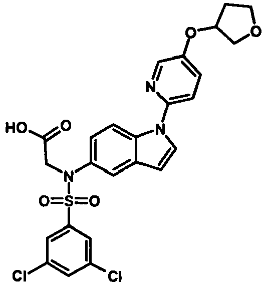 Mass spectrum (ESI⁺): m/z = 562 [M+H]⁺
   R_{f} value: 0.43 (silica gel: dichloromethane/methanol/acetic acid 95:5:0.1)
(84) ((3,5-dichloro-phenylsulphonyl)-{1-[5-(tetrahydro-pyran-4-yloxy)-pyridin-2-yl]-1H-indol-5-yl}-amino)-acetic acid 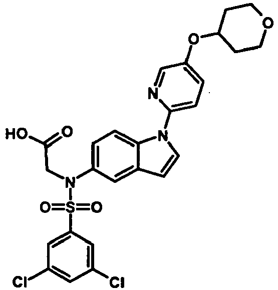 Mass spectrum (ESI⁺): m/z = 576 [M+H]⁺
   R_{f} value: 0.58 (silica gel: dichloromethane/methanol/acetic acid 95:5:0.1)
(85) ((3,5-dichloro-phenylsulphonyl)-{1-[4-(tetrahydro-furan-3-yloxy)-pyridin-2-yl]-1H-indol-5-yl}-amino)-acetic acid 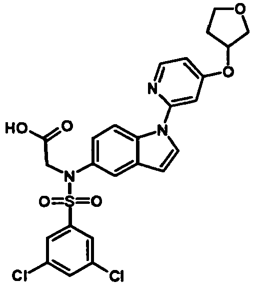 Mass spectrum (ESI⁺): m/z = 562 [M+H]⁺
   R_{f} value: 0.50 (silica gel: dichloromethane/methanol/acetic acid 95:5:0.1)
(86) [(3,5-dichloro-phenylsulphonyl)-(1-{5-[methyl-(tetrahydro-pyran-4-yl)-amino]-pyrazin-2-yl}-1H-indol-5-yl)-amino]-acetic acid 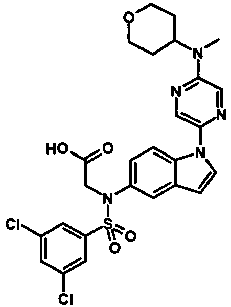 Mass spectrum (ESI⁺): m/z = 590 [M+H]⁺
   R_{f} value: 0.63 (silica gel: toluene/dioxane/ethanol/glacial acetic acid 90:10:10:6)
(87) ((3,5-dichloro-phenylsulphonyl)-{3-iodo-1-[6-(3-oxo-piperazin-1-yl)-pyridazin-3-yl]-1H-indol-5-yl}-amino)-acetic acid 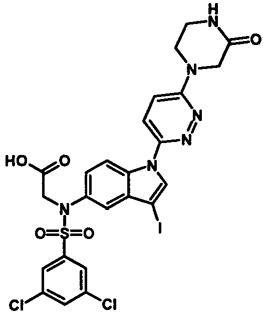 Mass spectrum (ESI⁺): m/z = 701 [M+H]⁺
(88) ((3,5-dichloro-phenylsulphonyl)-{1-[6-(3-oxo-piperazin-1-yl)-pyridazin-3-yl]-1H-indol-5-yl}-amino)-acetic acid 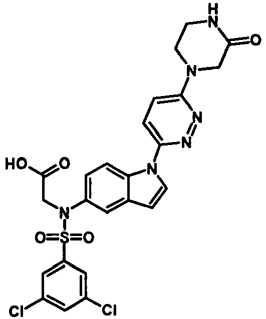 Mass spectrum (ESI⁺): m/z = 575 [M+H]⁺
(89) ((3,5-dichloro-phenylsulphonyl)-{1-[4-(tetrahydro-pyran-4-yloxy)-pyridin-2-yl]-1H-indol-5-yl}-amino)-acetic acid 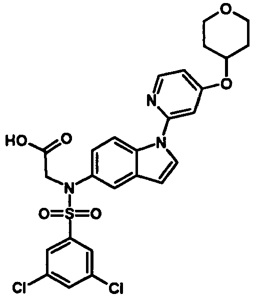 Mass spectrum (ESI⁻): m/z = 574 [M-H]⁻
   R_{f} value: 0.50 (silica gel: dichloromethane/methanol/acetic acid 95:5:0.1)
(90) [[6-chloro-1-(6-methyl-pyridazin-3-yl)-1H-indol-5-yl]-(3,5-dichlorophenylsulphonyl)-amino]-acetic acid 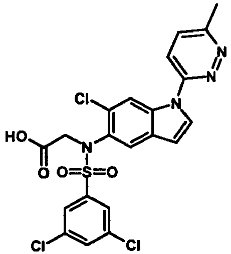 Mass spectrum (ESI⁺): m/z = 525 [M+H]⁺
   R_{f} value: 0.20 (silica gel: dichloromethane/methanol/acetic acid 98:2:0.1)
(91) [[3-methyl-1-(6-methyl-pyridazin-3-yl)-1H-indazol-5-yl]-(3,5-dichlorophenylsulphonyl)-amino]-acetic acid 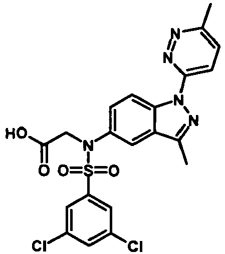 Mass spectrum (ESI⁺): m/z = 506 [M+H]⁺
   R_{f} value: 0.30 (silica gel: dichloromethane/methanol 95:5)
(92) {(3,5-dichloro-phenylsulphonyl)-[1-(5-trifluoromethyl-pyridazin-3-yl)-1H-indol-5-yl]-amino}-acetic acid 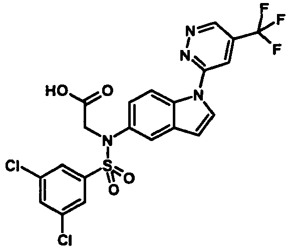 Mass spectrum (ESI⁺): m/z = 545 [M+H]⁺
   R_{f} value: 0.37 (silica gel: dichloromethane/methanol 95:5)
(93) {(3,5-dichloro-phenylsulphonyl)-[3-iodo-1-(5-trifluoromethyl-pyridazin-3-yl)-1H-indol-5-yl]-amino}-acetic acid 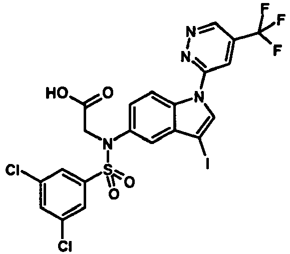 Mass spectrum (ESI⁺): m/z = 671 [M+H]⁺
   R_{f} value: 0.35 (silica gel: dichloromethane/methanol 95:5)
(94) {(3,5-dichloro-phenylsulphonyl)-[1-(6-thiazol-2-yl-pyrimidin-4-yl)-1H-indol-5-yl]-amino}-acetic acid 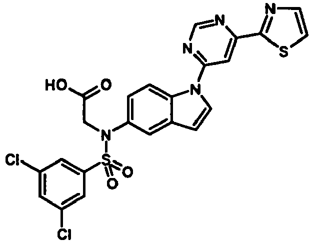 Mass spectrum (ESI⁺): m/z = 560 [M+H]⁺
   R_{f} value: 0.30 (silica gel: dichloromethane/methanol 95:5)
(95) [[6-ethyl-1-(6-methyl-pyridazin-3-yl)-1H-indol-5-yl]-(3,5-dichlorophenylsulphonyl)-amino]-acetic acid 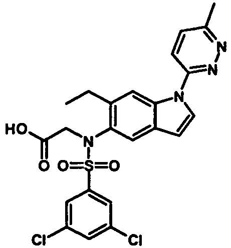 Mass spectrum (ESI⁺): m/z = 519 [M+H]⁺
   R_{f} value: 0.53 (silica gel: dichloromethane/methanol/acetic acid 95:5:0.1)
(96) {(3,5-dichloro-phenylsulphonyl)-[1-(6-methylamino-pyridazin-3-yl)-1H-indol-5-yl]-amino}-acetic acid · CF₃CO₂H 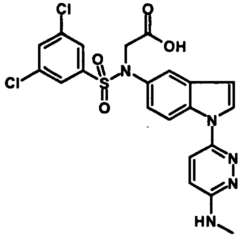 Mass spectrum (ESI⁺): m/z = 505 [M+H]⁺
(97) {(3,5-dichloro-phenylsulphonyl)-[3-iodo-1-(6-methylamino-pyridazin-3-yl)-1H-indol-5-yl]-amino}-acetic acid · CF₃CO₂H 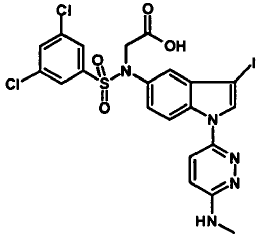 Mass spectrum (ESI⁺): m/z = 632 [M+H]⁺
(98) ((3,5-dichloro-phenylsulphonyl)-{3-iodo-1-[6-(2-oxo-imidazolidin-1-yl)-pyridazin-3-yl]-1H-indol-5-yl}-amino)-acetic acid 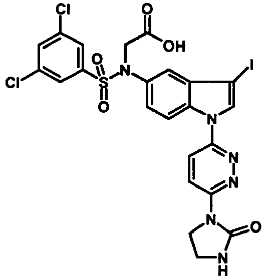 Mass spectrum (ESI⁺): m/z = 687 [M+H]⁺
(99) [[1-(5-cyano-pyridin-2-yl)-1H-indol-5-yl]-(3,5-dichloro-phenylsulphonyl)-amino]-acetic acid 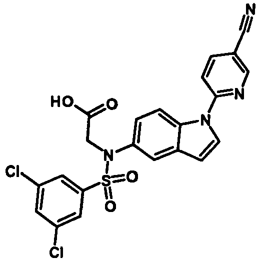 Mass spectrum (ESI⁺): m/z = 501 [M+H]⁺
(100) [[1-(4-amino-pyridin-2-yl)-1H-indol-5-yl]-(3,5-dichloro-phenylsulphonyl)-amino]-acetic acid · CF₃CO₂H 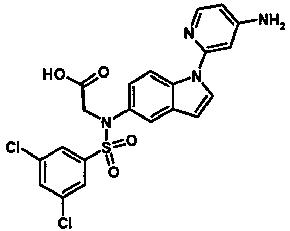 Mass spectrum (ESI⁺): m/z = 491 [M+H]⁺
(101) [[1-(6-cyano-pyridin-2-yl)-1H-indol-5-yl]-(3,5-dichloro-phenylsulphonyl)-amino]-acetic acid 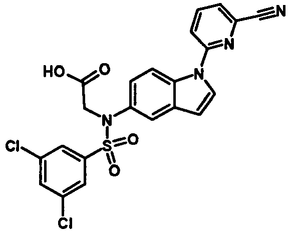 Mass spectrum (ESI⁺): m/z = 501 [M+H]⁺
(102) ((3,5-dichloro-phenylsulphonyl)-{1-[6-(2-dimethylamino-ethoxy)-pyridazin-3-yl]-1H-indol-5-yl}-amino)-acetic acid · CF₃CO₂H 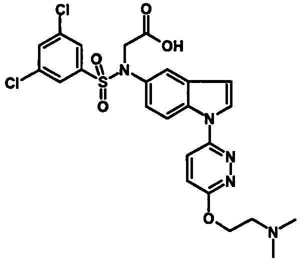 Mass spectrum (ESI⁺): m/z = 564 [M+H]⁺
(103) ethyl [[1-(6-[1,4]diazepan-1-yl-pyridazin-3-yl)-1H-indol-5-yl]-(3,5-dichlorophenylsulphonyl)-amino]-acetate · CF₃CO₂H 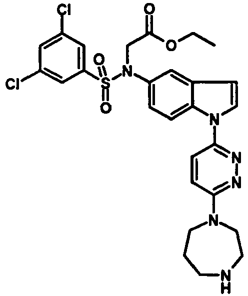 Mass spectrum (ESI⁺): m/z = 603 [M+H]⁺
(104) {(3,5-dichloro-phenylsulphonyl)-[1-(5-morpholin-4-yl-pyrazin-2-yl)-1H-indol-5-yl]-amino}-acetic acid 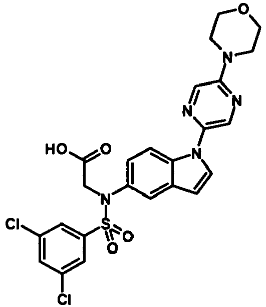 Mass spectrum (ESI⁺): m/z = 562 [M+H]⁺
(105) ((3,5-dichloro-phenylsulphonyl)-{1-[6-(3-methylamino-propylamino)-pyridazin-3-yl]-1H-indol-5-yl}-amino)-acetic acid · CF₃CO₂H 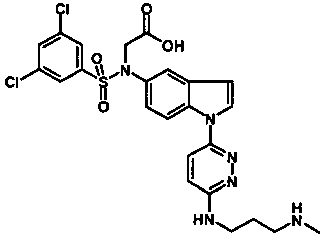 Mass spectrum (ESI⁺): m/z = 563 [M+H]⁺
(106) [(1-{6-[(3-amino-propyl)-methyl-amino]-pyridazin-3-yl}-1H-indol-5-yl)-(3,5-dichloro-phenylsulphonyl)-amino]-acetic acid · CF₃CO₂H 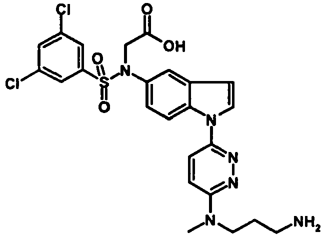 Mass spectrum (ESI⁺): m/z = 563 [M+H]⁺
(107) {(3,5-dichloro-phenylsulphonyl)-[1-(1,3-dimethyl-2,4-dioxo-1,2,3,4-tetrahydro-pyrimidin-5-yl)-1H-indol-5-yl]-amino}-acetic acid 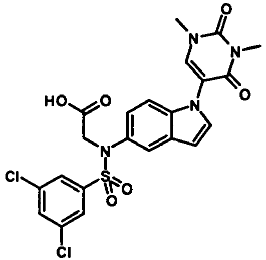 Mass spectrum (ESI⁻): m/z = 535 [M-H]⁻
(108) {(3,5-dichloro-phenylsulphonyl)-[1-(6-oxo-1,6-dihydro-pyridazin-3-yl)-1H-indol-5-yl]-amino}-acetic acid 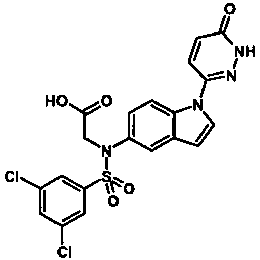 Mass spectrum (ESI⁺): m/z = 493 [M+H]⁺
(109) ((3,5-dichloro-phenylsulphonyl)-{1-[4-(morpholine-4-carbonyl)-pyridin-2-yl]-1H-indol-5-yl}-amino)-acetic acid 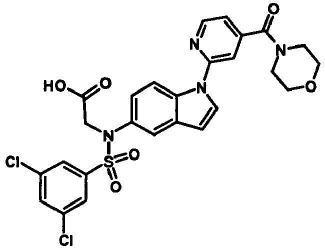 Mass spectrum (ESI⁺): m/z = 589 [M+H]⁺
(110) [[1-(3-cyano-pyridin-2-yl)-1H-indol-5-yl]-(3,5-dichloro-phenylsulphonyl)-amino]-acetic acid 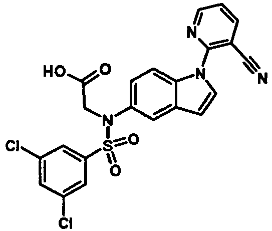 Mass spectrum (ESI⁻): m/z = 499 [M-H]⁻
(111) {(3,5-dichloro-phenylsulphonyl)-[4-methyl-1-(6-morpholin-4-yl-pyridazin-3-yl)-1H-indol-5-yl]-amino}-acetic acid 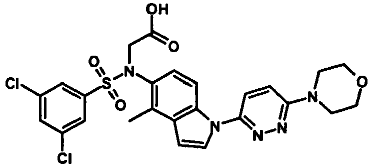 Mass spectrum (ESI⁺): m/z = 576 [M+H]⁺
(112) ((3,5-dichloro-phenylsulphonyl)-{1-[6-(2-hydroxy-ethoxy)-pyridazin-3-yl]-1H-indol-5-yl}-amino)-acetic acid 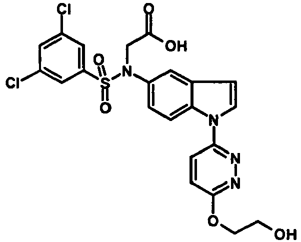 Mass spectrum (ESI⁺): m/z = 537 [M+H]⁺
(113) [[1-(2-chloro-9H-purin-6-yl)-1H-indol-5-yl]-(3,5-dichloro-phenylsulphonyl)-amino]-acetic acid 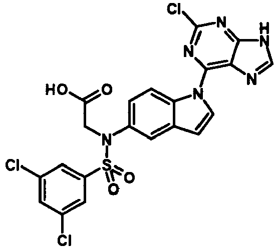 Mass spectrum (ESI⁻): m/z = 549 [M-H]⁻
(114) ((3,5-dichloro-phenylsulphonyl)-{1-[6-(2-pyrrolidin-1-yl-ethylamino)-pyridazin-3-yl]-1H-indol-5-yl}-amino)-acetic acid · CF₃CO₂H 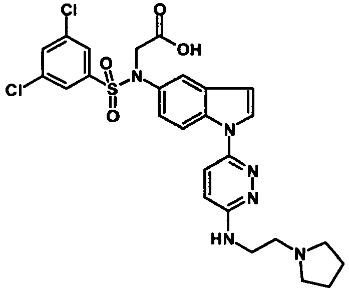 Mass spectrum (ESI⁺): m/z = 589 [M+H]⁺
(115) ((3,5-dichloro-phenylsulphonyl)-{1-[6-(2-oxo-imidazolidin-1-yl)-pyridazin-3-yl]-1H-indol-5-yl}-amino)-acetic acid 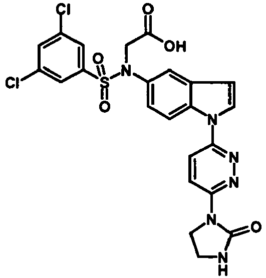 Mass spectrum (ESI⁺): m/z = 561 [M+H]⁺
(116) {(3,5-dichloro-phenylsulphonyl)-[1-(6-methylsulphanyl-pyridazin-3-yl)-1H-indol-5-yl]-amino}-acetic acid 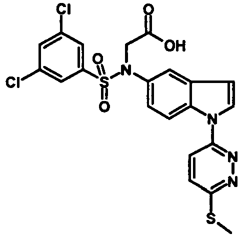 Mass spectrum (ESI⁺): m/z = 523 [M+H]⁺
(117) {(3,5-dichloro-phenylsulphonyl)-[1-(4-methylcarbamoyl-pyridin-2-yl)-1H-indol-5-yl]-amino}-acetic acid 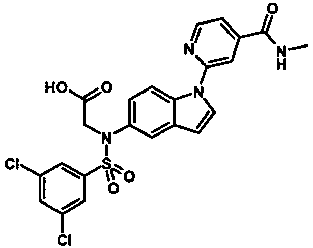 Mass spectrum (ESI⁻): m/z = 531 [M-H]⁻
(118) ((3,5-dichloro-phenylsulphonyl)-{1-[4-(2-methoxy-ethylcarbamoyl)-pyridin-2-yl]-1H-indol-5-yl}-amino)-acetic acid 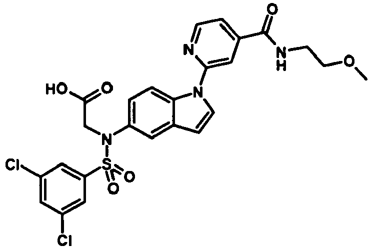 Mass spectrum (ESI⁺): m/z = 577 [M+H]⁺
(119) [[1-(4-cyano-pyridin-2-yl)-1H-indol-5-yl]-(3,5-dichloro-phenylsulphonyl)-amino]-acetic acid 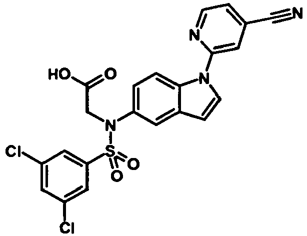 Mass spectrum (ESI⁻): m/z = 499 [M-H]⁻
(120) {(3,5-dichloro-phenylsulphonyl)-[3-methyl-1-(6-methyl-pyridazin-3-yl)-1H-indol-5-yl]-amino}-acetic acid 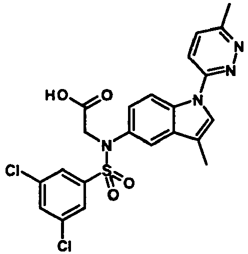 Mass spectrum (ESI⁺): m/z = 505 [M+H]⁺
(121) {(3,5-dichloro-phenylsulphonyl)-[1-(6-thiomorpholin-4-yl-pyridazin-3-yl)-1H-indol-5-yl]-amino}-acetic acid 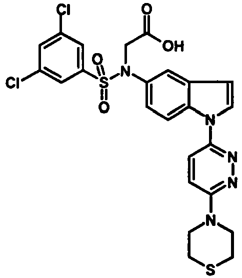 Mass spectrum (ESI⁺): m/z = 578 [M+H]⁺
(122) {(3,5-dichloro-phenylsulphonyl)-[1-(6-methanesulphinyl-pyridazin-3-yl)-1H-indol-5-yl]-amino}-acetic acid 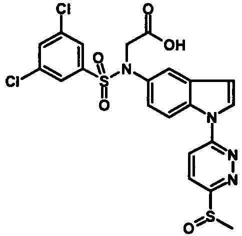 Mass spectrum (ESI⁻): m/z = 537 [M-H]⁻
(123) [[3-chloro-1-(6-methyl-pyridazin-3-yl)-1H-indol-5-yl]-(3,5-dichlorophenylsulphonyl)-amino]-acetic acid 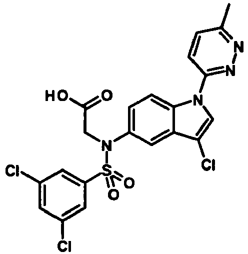 Mass spectrum (ESI⁺): m/z = 525 [M+H]⁺
(124) ((3,5-dichloro-phenylsulphonyl)-{1-[6-(1,1-dioxo-1λ⁶-thiomorpholin-4-yl)-pyridazin-3-yl]-1H-indol-5-yl}-amino)-acetic acid 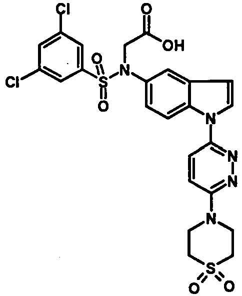 Mass spectrum (ESI⁺): m/z = 610 [M+H]⁺
(125) ((3,5-dichloro-phenylsulphonyl)-{1-[6-(2,3-dihydroxy-propoxy)-pyridazin-3-yl]-1H-indol-5-yl}-amino)-acetic acid 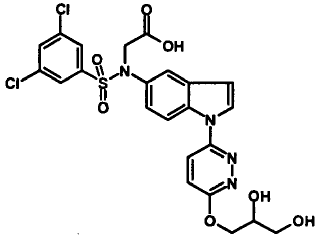 Mass spectrum (ESI⁺): m/z = 567 [M+H]⁺
(126) {(3,5-dichloro-phenylsulphonyl)-[1-(5-methylsulphanyl-pyrazin-2-yl)-1H-indol-5-yl]-amino}-acetic acid 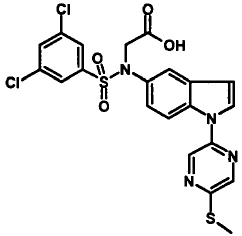 Mass spectrum (ESI⁻): m/z = 521 [M-H]⁻
(127) {(3,5-dichloro-phenylsulphonyl)-[1-(5-methylsulphanyl-pyridin-2-yl)-1H-indol-5-yl]-amino}-acetic acid 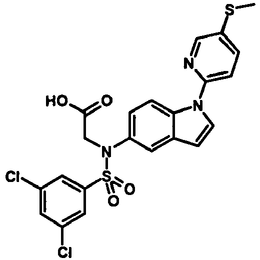 Mass spectrum (ESI⁺): m/z = 522 [M+H]⁺
(128) {(3,5-dichloro-phenylsulphonyl)-[1-(5-methanesulphinyl-pyrazin-2-yl)-1H-indol-5-yl]-amino}-acetic acid 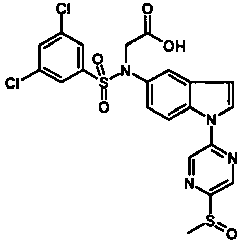 Mass spectrum (ESI⁺): m/z = 539 [M+H]⁺
(129) {(3,5-dichloro-phenylsulphonyl)-[1-(5-methanesulphonyl-pyrazin-2-yl)-1H-indol-5-yl]-amino}-acetic acid 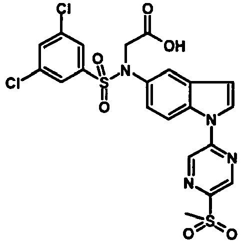 Mass spectrum (ESI⁻): m/z = 553 [M-H]⁻
(130) {(3,5-dichloro-phenylsulphonyl)-[1-(6-methanesulphonyl-pyridazin-3-yl)-1H-indol-5-yl]-amino}-acetic acid 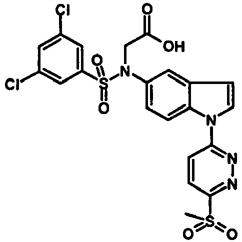 Mass spectrum (ESI⁺): m/z = 555 [M+H]⁺
(131) {(3,5-dichloro-phenylsulphonyl)-[1-(5-methanesulphinyl-pyridin-2-yl)-1H-indol-5-yl]-amino}-acetic acid 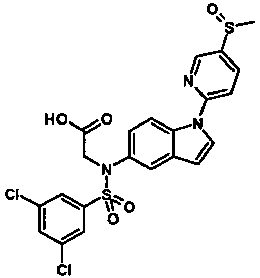 Mass spectrum (ESI⁺): m/z = 538 [M+H]⁺
(132) {(3,5-dichloro-phenylsulphonyl)-[1-(5-methanesulphonyl-pyridin-2-yl)-1H-indol-5-yl]-amino}-acetic acid 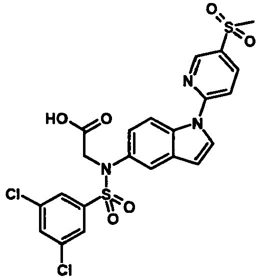 Mass spectrum (ESI⁻): m/z = 552 [M-H]⁻
(133) ((3,5-dichloro-phenylsulphonyl)-{1-[5-(2,3-dihydroxy-propoxy)-pyrazin-2-yl]-1H-indol-5-yl}-amino)-acetic acid 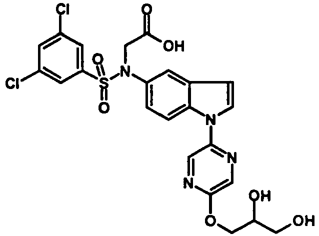 Mass spectrum (ESI⁺): m/z = 567 [M+H]⁺
(134) [(3,5-dichloro-phenylsulphonyl)-(1-{5-[2-(tetrahydro-pyran-2-yloxy)-ethylamino]-pyrazin-2-yl}-1H-indol-5-yl)-amino]-acetic acid 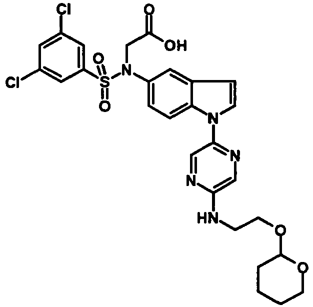 Mass spectrum (ESI⁺): m/z = 620 [M+H]⁺
(135) ((3,5-dichloro-phenylsulphonyl)-{1-[5-(2-dimethylamino-ethoxy)-pyrazin-2-yl]-1H-indol-5-yl}-amino)-acetic acid · CF₃CO₂H 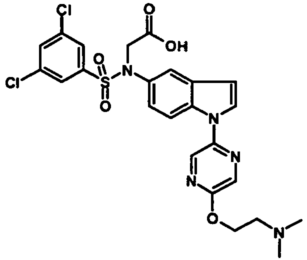 Mass spectrum (ESI⁺): m/z = 564 [M+H]⁺
(136) {(3,5-dichloro-phenylsulphonyl)-[1-(4-methanesulphinyl-pyridin-2-yl)-1H-indol-5-yl]-amino}-acetic acid 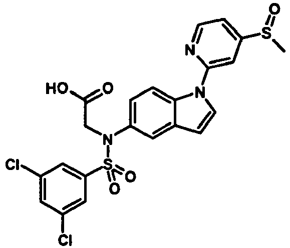 Mass spectrum (ESI⁺): m/z = 538 [M+H]⁺
(137) [{1-[5-(2-amino-ethoxy)-pyrazin-2-yl]-1H-indol-5-yl}-(3,5-dichlorophenylsulphonyl)-amino]-acetic acid · CF₃CO₂H 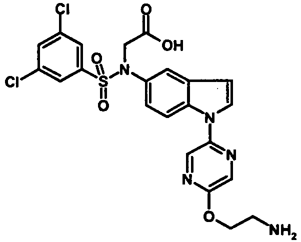 Mass spectrum (ESI⁺): m/z = 536 [M+H]⁺
(138) ((3,5-dichloro-phenylsulphonyl)-{1-[6-(1-oxo-1λ⁴-thiomorpholin-4-yl)-pyridazin-3-yl]-1H-indol-5-yl}-amino)-acetic acid 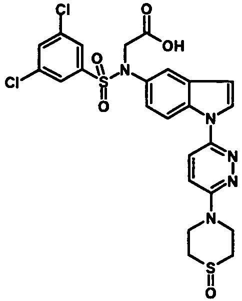 Mass spectrum (ESI⁺): m/z = 594 [M+H]⁺
(139) {(3,5-dichloro-phenylsulphonyl)-[3-ethyl-1-(6-methyl-pyridazin-3-yl)-1H-indol-5-yl]-amino}-acetic acid 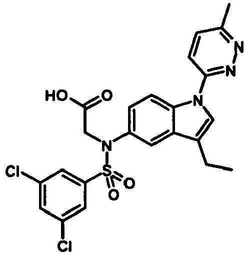 Mass spectrum (ESI⁺): m/z = 519 [M+H]⁺
(140) ((3,5-dichloro-phenylsulphonyl)-{1-[5-(1-oxo-1λ⁴-thiomorpholin-4-yl)-pyrazin-2-yl]-1H-indol-5-yl}-amino)-acetic acid 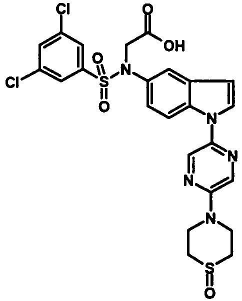 Mass spectrum (ESI⁺): m/z = 594 [M+H]⁺
(141) [[1-(5-carbamoyl-pyrazin-2-yl)-1H-indol-5-yl]-(3,5-dichlorophenylsulphonyl)-amino]-acetic acid 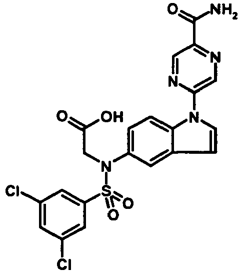 Mass spectrum (ESI⁺): m/z = 520 [M+H]⁺
(142) 2-{5-[carboxymethyl-(3,5-dichloro-phenylsulphonyl)-amino]-indol-1-yl}-isonicotinic acid 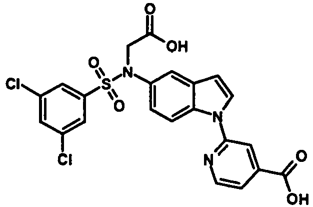 Mass spectrum (ESI⁺): m/z = 520 [M+H]⁺
(143) {(3,5-dichloro-phenylsulphonyl)-[1-(4-thiazol-2-yl-pyridin-2-yl)-1H-indol-5-yl]-amino}-acetic acid 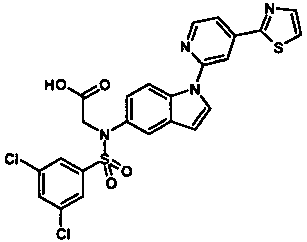 Mass spectrum (ESI⁺): m/z = 559 [M+H]⁺
(144) [{1-[5-(2-amino-ethylamino)-pyrazin-2-yl]-1H-indol-5-yl}-(3,5-dichlorophenylsulphonyl)-amino]-acetic acid · CF₃CO₂H 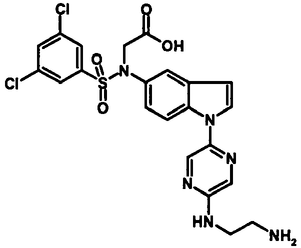 Mass spectrum (ESI⁺): m/z = 535 [M+H]⁺
(145) [[1-(4-cyclopropylcarbamoyl-pyridin-2-yl)-1H-indol-5-yl]-(3,5-dichlorophenylsulphonyl)-amino]-acetic acid 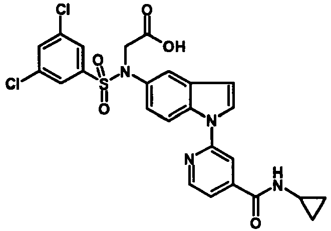 Mass spectrum (ESI⁺): m/z = 559 [M+H]⁺
(146) methyl 2-{5-[carboxymethyl-(3,5-dichloro-phenylsulphonyl)-amino]-indol-1-yl}-isonicotinate 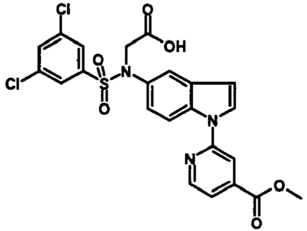 The crude product is purified by preparative HPLC.
   Mass spectrum (ESI⁺): m/z = 534 [M+H]⁺
(147) {(3,5-dichloro-phenylsulphonyl)-[1-(5-methylcarbamoyl-pyrazin-2-yl)-1H-indol-5-yl]-amino}-acetic acid 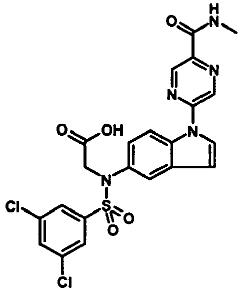 Mass spectrum (ESI⁺): m/z = 534 [M+H]⁺
(148) ((3,5-dichloro-phenylsulphonyl)-{1-[5-(morpholine-4-carbonyl)-pyrazin-2-yl]-1H-indol-5-yl}-amino)-acetic acid 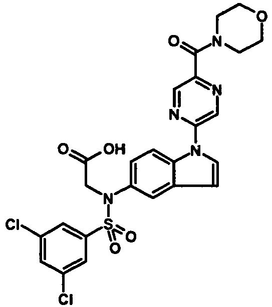 Mass spectrum (ESI⁺): m/z = 590 [M+H]⁺
(149) ((3,5-dichloro-phenylsulphonyl)-{1-[4-(methoxy-methyl-carbamoyl)-pyridin-2-yl]-1H-indol-5-yl}-amino)-acetic acid 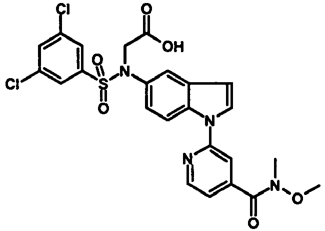 The crude product is purified by preparative HPLC.
   Mass spectrum (ESI⁺): m/z = 563 [M+H]⁺
(150) {(3,5-dichloro-phenylsulphonyl)-[1-(4-ethansulphinyl-pyridin-2-yl)-1H-indol-5-yl]-amino}-acetic acid 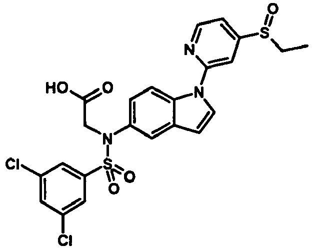 Mass spectrum (ESI⁺): m/z = 552 [M+H]⁺
(151) [(3,5-dichloro-phenylsulphonyl)-(1-imidazo[1,2-a]pyrazin-6-yl-1H-indol-5-yl)-amino]-acetic acid 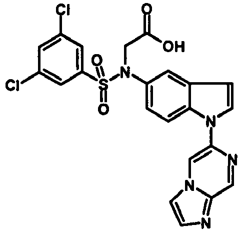 The crude product is purified by preparative HPLC.
   Mass spectrum (ESI⁺): m/z = 516 [M+H]⁺
(152) {(3,5-dichloro-phenylsulphonyl)-[1-(4-oxazol-2-yl-pyridin-2-yl)-1H-indol-5-yl]-amino}-acetic acid 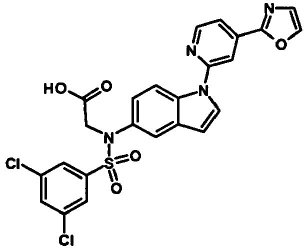 Mass spectrum (ESI⁺): m/z = 543 [M+H]⁺
(153) [[1-(4-acetyl-pyridin-2-yl)-1H-indol-5-yl]-(3,5-dichloro-phenylsulphonyl)-amino]-acetic acid 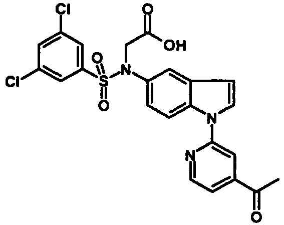 The crude product is purified by preparative HPLC.
   Mass spectrum (ESI⁺): m/z = 518 [M+H]⁺
(154) [[1-(5-cyano-pyrazin-2-yl)-1H-indol-5-yl]-(3,5-dichloro-phenylsulphonyl)-amino]-acetic acid 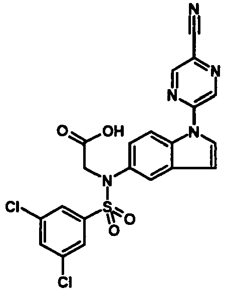 Mass spectrum (ESI⁻): m/z = 500 [M-H]⁻
(155) {(3,5-dichloro-phenylsulphonyl)-[1-(4-propionyl-pyridin-2-yl)-1H-indol-5-yl]-amino}-acetic acid 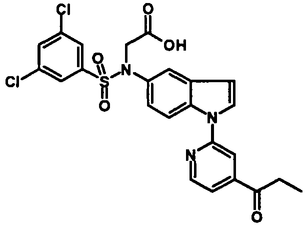 The crude product is purified by preparative HPLC.
   Mass spectrum (ESI⁺): m/z = 532 [M+H]⁺
(156) ((3,5-dichloro-phenylsulphonyl)-{1-[6-(morpholine-4-carbonyl)-pyridazin-3-yl]-1H-indol-5-yl}-amino)-acetic acid 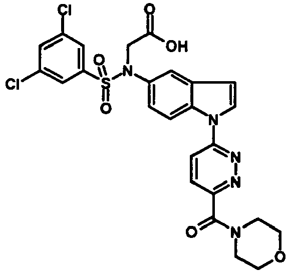 Mass spectrum (ESI⁺): m/z = 590 [M+H]⁺
(157) {(3,5-dichloro-phenylsulphonyl)-[1-(6-dimethylcarbamoyl-pyridazin-3-yl)-1H-indol-5-yl]-amino}-acetic acid 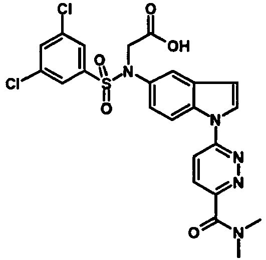 Mass spectrum (ESI⁺): m/z = 548 [M+H]⁺
(158) [[1-(4-cyclopropylcarbamoyl-pyridin-2-yl)-3-methyl-1H-indol-5-yl]-(3,5-dichloro-phenylsulphonyl)-amino]-acetic acid 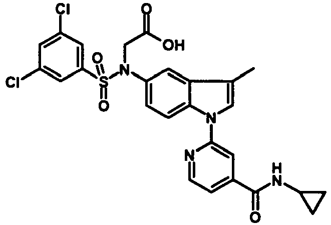 Mass spectrum (ESI⁺): m/z = 573 [M+H]⁺
(159) ((3,5-dichloro-phenylsulphonyl)-{1-[4-(1-methyl-1H-imidazol-2-yl)-pyridin-2-yl]-1H-indol-5-yl}-amino)-acetic acid CF₃CO₂H 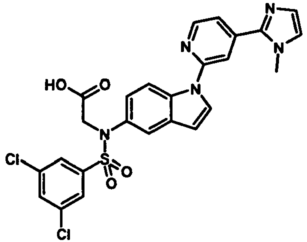 Mass spectrum (ESI⁺): m/z = 556 [M+H]⁺
(160) [[1-(4-aminomethyl-pyridin-2-yl)-1H-indol-5-yl]-(3,5-dichlorophenylsulphonyl)-amino]-acetic acid · CF₃CO₂H 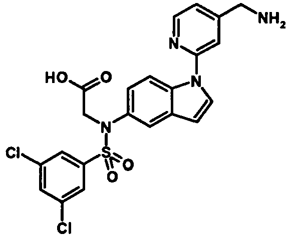 Mass spectrum (ESI⁺): m/z = 505 [M+H]⁺
(161) ((3,5-dichloro-phenylsulphonyl)-{1-[5-(2-dimethylamino-ethylamino)-pyrazin-2-yl]-3-methyl-1H-indol-5-yl}-amino)-acetic acid · CF₃CO₂H 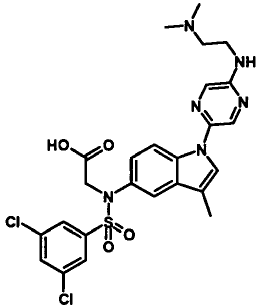 Mass spectrum (ESI⁺): m/z = 577 [M+H]⁺
(162) {(3,5-dichloro-phenylsulphonyl)-[1-(1-methyl-1H-pyrazol-4-yl)-1H-indol-5-yl]-amino}-acetic acid 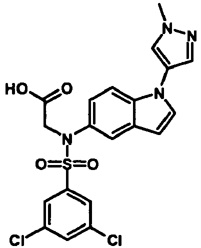 Mass spectrum (ESI⁺): m/z = 479 [M+H]⁺
   R_{f} value: 0.45 (silica gel: dichloromethane/methanol/acetic acid 95:5:0.1)
(163) {(3,5-dichloro-phenylsulphonyl)-[1-(5-thiazol-2-yl-pyridazin-3-yl)-1H-indol-5-yl]-amino}-acetic acid 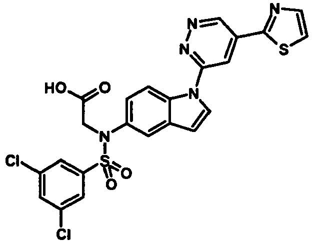 Mass spectrum (ESI⁺): m/z = 560 [M+H]⁺
   R_{f} value: 0.34 (silica gel: dichloromethane/methanol 95:5)
(164) {(3,5-dichloro-phenylsulphonyl)-[1-(1-methyl-1H-imidazol-4-yl)-1H-indol-5-yl]-amino}-acetic acid 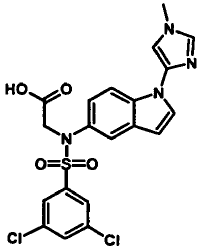 Mass spectrum (ESI⁺): m/z = 479 [M+H]⁺
   R_{f} value: 0.33 (silica gel: dichloromethane/methanol/acetic acid 95:5:0.1)
(165) {(3,5-dichloro-phenylsulphonyl)-[1-(6-trifluoromethyl-pyrimidin-4-yl)-1H-indol-5-yl]-amino}-acetic acid 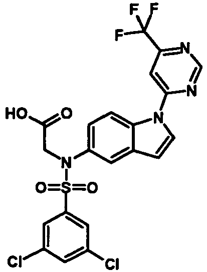 Mass spectrum (ESI⁺): m/z = 545 [M+H]⁺
   R_{f} value: 0.57 (silica gel: dichloromethane/methanol/acetic acid 95:5:0.1)
(166) [(3,5-dichloro-phenylsulphonyl)-(1-thiazol-2-yl-1H-indol-5-yl)-amino]-acetic acid 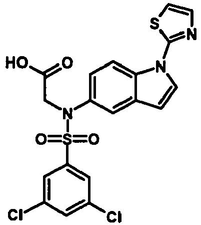 Mass spectrum (ESI⁺): m/z = 482 [M+H]⁺
   R_{f} value: 0.55 (silica gel: dichloromethane/methanol/acetic acid 95:5:0.1)
(167) [[1-(2-cyano-thiophen-3-yl)-1H-indol-5-yl]-(3,5-dichloro-phenylsulphonyl)-amino]-acetic acid 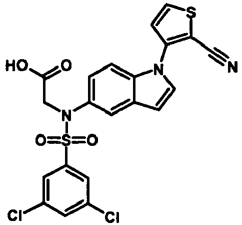 Mass spectrum (ESI⁻): m/z = 504 [M-H]⁻
   R_{f} value: 0.50 (silica gel: dichloromethane/methanol/acetic acid 95:5:0.1)
(168) [[1-(5-acetyl-thiophen-2-yl)-1H-indol-5-yl]-(3,5-dichloro-phenylsulphonyl)-amino]-acetic acid 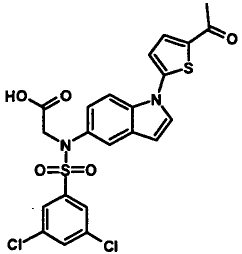 Mass spectrum (ESI⁺): m/z = 523 [M+H]⁺
   R_{f} value: 0.58 (silica gel: dichloromethane/methanol/acetic acid 95:5:0.1)
(169) {(3,5-dichloro-phenylsulphonyl)-[1-(2,6-dimethyl-pyrimidin-4-yl)-1H-indol-5-yl]-amino}-acetic acid 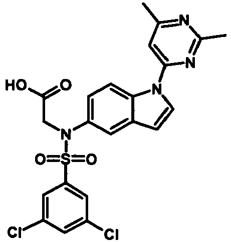 Mass spectrum (ESI⁺): m/z = 505 [M+H]⁺
   R_{f} value: 0.40 (silica gel: dichloromethane/methanol/acetic acid 95:5:0.1)
(170) {(3,5-dichloro-phenylsulphonyl)-[1-(5-methyl-[1,3,4]thiadiazol-2-yl)-1H-indol-5-yl]-amino}-acetic acid 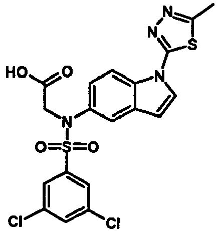 Mass spectrum (ESI⁺): m/z = 497 [M+H]⁺
   R_{f} value: 0.60 (silica gel: dichloromethane/methanol/acetic acid 95:5:0.1)
(171) {(3,5-dichloro-phenylsulphonyl)-[1-(6-methoxy-pyrimidin-4-yl)-1H-indol-5-yl]-amino}-acetic acid 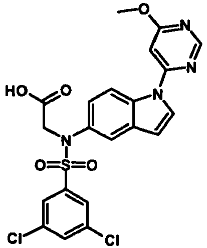 Mass spectrum (ESI⁺): m/z = 507 [M+H]⁺
   R_{f} value: 0.55 (silica gel: dichloromethane/methanol/acetic acid 95:5:0.1)
(172) {(3,5-dichloro-phenylsulphonyl)-[1-(2-thiazol-2-yl-pyrimidin-4-yl)-1H-indol-5-yl]-amino}-acetic acid 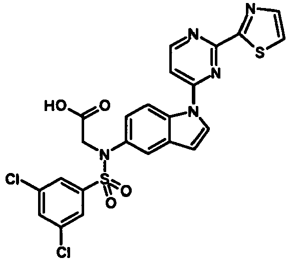 Mass spectrum (ESI⁺): m/z = 560 [M+H]⁺
   R_{f} value: 0.30 (silica gel: toluene/dioxane/ethanol/glacial acetic acid 90:10:10:6)
(173) ethyl 2-(5-{5-[carboxymethyl-(3,5-dichloro-phenylsulphonyl)-amino]-indol-1-yl}-thiophen-2-yl)-thiazole-4-carboxylate 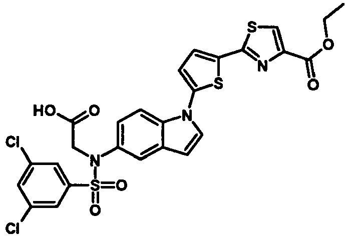 Mass spectrum (ESI⁺): m/z = 636 [M+H]⁺
   R_{f} value: 0.65 (silica gel: toluene/dioxane/ethanol/glacial acetic acid 90:10:10:6)
(174) ((3,5-dichloro-phenylsulphonyl)-{1-[6-(1-methyl-1H-imidazol-2-yl)-pyrimidin-4-yl]-1H-indol-5-yl}-amino)-acetic acid 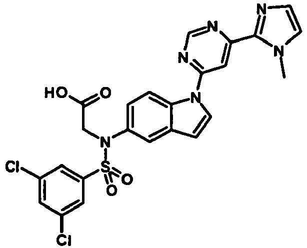 Mass spectrum (ESI⁺): m/z = 557 [M+H]⁺
   R_{f} value: 0.25 (silica gel: dichloromethane/methanol 90:10)
(175) ethyl 5-(5-{5-[carboxymethyl-(3,5-dichloro-phenylsulphonyl)-amino]-indol-1-yl}-thiophen-2-yl)-isoxazol-3-carboxylate 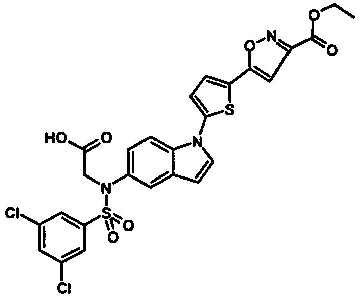 Mass spectrum (ESI⁺): m/z = 620 [M+H]⁺
   R_{f} value: 0.40 (silica gel: dichloromethane/methanol 95:5)
(176) {(3,5-dichloro-phenylsulphonyl)-[3-methyl-1-(4-methyl-pyridin-2-yl)-1H-indol-5-yl]-amino}-acetic acid 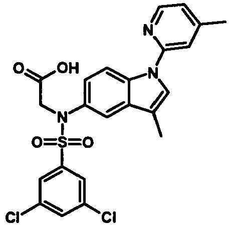 Mass spectrum (ESI⁺): m/z = 507 [M+H]⁺
   R_{f} value: 0.39 (silica gel: dichloromethane/methanol 90:10)
(177) [{3-bromo-1-[6-(2-dimethylamino-ethylamino)-pyridazin-3-yl]-1H-indol-5-yl}-(3,5-dichloro-phenylsulphonyl)-amino]-acetic acid 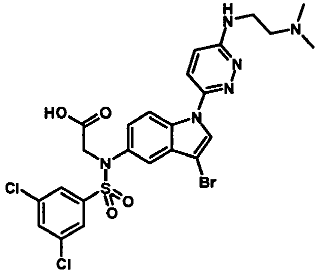 Mass spectrum (ESI⁺): m/z = 641 [M+H]⁺
(178) [(3,5-dichloro-phenylsulphonyl)-(3-pyrimidin-2-yl-3H-benzimidazol-5-yl)-amino]-acetic acid 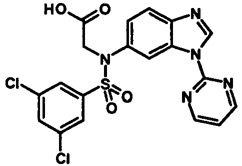 Mass spectrum (ESI⁺): m/z = 478 [M+H]⁺
(179) [(3,5-dichloro-phenylsulphonyl)-(1-pyrimidin-2-yl-1H-benzimidazol-5-yl)-amino]-acetic acid 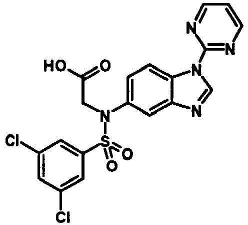 Mass spectrum (ESI⁺): m/z = 478 [M+H]⁺
(180) {(2,6-dimethyl-pyridin-4-sulphonyl)-[1-(6-methyl-pyridazin-3-yl)-1H-indol-5-yl]-amino}-acetic acid · CF₃CO₂H 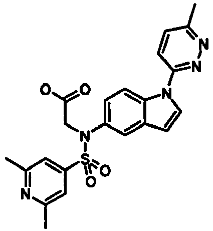 Mass spectrum (ESI⁺): m/z = 452 [M+H]⁺
(181) {(2,6-dichloro-pyridin-4-sulphonyl)-[1-(6-methyl-pyridazin-3-yl)-1H-indol-5-yl]-amino}-acetic acid 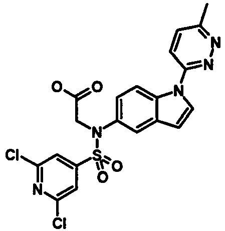 Mass spectrum (ESI⁺): m/z = 492 [M+H]⁺
(182) {(2-chloro-6-methyl-pyridin-4-sulphonyl)-[1-(6-methyl-pyridazin-3-yl)-1H-indol-5-yl]-amino}-acetic acid 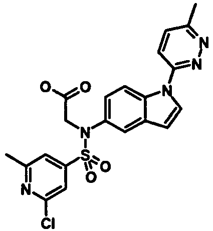 Mass spectrum (ESI⁺): m/z = 472 [M+H]⁺
(183) {(2,6-dichloro-pyridin-4-sulphonyl)-[1-(4-methylcarbamoyl-pyridin-2-yl)-1H-indol-5-yl]-amino}-acetic acid 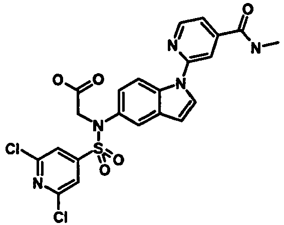 The product is purified by preparative HPLC (Kromasil-C-18 column, water/acetonitrile 90:10 to 0:100).
   Mass spectrum (ESI⁺): m/z = 534 [M+H]⁺

### Example 2

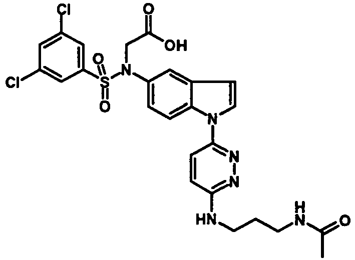

### [{1-[6-(3-acetylamino-propylamino)-pyridazin-3-yl]-1H-indol-5-yl}-(3,5-dichlorophenylsulphonyl)-amino]-acetic acid

130 mg [{1-[6-(3-amino-propylamino)-pyridazin-3-yl]-1H-indol-5-yl}-(3,5-dichlorophenylsulphonyl)-amino]- acetic acid. CF₃CO₂H are dissolved in 2 ml of tetrahydrofuran. 100 µl pyridine and 20 µl acetanhydride are added and the mixture is stirred for 3 hours at ambient temperature. The volatile constituents are eliminated in vacuo, the residue is extracted from water and the solid thus obtained is purified by chromatography on silica gel with dichloromethane/methanol (99:5 to 60:40). The product thus obtained is extracted from diethyl ether.
Yield: 70 mg (60 % of theory)
Mass spectrum (ESI⁺): m/z = 591 [M+H]⁺

The following compounds are obtained analogously to Example 2:
(1) [(1-{6-[(3-acetylamino-propyl)-methyl-amino]-pyridazin-3-yl}-1H-indol-5-yl)-(3,5-dichloro-phenylsulphonyl)-amino]-acetic acid 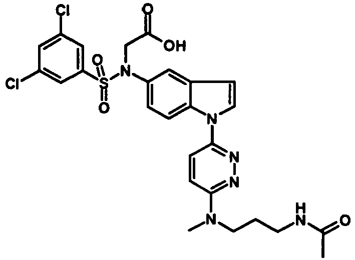 The reaction is carried out in acetic anhydride at 50°C.
   Mass spectrum (ESI⁺): m/z = 605 [M+H]⁺
(2) [[1-(5-acetylamino-pyrazin-2-yl)-1H-indol-5-yl]-(3,5-dichloro-phenylsulphonyl)-amino]-acetic acid 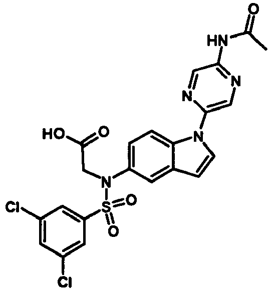 The reaction is carried out in the presence of 3 equivalents of pyridine in tetrahydrofuran.
   Mass spectrum (ESI⁻): m/z = 532 [M-H]⁻
(3) [[1-(4-acetylamino-pyridin-2-yl)-1H-indol-5-yl]-(3,5-dichloro-phenylsulphonyl)-amino]-acetic acid 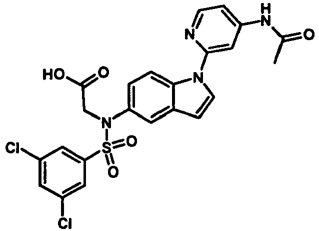 The reaction is carried out in pyridine.
   Mass spectrum (ESI⁺): m/z = 533 [M+H]⁺
(4) [{1-[6-(4-acetyl-piperazin-1-yl)-pyridazin-3-yl]-1H-indol-5-yl}-(3,5-dichlorophenylsulphonyl)-amino]-acetic acid 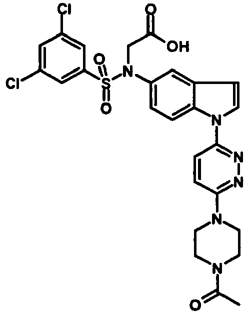
The reaction is carried out in the presence of 5.5 equivalents pyridine in tetrahydrofuran. The crude product is purified by preparative HPLC
Mass spectrum (ESI⁺): m/z = 603 [M+H]⁺

### Example 3

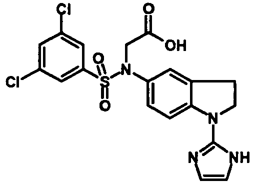

### {(3,5-dichloro-phenylsulphonyl)-[1-(1H-imidazol-2-yl)-2,3-dihydro-1H-indol-5-yl]-amino}-acetic acid · HCl

300 mg tert-butyl 2-{5-[tert-butoxycarbonylmethyl-(3,5-dichloro-phenylsulphonyl)-amino]-2,3-dihydro-indol-1-yl}-imidazole-1-carboxylate are dissolved in 2 ml dichloromethane, 2 ml trifluoroacetic acid are added and the mixture is stirred overnight at ambient temperature. The solvents are eliminated in vacuo and the residue is purified by chromatography on silica gel with dichloromethane/methanol (99:1 to 60:40). The product thus obtained is dissolved in a little dioxane and combined with a 4 N solution of HCl in dioxane. Then some diisopropylether is added and the solvents are eliminated in vacuo. The residue is extracted from diethyl ether and dried in vacuo.
Mass spectrum (ESI⁺): m/z = 467 [M+H]⁺

### Example 4

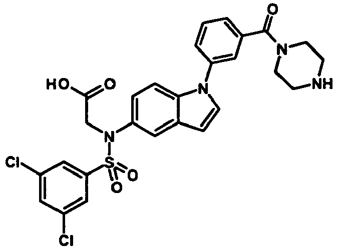

### ((3,5-dichloro-phenylsulphonyl)-{1-[3-(piperazine-1-carbonyl)-phenyl]-1H-indol-5-yl}-amino)-acetic acid · HCl

90 mg tert-butyl 4-(3-{5-[tert-butoxycarbonylmethyl-(3,5-dichlorophenylsulphonyl)-amino]-indol-1-yl}-benzoyl)-piperazine-1-carboxylate are dissolved in 2 ml of a 4 N solution of HCl in dioxane. The mixture is stirred for 7 hours at RT and then combined with diethyl ether. After stirring overnight the precipitated solid is suction filtered and dried in vacuo.
Yield: 55 mg (73 % of theory)
Mass spectrum (ESI⁺): m/z = 578 [M+H]⁺

The following compounds are obtained analogously to Example 4:
(1) [(3,5-dichloro-phenylsulphonyl)-(1-pyridin-4-yl-2,3-dihydro-1H-indol-5-yl)-amino]-acetic acid · HCl 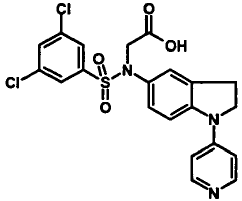 Carried out in dioxane with 1.05 equivalents of HCl at 70°C for 24 hours. Crude product purified by preparative HPLC.
   Mass spectrum (ESI⁺): m/z = 478 [M+H]⁺
(2) ((3,5-dichloro-phenylsulphonyl)-(1-[5-(2-dimethylamino-ethylamino)-pyrazin-2-yl]-1H-indol-5-yl}-amino)-acetic acid · HCl 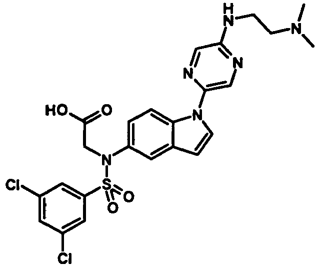 Carried out at 70°C for 3 hours.
   Mass spectrum (ESI⁺): m/z = 563 [M+H]⁺
(3) ((3,5-dichloro-phenylsulphonyl)-{1-[4-(piperazine-1-carbonyl)-phenyl]-1H-indol-5-yl}-amino)-acetic acid · HCl 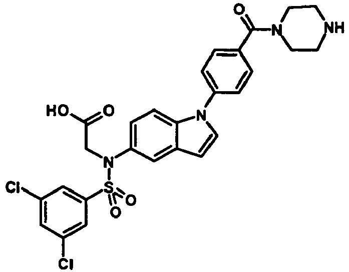 Mass spectrum (ESI⁺): m/z = 587 [M+H]⁺
(4) {(3,5-dichloro-phenylsulphonyl)-[1-(4-piperazin-1-yl-pyrimidin-2-yl)-2,3-dihydro-1H-indol-5-yl]-amino}-acetic acid · HCl 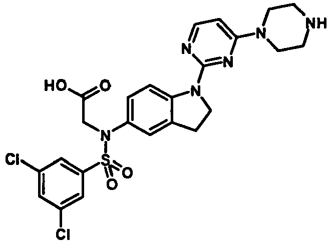 The crude product obtained initially is taken up in diisopropylether/methanol. The solvents are eliminated in vacuo. The remainder is taken up in water and this is in turn eliminated by freeze-drying. Then the residue is dissolved in 2 ml of methanol and 1 ml of 1 N NaOH is added. The mixture is stirred for 1.5 hours at ambient temperature and the solvents are eliminated in vacuo. 2 ml of 1 N HCl are added and the solvents are again eliminated in vacuo. The residue is dried under a high vacuum, taken up in 2 ml of methanol and the precipitated solid is filtered off. The methanol is eliminated in vacuo.
   Mass spectrum (ESI⁺): m/z = 563 [M+H]⁺
(5) ethyl {(3,5-dichloro-phenylsulphonyl)-[1-(4-piperazin-1-yl-pyrimidin-2-yl)-2,3-dihydro-1H-indol-5-yl]-amino}-acetate · HCl 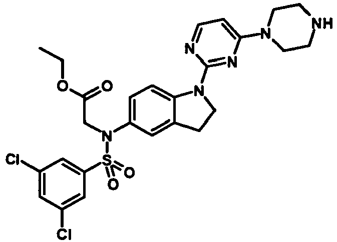 Carried out at 70°C for 3 hours.
   Mass spectrum (ESI⁺): m/z = 591 [M+H]⁺
(6) {(3,5-dichloro-phenylsulphonyl)-[1-(4-piperazin-1-yl-pyrimidin-2-yl)-1H-indol-5-yl]-amino}-acetic acid · HCl 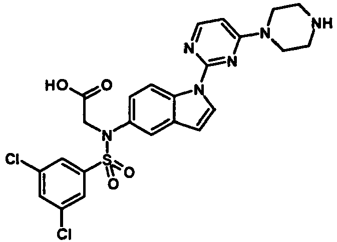 Carried out at 70°C for 3 hours.
   Mass spectrum (ESI⁺): m/z = 561 [M+H]⁺
(7) ethyl {(3,5-dichloro-phenylsulphonyl)-[1-(4-piperazin-1-yl-pyrimidin-2-yl)-1H-indol-5-yl]-amino}-acetate · HCl 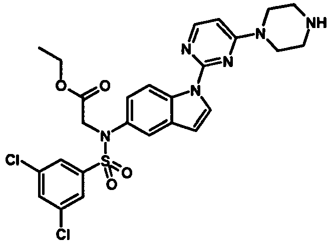 Carried out at 70°C for 3 hours.
   Mass spectrum (ESI⁺): m/z = 589 [M+H]⁺
(8) ((3,5-dichloro-phenylsulphonyl)-{1-[5-(piperazine-1-carbonyl)-pyrazin-2-yl]-2,3-dihydro-1H-indol-5-yl}-amino)-acetic acid · HCl 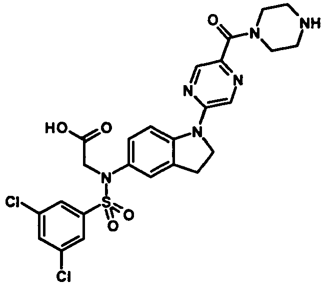 Carried out at 70°C for 3 hours.
   Mass spectrum (ESI⁺): m/z = 591 [M+H]⁺
(9) ((3,5-dichloro-phenylsulphonyl)-{1-[5-(piperazine-1-carbonyl)-pyrazin-2-yl]-1H-indol-5-yl}-amino)-acetic acid · HCl 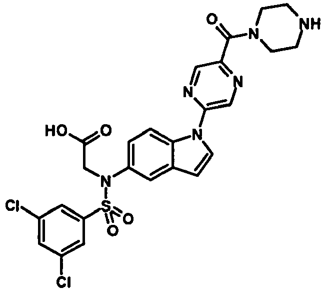 Carried out at 70°C for 3 hours.
   Mass spectrum (ESI⁺): m/z = 589 [M+H]⁺
(10) {(3,5-dichloro-phenylsulphonyl)-[1-(3,4,5,6-tetrahydro-2H-[1,2']bipyrazinyl-6'-yl)-1H-indol-5-yl]-amino}-acetic acid · HCl 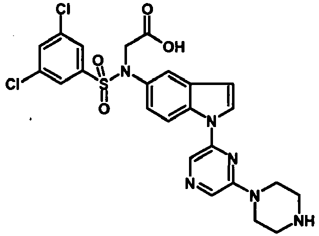 Mass spectrum (ESI⁺): m/z = 561 [M+H]⁺
(11) {(3,5-dichloro-phenylsulphonyl)-[1-(3,4,5,6-tetrahydro-2H-[1,2']bipyrazinyl-6'-yl)-2,3-dihydro-1H-indol-5-yl]-amino}-acetic acid · HCl 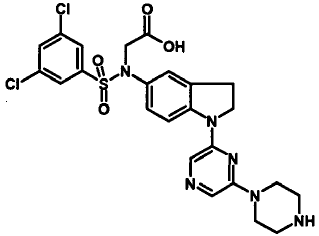 Mass spectrum (ESI⁺): m/z = 563 [M+H]⁺
(12) [(3,5-dichloro-phenylsulphonyl)-(1-pyrimidin-2-yl-2,3-dihydro-1H-indol-4-yl)-amino]-acetic acid 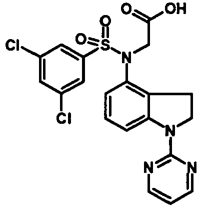 Mass spectrum (ESI⁺): m/z = 479 [M+H]⁺
(13) [(1-{4-[carboxymethyl-(2-dimethylamino-ethyl)-amino]-pyrimidin-2-yl}-2,3-dihydro-1H-indol-5-yl)-(3,5-dichloro-phenylsulphonyl)-amino]-acetic acid · HCl 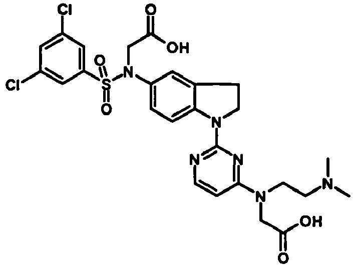 Mass spectrum (ESI⁺): m/z = 623 [M+H]⁺
(14) {(3,5-dichloro-phenylsulphonyl)-[1-(6-piperazin-1-yl-pyridazin-3-yl)-2,3-dihydro-1H-indol-5-yl]-amino}-acetic acid · HCl 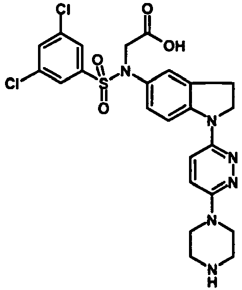 The reaction is carried out for 12 hours at 40°C.
   Mass spectrum (ESI⁺): m/z = 563 [M+H]⁺
(15) ((3,5-dichloro-phenylsulphonyl)-{1-[4-(2-dimethylaminoethylamino)-pyrimidin-2-yl]-2,3-dihydro-1H-indol-5-yl}-amino)-acetic acid · HCl 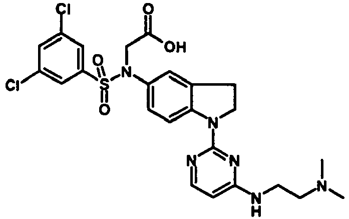 Mass spectrum (ESI⁺): m/z = 565 [M+H]⁺
(16) carboxymethyl-[2-(6-{5-[carboxymethyl-(3,5-dichloro-phenylsulphonyl)-amino]-2,3-dihydro-indol-1-yl}-pyridazin-3-ylamino)ethyl]-dimethyl-ammonium chloride 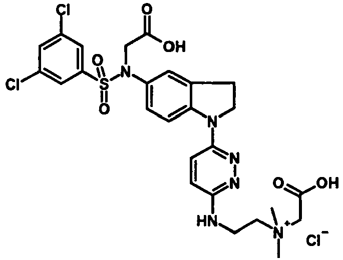 Mass spectrum (ESI⁺): m/z = 623 [M+H]⁺
(17) carboxymethyl-[2-(2-{5-[carboxymethyl-(3,5-dichloro-phenylsulphonyl)-amino]-2,3-dihydro-indol-1-yl}-pyrimidin-4-ylamino)ethyl]-dimethyl-ammonium chloride 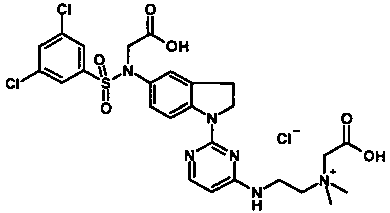 Mass spectrum (ESI⁺): m/z = 623 [M+H]⁺
(18) [(3,5-dichloro-phenylsulphonyl)-(1-pyridin-2-yl-1H-indol-5-yl]-amino]-acetic acid · HCl 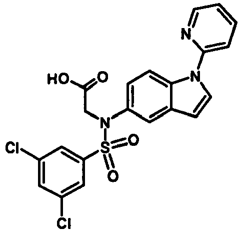 Mass spectrum (ESI⁻): m/z = 474 [M-H]⁻
(19) {(3,5-dichloro-phenylsulphonyl)-[1-(3-trifluoromethyl-pyridin-2-yl)-1H-indol-5-yl]-amino}-acetic acid · HCl 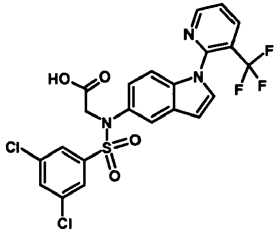 Mass spectrum (ESI⁻): m/z = 542 [M-H]⁻
(20) [{1-[6-(2-amino-ethylamino)-pyridazin-3-yl]-2,3-dihydro-1H-indol-5-yl}-(3,5-dichloro-phenylsulphonyl)-amino]-acetic acid · HCl 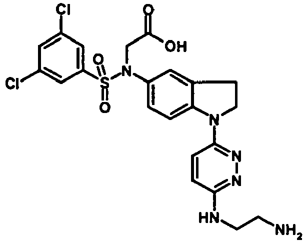 Mass spectrum (ESI⁺): m/z = 537 [M+H]⁺
(21) [{1-[6-(2-amino-ethylamino)-pyridazin-3-yl]-1H-indol-5-yl}-(3,5-dichlorophenylsulphonyl)-amino]-acetic acid · HCl 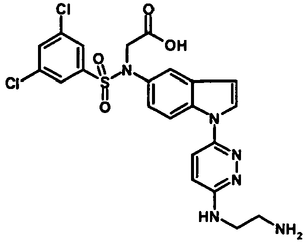 Mass spectrum (ESI⁺): m/z = 535 [M+H]⁺
(22) ((3,5-dichloro-phenylsulphonyl)-{1-[6-(2-oxo-imidazolidin-1-yl)-pyridazin-3-yl]-2,3-dihydro-1H-indol-5-yl}amino)-acetic acid 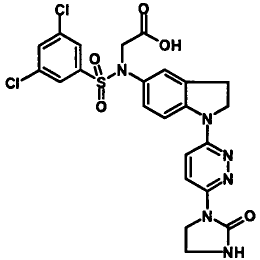 Mass spectrum (ESI⁺): m/z = 563 [M+H]⁺
(23) {(3,5-dichloro-phenylsulphonyl)-[1-(6-methyl-pyridazin-3-yl)-1H-indol-5-yl]-amino}-acetic acid 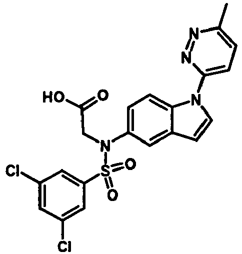 Mass spectrum (ESI⁺): m/z = 490 [M+H]⁺
(24) ((3,5-dichloro-phenylsulphonyl)-{1-[6-(2-dimethylamino-ethylamino)-pyridazin-3-yl]-2,3-dimethyl-1H-indol-5-yl}-amino)-acetic acid · HCl 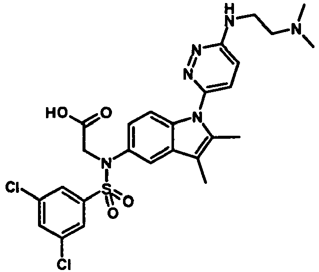 Mass spectrum (ESI⁺): m/z = 591 [M+H]⁺
(25) ((3,5-dichloro-phenylsulphonyl)-{1-[6-(2-dimethylamino-ethylamino)-pyridazin-3-yl]-2-methyl-1H-indol-5-yl}-amino)-acetic acid · HCl 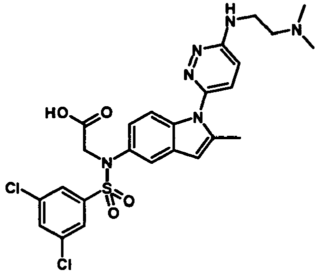 Mass spectrum (ESI⁺): m/z = 577 [M+H]⁺
(26) ((3,5-dichloro-phenylsulphonyl)-{1-[4-(2-dimethylamino-ethylcarbamoyl)-pyridin-2-yl]-1H-indol-5-yl}-amino)-acetic acid · HCl 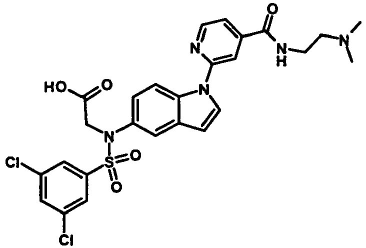 Mass spectrum (ESI⁺): m/z = 590 [M+H]⁺
(27) {(3,5-dichloro-phenylsulphonyl)-[3-iodo-1-(6-methyl-pyridazin-3-yl)-1H-indol-5-yl]-amino}-acetic acid 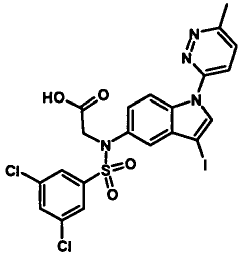 Mass spectrum (ESI⁺): m/z = 617 [M+H]⁺
(28) {(3,5-dichloro-phenylsulphonyl)-[1-(3,4,5,6-tetrahydro-2H-[1,2']bipyrazinyl-5'-yl)-1H-indol-5-yl]-amino}-acetic acid · HCl 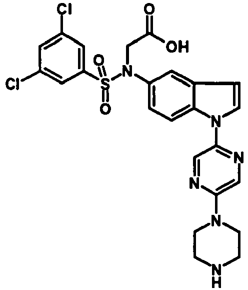 Mass spectrum (ESI⁺): m/z = 561 [M+H]⁺
(29) ((3,5-dichloro-phenylsulphonyl)-{1-[4-(piperazine-1-carbonyl)-pyridin-2-yl]-1H-indol-5-yl}-amino)-acetic acid · HCl 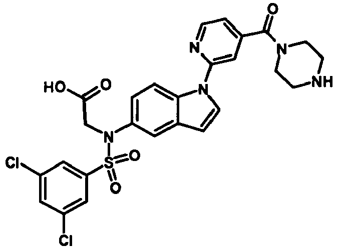 Mass spectrum (ESI⁺): m/z = 588 [M+H]⁺
(30) [(1-[4-(2-amino-ethylamino)-pyrimidin-2-yl]-1H-indol-5-yl}-{(3,5-dichlorophenylsulphonyl)-amino]-acetic acid · HCl 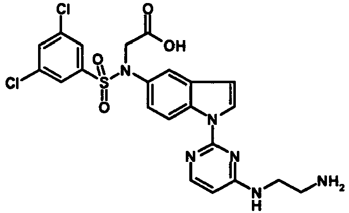 Mass spectrum (ESI⁺): m/z = 535 [M+H]⁺
(31) [[1-(6-amino-pyridazin-3-yl)-1H-indol-5-yl]-(3,5-dichloro-phenylsulphonyl)-amino]-acetic acid · HCl 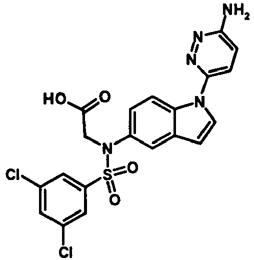 Mass spectrum (ESI⁺): m/z = 492 [M+H]⁺
(32) {(3,5-dichloro-phenylsulphonyl)-[1-(5-hydroxymethyl-pyridin-2-yl)-1H-indol-5-yl]-amino}-acetic acid 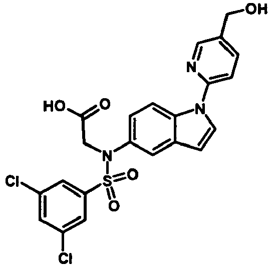 Mass spectrum (ESI⁺): m/z = 506 [M+H]⁺
(33) ((3,5-dichloro-phenylsulphonyl)-{1-(6-(3-hydroxy-propyl)-pyridazin-3-yl]-1H-indol-5-yl}-amino)-acetic acid 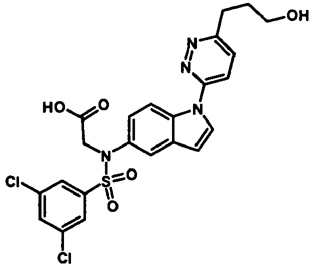 The crude product is purified by chromatography on silica gel with dichloromethane/methanol (9:1 to 6:1).
   Mass spectrum (ESI⁺): m/z = 535 [M+H]⁺
(34) ((3,5-dichloro-phenylsulphonyl)-{1-[5-(2-hydroxy-1-hydroxymethylethylamino)-pyridin-2-yl]-1H-indol-5-yl}-amino)-acetic acid 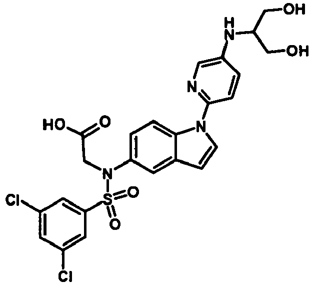 Mass spectrum (ESI⁺): m/z = 565 [M+H]⁺
(35) ((3,5-dichloro-phenylsulphonyl)-{1-[5-(2-hydroxy-ethylamino)-pyrazin-2-yl]-1H-indol-5-yl}-amino)-acetic acid 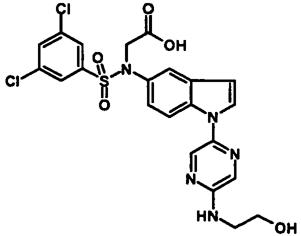 The crude product is purified by preparative HPLC.
   Mass spectrum (ESI⁺): m/z = 536 [M+H]⁺

### Example 5

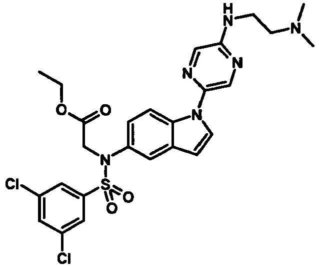

### Ethyl ((3,5-dichloro-phenylsulphonyl)-{1-[5-(2-dimethylamino-ethylamino)-pyrazin-2,yl]-1H-indol-5-yl}-amino)-acetate · HCl

Under argon 200 mg tert.butyl [(3,5-dichloro-phenylsulphonyl)-(1H-indol-5-yl)-amino]-acetate, 9 mg copper iodide and 298 mg potassium phosphate are dissolved in 3 ml of toluene. 229 mg N'-(5-bromo-pyrazin-2-yl)-N,N-dimethylethan-1,2-diamine and 15 µl N,N'-dimethyl-trans-cyclohexanediamine are added. Then the mixture is heated to 110°C for 8 hours. It is divided between water and ethyl acetate and the aqueous phase is extracted twice with ethyl acetate. After drying with magnesium sulphate the solvents are eliminated in vacuo. The residue is purified by chromatography on silica gel (dichloromethane/ methanol 9:1 to 1:2). The product is taken up in 10 ml diisopropylether and combined with 500 pl of 4 N HCl in dioxane. The precipitated solid is suction filtered and dried in vacuo.
Yield: 108 mg (39 % of theory)
Mass spectrum (ESI⁺): m/z = 591 [M+H]⁺

The following compounds are obtained analogously to Example 5:
(1) ethyl {(3,5-dichloro-phenylsulphonyl)-[1-(6-methyl-pyridazin-3-yl)-1H-indol-5-yl]-amino}-acetate 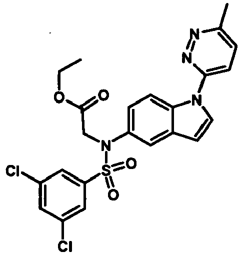 Mass spectrum (ESI⁺): m/z = 519 [M+H]⁺
(2) ethyl [[1-(5-amino-pyrazin-2-yl)-1H-indol-5-yl]-(3,5-dichloro-phenylsulphonyl)-amino]-acetate 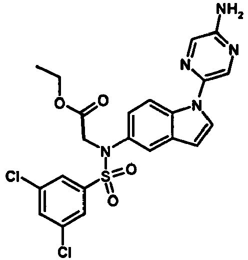 Mass spectrum (ESI⁺): m/z = 520 [M+H]⁺
(3) ethyl {(3,5-dichloro-phenylsulphonyl)-[1-(6-morpholin-4-yl-pyridazin-3-yl)-1H-indol-5-yl]-amino}-acetate 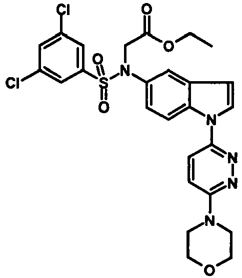 Mass spectrum (ESI⁺): m/z = 590 [M+H]⁺

### Example 6

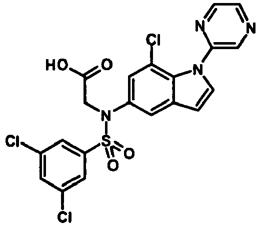

### [(7-chloro-1-pyrazin-2-yl-1H-indol-5-yl)-(3,5-dichloro-phenylsulphonyl)-amino]-acetic acid

75 mg tert.butyl [(3,5-dichloro-phenylsulphonyl)-(1-pyrazin-2-yl-1H-indol-5-yl)-amino]-acetate are dissolved in 2 ml dichloromethane under argon. 19 mg of N-chloro-succinimide are added and the mixture is stirred for 1 hour at ambient temperature. The solvent is eliminated in vacuo and the residue is taken up in 1 ml acetic acid. It is heated to 70°C and 150 µl phosphoric acid are added. Then the mixture is refluxed for 2 hours. It is divided between water and ethyl acetate, the aqueous phase is extracted twice with ethyl acetate and the combined organic phases are dried with sodium sulphate. The solvents are eliminated in vacuo and the residue is chromatographed on silica gel (dichloromethane/methanol 9:1 to 3:2).
Yield: 17 mg (24 % of theory)
Mass spectrum (ESI⁺): m/z = 511 [M+H]⁺

### Example 7

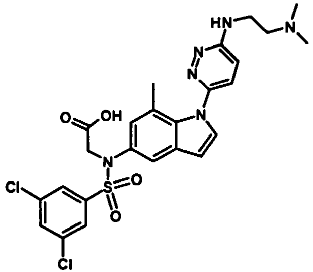

### ((3,5-dichloro-phenylsulphonyl)-{1-[6-(2-dimethylamino-ethylamino)-pyridazin-3-yl]-7-methyl-1H-indol-5-yl}-amino)-acetic acid · CF₃CO₂H

90 mg tert.butyl [(3,5-dichloro-phenylsulphonyl)-(7-methyl-1H-indol-5-yl)-amino]-acetate, 122 mg potassium phosphate and 7.3 mg copper iodide are suspended in 2 ml anhydrous toluene. To this is added a solution of 45 mg N'-(6-bromo-pyridazin-3-yl)-N,N-dimethyl-ethan-1 ,2-diamine in 1 ml anhydrous toluene. After the addition of 13 µl N,N'-dimethyl-trans-cyclohexane-diamine the mixture is heated to 100°C for 5 hours. Then it is divided between water and ethyl acetate. The aqueous phase is extracted twice with ethyl acetate and the combined organic phases are dried on magnesium sulphate. The solvents are eliminated in vacuo and the residue is chromatographed on silica gel [dichloromethane/(methanol/acetic acid/water 8:1:1) 95:5 to 75:25]. The product thus obtained is taken up in 3 ml dichloromethane. 1.5 ml trifluoroacetic acid are added and the mixture is stirred for 3 hours at ambient temperature. The solvents are then eliminated in vacuo and the residue is taken up in 2 ml of water. After freeze-drying the product is obtained as a solid.
Yield: 31 mg (28 % of theory)
Mass spectrum (ESI⁺): m/z = 577 [M+H]⁺

The following compounds are obtained analogously to Example 7:
(1) ((3,5-dichloro-phenylsulphonyl)-{1-[6-(2-dimethylamino-ethylamino)-pyridazin-3-yl]-6-methyl-1H-indol-5-yl}-amino)-acetic acid · CF₃CO₂H 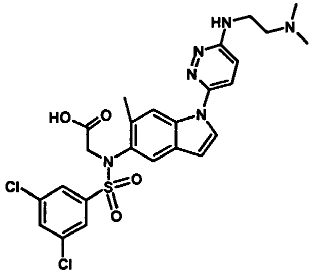 Mass spectrum (ESI⁺): m/z = 577 [M+H]⁺

### Example 8

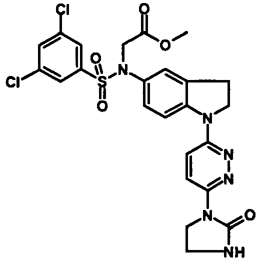

### Methyl ((3,5-dichloro-phenylsulphonyl)-{1-[6-(2-oxo-imidazolidin-1-yl)-pyridazin-3-yl]-2,3-dihydro-1H-indol-5-yl}-amino)-acetate

60 mg ((3,5-dichloro-phenylsulphonyl)-{1-[6-(2-oxo-imidazolidin-1-yl)-pyridazin-3-yl]-2,3-dihydro-1H-indol-5-yl}-amino)-acetic acid are suspended in 1 ml of methanol, 150 µl of a 2 N solution of trimethylsilyldiazomethan in hexane and stirred for 12 hours at ambient temperature. Then the mixture is diluted with 2 ml of methanol and 10 drops of 2 N hydrochloric acid are added. The solvents are eliminated in vacuo and the residue is extracted from diethyl ether.
Yield: 45 mg (73 % of theory)
Mass spectrum (ESI⁺): m/z = 577 [M+H]⁺

### Example 9

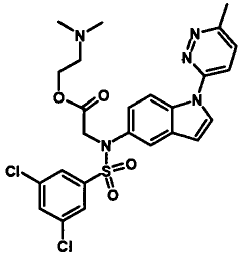

### 2-dimethylamino-ethyl {(3,5-dichloro-phenylsulphonyl)-[1-(6-methyl-pyridazin-3-yl)-1H-indol-5-yl]-amino}-acetate

1.4 g of ethyl{(3,5-dichloro-phenylsulphonyl)-[1-(6-methyl-pyridazin-3-yl)-1H-indol-5-yl]-amino}-acetate are dissolved in 5 ml 2-dimethylaminoethanol. 100 µl of titanium tetraisopropoxide are added and the mixture is heated for 4 hours to 120°C. Then the excess 2-dimethylaminoethanol is eliminated in vacuo and the residue is divided between water and diethyl ether. The aqueous phase is extracted 3 times with diethyl ether and the combined organic phases are dried on magnesium sulphate. Then the solvent is eliminated in vacuo until crystallisation sets in. After crystallisation has ended the solid is suction filtered and dried in vacuo.
Yield: 780 mg (51 % of theory)
Mass spectrum (ESI⁺): m/z = 562 [M+H]⁺

### Example 10

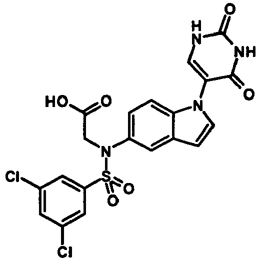

### {(3,5-dichloro-phenylsulphonyl)-[1-(2,4-dioxo-1,2,3,4-tetrahydro-pyrimidin-5-yl)-1H-indol-5-yl]-amino}-acetic acid

116 mg tert.butyl {(3,5-dichloro-phenylsulphonyl)-[1-(2,4-dimethoxy-pyrimidin-5-yl)-1H-indol-5-yl]-amino}-acetate are dissolved in 18 ml acetic acid. To this are added 230 µl 2 N hydrochloric acid and the mixture is refluxed for 1 hour. The solvents are then eliminated in vacuo and the residue is purified by preparative HPLC.
Yield: 5 mg (5 % of theory)
Mass spectrum (ESI⁻): m/z = 507 [M⁻H]⁻

The following compounds are obtained analogously to Example 10:
(1) {(3,5-dichloro-phenylsulphonyl)-[1-(2',4'-dioxo-1'.2'.3'.4'-tetrahydro-[4.5']bipyrimidinyl-6-yl)-1H-indol-5-yl]-amino}-acetic acid 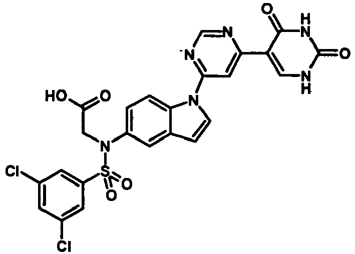 Prepared by treating tert.butyl [[1-(2',4'-di-tert-butoxy-[4.5']bipyrimidinyl-6-yl)-1H-indol-5-yl]-(3,5-dichloro-phenylsulphonyl)-amino]-acetate with hydrochloric acid in methanol at ambient temperature and subsequent treatment with trifluoroacetic acid in dichloromethane at ambient temperature.
   Mass spectrum (ESI⁺): m/z = 587 [M+H]⁺
   R_{f} value: 0.35 (silica gel: dichloromethane/methanol 90:10)

### Example 11

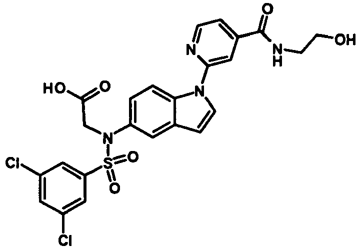

### ((3,5-dichloro-phenylsulphonyl)-{1-[4-(2-hydroxy-ethylcarbamoyl)-pyridin-2-yl]-1H-indol-5-yl}-amino)-acetic acid

100 mg tert.butyl ((3,5-dichloro-phenylsulphonyl)-{1-[4-(2-hydroxyethylcarbamoyl)-pyridin-2-yl]-1H-indol-5-yl}-amino)-acetate are dissolved in 1 ml dichloromethane and 0.5 ml trifluoroacetic acid and stirred for 5 hours at ambient temperature. Then the solvents are eliminated in vacuo and the residue is taken up in 0.5 ml of tetrahydrofuran. 140 µl 1 N sodium hydroxide solution are added and the mixture is stirred for 1 hour at ambient temperature. Then the tetrahydrofuran is eliminated in vacuo and the residue is combined with 140 µl of 1 N hydrochloric acid. The precipitated solid is suction filtered and dried in vacuo.
Yield: 54 mg (59 % of theory)
Mass spectrum (ESI⁺): m/z = 563 [M+H]⁺

The following compounds are obtained analogously to Example 11:
(1) ((3,5-dichloro-phenylsulphonyl)-{1-[5-(2-hydroxy-ethylamino)-pyridin-2-yl]-1H-indol-5-yl}-amino)-acetic acid 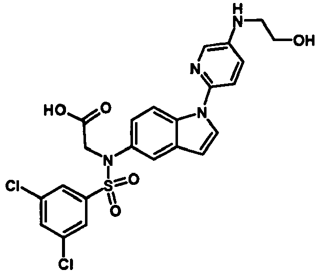 Mass spectrum (ESI⁻): m/z = 533 [M-H]⁻
(2) ((3,5-dichloro-phenylsulphonyl)-{1-[4-(3-hydroxy-propyl)-pyridin-2-yl]-1H-indol-5-yl}-amino)-acetic acid 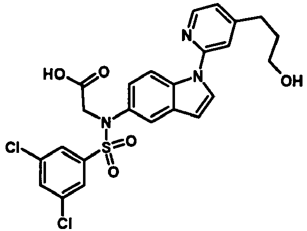 Mass spectrum (ESI⁺): m/z = 534 [M+H]⁺

### Example 12

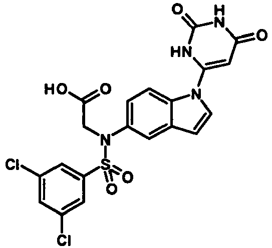

### {(3,5-dichloro-phenylsulphonyl)-[1-(2,6-dioxo-1,2,3,6-tetrahydro-pyrimidin-4-yl)-1H-indol-5-yl]-amino}-acetic acid

31 mg {(3,5-dichloro-phenylsulphonyl)-[1-(2,6-dichloro-pyrimidin-4-yl)-1H-indol-5-yl]-amino}-acetic acid are dissolved in 600 µl tetrahydrofuran. 250 µl of 4 N sodium hydroxide solution and 15 drops of a 30% hydrogen peroxide solution are added and the mixture is heated for 6 hours to 50°C. The volatile constituents are eliminated in vacuo and the residue is purified by preparative HPLC.
Yield: 2.6 mg (11 % of theory) {(3,5-dichloro-phenylsulphonyl)-[1-(2,6-dioxo-1,2,3,6-tetrahydro-pyrimidin-4-yl)-1H-indol-5-yl]-amino}-acetic acid Mass spectrum (ESI⁺): m/z = 509 [M+H]⁺ and
5 mg (20 % of theory) of 3-{5-[carboxymethyl-(3,5-dichloro-phenylsulphonyl)-amino]-indol-1-yl}-3-ureido-acrylic acid
Mass spectrum (ESI⁺): m/z = 527 [M+H]⁺

### Example 13

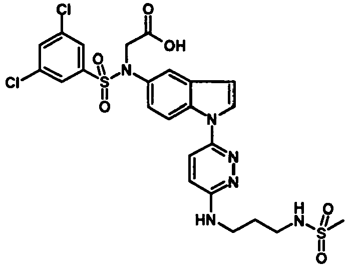

### ((3,5-dichloro-phenylsulphonyl)-{1-[6-(3-methanesulphonylamino-propylamino)-pyridazin-3-yl]-1H-indol-5-yl}-amino)-acetic acid

25 mg [{1-[6-(3-amino-propylamino)-pyridazin-3-yl]-1H-indol-5-yl}-(3,5-dichlorophenylsulphonyl)-amino]-acetic acid are dissolved in 2 ml of tetrahydrofuran. The mixture is cooled to 0°C, 250 µl 1 N sodium hydroxide solution are added and then 10 µl methanesulphonic acid chloride are added thereto. After stirring for 3 hours, 250 µl of 1 N hydrochloric acid are added and the volatile constituents are removed in vacuo. The residue is taken up in dichloromethane/methanol 2:1 and the solid is suction filtered. The mother liquor is evaporated down in vacuo and the residue is purified by preparative HPLC.
Yield: 6.5 mg (23 % of theory)
Mass spectrum (ESI⁺): m/z = 627 [M+H]⁺

The following compounds are obtained analogously to Example 13:
(1) ((3,5-dichloro-phenylsulphonyl)-{1-[6-(4-methanesulphonyl-piperazin-1-yl)-pyridazin-3-yl]-1H-indol-5-yl}-amino)-acetic acid 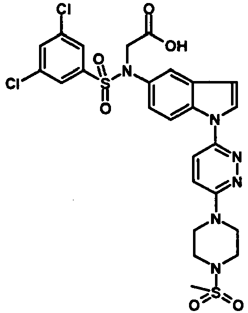 The crude product is purified by preparative HPLC.
   Mass spectrum (ESI⁺): m/z = 639 [M+H]⁺
(2) ((3,5-dichloro-phenylsulphonyl)-{1-[5-(2-methanesulphonylamino-ethoxy)-pyrazin-2-yl]-1H-indol-5-yl}-amino)-acetic acid 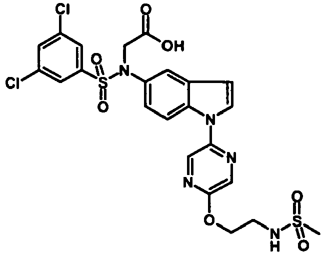 The crude product is purified by preparative HPLC.
   Mass spectrum (ESI⁺): m/z = 614 [M+H]⁺
(3) [{1-[5-(2-acetylamino-ethoxy)-pyrazin-2-yl]-1H-indol-5-yl}-(3,5-dichlorophenylsulphonyl)-amino]-acetic acid 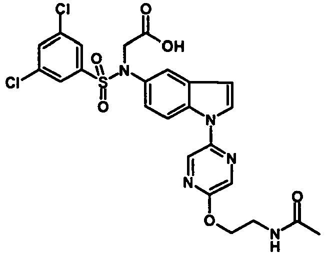 Instead of methanesulphonic acid chloride acetic anhydride is used. The crude product is purified by preparative HPLC.
   Mass spectrum (ESI⁻): m/z = 576 [M-H]⁻
(4) {(3,5-dichloro-phenylsulphonyl)-[1-(4-methanesulphonyl-3,4,5,6-tetrahydro-2H-[1,2']bipyrazinyl-5'-yl)-1H-indol-5-yl]-amino}-acetic acid 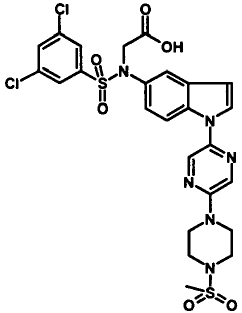 Mass spectrum (ESI⁺): m/z = 639 [M+H]⁺
(5) [{1-[5-(2-acetylamino-ethylamino)-pyrazin-2-yl]-1H-indol-5-yl}-(3,5-dichlorophenylsulphonyl)-amino]-acetic acid 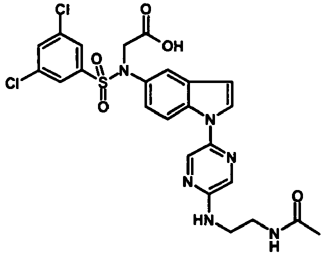 Instead of methanesulphonic acid chloride acetic anhydride is used. The crude product is purified by preparative HPLC.
   Mass spectrum (ESI⁺): m/z = 577 [M+H]⁺
(6) ((3,5-dichloro-phenylsulphonyl)-{1-[5-(2-methanesulphonylamino-ethylamino)-pyrazin-2-yl]-1H-indol-5-yl}-amino)-acetic acid 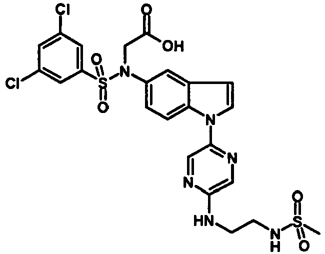 The crude product is purified by preparative HPLC.
   Mass spectrum (ESI⁺): m/z = 613 [M+H]⁺
(7) [[1-(4-acetyl-3,4,5,6-tetrahydro-2H-[1,2']bipyrazinyl-5'-yl)-1H-indol-5-yl]-(3,5-dichloro-phenylsulphonyl)-amino]-acetic acid 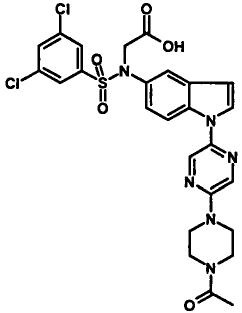 Instead of methanesulphonic acid chloride acetic anhydride is used. The crude product is purified by preparative HPLC.
   Mass spectrum (ESI⁺): m/z = 603 [M+H]⁺

### Example 14

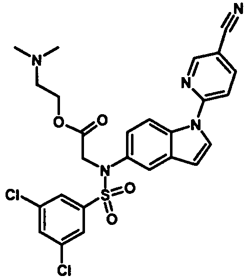

### 2-dimethylamino-ethyl [[1-(5-cyano-pyridin-2-yl)-1H-indol-5-yl]-(3,5-dichlorophenylsulphonyl)-amino]-acetate

250 mg [[1-(5-cyano-pyridin-2-yl)-1H-indol-5-yl]-(3,5-dichloro-phenylsulphonyl)-amino]-acetic acid are dissolved in 4 ml dichloromethane. 217 µl oxalyl chloride and 1 drop of DMF are added successively and the mixture is stirred for 1 hour at ambient temperature. Then the volatile constituents are eliminated in vacuo and the residue is taken up in 4 ml dichloromethane. To this is added dropwise a solution of 80 µl pyridine and 74 µl 2-dimethylamino-ethanol in 1 ml dichloromethane. The mixture is stirred for 2 hours and divided between saturated sodium hydrogen carbonate solution and dichloromethane. The aqueous phase is extracted twice with dichloromethane and the combined organic phases are dried on magnesium sulphate. The solvent is eliminated in vacuo and the residue is extracted from diisopropylether.
Yield: 239 mg (84 % of theory)
Mass spectrum (ESI⁺): m/z = 572 [M+H]⁺

The following compounds are obtained analogously to Example 14:
(1) 2-dimethylamino-ethyl {(3,5-dichloro-phenylsulphonyl)-[3-methyl-1-(6-methylpyridazin-3-yl)-1H-indol-5-yl]-amino}-acetate 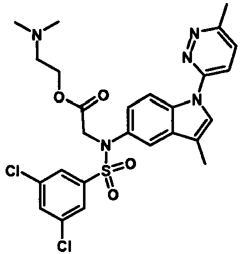 Mass spectrum (ESI⁺): m/z = 576 [M+H]⁺

### Example 15

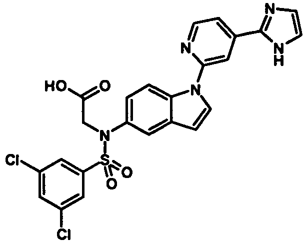

### ((3,5-dichloro-phenylsulphonyl)-{1-[4-(1H-imidazol-2-yl)-pyridin-2-yl]-1H-indol-5-yl}-amino)-acetic acid

25 mg tert.butyl ((3,5-dichloro-phenylsulphonyl)-{1-[4-(1-ethoxymethyl-1H-imidazol-2-yl)-pyridin-2-yl]-1H-indol-5-yl}-amino)-acetate are dissolved in 2 ml trifluoroacetic acid and 200 µl anisole. The mixture is heated for 36 hours to 70°C. Then the solvents are eliminated in vacuo and the residue is purified by preparative thin layer chromatography (silica gel, dichloromethane/methanol 9:1).
Yield: 3.7 mg (18 % of theory)
Mass spectrum (ESI⁺): m/z = 542 [M+H]⁺

### Example 16

Coated tablets containing 75 mg of active substance

| 1 tablet core contains: | |
|---|---|
| active substance | 75.0 mg |
| calcium phosphate | 93.0 mg |
| corn starch | 35.5 mg |
| polyvinylpyrrolidone | 10.0 mg |
| hydroxypropylmethylcellulose | 15.0 mg |
| magnesium stearate | 1.5 mg |
| | 230.0 mg |

### Preparation:

The active substance is mixed with calcium phosphate, corn starch, polyvinylpyrrolidone, hydroxypropylmethylcellulose and half the specified amount of magnesium stearate. Blanks 13 mm in diameter are produced in a tablet-making machine and these are then rubbed through a screen with a mesh size of 1.5 mm using a suitable machine and mixed with the rest of the magnesium stearate. This granulate is compressed in a tablet-making machine to form tablets of the desired shape.
- Weight of core:: 230 mg
- die:: 9 mm, convex

The tablet cores thus produced are coated with a film consisting essentially of hydroxypropylmethylcellulose. The finished film-coated tablets are polished with beeswax.
Weight of coated tablet: 245 mg.

### Example 17

Tablets containing 100 mg of active substance

**Composition:**

| 1 tablet contains: | |
|---|---|
| active substance | 100.0 mg |
| lactose | 80.0 mg |
| corn starch | 34.0 mg |
| polyvinylpyrrolidone | 4.0 mg |
| magnesium stearate | 2.0 mg |
| | 220.0 mg |

### Method of Preparation:

The active substance, lactose and starch are mixed together and uniformly moistened with an aqueous solution of the polyvinylpyrrolidone. After the moist composition has been screened (2.0 mm mesh size) and dried in a rack-type drier at 50°C it is screened again (1.5 mm mesh size) and the lubricant is added. The finished mixture is compressed to form tablets.
Weight of tablet: 220 mg
Diameter: 10 mm, biplanar, facetted on both sides and notched on one side.

### Example 18

Tablets containing 150 mg of active substance

**Composition:**

| 1 tablet contains: | |
|---|---|
| active substance | 50.0 mg |
| powdered lactose | 89.0 mg |
| corn starch | 40.0 mg |
| colloidal silica | 10.0 mg |
| polyvinylpyrrolidone | 10.0 mg |
| magnesium stearate | 1.0 mg |
| | 300.0 mg |

### Preparation:

The active substance mixed with lactose, corn starch and silica is moistened with a 20% aqueous polyvinylpyrrolidone solution and passed through a screen with a mesh size of 1.5 mm. The granules, dried at 45°C, are passed through the same screen again and mixed with the specified amount of magnesium stearate. Tablets are pressed from the mixture.
- Weight of tablet:: 300 mg
- die:: 10 mm, flat

### Example 19

Hard gelatine capsules containing 150 mg of active substance

| 1 capsule contains: | | |
|---|---|---|
| active substance | | 50.0 mg |
| corn starch (dried | approx. | 80.0 mg |
| lactose (powdered) | approx. | 87.0 mg |
| magnesium stearate | | 3.0 mg |
| | approx. | 420.0 mg |

### Preparation:

The active substance is mixed with the excipients, passed through a screen with a mesh size of 0.75 mm and homogeneously mixed using a suitable apparatus. The finished mixture is packed into size 1 hard gelatine capsules.
- Capsule filling: approx.: 320 mg
- Capsule shell:: size 1 hard gelatine capsule.

### Example 20

Suppositories containing 150 mg of active substance

| 1 suppository contains: | |
|---|---|
| active substance | 150.0 mg |
| polyethyleneglycol 1500 | 550.0 mg |
| polyethyleneglycol 6000 | 460.0 mg |
| polyoxyethylene sorbitan monostearate | 840.0 mg |
| | 2,000.0 mg |

### Preparation:

After the suppository mass has been melted the active substance is homogeneously distributed therein and the melt is poured into chilled moulds.

### Example 21

Suspension containing 50 mg of active substance

| 100 ml of suspension contain: | |
|---|---|
| active substance | 1.00 g |
| carboxymethylcellulose-Na-salt | 0.10 g |
| methyl p-hydroxybenzoate | 0.05 g |
| propyl p-hydroxybenzoate | 0.01 g |
| glucose | 10.00 g |
| glycerol | 5.00 g |
| 70% sorbitol solution | 20.00 g |
| flavouring | 0.30 g |
| dist. water ad | 100 ml |

### Preparation:

The distilled water is heated to 70°C. The methyl and propyl p-hydroxybenzoates together with the glycerol and sodium salt of carboxymethylcellulose are dissolved therein with stirring. The solution is cooled to ambient temperature and the active substance is added and homogeneously dispersed therein with stirring. After the sugar, the sorbitol solution and the flavouring have been added and dissolved, the suspension is evacuated with stirring to eliminate air.

5 ml of suspension contain 50 mg of active substance.

### Example 22

Ampoules containing 10 mg active substance

**Composition:**

| | |
|---|---|
| active substance | 10.0 mg |
| 0.01 N hydrochloric acid q.s. | |
| double-distilled water ad | 2.0 ml |

### Preparation:

The active substance is dissolved in the necessary amount of 0.01 N HCl, made isotonic with common salt, filtered sterile and transferred into 2 ml ampoules.

### Example 23

Ampoules containing 50 mg of active substance

**Composition:**

| | |
|---|---|
| active substance | 50.0 mg |
| 0.01 N hydrochloric acid q.s. | |
| double-distilled water ad | 10.0 ml |

### Preparation:

The active substance is dissolved in the necessary amount of 0.01 N HCl, made isotonic with common salt, filtered sterile and transferred into 10 ml ampoules.

## Claims

1. Arylsulphonylglycines of general formula 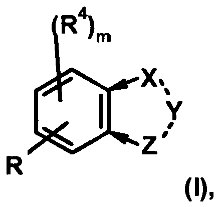 wherein
R denotes a group of formula 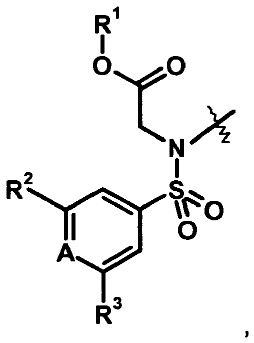 wherein
R¹ denotes H, C₁₋₆-alkyl or a group of formula 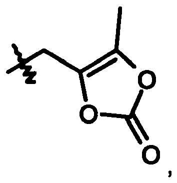 wherein the C₁₋₆-alkyl group mentioned hereinbefore for R¹ may be substituted by C₁₋₆-alkyl-carbonyloxy, C₁₋₆-alkoxy-carbonyloxy, C₁₋₆-alkoxy, hydroxy, amino, C₁₋₃-alkyl-amino, di-(C₁₋₃-alkyl)-amino, pyrrolidin-1-yl, piperidin-1-yl, morpholin-4-yl, piperazin-1-yl, 4-(C₁₋₃-alkyl)-piperazin-1-yl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, tetrahydrofuran-3-yl-oxy, C₁₋₃-alkylamino-C₁₋₃-alkyloxy, di-(C₁₋₃-alkyl)-amino- C₁₋₃-alkyloxy, pyrrolidin-1-yl-C₁₋₃-alkyloxy, piperidin-1-yl-C₁₋₃-alkyloxy, morpholin-4-yl-C₁₋₃-alkyloxy, piperazin-1-yl-C₁₋₃-alkyloxy or 4-(C₁₋₃-alkyl)-piperazin-1-yl-C₁₋₃-alkyloxy,
R² and R³ independently of one another denote H, halogen, C₁₋₃-alkyl, C₁₋₃-perfluoroalkyl, C₁₋₃-perfluoroalkoxy, C₁₋₃-alkoxy, cyano, nitro or hydroxy,
and
A denotes CH or N,
m denotes 0, 1 or 2,
R⁴ denotes halogen, C₁₋₃-alkyl, C₁₋₃-perfluoroalkyl, C₁₋₃-perfluoroalkoxy, cyano, hydroxy or C₁₋₃-alkoxy, while, if m denotes the number 2, the R⁴ groups may be identical or different,
and the heterocyclic group 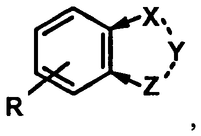 which may be substituted by R⁴ as described hereinbefore, denotes a group of formula 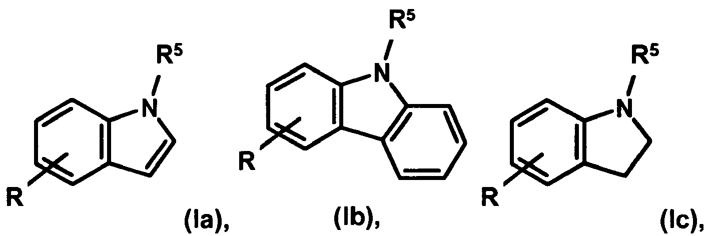 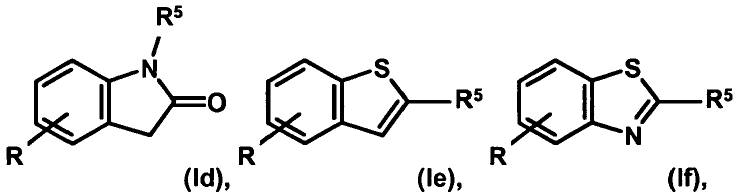 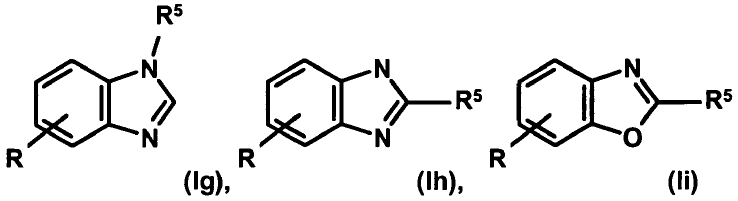 or 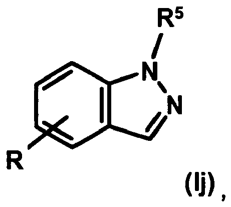
wherein the above-mentioned heterocycles of formulae (Ia), (Ic), (Id), (Ie), (Ig) and (Ij) may optionally be substituted at the carbon atoms of the 5 ring by one or two groups selected from among halogen, C₁₋₃-alkyl, cyano, C₁₋₃ -perfluoroalkyl, C₃₋₆-cycloalkyl, C₂₋₄-alkynyl, C₂₋₄-alkenyl, C₁₋₃-alkylcarbonyl, C₁₋₃-perfluoroalkyl-carbonyl, carboxyl, aminomethyl, C₁₋₃-alkylaminomethyl, di-(C₁₋₃-alkyl)-aminomethyl, aminocarbonyl, C₁₋₃-alkylaminocarbonyl or di-(C₁₋₃-alkyl)-aminocarbonyl, wherein the groups may be identical or different and each carbon atom may carry only one group, and
wherein R⁵ denotes a 1H-pyrimidin-2,4-dionyl, 2H-pyridazin-3-onyl or 1H-pyridin-2-onyl group optionally mono- or disubstituted by one or two methyl groups or
a mono- or bicyclic 5- to 14-membered ring system, which may contain 0 to 4 heteroatoms selected from among N, O or S, wherein not more than one oxygen atom and/or one sulphur atom may be present, is aromatic, saturated or partially unsaturated and may be mono-, di- or trisubstituted independently of one another by a group selected from among
halogen, cyano, nitro,
C₁₋₆-alkyl, C₃₋₆-cycloalkyl, C₁₋₃-perfluoroalkyl, C₂₋₆-alkynyl, C₂₋₆-alkenyl,
hydroxy, C₁₋₆-alkoxy, C₃₋₆-cycloalkoxy, C₁₋₃-perfluoroalkoxy,
carboxyl, C₁₋₃-alkyl-carbonyl, C₁₋₄-alkoxy-carbonyl, C₃₋₆-cycloalkoxy-carbonyl, aminocarbonyl, C₁₋₆-alkyl-aminocarbonyl, di-(C₁₋₆-alkyl)-aminocarbonyl, N-(C₁₋₆-alkyl)-N-(C₁₋₆-alkoxy)-aminocarbonyl, C₃₋₆-cycloalkyl-aminocarbonyl, N-(C₃₋₆-cycloalkyl)-N-(C₁₋₆-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, 4-(C₁₋₃-alkyl-carbonyl)-piperazin-1-yl-carbonyl, 4-(C₃₋₆-cycloalkyl-carbonyl)-piperazin-1-yl-carbonyl, 4-(C₁₋₃ -alkylsulphonyl)-piperazin-1-yl-carbonyl, 4-(C₃₋₆-cycloalkylsulphonyl)-piperazin-1-yl-carbonyl, C₁₋₃-alkylsulphanyl, C₃₋₆-cycloalkylsulphanyl, C₁₋₃-alkylsulphinyl, C₃₋₆ -cycloalkylsulphinyl, C₁₋₃-alkylsulphonyl, C₃₋₆-cycloalkylsulphonyl,
amino, C₁₋₆-alkyl-amino, di-(C₁₋₆-alkyl)-amino, C₃₋₆-cycloalkylamino, N-(C₃₋₆-cycloalkyl)-N-(C₁₋₆-alkyl)-amino, (C₁₋₄-alkylcarbonyl)-amino, (C₃₋₆-cycloalkyl-carbonyl)-amino, (C₁₋₄-alkylcarbonyl)-(C₁₋₃-alkyl)-amino, (C₃₋₆-cycloalkyl-carbonyl)-(C₁₋₃-alkyl)-amino,
pyrrolidin-1-yl, piperidin-1-yl, 3-amino-piperidin-1-yl, 4-amino-piperidin-1-yl, morpholin-4-yl, thiomorpholin-4-yl, 1-oxo-1λ⁴-thiomorpholin-4-yl, 1,1-dioxo-1λ⁶-thiomorpholin-4-yl, piperazin-1-yl, homopiperazin-1-yl, 4-(C₁₋₃-alkyl)-piperazin-1-yl, 4-(C₁₋₃-alkylcarbonyl)-piperazin-1-yl, 4-(C₃₋₆-cycloalkyl-carbonyl)-piperazin-1-yl, 4-(C₁₋₃-alkylsulphonyl)-piperazin-1-yl, 4-(C₃₋₆-cycloalkyl-sulphonyl)-piperazin-1-yl,
tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidin-1-yl, 2-oxo-tetrahydropyrimidin-1-yl and heteroaryl,
wherein the C₁₋₆-alkyl, C₃₋₆-cycloalkyl, C₂₋₆-alkynyl, C₂₋₆-alkenyl, C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl, C₃₋₆-cycloalkylsulphonyl, C₁₋₆-alkoxy, C₃₋₆-cycloalkoxy, C₁₋₆-alkyl-amino, di-(C₁₋₆-alkyl)-amino, C₃₋₆-cycloalkyl-amino- N-(C₃₋₆-cycloalkyl)-N-(C₁₋₆-alkyl)-amino, (C₁₋₄-alkyl-carbonyl)-amino, (C₃₋₆-cycloalkyl-carbonyl)-amino, (C₁₋₄-alkylcarbonyl)-(C₁₋₃-alkyl)-amino- and (C₃₋₆-cycloalkyl-carbonyl)-(C₁₋₃-alkyl)-amino groups mentioned above in the definition of R⁵ may each be mono- or disubstituted independently of one another in the carbon skeleton by a group selected from among
cyano, hydroxy, C₁₋₃-alkoxy, tetrahydro-pyran-2-yloxy,
amino, C₁₋₆-alkyl-amino, di-(C₁₋₆-alkyl)-amino, C₃₋₆-cycloalkyl-amino, N-(C₃₋₆-cycloalkyl)-N-(C₁₋₆-alkyl)-amino, (C₁₋₄-alkyl-carbonyl)-amino, (C₃₋₆-cycloalkyl-carbonyl)-amino, (C₁₋₄-alkyl-carbonyl)-(C₁₋₃-alkyl)-amino, (C₃₋₆-cycloalkylcarbonyl)-(C₁₋₃-alkyl)-amino, (C₁₋₄-alkylsulphonyl)-amino, (C₃₋₆ -cycloalkylsulphonyl)-amino, (C₁₋₄-alkylsulphonyl)-(C₁₋₃-alkyl)-amino, (C₃₋₆-cycloalkylsulphonyl)-(C₁₋₃-alkyl)-amino,
pyrrolidin-1-yl, piperidin-1-yl, morpholin-4-yl, piperazin-1-yl, 4-(C₁₋₃-alkyl)-piperazin-1-yl,
carboxyl, aminocarbonyl, C₁₋₆-alkyl-aminocarbonyl, di-(C₁₋₆-alkyl)-aminocarbonyl, C₁₋₂-alkoxy-carbonyl, C₃₋₆-cycloalkylaminocarbonyl, N-(C₃₋₆-cycloalkyl)-N-(C₁₋₆-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl,
C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl, and C₃₋₆-cycloalkylsulphonyl, wherein the substituents must not be bound to a common carbon atom, and
wherein the pyrrolidin-1-yl and piperidin-1-yl groups mentioned above in the definition of R⁵ may be substituted by amino or hydroxy, and
wherein the C₁₋₆-alkyl-aminocarbonyl, di-(C₁₋₆-alkyl)-aminocarbonyl, C₃₋₆-cycloalkyl-aminocarbonyl, N-(C₃₋₆-cycloalkyl)-N-(C₁₋₆-alkyl)-aminocarbonyl groups mentioned hereinbefore for R⁵ may each be mono- or disubstituted independently of one another in the carbon skeleton by a group selected from among
cyano, hydroxy, C₁₋₃-alkoxy,
amino, C₁₋₃-alkyl-amino, di-(C₁₋₃-alkyl)-amino, C₃₋₆-cycloalkyl-amino, N-(C₃₋₆-cycloalkyl)-N-(C₁₋₆-alkyl)-amino, (C₁₋₄-alkyl-carbonyl)-amino, (C₃₋₆-cycloalkyl-carbonyl)-amino, (C₁₋₄-alkyl-carbonyl)-(C₁₋₃-alkyl)-amino, (C₃₋₆-cycloalkylcarbonyl)-(C₁₋₃-alkyl)-amino, (C₁₋₄-alkylsulphonyl)-amino, (C₃₋₆ -cycloalkylsulphonyl)-amino, (C₁₋₄-alkylsulphonyl)-(C₁₋₃-alkyl)-amino, (C₃₋₆-cycloalkylsulphonyl)-(C₁₋₃-alkyl)-amino
pyrrolidin-1-yl, piperidin-1-yl, morpholin-4-yl, piperazin-1-yl, 4-(C₁₋₃-alkyl)-piperazin-1-yl,
C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl and C₃₋₆-cycloalkylsulphonyl, wherein the substituents must not be bound to a common carbon atom, and
wherein the heteroaryl group mentioned hereinbefore for R⁵ denotes a monocyclic five-membered aromatic system with 1 to 4 heteroatoms selected from among N, O or S, wherein not more than one oxygen atom and/or one sulphur atom may be present, or a six-membered aromatic system with 1 to 3 nitrogen atoms and may be mono- or disubstituted independently of one another by halogen, cyano, hydroxy, amino, C₁₋₃-alkyl or C₁₋₃-alkyloxycarbonyl, and
wherein in the above-mentioned morpholin-4-yl and piperazin-1-yl groups in each case a methylene unit may be replaced by a carbonyl or sulphonyl group,
and the tautomers, stereoisomers, mixtures thereof and the salts thereof.

2. Compounds of general formula I according to claim 1, wherein
R denotes a group of the formula mentioned in claim 1, wherein
R¹ denotes H, C₁₋₆-alkyl or a group of formula 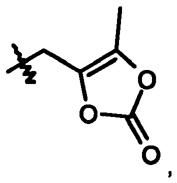 wherein the C₁₋₆-alkyl group mentioned hereinbefore for R¹ may be substituted by C₁₋₆-alkyl-carbonyloxy, C₁₋₆-alkoxy-carbonyloxy, C₁₋₆-alkoxy, hydroxy, amino, C₁₋₃-alkyl-amino, di-(C₁₋₃-alkyl)-amino, pyrrolidin-1-yl, piperidin-1-yl, morpholin-4-yl, piperazin-1-yl, 4-(C₁₋₃-alkyl)-piperazin-1-yl, aminocarbonyl, C₁₋₃-alkyl-aminocarbonyl, di-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl or 4-(C₁₋₃ -alkyl)-piperazin-1-yl-carbonyl,
R² and R³ independently of one another denote halogen, C₁₋₃-alkyl, C₁₋₃-perfluoroalkyl, C₁₋₂-alkoxy or cyano and
A denotes CH or N,
m denotes 0, 1 or 2,
R⁴ denotes halogen, C₁₋₃-alkyl, trifluoromethyl or cyano, while, if m denotes the number 2, the groups R⁴ may be identical or different,
and the heterocyclic group 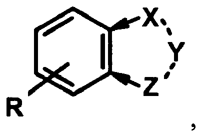 which may be substituted by R⁴ as described hereinbefore denotes a group of formula 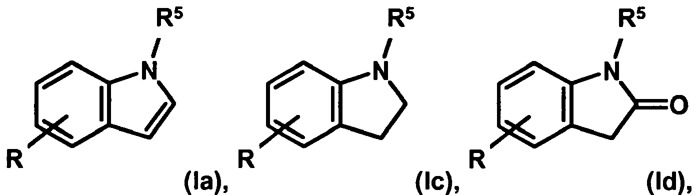 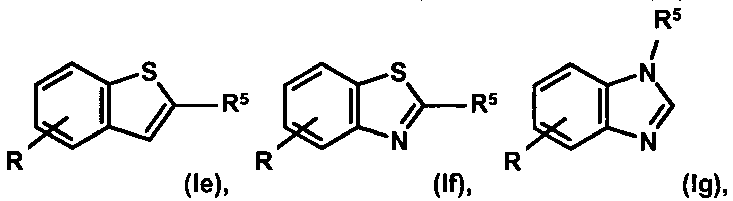 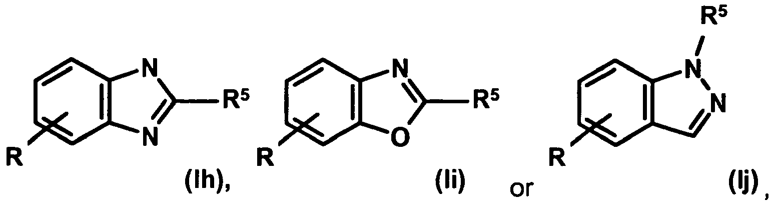
wherein the above-mentioned heterocycles of formulae (Ia), (Ic), (Id), (Ie), (Ig) and (Ij) may optionally be substituted at the carbon atoms of the 5 ring by one or two groups selected from among halogen, C₁₋₃-alkyl, cyano, C₁₋₃ -perfluoroalkyl, C₃₋₆-cycloalkyl, C₁₋₃-alkyl-carbonyl, C₁₋₃-perfluoroalkyl-carbonyl, aminocarbonyl, C₁₋₃-alkyl-aminocarbonyl or di-(C₁₋₃-alkyl)-aminocarbonyl, wherein the groups may be identical or different and each carbon atom carries at most one group, and
R⁵ denotes 1,3-dimethyl-1H-pyrimidin-2,4-dion-5-yl, 1H-pyrimidin-2,4-dion-6-yl, 1H-pyrimidin-2,4-dion-5-yl, 2H-pyridazin-3-on-6-yl, 1H-pyridin-2-on-3-yl, 1H-pyridin-2-on-5-yl, 1H-pyridin-2-on-4-yl or
a mono- or bicyclic 5- to 14-membered ring system, which may contain 0 to 4 heteroatoms selected from among N, O or S, wherein not more than one oxygen atom and/or one sulphur atom may be present, is aromatic, saturated or partially unsaturated and may be mono-, di- or trisubstituted independently of one another by a group selected from among
halogen, cyano,
C₁₋₆-alkyl, C₃₋₆-cycloalkyl, C₁₋₃-perfuoroalkyl, C₂₋₆-alkynyl, C₂₋₆-alkenyl,
hydroxy, C₁₋₆-alkoxy, C₃₋₆-cycloalkoxy, trifluoromethoxy,
carboxyl, C₁₋₃-alkyl-carbonyl, C₁₋₄-alkoxy-carbonyl, cyclopropoxycarbonyl, aminocarbonyl, C₁₋₆-alkyl-aminocarbonyl, di-(C₁₋₆-alkyl)-aminocarbonyl, N-(C₁₋₃-alkyl)-N-(C₁₋₃-alkoxy)-aminocarbonyl, C₃₋₆-cycloalkyl-aminocarbonyl, N-(C₃₋₆-cycloalkyl)-N-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl, 4-(C₁₋₃-alkyl-carbonyl)-piperazin-1-yl-carbonyl, 4-(C₃₋₆-cycloalkyl-carbonyl)-piperazin-1-yl-carbonyl, 4-(C₁₋₃ -alkylsulphonyl)-piperazin-1-yl-carbonyl, 4-(C₃₋₆-cycloalkyl-sulphonyl)-piperazin-1-yl-carbonyl,
C₁₋₃-alkylsulphanyl, C₃₋₆-cycloalkylsulphanyl, C₁₋₃-alkylsulphinyl, C₃₋₆ -cycloalkylsulphinyl, C₁₋₃-alkylsulphonyl, C₃₋₆-cycloalkylsulphonyl, amino, C₁₋₆-alkyl-amino, di-(C₁₋₆-alkyl)-amino, C₃₋₆-cycloalkylamino, N-(C₃₋₆-cycloalkyl)-N-(C₁₋₃-alkyl)-amino, (C₁₋₄-alkylcarbonyl)-amino, (C₃₋₆-cycloalkyl-carbonyl)-amino, (C₁₋₄-alkylcarbonyl)-(C₁₋₃-alkyl)-amino, (C₃₋₆-cycloalkyl-carbonyl)-(C₁₋₃-alkyl)-amino, pyrrolidin-1-yl, piperidin-1-yl, 3-amino-piperidin-1-yl, 4-amino-piperidin-1-yl, morpholin-4-yl, thiomorpholin-4-yl, 1-oxo-1λ⁴-thiomorpholin-4-yl, 1,1-dioxo-1λ⁶-thiomorpholin-4-yl, piperazin-1-yl, homopiperazin-1-yl, 4-(C₁₋₃-alkyl)-piperazin-1-yl, 4-(C₁₋₃-alkylcarbonyl)-piperazin-1-yl, 4-(C₃₋₆-cycloalkyl-carbonyl)-piperazin-1-yl, 4-(C₁₋₃-alkylsulphonyl)-piperazin-1-yl, 4-(C₃₋₆-cycloalkylsulphonyl)-piperazin-1-yl,
tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, 2-oxo-tetrahydropyrimidinyl and heteroaryl,
wherein the C₁₋₆-alkyl, C₃₋₆-cycloalkyl, C₂₋₆-alkynyl, C₂₋₆-alkenyl, C₁₋₃ -alkylsulphanyl, C₃₋₆-cycloalkylsulphanyl, C₁₋₃-alkylsulphinyl, C₃₋₆-cycloalkylsulphinyl, C₁₋₃-alkylsulphonyl, C₃₋₆-cycloalkylsulphonyl, C₁₋₆-alkoxy, C₃₋₆-cycloalkoxy, C₁₋₆-alkyl-amino, di-(C₁₋₆-alkyl)-amino, C₃₋₆-cycloalkyl-amino, N-(C₃₋₆-cycloalkyl)-N-(C₁₋₃-alkyl)-amino, (C₁₋₄ -alkyl-carbonyl)-amino, (C₃₋₆-cycloalkyl-carbonyl)-amino, (C₁₋₄-alkyl-carbonyl)-(C₁₋₃-alkyl)-amino- and (C₃₋₆-cycloalkyl-carbonyl)-(C₁₋₃-alkyl)-amino groups mentioned above in the definition of R⁵ may each be mono- or disubstituted independently of one another in the carbon skeleton by a group selected from among
cyano, hydroxy, C₁₋₃-alkoxy, tetrahydro-pyran-2-yloxy,
amino, C₁₋₆-alkyl-amino, di-(C₁₋₆-alkyl)-amino, C₃₋₆-cycloalkyl-amino, N-(C₃₋₆-cycloalkyl)-N-(C₁₋₃-alkyl)-amino, (C₁₋₄-alkyl-carbonyl)-amino, (C₃₋₆-cycloalkyl-carbonyl)-amino, (C₁₋₄-alkyl-carbonyl)-(C₁₋₃-alkyl)-amino, (C₃₋₆-cycloalkylcarbonyl)-(C₁₋₃-alkyl)-amino, (C₁₋₄-alkylsulphonyl)-amino, (C₃₋₆ -cycloalkylsulphonyl)-amino, (C₁₋₄-alkylsulphonyl)-(C₁₋₃-alkyl)-amino, (C₃₋₆-cycloalkylsulphonyl)-(C₁₋₃-alkyl)-amino
pyrrolidin-1-yl, piperidin-1-yl, morpholin-4-yl, piperazin-1-yl, 4-(C₁₋₃-alkyl)-piperazin-1-yl,
carboxyl, C₁₋₂-alkoxy-carbonyl, aminocarbonyl, C₁₋₆-alkylaminocarbonyl, di-(C₁₋₆-alkyl)-aminocarbonyl, C₃₋₆-cycloalkylaminocarbonyl, N-(C₃₋₆-cycloalkyl)-N-(C₁₋₃-alkyl)-aminocarbonyl, pyrrolidin-1-yl-carbonyl, piperidin-1-yl-carbonyl, morpholin-4-yl-carbonyl, piperazin-1-yl-carbonyl, 4-(C₁₋₃-alkyl)-piperazin-1-yl-carbonyl,
C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl and C₃₋₆-cycloalkylsulphonyl, wherein the substituents must not be bound to a common carbon atom, and
wherein the pyrrolidin-1-yl and piperidin-1-yl groups mentioned above in the definition of R⁵ may be substituted by amino or hydroxy, and
wherein the C₁₋₆-alkyl-aminocarbonyl, di-(C₁₋₆-alkyl)-aminocarbonyl, C₃₋₆-cycloalkyl-aminocarbonyl, N-(C₃₋₆-cycloalkyl)-N-(C₁₋₃-alkyl)-aminocarbonyl groups mentioned above in the definition of R⁵ may each be mono- or disubstituted independently of one another in the carbon skeleton by a group selected from among
amino, hydroxy, C₁₋₃-alkoxy, C₁₋₃-alkyl-amino, di-(C₁₋₃-alkyl)-amino, C₃₋₆-cycloalkyl-amino, N-(C₃₋₆-cycloalkyl)-N-(methyl)-amino, (C₁₋₄-alkyl-carbonyl)-amino, (C₃₋₆-cycloalkylcarbonyl)-amino, (C₁₋₄-alkyl-carbonyl)-(methyl)-amino, (C₃₋₆-cycloalkyl-carbonyl)-(methyl)-amino, (C₁₋₄-alkylsulphonyl)-amino, (C₃₋₆-cycloalkylsulphonyl)-amino, (C₁₋₄-alkylsulphonyl)-(methyl)-amino or (C₃₋₆-cycloalkylsulphonyl)-(methyl)-amino,
pyrrolidin-1-yl, piperidin-1-yl, morpholin-4-yl, piperazin-1-yl, 4-(C₁₋₃-alkyl)-piperazin-1-yl,
C₁₋₃-alkylsulphanyl, C₃₋₆-cycloalkylsulphanyl, C₁₋₃-alkylsulphinyl, C₃₋₆-cycloalkylsulphinyl, C₁₋₃-alkylsulphonyl and C₃₋₆-cycloalkylsulphonyl, wherein the substituents must not be bound to a common carbon atom, and
wherein the heteroaryl group mentioned hereinbefore for R⁵ denotes a monocyclic five-membered aromatic system with 1 to 3 heteroatoms selected from among N, O or S, wherein not more than one oxygen and/or one sulphur atom may be present, or denotes a monocyclic five-membered aromatic system with 4 nitrogen atoms or a six-membered aromatic system with 1 to 3 nitrogen atoms and may be mono- or disubstituted independently of one another by fluorine, chlorine, cyano, C₁₋₃-alkyl or C₁₋₃-alkyloxycarbonyl, and
wherein in the above-mentioned morpholin-4-yl and piperazin-1-yl groups in each case a methylene unit may be replaced by a carbonyl or sulphonyl group,
and the tautomers, stereoisomers, mixtures thereof and the salts thereof.

3. Compounds of general formula I according to claim 2, wherein
R denotes a group of the formula mentioned in claim 1, wherein
R¹ denotes H, C₁₋₄-alkyl or a group of formula 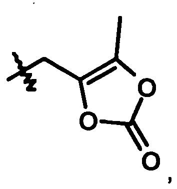 wherein the C₁₋₄-alkyl group mentioned hereinbefore for R¹ may be substituted by C₁₋₄-alkoxy, hydroxy, di-(C₁₋₃-alkyl)-amino, pyrrolidin-1-yl, piperidin-1-yl, morpholin-4-yl, piperazin-1-yl or 4-(methyl)-piperazin-1-yl,
R² and R³ independently of one another denote chlorine, bromine or C₁₋₂-alkyl and
A denotes CH or N,
m denotes 0 or 1,
R⁴ denotes fluorine, chlorine, methyl or ethyl,
and the heterocyclic group 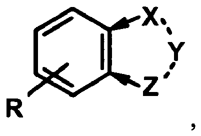 which may be substituted by R⁴ as described hereinbefore denotes a group of formula 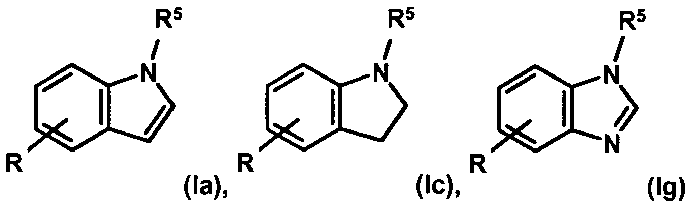 or 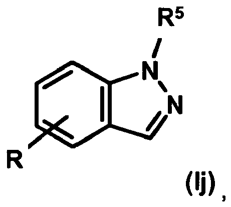 while the heterocycles mentioned hereinbefore may optionally be substituted at the carbon atoms of the 5 ring by one or two groups selected from among chlorine, bromine, iodine, C₁₋₃-alkyl, cyano and trifluoromethyl, wherein the groups may be identical or different and each carbon atom carries at most one group, and
wherein R⁵ denotes 1,3-dimethyl-1H-pyrimidin-2,4-dion-5-yl, 1H-pyrimidin-2,4-dion-6-yl, 1H-pyrimidin-2,4-dion-5-yl, 2H-pyridazin-3-on-6-yl, 1H-pyridin-2-on-3-yl, 1H-pyridin-2-on-5-yl or 1H-pyridin-2-on-4-yl,
phenyl, pyridazin-3-yl, pyrimidin-2-yl, pyrimidin-4-yl, pyrimidin-5-yl, pyrazin-2-yl, 1.3,5-triazin-2-yl, pyridin-2-yl, pyridin-4-yl, imidazol-2-yl, imidazol-4-yl, pyrazol-3-yl, pyrazol-4-yl, thiazol-2-yl, [1,3,4]thiadiazol-2-yl, thiophen-2-yl, thiophen-3-yl, naphthalin-1-yl, naphthalin-2-yl, purin-6-yl, purin-2-yl, 1-imidazo[1,2-a]pyrazin-6-yl, quinolin-6-yl, quinolin-8-yl, quinolin-2-yl or isoquinolin-1-yl, which may each be mono- or disubstituted independently of one another by a group selected from among
fluorine, chlorine, C₁₋₄-alkyl, cyclopropyl, trifluoromethyl, cyano, hydroxy, C₁₋₃-alkoxy, cyclopropoxy,
carboxyl, C₁₋₂-alkyl-carbonyl, C₁₋₂-alkoxy-carbonyl, aminocarbonyl, C₁₋₄-alkyl-aminocarbonyl, di-(C₁₋₂-alkyl)-aminocarbonyl, N-methoxy-N-methyl-aminocarbonyl, cyclopropyl-aminocarbonyl, N-( cyclopropyl)-N-(methyl)-aminocarbonyl, pyrrolidin-1-yl-carbonyl, piperidin-1-yl-carbonyl, morpholin-4-yl-carbonyl, piperazin-1-yl-carbonyl, 4-(methyl)-piperazin-1-yl-carbonyl, 4-(C₁₋₃-alkyl-carbonyl)-piperazin-1-yl-carbonyl, 4-(C₃₋₆-cycloalkyl-carbonyl)-piperazin-1-yl-carbonyl, 4-(C₁₋₃-alkylsulphonyl)-piperazin-1-yl-carbonyl, 4-(C₃₋₆-cycloalkylsulphonyl)-piperazin-1-yl-carbonyl,
C₁₋₂-alkylsulphanyl, cyclopropylsulphanyl, C₁₋₂-alkylsulphinyl, cyclopropylsulphinyl, C₁₋₂-alkylsulphonyl, cyclopropylsulphonyl,
amino, C₁₋₄-alkyl-amino, di-(C₁₋₃-alkyl)-amino, cyclopropyl-amino, N-( cyclopropyl)-N-(methyl)-amino, C₁₋₃-alkyl-carbonyl-amino,
pyrrolidin-1-yl, piperidin-1-yl, 3-amino-piperidin-1-yl, 4-amino-piperidin-1-yl, morpholin-4-yl, thiomorpholin-4-yl, 1-oxo-1λ⁴-thiomorpholin-4-yl, 1,1-dioxo-1λ⁶-thiomorpholin-4-yl, piperazin-1-yl, homopiperazin-1-yl, 4-(methyl)-piperazin-1-yl, 4-(C₁₋₂-alkylcarbonyl)-piperazin-1-yl, 4-(C₁₋₂-alkylsulphonyl)-piperazin-1-yl,
tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, 2-oxotetrahydropyrimidinyl, imidazol-2-yl, 1-methyl-imidazol-2-yl, thiazol-2-yl, 4-ethoxycarbonyl-thiazol-2-yl, 3-ethoxycarbonyl-isoxazol-5-yl, oxazol-2-yl, 2,4-dihydroxy-pyrimidin-5-yl.1.2,4-triazol-3-yl and tetrazol-5-yl, or
wherein the C₁₋₄-alkyl, C₁₋₃-alkoxy, C₁₋₄-alkyl-amino, di-(C₁₋₃-alkyl)-amino- and C₁₋₃-alkyl-carbonyl-amino groups mentioned hereinbefore for R⁵ may each be mono- or disubstituted independently of one another in the carbon skeleton by a group selected from among
cyano, hydroxy, C₁₋₂-alkoxy, tetrahydro-pyran-2-yloxy,
amino, C₁₋₃-alkyl-amino, di-(C₁₋₃-alkyl)-amino, (C₁₋₃-alkylcarbonyl)-amino, (C₁₋₃-alkylsulphonyl)-amino,
pyrrolidin-1-yl, piperidin-1-yl, morpholin-4-yl, piperazin-1-yl, 4-(methyl)-piperazin-1-yl,
carboxyl, C₁₋₂-alkoxy-carbonyl, aminocarbonyl, C₁₋₃-alkylaminocarbonyl, di-(C₁₋₃-alkyl)-aminocarbonyl and C₃₋₆-cycloalkyl-aminocarbonyl, wherein the substituents must not be bound to a common carbon atom, and
wherein the C₁₋₄-alkyl-aminocarbonyl and di-(C₁₋₂-alkyl)-aminocarbonyl groups mentioned hereinbefore for R⁵ may each be mono- or disubstituted independently of one another in the carbon skeleton by amino, hydroxy, C₁₋₃-alkoxy, C₁₋₃-alkyl-amino or di-(C₁₋₃ -alkyl)-amino, wherein the substituents must not be bound to a common carbon atom, and
wherein in the above-mentioned morpholin-4-yl and piperazin-1-yl groups in each case a methylene unit may be replaced by a carbonyl group,
and the tautomers, stereoisomers, mixtures thereof and the salts thereof.

4. Compounds of general formula I according to claim 3, wherein
R denotes a group of the formula mentioned in claim 1, wherein
R¹ denotes H or a C₁₋₃-alkyl group optionally substituted by a di-(C₁₋₃-alkyl)-amino group,
R² and R³ independently of one another denote chlorine, bromine or methyl and
A denotes CH or N,
m denotes 0 or 1,
R⁴ denotes chlorine, methyl or ethyl,
and the heterocyclic group 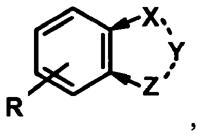 which may be substituted by R⁴ as described hereinbefore denotes a group of formula 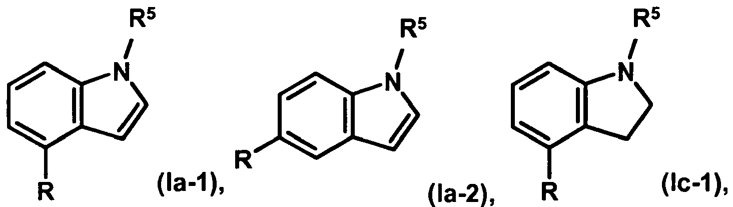 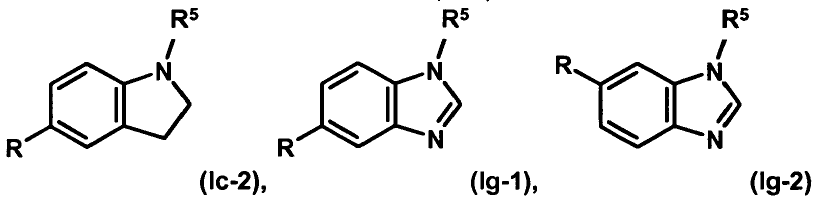 or 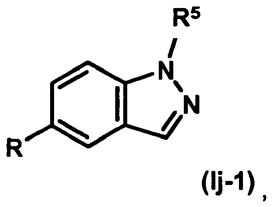 while the heterocycles mentioned hereinbefore may optionally be substituted at the carbon atoms of the 5 ring by one or two groups selected from among chlorine, bromine, iodine, C₁₋₂-alkyl, cyano and trifluoromethyl, wherein the groups may be identical or different and each carbon atom carries at most one group, and
R⁵ is defined as in claim 3,
and the tautomers, stereoisomers, mixtures thereof and the salts thereof.

5. Compounds of general formula I according to claim 4, wherein
R denotes a group of the formula mentioned in claim 1, wherein
R¹ denotes H, methyl, ethyl or 2-dimethylamino-ethyl,
R² and R³ independently of one another denote chlorine, bromine or methyl and
A denotes CH or N,
m denotes 0 or 1,
R⁴ denotes chlorine, methyl or ethyl,
and the heterocyclic group 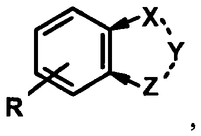 which may be substituted by R⁴ as described hereinbefore denotes a group of formula 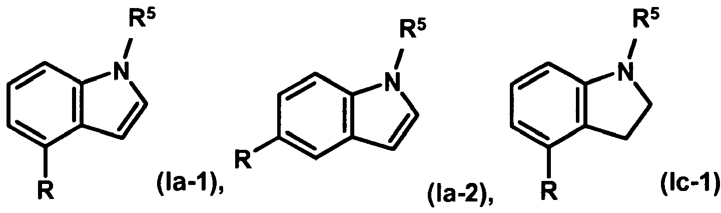 or 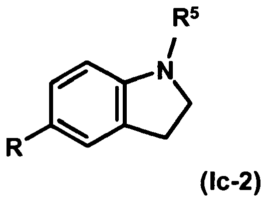
while the heterocycles mentioned hereinbefore may optionally be substituted at the carbon atoms of the 5 ring by one or two methyl or ethyl groups, wherein the groups may be identical or different and each carbon atom carries at most one group, and wherein
R⁵ denotes 1,3-dimethyl-1H-pyrimidin-2,4-dion-5-yl, 1H-pyrimidin-2,4-dion-6-yl, 1H-pyrimidin-2,4-dion-5-yl, 2H-pyridazin-3-on-6-yl, 1H-pyridin-2-on-3-yl, 1H-pyridin-2-on-5-yl, 1H-pyridin-2-on-4-yl or
phenyl, pyridazin-3-yl, pyrimidin-2-yl, pyrimidin-4-yl, pyrimidin-5-yl, pyrazin-2-yl, pyridin-2-yl, pyridin-4-yl, imidazol-2-yl, imidazol-4-yl, pyrazol-3-yl, pyrazol-4-yl, 1.3,5-triazin-2-yl, thiazol-2-yl, [1,3,4]thiadiazol-2-yl, thiophen-2-yl, thiophen-3-yl, purin-6-yl, purin-2-yl or 1-imidazo[1,2-a]pyrazin-6-yl, which may be mono- or disubstituted independently of one another by a group selected from among
chlorine, cyano, methyl, aminomethyl, morpholin-4-ylmethyl, hydroxymethyl, 3-hydroxypropyl, trifluoromethyl,
hydroxy, methoxy, 2-hydroxyethoxy, 2-aminoethoxy, 2-dimethylaminoethoxy, 2-methylsulphonylamino-ethoxy, 2-acetylamino-ethoxy, 2,3-dihydroxy-propoxy,
carboxyl, acetyl, ethylcarbonyl, aminocarbonyl, methyl-aminocarbonyl, dimethyl-aminocarbonyl, N-methoxy-N-methyl-aminocarbonyl, 2-dimethylamino-ethyl-aminocarbonyl, 2-hydroxy-ethyl-aminocarbonyl, 2-methoxy-ethyl-aminocarbonyl, cyclo-aminocarbonyl,
morpholin-4-yl-carbonyl, piperazin-1-yl-carbonyl, methoxy-carbonyl,
methylsulphanyl, methylsulphinyl, ethylsulphinyl, methylsulphonyl,
amino, methyl-amino, acetylamino, 2-aminoethyl-amino, 2-dimethylaminoethyl-amino, 2-hydroxyethyl-amino, 2-(methylamino)-ethyl-amino, N-carboxymethyl-N-[2-(dimethylamino)-ethyl]-amino, 2-(acetylamino)ethyl-amino, 2-(methylsulphonylamino)-ethyl-amino, 2-(pyrrolidin-1-yl)-ethyl-amino, 2-(piperidin-1-yl)-ethyl-amino, 2-(tetrahydro-pyran-2-yloxy)-ethylamino, 3-aminopropyl-amino, 3-(methylamino)-propyl-amino, 2-amino-2-methyl-propyl-amino, 1,3-dihydroxy-2-propyl-amino, 3-(acetylamino)-propyl-amino, 3-(methylsulphonylamino)-propyl-amino, dimethyl-amino, N-methyl-N-2-aminoethyl-amino, N,N-bis-2-(hydroxyethyl)-amino, N-methyl-N-3-aminopropyl-amino, N-methyl-N-[3-(acetylamino)-propyl]-amino, N-methyl-N-[3-(methylsulphonylamino)-propyl]-amino, cyclopropyl-amino,
morpholin-4-yl, thiomorpholin-4-yl, 1-oxo-1λ⁴-thiomorpholin-4-yl, 1,1-dioxo-1λ⁶-thiomorpholin-4-yl, 3-amino-piperidin-1-yl, piperazin-1-yl, homopiperazin-1-yl, 4-acetyl-piperazin-1-yl, 4-methylsulphonyl-piperazin-1-yl,
tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, imidazol-2-yl, 1-methyl-imidazol-2-yl, thiazol-2-yl, 4-ethoxycarbonylthiazol-2-yl, 3-ethoxycarbonyl-isoxazol-5-yl, and oxazol-2-yl,
wherein in the above-mentioned morpholin-4-yl and piperazin-1-yl groups in each case a methylene unit may be replaced by a carbonyl group,
and the tautomers, stereoisomers, mixtures thereof and the salts thereof.

6. Compounds of general formula I according to claim 5, wherein
R denotes a group of the formula mentioned in claim 1, wherein
R¹ denotes hydrogen,
R² and R³ in each case represent chlorine and
A denotes CH ,
m denotes 0 and
and the heterocyclic group 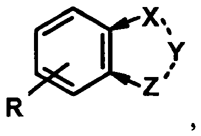 denotes a group of formula 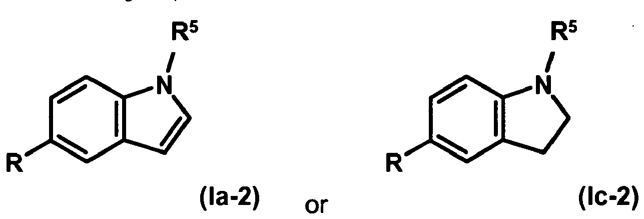 while the heterocycles mentioned hereinbefore may optionally be substituted at the carbon atoms of the 5 ring by one or two methyl groups, each carbon atom carries at most one group, and wherein
R⁵ denotes phenyl, pyridazin-3-yl, pyrimidin-2-yl, pyrimidin-4-yl, pyrimidin-5-yl, pyrazin-2-yl, pyridin-2-yl or pyridin-4-yl, which may be substituted by a group selected from among
cyano, methyl, aminomethyl, hydroxymethyl, 3-hydroxypropyl, trifluoromethyl, hydroxy, methoxy, 2-hydroxyethoxy, 2-aminoethoxy, 2-(dimethylamino)-ethoxy, 2-(methylsulphonyl)-amino-ethoxy, 2-(acetylamino)-ethoxy, 2,3-dihydroxy-propoxy,
carboxyl, acetyl, ethylcarbonyl, aminocarbonyl, methyl-aminocarbonyl, dimethyl-aminocarbonyl, N-methoxy-N-methyl-aminocarbonyl, 2-(dimethylamino)-ethyl-aminocarbonyl, 2-hydroxy-ethyl-aminocarbonyl, 2-methoxy-ethyl-aminocarbonyl, cyclopropyl-aminocarbonyl,
morpholin-4-yl-carbonyl, piperazin-1-yl-carbonyl, methoxy-carbonyl,
methylsulphanyl, methylsulphinyl, ethylsulphinyl, methylsulphonyl,
amino, methyl-amino, acetylamino, 2-aminoethyl-amino, 2-(dimethylamino)-ethyl-amino, 2-hydroxyethyl-amino, 2-(methylamino)-ethyl-amino, N-carboxymethyl-N-[2-(dimethylamino)-ethyl]-amino, 2-(acetylamino)-ethyl-amino, 2-(methylsulphonylamino)-ethyl-amino, 2-(pyrrolidin-1-yl)-ethyl-amino, 2-(piperidin-1-yl)-ethyl-amino, 3-aminopropyl-amino, 3-(methylamino)-propyl-amino, 3-(acetylamino)-propyl-amino, 3-(methylsulphonylamino)-propyl-amino, dimethyl-amino, N-methyl-N-(2-aminoethyl)-amino, N,N-bis-(2-hydroxyethyl)-amino, N-methyl-N-(3-aminopropyl)-amino, N-methyl-N-[3-(acetylamino)-propyl]-amino, N-methyl-N-[3-(methylsulphonylamino)-propyl]-amino, cyclopropyl-amino,
morpholin-4-yl, thiomorpholin-4-yl, 1-oxo-1λ⁴-thiomorpholin-4-yl, 1,1-dioxo-1λ⁶-thiomorpholin-4-yl, 3-amino-piperidin-1-yl, piperazin-1-yl, homopiperazin-1-yl , 4-acetyl-piperazin-1-yl, 4-methylsulphonyl-piperazin-1-yl,
tetrahydrofuran-3-yl-oxy, tetrahydrofuran-3-yl-amino, tetrahydropyran-4-yl-oxy, tetrahydropyran-4-yl-amino, N-tetrahydropyran-4-yl-N-methyl-amino, 2-oxo-imidazolidinyl, imidazol-2-yl, 1-methyl-imidazol-2-yl, thiazol-2-yl, 4-ethoxycarbonylthiazol-2-yl, 3-ethoxycarbonyl-isoxazol-5-yl, and oxazol-2-yl,
and the tautomers, stereoisomers, mixtures thereof and the salts thereof.

7. The following compounds of general formula I according to claim 1:
(1) ((3,5-dichloro-phenylsulphonyl)-{1-[5-(2-dimethylamino-ethylamino)-pyrazin-2-yl]-1H-indol-5-yl}-amino)-acetic acid
(2) {(3,5-dichloro-phenylsulphonyl)-[1-(6-piperazin-1-yl-pyridazin-3-yl)-2,3-dihydro-1H-indol-5-yl]-amino}-acetic acid
(3) ((3,5-dichloro-phenylsulphonyl)-{1-[6-(2-dimethylamino-ethylamino)-pyridazin-3-yl]-1H-indol-5-yl}-amino)-acetic acid
(4) {(3,5-dichloro-phenylsulphonyl)-[1-(6-methyl-pyridazin-3-yl)-1H-indol-5-yl]-amino}-acetic acid
(5) ((3-bromo-5-methyl-phenylsulphonyl)-{1-[6-(2-dimethylamino-ethylamino)-pyridazin-3-yl]-1H-indol-5-yl}-amino)-acetic acid
(6) {(3,5-dichloro-phenylsulphonyl)-[1-(6-piperazin-1-yl-pyridazin-3-yl)-1H-indol-5-yl]-amino}-acetic acid
(7) [[1-(6-[1,4]diazepan-1-yl-pyridazin-3-yl)-1H-indol-5-yl]-(3,5-dichlorophenylsulphonyl)-amino]-acetic acid
(8) {(3,5-dichloro-phenylsulphonyl)-[1-(4-methyl-pyridin-2-yl)-1H-indol-5-yl]-amino}-acetic acid
(9) ((3,5-dichloro-phenylsulphonyl)-{1-[6-(2-dimethylamino-ethylamino)-pyridazin-3-yl]-3-methyl-1H-indol-5-yl}-amino)-acetic acid
(10) [(3,5-dichloro-phenylsulphonyl)-(1-{6-[methyl-(tetrahydro-pyran-4-yl)-amino]-pyridazin-3-yl}-1H-indol-5-yl)-amino]-acetic acid
(11) ((3,5-dichloro-phenylsulphonyl)-{1-[5-(3-oxo-piperazin-1-yl)-pyrazin-2-yl]-1H-indol-5-yl}-amino)-acetic acid
(12) [[6-ethyl-1-(6-methyl-pyridazin-3-yl)-1H-indol-5-yl]-(3,5-dichlorophenylsulphonyl)-amino]-acetic acid
(13) [[1-(4-amino-pyridin-2-yl)-1H-indol-5-yl]-(3,5-dichloro-phenylsulphonyl)-amino]-acetic acid
(14) ((3,5-dichloro-phenylsulphonyl)-{1-[6-(3-hydroxy-propyl)-pyridazin-3-yl]-1H-indol-5-yl}-amino)-acetic acid
(15) [{1-[6-(4-acetyl-piperazin-1-yl)-pyridazin-3-yl]-1H-indol-5-yl}-(3,5-dichlorophenylsulphonyl)-amino]-acetic acid
(16) {(3,5-dichloro-phenylsulphonyl)-[1-(4-methylcarbamoyl-pyridin-2-yl)-1H-indol-5-yl]-amino}-acetic acid
(17) {(3,5-dichloro-phenylsulphonyl)-[3-methyl-1-(6-methyl-pyridazin-3-yl)-1H-indol-5-yl]-amino}-acetic acid
(18) {(3,5-dichloro-phenylsulphonyl)-[1-(4-methanesulphinyl-pyridin-2-yl)-1H-indol-5-yl]-amino}-acetic acid
(19) [[1-(4-cyclopropylcarbamoyl-pyridin-2-yl)-1H-indol-5-yl]-(3,5-dichlorophenylsulphonyl)-amino]-acetic acid
(20) {(2,6-dichloro-pyridin-4-sulphonyl)-[1-(4-methylcarbamoyl-pyridin-2-yl)-1H-indol-5-yl]-amino}-acetic acid,
and the enantiomers, mixtures thereof and salts thereof.

8. Physiologically acceptable salts of the compounds according to claims 1 to 7 with inorganic or organic acids or bases.

9. Pharmaceutical compositions, containing a compound according to at least one of claims 1 to 7 or a salt according to claim 8 optionally together with one or more inert carriers and/or diluents.

10. Use of a compound according to at least one of claims 1 to 7 or a salt according to claim 8 for preparing a pharmaceutical composition that is suitable for the treatment of type I and type II diabetes mellitus.

11. Process for preparing a pharmaceutical composition according to claim 9, **characterised in that** a compound according to at least one of claims 1 to 7 or a salt according to claim 8 is incorporated in one or more inert carriers and/or diluents by a non-chemical method.

12. Process for preparing the compounds of general formula I according to claims 1 to 8, **characterised in that**
a) a compound of general formula 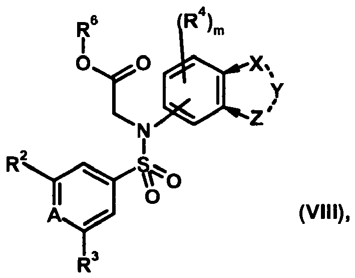 wherein R² to R⁵, m, X, Y, Z and A are defined as mentioned in claim 1 and R⁶ denotes a carboxy protective group, is deprotected and the acid thus obtained is optionally converted by alkylation into a compound of general formula (I) according to claim 1 and
b) if desired any protective group used to protect reactive groups during the reactions is cleaved afterwards or simultaneously and/or
c) a compound of general formula I thus obtained is resolved into its stereoisomers and/or
d) a compound of general formula I thus obtained is converted into the salts thereof, particularly for pharmaceutical use into the physiologically acceptable salts thereof with an inorganic or organic acid or base.

## Patentansprüche

1. Arylsulphonylglycine der allgemeinen Formel 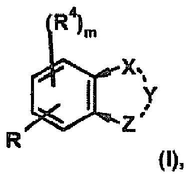 worin
R eine Gruppe der Formel 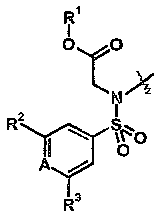 bezeichnet, worin
R¹ H, C₁₋₆-Alkyl oder eine Gruppe der Formel 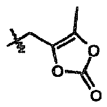 bezeichnet, worin die hier vorstehend für R¹ erwähnte C₁₋₆-Alkylgruppe substituiert sein kann mit C₁₋₆-Alkyl-carbonyloxy, C₁₋₆-Alkoxy-carbonyloxy, C₁₋₆-Alkoxy, Hydroxy, Amino, C₁₋₃-Alkyl-amino, Di-(C₁₋₃-alkyl)-amino, Pyrrolidin-1-yl, Piperidin-1-yl, Morpholin-4-yl, Piperazin-1-yl, 4-(C₁₋₃-Alkyl)-piperazin-1-yl, Aminocarbonyl, C₁₋₃-Alkyl-aminocarbonyl, Di-(C₁₋₃-alkyl)-aminocarbonyl, Pyrrolidin-1-yl-carbonyl, Piperidin-1-yl-carbonyl, Morpholin-4-yl-carbonyl, Piperazin-1-yl-carbonyl, 4-(C₁₋₃-Alkyl)-piperazin-1-yl-carbonyl, Tetrahydrofuran-3-yl-oxy, C₁₋₃-Alkylamino-C₁₋₃-alkyloxy, Di-(C₁₋₃-alkyl)-amino-C₁₋₃-alkyloxy, Pyrrolidin-1-yl-C₁₋₃-alkyloxy, Piperidin-1-yl-C₁₋₃-alkyloxy, Morpholin-4-yl-C₁₋₃-alkyloxy, Piperazin-1-yl-C₁₋₃-al-kyloxy oder 4-(C₁₋₃-Alkyl)-piperazin-1-yl-C₁₋₃-alkyloxy,
R² und R³ bezeichnen unabhängig voneinander H, Halogen, C₁₋₃-Alkyl, C₁₋₃-Perfluoroalkyl, C₁₋₃-Perfluoroalkoxy, C₁₋₃-Alkoxy, Cyano, Nitro oder Hydroxy,
und
A bezeichnet CH oder N,
m bezeichnet 0,1 oder 2,
R⁴ bezeichnet Halogen, C₁₋₃-Alkyl, C₁₋₃-Perfluoroalkyl, C₁₋₃-Perfluoroalkoxy, Cyano, Hydroxy oder C₁₋₃-Alkoxy, wobei, wenn m die Zahl 2 bezeichnet, die R⁴-Gruppen gleich oder verschieden sein können,
und die heterocyclische Gruppe 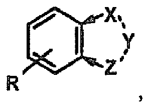 die substituiert sein kann mit R⁴ wie hier zuvor beschrieben, bezeichnet eine Gruppe der Formel 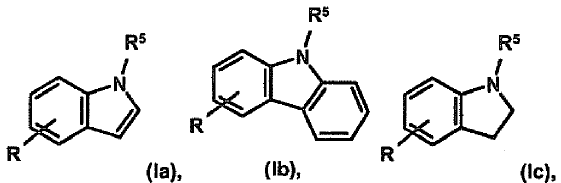 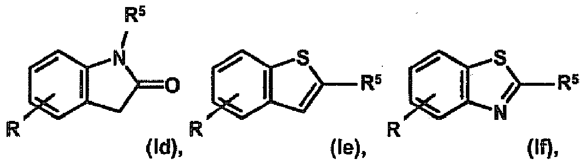 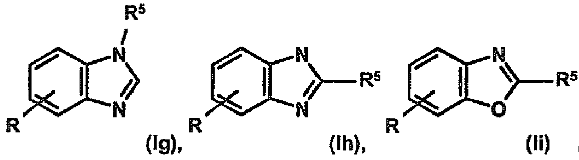 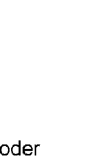 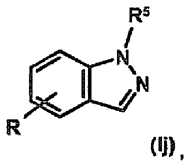
worin die oben erwähnten Heterocyclen der Formeln (la), (Ic), (Id), (Ie), (Ig) und (Ij) gegebenenfalls an den Kohlenstoffatomen des Fünfrings mit ein oder zwei Gruppen substituiert sein können, ausgewählt aus Halogen, C₁₋₃-Alkyl, Cyano, C₁₋₃-Perfluoroalkyl, C₃₋₆-Cycloalkyl, C₂₋₄-Alkinyl, C₂₋₄-Alkenyl, C₁₋₃-Alkyl-carbonyl, C₁₋₃-Perfluoroalkyl-carbonyl, Carboxyl, Aminomethyl, C₁₋₃-Alkylaminomethyl, Di-(C₁₋₃-alkyl)-aminomethyl, Aminocarbonyl, C₁₋₃-Alkyl-aminocarbonyl oder Di-(C₁₋₃-alkyl)-aminocarbonyl, wobei die Gruppen gleich oder verschieden sein können und jedes Kohlenstoffatom nur eine Gruppe tragen kann, und
worin R⁵ eine 1H-Pyrimidin-2,4-dionyl-, 2H-Pyridazin-3-onyl- oder 1H-Pyridin-2-onyl-Gruppe bezeichnet, gegebenenfalls mono- oder disubstituiert mit ein oder zwei Methylgruppen, oder
ein mono- oder bicyclisches, 5- bis 14-gliedriges Ringsystem, das 0 bis 4 Heteroatome enthalten kann, ausgewählt aus N, O oder S, worin nicht mehr als ein Sauerstoffatom und/oder ein Schwefelatom vorhanden sein kann, das aromatisch, gesättigt oder teilweise ungesättigt ist und das unabhängig voneinander mono-, di- oder trisubstituiert sein kann mit einer Gruppe, ausgewählt aus,
Halogen, Cyano, Nitro,
C₁₋₆-Alkyl, C₃₋₆-Cycloalkyl, C₁₋₃-Perfluoroalkyl, C₂₋₆-Alkinyl, C₂₋₆-Alkenyl,
Hydroxy, C₁₋₆-Alkoxy, C₃₋₆-Cycloalkoxy, C₁₋₃-Perfluoroalkoxy,
Carboxyl, C₁₋₃-Alkyl-carbonyl, C₁₋₄-Alkoxy-carbonyl, C₃₋₆-Cycloalkoxy-carbonyl, Aminocarbonyl, C₁₋₆-Alkyl-aminocarbonyl, Di-(C₁₋₆-alkyl)-aminocarbonyl, N-(C₁₋₆-Alkyl)-N-(C₁₋₆-alkoxy)-amino-carbonyl, C₃₋₆-Cycloalkyl-aminocarbonyl, N-(C₃₋₆-Cycloalkyl)-N-(C₁₋₆-alkyl)-aminocarbonyl, Pyrrolidin-1-yl-carbonyl, Piperidin-1-yl-carbonyl, Morpholin-4-yl-carbonyl, Piperazin-1-yl-carbonyl, 4-(C₁₋₃-Alkyl)-piperazin-1-yl-carbonyl, 4-(C₁₋₃-Alkyl-carbonyl)-piperazin-1-yl-carbonyl, 4-(C₃₋₆-Cycloalkyl-carbonyl)-piperazin-1-yl-carbonyl, 4-(C₁₋₃-Alkylsulphonyl)-piperazin-1-yl-carbonyl, 4-(C₃₋₆-Cycloalkylsulphonyl)-piperazin-1-yl-carbonyl, C₁₋₃-Alkylsulphanyl, C₃₋₆-Cycloalkylsulphonyl, C₁₋₃-Alkylsulphinyl, C₃₋₆-Cycloalkylsulphinyl, C₁₋₃-Alkylsulphonyl, C₃₋₆-Cycloalkylsulphonyl,
Amino, C₁₋₆-Alkyl-amino, Di-(C₁₋₆-alkyl)-amino, C₃₋₆-Cycloalkyl-amino, N-(C₃₋₆-Cycloalkyl)-N-(C₁₋₆-alkyl)-amino, (C₁₋₄-Alkyl-carbonyl)-amino, (C₃₋₆-Cycloalkyl-carbonyl)-amino, (C₁₋₄-Alkyl-carbonyl)-(C₁₋₃-alkyl)-amino, (C₃₋₆-Cycloalkyl-carbonyl)-(C₁₋₃-alkyl)-amino,
Pyrrolidin-1-yl, Piperidin-1-yl, 3-Amino-piperidin-1-yl, 4-Amino-piperidin-1-yl, Morpholin-4-yl, Thiomorpholin-4-yl, 1-Oxo-1λ⁴-thiomorpholin-4-yl, 1,1-Dioxo-1λ⁶-thiomorpholin-4-yl, Piperazin-1-yl, Homopiperazin-1-yl, 4-(C₁₋₃-Alkyl)-piperazin-1-yl, 4-(C₁₋₃-Alkyl-carbonyl)-piperazin-1-yl, 4-(C₃₋₆-Cycloalkyl-carbonyl)-piperazin-1-yl, 4-(C₁₋₃-Alkylsulphonyl)-piperazin-1-yl, 4-(C₃₋₆-Cycloalkyl-sulphonyl)-piperazin-1-yl,
Tetrahydrofuran-3-yl-oxy, Tetrahydrofuran-3-yl-amino, Tetrahydropyran-4-yl-oxy, Tetrahydropyran-4-yl-amino, N-Tetrahydropyran-4-yl-N-methyl-amino, 2-Oxo-imidazolidin-1-yl, 2-Oxo-tetrahydropyrimidin-1-yl und Heteroaryl,
wobei die oben bei der Definition von R⁵ erwähnten C₁₋₆-Alkyl-, C₃₋₆-Cycloalkyl-, C₂₋₆-Alkinyl-, C₂₋₆-Alkenyl-, C₁₋₃-Alkylsulphanyl-, C₃₋₆-Cycloal kylsulphanyl-, C₁₋₃-Alkylsulphinyl-, C₃₋₆-Cycloalkylsulphinyl-, C₁₋₃-Alkylsulphonyl-, C₃₋₆-Cycloalkylsulphonyl-, C₁₋₆-Alkoxy-, C₃₋₆-Cycloalkoxy-, C₁₋₆-Alkyl-amino-, Di-(C₁₋₆-alkyl)-amino-, C₃₋₆-Cycloalkyl-amino-, N-(C₃₋₆-cycloalkyl)-N-(C₁₋₆-alkyl)-amino-, (C₁₋₄-Alkyl-carbonyl)-amino-, (C₃₋₆-Cycloalkyl-carbonyl)-amino-, (C₁₋₄-Alkyl-carbonyl)-(C₁₋₃-alkyl)-amino- und (C₃₋₆-Cycloalkyl-carbonyl)-(C₁₋₃-alkyl)-amino-Gruppen jeweils unabhängig voneinander im Kohlenstoffskelett mono- oder disubstituiert sein können mit einer Gruppe, ausgewählt aus
Cyano, Hydroxy, C₁₋₃-Alkoxy, Tetrahydro-pyran-2-yloxy,
Amino, C₁₋₆-Alkyl-amino, Di-(C₁₋₆-alkyl)-amino, C₃₋₆-Cycloalkyl-amino, N-(C₃₋₆-Cycloalkyl)-N-(C₁₋₆-alkyl)-amino, (C₁₋₄-Alkyl-carbonyl)-amino, (C₃₋₆-Cycloalkyl-carbonyl)-amino, (C₁₋₄-Alkyl-carbonyl)-(C₁₋₃-alkyl)-amino, (C₃₋₆-Cycloalkyl-carbonyl)-(C₁₋₃-alkyl)-amino, (C₁₋₄-Alkylsulphonyl)-amino, (C₃₋₆-Cycloalkylsulphonyl)-amino, (C₁₋₄-Alkylsulphonyl)-(C₁₋₃-alkyl)-amino, (C₃₋₆-Cycloalkylsulphonyl)-(C₁₋₃-alkyl)-amino,
Pyrrolidin-1-yl, Piperidin-1-yl, Morpholin-4-yl, Piperazin-1-yl, 4-(C₁₋₃-Alkyl)-piperazin-1-yl,
Carboxyl, Aminocarbonyl, C₁₋₆-Alkyl-aminocarbonyl, Di-(C₁₋₆-alkyl)-aminocarbonyl, C₁₋₂-Alkoxy-carbonyl, C₃₋₆-Cycloalkyl-aminocarbonyl, N-(C₃₋₆-Cycloalkyl)-N-(C₁₋₆-alkyl)-amino-carbonyl, Pyrrolidin-1-yl-carbonyl, Piperidin-1-yl-carbonyl, Morpholin-4-yl-carbonyl, Piperazin-1-yl-carbonyl, 4-(C₁₋₃-Alkyl)-piperazin-1-yl-carbonyl,
C₁₋₃-Alkylsulphanyl, C₃₋₆-Cycloalkylsulphanyl, C₁₋₃-Alkylsulphinyl, C₃₋₆-Cycloalkylsulphinyl, C₁₋₃-Alkylsulphonyl und C₃₋₆-Cycloalkylsulphonyl, wobei die Substituenten nicht an ein gemeinsames Kohlenstoffatom gebunden sein müssen, und
wobei die oben bei der Definition von R⁵ erwähnten Pyrrolidin-1-yl- und Piperidin-1-yl-Gruppen substituiert sein können mit Amino oder Hydroxy, und
wobei die hier vorher für R⁵ erwähnten C₁₋₆-Alkyl-aminocarbonyl-, Di-(C₁₋₆ -alkyl)-aminocarbonyl-, C₃₋₆-Cycloalkyl-aminocarbonyl-, N-(C₃₋₆-Cycloalkyl)-N-(C₁₋₆-alkyl)-aminocarbonyl-Gruppen jeweils unabhängig voneinander im Kohlenstoffskelett mono- oder disubstituiert sein können mit einer Gruppe, ausgewählt aus
Cyano, Hydroxy, C₁₋₃-Alkoxy,
Amino, C₁₋₃-Alkyl-amino, Di-(C₁₋₃-alkyl)-amino, C₃₋₆-Cycloalkylamino, N-(C₃₋₆-Cycloalkyl)-N-(C₁₋₆-alkyl)-amino, (C₁₋₄-Alkyl-carbonyl)-amino, (C₃₋₆-Cycloalkyl-carbonyl)-amino, (C₁₋₄-Alkyl-carbonyl)-(C₁₋₃-alkyl)-amino, (C₃₋₆-Cycloalkyl-carbonyl)-(C₁₋₃-alkyl)-amino, (C₁₋₄-Alkylsulphonyl)-amino, (C₃₋₆-Cycloalkylsulphonyl)-amino, (C₁₋₄-Alkylsulphonyl)-(C₁₋₃-alkyl)-amino, (C₃₋₆-Cycloalkylsulphonyl)-(C₁₋₃-alkyl)-amino,
Pyrrolidin-1-yl, Piperidin-1-yl, Morpholin-4-yl, Piperazin-1-yl, 4-(C₁₋₃-Alkyl)-piperazin-1-yl,
C₁₋₃-Alkylsulphanyl, C₃₋₆-Cycloalkylsulphanyl, C₁₋₃-Alkylsulphinyl, C₃₋₆-Cycloalkylsulphinyl, C₁₋₃-Alkylsulphonyl und C₃₋₆-Cycloalkylsulphonyl, wobei die Substituenten nicht an ein gemeinsames Kohlenstoffatom gebunden sein müssen, und
wobei die hier zuvor für R⁵ erwähnte Heteroarylgruppe ein monocyclisches fünfgliedriges aromatisches System mit 1 bis 4 Heteroatomen, ausgewählt aus N, O oder S, bezeichnet, worin nicht mehr als ein Sauerstoffatom und/oder ein Schwefelatom vorhanden sein kann, oder ein sechsgliedriges aromatisches System mit 1 bis 3 Stickstoffatomen, und die unabhängig voneinander mono- oder disubstituiert sein können mit Halogen, Cyano, Hydroxy, Amino, C₁₋₃-Alkyl oder C₁₋₃-Alkyloxycarbonyl, und
wobei in den oben erwähnten Morpholin-4-yl- und Piperazin-1-yl-Gruppen in jedem Fall eine Methyleneinheit durch eine Carbonyl- oder Sulphonyl-Gruppe ersetzt sein kann,
und die Tautomeren, Stereoisomeren, Mischungen davon und Salze davon.

2. Verbindungen der allgemeinen Formel nach Anspruch 1, worin R eine Gruppe der in Anspruch 1 erwähnten Formel bezeichnet, worin
R¹ H, C₁₋₆-Alkyl oder eine Gruppe der Formel 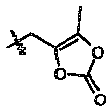 bezeichnet, worin die hier zuvor für R¹ erwähnte C₁₋₆-Alkylgruppe substituiert sein kann mit C₁₋₆-Alkyl-carbonyloxy, C₁₋₆-Alkoxy-carbonyloxy, C₁₋₆-Alkoxy, Hydroxy, Amino, C₁₋₃-Alkyl-amino, Di-(C₁₋₃-alkyl)-amino, Pyrrolidin-1-yl, Piperidin-1-yl, Morpholin-4-yl, Piperazin-1-yl, 4-(C₁₋₃-Alkyl)-piperazin-1-yl, Aminocarbonyl, C₁₋₃-Alkyl-aminocarbonyl, Di-(C₁₋₃-alkyl)-aminocarbonyl, Pyrrolidin-1-yl-carbonyl, Piperidin-1-yl-carbonyl, Morpholin-4-yl-carbonyl, Piperazin-1-yl-carbonyl oder 4-(C₁₋₃-Alkyl)-piperazin-1-yl-carbonyl,
R² und R³ bezeichnen unabhängig voneinander Halogen, C₁₋₃-Alkyl, C₁₋₃-Perfluoroalkyl, C₁₋₂-Alkoxy oder Cyano, und
A bezeichnet CH oder N,
m bezeichnet 0,1 oder 2,
R⁴ bezeichnet Halogen, C₁₋₃-Alkyl, Trifluormethyl oder Cyano, während, wenn m die Zahl 2 bezeichnet, die Gruppen R⁴ gleich oder verschieden sein können,
und die heterocyclische Gruppe 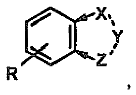 die substituiert sein kann mit R⁴ wie hier zuvor beschrieben, bezeichnet eine Gruppe der Formel 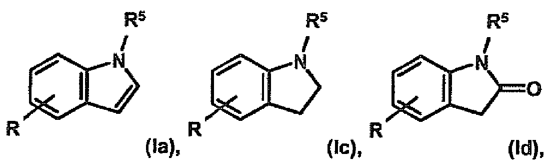 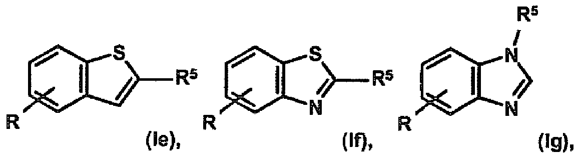 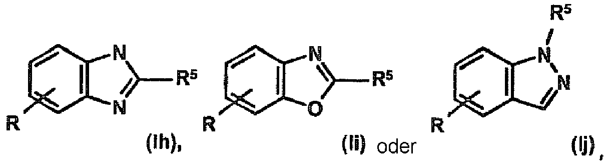
worin die oben erwähnten Heterocyclen der Formeln (la), (Ic), (Id), (Ie), (Ig) und (Ij) gegebenenfalls an den Kohlenstoffatomen des Fünfrings substituiert sein können mit ein oder zwei Gruppen, ausgewählt aus Halogen, C₁₋₃-Alkyl, Cyano, C₁₋₃-Perfluoroalkyl, C₃₋₆-Cycloalkyl, C₁₋₃-Alkyl-carbonyl, C₁₋₃-Perfluoroalkyl-carbonyl, Aminocarbonyl, C₁₋₃-Alkyl-aminocarbonyl oder Di-(C₁₋₃-alkyl)-aminocarbonyl, worin die Gruppen gleich oder verschieden sein können und jedes Kohlenstoffatom trägt höchstens eine Gruppe, und
R⁵ bezeichnet 1,3-Dimethyl-1H-pyrimidin-2,4-dion-5-yl, 1H-Pyrimidin-2,4-dion-6-yl, 1H-Pyrimidin-2,4-dion-5-yl, 2H-Pyridazin-3-on-6-yl, 1H-Pyridin-2-on-3-yl, 1H-Pyridin-2-on-5-yl, 1H-Pyridin-2-on-4-yl oder
ein mono- oder bicyclisches, 5- bis 14-gliedriges Ringsystem, das 0 bis 4 Heteroatome enthalten kann, ausgewählt aus N, O oder S, worin nicht mehr als ein Sauerstoffatom und/oder ein Schwefelatom vorhanden sein kann, das aromatisch, gesättigt oder teilweise ungesättigt ist und das unabhängig voneinander mono-, di- oder trisubstituiert sein kann mit einer Gruppe, ausgewählt aus,
Halogen, Cyano,
C₁₋₆-Alkyl, C₃₋₆-Cycloalkyl, C₁₋₃-Perfluoroalkyl, C₂₋₆-Alkinyl, C₂₋₆-Alkenyl,
Hydroxy, C₁₋₆-Alkoxy, C₃₋₆-Cycloalkoxy, Trifluormethoxy,
Carboxyl, C₁₋₃-Alkyl-carbonyl , C₁₋₄-Alkoxy-carbonyl, Cyclopropoxy-carbonyl, Aminocarbonyl, C₁₋₆-Alkyl-aminocarbonyl, Di-(C₁₋₆-alkyl)-aminocarbonyl, N-(C₁₋₃-Alkyl)-N-(C₁₋₃-alkoxy)-aminocarbonyl, C₃₋₆-Cycloalkyl-aminocarbonyl, N-(C₃₋₆-Cycloalkyl)-N-(C₁₋₃-Alkyl)-aminocarbonyl, Pyrrolidin-1-yl-carbonyl, Piperidin-1-yl-carbonyl, Morpholin-4-yl-carbonyl, Piperazin-1-yl-carbonyl, 4-(C₁₋₃-Alkyl)-piperazin-1-yl-carbonyl, 4-(C₁₋₃-Alkyl-carbonyl)-piperazin-1-yl-carbonyl, 4-(C₃₋₆-Cycloalkyl-carbonyl)-piperazin-1-yl-carbonyl, 4-(C₁₋₃-Alkylsulphonyl)-piperazin-1-yl-carbonyl, 4-(C₃₋₆-Cycloalkylsulphonyl)-piperazin-1-yl-carbonyl,
C₁₋₃-Alkylsulphanyl, C₃₋₆-Cycloalkylsulphanyl, C₁₋₃-Alkylsulphinyl, C₃₋₆-Cycloalkylsulphinyl, C₁₋₃-Alkylsulphonyl, C₃₋₆-Cycloalkylsulphonyl, Amino, C₁₋₆-Alkyl-amino, Di-(C₁₋₆-alkyl)-amino, C₃₋₆-Cycloalkyl-amino, N-(C₃₋₆-Cycloalkyl)-N-(C₁₋₃-alkyl)-amino, (C₁₋₄-Alkyl-carbonyl)-amino, (C₃₋₆-Cycloalkyl-carbonyl)-amino, (C₁₋₄-Alkyl-carbonyl)-(C₁₋₃-alkyl)-amino, (C₃₋₆-Cycloalkyl-carbonyl)-(C₁₋₃-alkyl)-amino, Pyrrolidin-1-yl, Piperidin-1-yl, 3-Amino-piperidin-1-yl, 4-Amino-piperidin-1-yl, Morpholin-4-yl, Thiomorpholin-4-yl, 1-Oxo-1λ⁴-thiomorpholin-4-yl, 1,1-Dioxo-1λ⁶-thiomorpholin-4-yl, Piperazin-1-yl, Homopiperazin-1-yl, 4-(C₁₋₃-Alkyl)-piperazin-1-yl, 4-(C₁₋₃-Alkyl-carbonyl)-piperazin-1-yl, 4-(C₃₋₆-Cycloalkyl-carbonyl)-piperazin-1-yl, 4-(C₁₋₃-Alkylsulphonyl)-piperazin-1-yl, 4-(C₃₋₆-Cycloalkylsulphonyl)-piperazin-1-yl,
Tetrahydrofuran-3-yl-oxy, Tetrahydrofuran-3-yl-amino, Tetrahydropyran-4-yl-oxy, Tetrahydropyran-4-yl-amino, N-Tetrahydropyran-4-yl-N-methyl-amino, 2-Oxo-imidazolidinyl, 2-Oxo-tetrahydropyrimidinyl und Heteroaryl,
worin die oben bei der Definition von R⁵ erwähnten C₁₋₆-Alkyl-, C₃₋₆-Cycloalkyl-, C₂₋₆-Alkinyl-, C₂₋₆-Alkenyl-, C₁₋₃-Alkylsulphanyl-, C₃₋₆-Cycloalkylsulphanyl-, C₁₋₃-Alkylsulphinyl-, C₃₋₆-Cycloalkylsulphinyl-, C₁₋₃-Alkylsulphonyl-, C₃₋₆-Cycloalkylsulphonyl-, C₁₋₆-Alkoxy-, C₃₋₆-Cycloalkoxy-, C₁₋₆-Alkyl-amino-, Di-(C₁₋₆-alkyl)-amino-, C₃₋₆-Cycloalkyl-amino-, N-(C₃₋₆-Cycloalkyl)-N-(C₁₋₃-alkyl)-amino-, (C₁₋₄-Alkyl-carbonyl)-amino-, (C₃₋₆-Cycloalkylcarbonyl)-amino-, (C₁₋₄-Alkyl-carbonyl)-(C₁₋₃-alkyl)-amino- und (C₃₋₆-Cycloalkyl-carbonyl)-(C₁₋₃-alkyl)-amino-Gruppen im Kohlenstoffskelett unabhängig voneinander mono- oder disubstituiert sein können mit einer Gruppe, ausgewählt aus
Cyano, Hydroxy, C₁₋₃-Alkoxy, Tetrahydro-pyran-2-yloxy,
Amino, C₁₋₆-Alkyl-amino, Di-(C₁₋₆-alkyl)-amino, C₃₋₆-Cycloalkyl-amino, N-(C₃₋₆-Cycloalkyl)-N-(C₁₋₃-alkyl)-amino, (C₁₋₄-Alkyl-carbonyl)-amino, (C₃₋₆-Cycloalkyl-carbonyl)-amino, (C₁₋₄-Alkyl-carbonyl)-(C₁₋₃-alkyl)-amino, (C₃₋₆-Cycloalkyl-carbonyl)-(C₁₋₃-alkyl)-amino, (C₁₋₄-Alkylsulphonyl)-amino, (C₃₋₆-Cycloalkylsulphonyl)-amino, (C₁₋₄-Alkylsulphonyl)-(C₁₋₃-alkyl)-amino, (C₃₋₆-Cycloalkylsulphonyl)-(C₁₋₃-alkyl)-amino, Pyrrolidin-1-yl, Piperidin-1-yl, Morpholin-4-yl, Piperazin-1-yl, 4-(C₁₋₃-Alkyl)-piperazin-1-yl,
Carboxyl, C₁₋₂-Alkoxy-carbonyl, Aminocarbonyl, C₁₋₆-Alkyl-aminocarbonyl, Di-(C₁₋₆-alkyl)-aminocarbonyl, C₃₋₆-Cycloalkyl-aminocarbonyl, N-(C₃₋₆-Cycloalkyl)-N-(C₁₋₃-alkyl)-amino-carbonyl, Pyrrolidin-1-yl-carbonyl, Piperidin-1-yl-carbonyl, Morpholin-4-yl-carbonyl, Piperazin-1-yl-carbonyl, 4-(C₁₋₃-Alkyl)-piperazin-1-yl-carbonyl,
C₁₋₃-Alkylsulphanyl, C₃₋₆-Cycloalkylsulphanyl, C₁₋₃-Alkylsulphinyl, C₃₋₆-Cycloalkylsulphinyl, C₁₋₃-Alkylsulphonyl und C₃₋₆-Cycloalkylsulphonyl, wobei die Substituenten nicht an ein gemeinsames Kohlenstoffatom gebunden sein müssen, und
wobei die oben bei der Definition von R⁵ erwähnten Pyrrolidin-1-yl- und Piperidin-1-yl-Gruppen substituiert sein können mit Amino oder Hydroxy, und
wobei die oben bei der Definition von R⁵ erwähnten C₁₋₆-alkyl-aminocarbonyl-, Di-(C₁₋₆-alkyl)-aminocarbonyl-, C₃₋₆-Cycloalkyl-aminocarbonyl-, N-(C₃₋₆-Cycloalkyl)-N-(C₁₋₃-alkyl)-aminocarbonyl-Gruppen im Kohlenstoffskelett jeweils unabhängig voneinander mono- oder disubstituiert sein können mit einer Gruppe, ausgewählt aus
Amino, Hydroxy, C₁₋₃-Alkoxy, C₁₋₃-Alkyl-amino, Di-(C₁₋₃-alkyl)-amino, C₃₋₆-Cycloalkyl-amino, N-(C₃₋₆-Cycloalkyl)-N-(methyl)-amino, (C₁₋₄-Alkyl-carbonyl)-amino, (C₃₋₆-Cycloalkyl-carbonyl)-amino, (C₁₋₄-Alkyl-carbonyl)-(methyl)-amino, (C₃₋₆-Cycloalkyl-carbonyl)-(methyl)-amino, (C₁₋₄-Alkylsulphonyl)-amino, (C₃₋₆-Cycloalkylsulphonyl)-amino, (C₁₋₄-Alkylsulphonyl)-(methyl)-amino oder (C₃-₆-Cycloalkylsulphonyl)-(methyl)-amino,
Pyrrolidin-1-yl, Piperidin-1-yl, Morpholin-4-yl, Piperazin-1-yl, 4-(C₁₋₃-Alkyl)-piperazin-1-yl,
C₁₋₃-Alkylsulphanyl, C₃₋₆-Cycloalkylsulphanyl, C₁₋₃-Alkylsulphinyl, C₃₋₆-Cycloalkylsulphinyl, C₁₋₃-Alkylsulphonyl und C₃₋₆-Cycloalkylsulphonyl, wobei die Substituenten nicht an ein gemeinsames Kohlenstoffatom gebunden sein müssen, und
wobei die oben bei der Definition von R⁵ erwähnte Heteroarylgruppe ein monocyclisches fünfgliedriges, aromatisches System mit 1 bis 3 Heteroatomen, ausgewählt aus N, O oder S, bezeichnet, wobei nicht mehr als ein Sauerstoff und/oder ein Schwefelatom vorhanden sein kann, oder ein monocyclisches fünfgliedriges aromatisches System mit 4 Stickstoffatomen oder ein sechsgliedriges aromatisches System mit 1 bis 3 Stickstoffatomen, und die unabhängig voneinander mono- oder disubstituiert sein können mit Fluor, Chlor, Cyano, C₁₋₃-Alkyl oder C₁₋₃-Alkyloxycarbonyl, und
wobei in den oben erwähnten Morpholin-4-yl- und Piperazin-1-yl-Gruppen in jedem Fall eine Methyleneinheit ersetzt sein kann durch eine Carbonyl- oder Sulphonyl-Gruppe,
und die Tautomeren, Stereoisomeren, Mischungen davon und Salze davon.

3. Verbindungen der allgemeinen Formel I nach Anspruch 2, worin R eine Gruppe der in Anspruch 1 erwähnten Formel bezeichnet, worin
R¹ H, C₁₋₄-Alkyl oder eine Gruppe der Formel 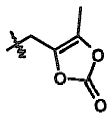 bezeichnet, worin die hier zuvor für R¹ erwähnte C₁₋₄-Alkylgruppe substituiert sein kann mit C₁₋₄-Alkoxy, Hydroxy, Di-(C₁₋₃-alkyl)-amino, Pyrrolidin-1-yl, Piperidin-1-yl, Morpholin-4-yl, Piperazin-1-yl oder 4-(Methyl)-piperazin-1-yl,
R² und R³ bezeichnen unabhängig voneinander Chlor, Brom oder C₁₋₂-Alkyl und
A bezeichnet CH oder N,
m bezeichnet 0 oder 1,
R⁴ bezeichnet Fluor, Chlor, Methyl oder Ethyl,
und die heterocyclische Gruppe 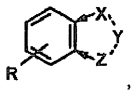 die substituiert sein kann mit R⁴ wie hier zuvor beschrieben, bezeichnet eine Gruppe der Formel 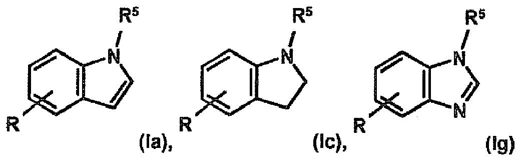 oder 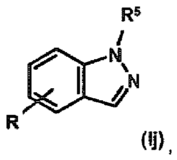 wobei die hier zuvor erwähnten Heterocyclen gegebenenfalls an den Kohlenstoffatomen des Fünfrings substituiert sein können mit ein oder zwei Gruppen, ausgewählt aus Chlor, Brom, lod, C₁₋₃-Alkyl, Cyano und Trifluormethyl, wobei die Gruppen gleich oder verschieden sein können und jedes Kohlenstoffatom höchstens eine Gruppe trägt, und
worin R⁵ 1,3-Dimethyl-1H-pyrimidin-2,4-dion-5-yl, 1H-Pyrimidin-2,4-dion-6-yl, 1H-Pyrimidin-2,4-dion-5-yl, 2H-Pyridazin-3-on-6-yl, 1H-Pyridin-2-on-3-yl, 1H-Pyridin-2-on-5-yl oder 1H-Pyridin-2-on-4-yl,
Phenyl, Pyridazin-3-yl, Pyrimidin-2-yl, Pyrimidin-4-yl, Pyrimidin-5-yl, Pyrazin-2-yl, 1,3,5-Triazin-2-yl, Pyridin-2-yl, Pyridin-4-yl, Imidazol-2-yl, Imidazol-4-yl, Pyrazol-3-yl, Pyrazol-4-yl, Thiazol-2-yl, [1,3,4]Thiadiazol-2-yl, Thiophen-2-yl, Thiophen-3-yl, Naphthalin-1-yl, Naphthalin-2-yl, Purin-6-yl, Purin-2-yl, 1-lmidazo[1,2-a]pyrazin-6-yl, Chinolin-6-yl, Chinolin-8-yl, Chinolin-2-yl oder Isochinolin-1-yl bezeichnet, die jeweils unabhängig voneinander mono- oder disubstituiert sein können mit einer Gruppe, ausgewählt aus
Fluor, Chlor, C₁₋₄-Alkyl, Cyclopropyl, Trifluormethyl, Cyano, Hydroxy, C₁₋₃-Alkoxy, Cyclopropoxy,
Carboxyl, C₁₋₂-Alkyl-carbonyl, C₁₋₂-Alkoxy-carbonyl, Aminocarbonyl, C₁₋₄-Alkyl-aminocarbonyl, Di-(C₁₋₂-alkyl)-aminocarbonyl, N-Methoxy-N-methyl-aminocarbonyl, Cyclopropyl-aminocarbonyl, N-(Cyclopropyl)-N-(methyl)-aminocarbonyl, Pyrrolidin-1-yl-carbonyl, Piperidin-1-yl-carbonyl, Morpholin-4-yl-carbonyl, Piperazin-1-yl-carbonyl, 4-(Methyl)-piperazin-1-yl-carbonyl, 4-(C₁₋₃-Alkyl-carbonyl)-piperazin-1-yl-carbonyl, 4-(C₃₋₆-Cycloalkyl-carbonyl)-piperazin-1-yl-carbonyl, 4-(C₁₋₃-Alkylsulphonyl)-piperazin-1-yl-carbonyl, 4-(C₃₋₆-Cycloalkylsulphonyl)-piperazin-1-yl-carbonyl,
C₁₋₂-Alkylsulphanyl, Cyclopropylsulphanyl, C₁₋₂-Alkylsulphinyl, Cyclopropylsulphinyl, C₁₋₂-Alkylsulphonyl, Cyclopropylsulphonyl,
Amino, C₁₋₄-Alkyl-amino, Di-(C₁₋₃-alkyl)-amino, Cyclopropyl-amino, N-(Cyclopropyl)-N-(methyl)-amino, C₁₋₃-Alkyl-carbonyl-amino,
Pyrrolidin-1-yl, Piperidin-1-yl, 3-Amino-piperidin-1-yl, 4-Amino-piperidin-1-yl, Morpholin-4-yl, Thiomorpholin-4-yl, 1-Oxo-1λ⁴-thiomorpholin-4-yl, 1,1-Dioxo-1λ⁶-thiomorpholin-4-yl, Piperazin-1-yl, Homopiperazin-1-yl, 4-(Methyl)-piperazin-1-yl, 4-(C₁₋₂-Alkyl-carbonyl)-piperazin-1-yl, 4-(C₁₋₂-Alkylsulphonyl)-piperazin-1-yl,
Tetrahydrofuran-3-yl-oxy, Tetrahydrofuran-3-yl-amino, Tetrahydropyran-4-yl-oxy, Tetrahydropyran-4-yl-amino, N-Tetrahydropyran-4-yl-N-methyl-amino, 2-Oxo-imidazolidinyl, 2-Oxotetrahydropyrimidinyl, Imidazol-2-yl, 1-Methyl-imidazol-2-yl, Thiazol-2-yl, 4-Ethoxycarbonyl-thiazol-2-yl, 3-Ethoxycarbonyl-isoxazol-5-yl, Oxazol-2-yl, 2,4-Dihydroxy-pyrimidin-5-yl, 1,2,4-Triazol-3-yl und Tetrazol-5-yl, oder
wobei die hier zuvor für R⁵ erwähnten C₁₋₄-Alkyl-, C₁₋₃-Alkoxy-, C₁₋₄-Alkylamino-, Di-(C₁₋₃-alkyl)-amino- und C₁₋₃-Alkyl-carbonyl-amino-Gruppen jeweils unabhängig voneinander im Kohlenstoffskelett mono- oder disubstituiert sein können mit einer Gruppe, ausgewählt aus
Cyano, Hydroxy, C₁₋₂-Alkoxy, Tetrahydro-pyran-2-yloxy,
Amino, C₁₋₃-Alkyl-amino, Di-(C₁₋₃-alkyl)-amino, (C₁₋₃-Alkyl-carbonyl)-amino, (C₁₋₃-Alkylsulphonyl)-amino,
Pyrrolidin-1-yl, Piperidin-1-yl, Morpholin-4-yl, Piperazin-1-yl, 4-(Methyl)-piperazin-1-yl,
Carboxyl, C₁₋₂-Alkoxy-carbonyl, Aminocarbonyl, C₁₋₃-Alkyl-aminocarbonyl, Di-(C₁₋₃-alkyl)-aminocarbonyl und C₃₋₆-Cycloalkyl-aminocarbonyl, wobei die Substituenten nicht an ein gemeinsames Kohlenstoffatom gebunden sein müssen, und
wobei die hier zuvor für R⁵ erwähnten C₁₋₄-Alkyl-aminocarbonyl- und Di-(C₁₋₂-alkyl)-aminocarbonyl-Gruppen jeweils unabhängig voneinander im Kohlenstoffskelett mono- oder disubstituiert sein können mit Amino, Hydroxy, C₁₋₃-Alkoxy, C₁₋₃-Alkyl-amino oder Di-(C₁₋₃-alkyl)-amino, wobei die Substituenten nicht an ein gemeinsames Kohlenstoffatom gebunden sein müssen, und
wobei in den oben genannten Morpholin-4-yl- und Piperazin-1-yl-Gruppen in jedem Fall eine Methyleneinheit ersetzt sein kann durch eine Carbonylgruppe,
und die Tautomeren, Stereoisomeren, Mischungen davon und Salze davon.

4. Verbindungen der allgemeinen Formel I nach Anspruch 3, worin R eine Gruppe der in Anspruch 1 genannten Formel bezeichnet, worin
R¹ H oder eine C₁₋₃-Alkylgruppe bezeichnet, gegebenenfalls substituiert mit einer Di-(C₁₋₃ -alkyl)-amino-Gruppe,
R² und R³ unabhängig voneinander Chlor, Brom oder Methyl bezeichnen, und
A bezeichnet CH oder N,
m bezeichnet 0 oder 1,
R⁴ bezeichnet Chlor, Methyl oder Ethyl, und
die heterocyclische Gruppe 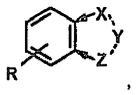 die substituiert sein kann mit R⁴wie hier zuvor beschrieben, bezeichnet eine Gruppe der Formel 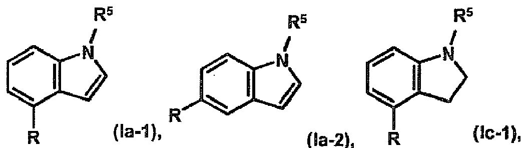 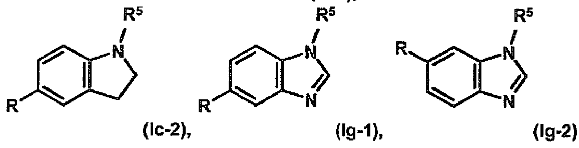 oder 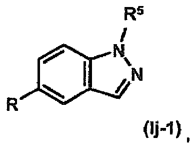 wobei die hier zuvor erwähnten Heterocyclen gegebenenfalls an den Kohlenstoffatomen des Fünfrings substituiert sein können mit ein oder zwei Gruppen, ausgewählt aus Chlor, Brom, lod, C₁₋₂-Alkyl, Cyano und Trifluormethyl, wobei die Gruppen gleich oder verschieden sein können und jedes Kohlenstoffatom höchstens eine Gruppe trägt, und
R⁵ wie in Anspruch 3 definiert ist,
und die Tautomeren, Stereoisomeren, Mischungen davon und Salze davon.

5. Verbindungen der allgemeinen Formel I nach Anspruch 4, worin R eine Gruppe der in Anspruch 1 genannten Formel bezeichnet, wobei
R¹ H, Methyl, Ethyl oder 2-Dimethylamino-ethyl bezeichnet,
R² und R³ unabhängig voneinander Chlor, Brom oder Methyl bezeichnen,
und
A bezeichnet CH oder N,
m bezeichnet 0 oder 1,
R⁴ bezeichnet Chlor, Methyl oder Ethyl, und
die heterocyclische Gruppe 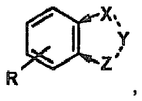 die substituiert sein kann mit R⁴ wie hier zuvor beschrieben, bezeichnet eine Gruppe der Formel 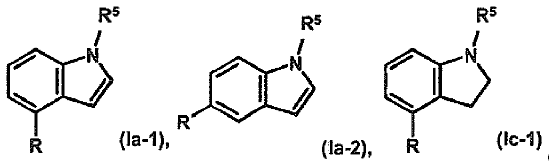 oder 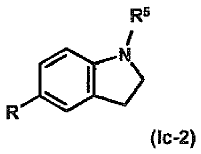
wobei die hier zuvor beschriebenen Heterocyclen gegebenenfalls an den Kohlenstoffatomen des Fünfrings substituiert sein können mit ein oder zwei Methyl- oder Ethylgruppen, wobei die Gruppen gleich oder verschieden sein können und jedes Kohlenstoffatom höchstens eine Gruppe trägt, und wobei
R⁵ 1,3-Dimethyl-1H-pyrimidin-2,4-dion-5-yl , 1H-Pyrimidin-2,4-dion-6-yl, 1H-Pyrimidin-2,4-dion-5-yl, 2H-Pyridazin-3-on-6-yl, 1H-Pyridin-2-on-3-yl, 1H-Pyridin-2-on-5-yl, 1H-Pyridin-2-on-4-yl oder
Phenyl, Pyridazin-3-yl, Pyrimidin-2-yl, Pyrimidin-4-yl, Pyrimidin-5-yl, Pyrazin-2-yl, Pyridin-2-yl, Pyridin-4-yl, Imidazol-2-yl, Imidazol-4-yl, Pyrazol-3-yl, Pyrazol-4-yl, 1,3,5-Triazin-2-yl, Thiazol-2-yl, [1,3,4]Thiadiazol-2-yl, Thiophen-2-yl, Thiophen-3-yl, Purin-6-yl , Purin-2-yl oder 1-Imidazo[1,2-a]pyrazin-6-yl bezeichnet, die unabhängig voneinander mono- oder disubstituiert sein können mit einer Gruppe, ausgewählt aus
Chlor, Cyano, Methyl, Aminomethyl, Morpholin-4-ylmethyl, Hydroxymethyl, 3-Hydroxypropyl, Trifluormethyl,
Hydroxy, Methoxy, 2-Hydroxyethoxy, 2-Aminoethoxy, 2-Dimethylaminoethoxy, 2-Methylsulphonylamino-ethoxy, 2-Acetylamino-ethoxy, 2,3-Dihydroxy-propoxy,
Carboxyl, Acetyl, Ethylcarbonyl, Aminocarbonyl, Methyl-aminocarbonyl, Dimethyl-aminocarbonyl, N-Methoxy-N-methyl-aminocarbonyl, 2-Dimethylamino-ethyl-aminocarbonyl, 2-Hydroxy-ethyl-aminocarbonyl, 2-Methoxy-ethyl-aminocarbonyl, Cyclo-aminocarbonyl,
Morpholin-4-yl-carbonyl, Piperazin-1-yl-carbonyl, Methoxy-carbonyl,
Methylsulphanyl, Methylsulphinyl, Ethylsulphinyl, Methylsulphonyl,
Amino, Methyl-amino, Acetylamino, 2-Aminoethyl-amino, 2-Dimethylaminoethyl-amino, 2-Hydroxyethyl-amino, 2-(Methylamino)-ethyl-amino, N-Carboxymethyl-N-[2-(dimethylamino)-ethyl]-amino, 2-(Acetylamino)ethylamino, 2-(Methylsulphonylamino)-ethyl-amino, 2-(Pyrrolidin-1-yl)-ethylamino, 2-(Piperidin-1-yl)-ethyl-amino, 2-(Tetrahydro-pyran-2-yloxy)-ethylamino, 3-Aminopropyl-amino, 3-(Methylamino)-propyl-amino, 2-Amino-2-methyl-propyl-amino, 1,3-Dihydroxy-2-propyl-amino, 3-(Acetylamino)-propyl-amino, 3-(Methylsulphonylamino)-propyl-amino, Dimethyl-amino, N-Methyl-N-2-aminoethyl-amino, N,N-bis-2-(Hydroxyethyl)-amino, N-Methyl-N-3-aminopropyl-amino, N-Methyl-N-[3-(acetylamino)-propyl]-amino, N-Methyl-N-[3-(methylsulphonylamino)-propyl]-amino, Cyclopropyl-amino,
Morpholin-4-yl, Thiomorpholin-4-yl, 1-Oxo-1λ⁴-thlomorpholin-4-yl, 1,1-Dioxo-1λ⁶-thiomorpholin-4-yl, 3-Amino-piperidin-1-yl, Piperazin-1-yl, Homopiperazin-1-yl, 4-Acetyl-piperazin-1-yl, 4-Methylsulphonyl-piperazin-1-yl,
Tetrahydrofuran-3-yl-oxy, Tetrahydrofuran-3-yl-amino, Tetrahydropyran-4-yl-oxy, Tetrahydropyran-4-yl-amino, N-Tetrahydropyran-4-yl-N-methyl-amino, 2-Oxo-imidazolidinyl, Imidazol-2-yl, 1-Methyl-imidazol-2-yl, Thiazol-2-yl, 4-Ethoxycarbonyl-thiazol-2-yl, 3-Ethoxycarbonyl-isoxazol-5-yl, und Oxazol-2-yl,
wobei in den oben erwähnten Morpholin-4-yl- und Piperazin-1-yl-Gruppen in jedem Fall eine Methyleneinheit durch eine Carbonylgruppe ersetzt sein kann, und die Tautomeren, Stereoisomeren, Mischungen davon und Salze davon.

6. Verbindungen der allgemeinen Formel I nach Anspruch 5, wobei
R eine Gruppe der in Anspruch 1 genannten Formel bezeichnet, wobei
R¹ Wasserstoff bezeichnet,
R² und R³ in jedem Falle Chlor darstellen und
A CH bezeichnet,
m 0 bezeichnet und
die heterocyclische Gruppe 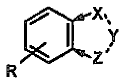 bezeichnet eine Gruppe der Formel 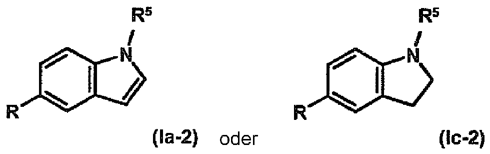 wobei die hier zuvor erwähnten Heterocyclen gegebenenfalls an den Kohlenstoffatomen des Fünfrings mit ein oder zwei Methylgruppen substituiert sein können, jedes Kohlenstoffatom trägt höchstens eine Gruppe, und wobei
R⁵ Phenyl, Pyridazin-3-yl, Pyrimidin-2-yl, Pyrimidin-4-yl, Pyrimidin-5-yl, Pyrazin-2-yl, Pyridin-2-yl oder Pyridin-4-yl bezeichnet, das mit einer Gruppe substituiert sein kann, ausgewählt aus
Cyano, Methyl, Aminomethyl, Hydroxymethyl, 3-Hydroxypropyl, Trifluormethyl,
Hydroxy, Methoxy, 2-Hydroxyethoxy, 2-Aminoethoxy, 2-(Dimethyl-amino)-ethoxy, 2-(Methylsulphonyl)-amino-ethoxy, 2-(Acetylamino)-ethoxy, 2,3-Dihydroxy-propoxy,
Carboxyl, Acetyl, Ethylcarbonyl, Aminocarbonyl, Methyl-aminocarbonyl, Dimethyl-aminocarbonyl, N-Methoxy-N-methyl-aminocarbonyl, 2-(Dimethylamino)-ethyl-aminocarbonyl, 2-Hydroxy-ethyl-aminocarbonyl, 2-Methoxy-ethyl-aminocarbonyl, Cyclopropyl-aminocarbonyl,
Morpholin-4-yl-carbonyl, Piperazin-1-yl-carbonyl, Methoxy-carbonyl,
Methylsulphanyl, Methylsulphinyl, Ethylsulphinyl, Methylsulphonyl,
Amino, Methyl-amino, Acetylamino, 2-Aminoethyl-amino, 2-(Dimethylamino)-ethyl-amino, 2-Hydroxyethyl-amino, 2-(Methylamino)-ethyl-amino, N-Carboxymethyl-N-[2-(dimethylamino)-ethyl]-amino, 2-(Acetylamino)-ethylamino, 2-(Methylsulphonylamino)-ethyl-amino, 2-(Pyrrolidin-1-yl)-ethylamino, 2-(Piperidin-1-yl)-ethyl-amino, 3-Aminopropyl-amino, 3-(Methylamino)-propyl-amino, 3-(Acetylamino)-propyl-amino, 3-(Methylsulphonylamino)-propyl-amino, Dimethyl-amino, N-Methyl-N-(2-aminoethyl)-amino, N,N-bis-(2-Hydroxyethyl)-amino, N-Methyl-N-(3-aminopropyl)-amino, N-Methyl-N-[3-(acetylamino)-propyl]-amino, N-Methyl-N-[3-(methylsulphonylamino)-propyl]-amino, Cyclopropyl-amino,
Morpholin-4-yl, Thiomorpholin-4-yl, 1-Oxo-1λ⁴-thiomorpholin-4-yl, 1,1-Dioxo-1λ⁶-thiomorpholin-4-yl, 3-Amino-piperidin-1-yl, Piperazin-1-yl, Homopiperazin-1-yl, 4-Acetyl-piperazin-1-yl, 4-Methylsulphonyl-piperazin-1-yl,
Tetrahydrofuran-3-yl-oxy, Tetrahydrofuran-3-yl-amino, Tetrahydropyran-4-yl-oxy, Tetrahydropyran-4-yl-amino, N-Tetrahydropyran-4-yl-N-methyl-amino, 2-Oxo-imidazolidinyl, Imidazol-2-yl, 1-Methyl-imidazol-2-yl, Thiazol-2-yl, 4-Ethoxycarbonyl-thiazol-2-yl, 3-Ethoxycarbonyl-isoxazol-5-yl und Oxazol-2-yl,
und die Tautomeren, Stereoisomeren, Mischungen davon und Salze davon.

7. Die folgenden Verbindungen der allgemeinen Formel I nach Anspruch 1:
(1) ((3,5-Dichlor-phenylsulphonyl)-(1-[5-(2-dimethylamino-ethylamino)-pyrazin-2-yl]-1H-indol-5-yl)-amino)-essigsäure
(2) {(3,5-Dichlor-phenylsulphonyl)-[1-(6-piperazin-1-yl-pyridazin-3-yl)-2,3-dihydro-1H-indol-5-yl]-amino)-essigsäure
(3) ((3,5-Dichlor-phenylsulphonyl)-(1-[6-(2-dimethylamino-ethylamino)-pyridazin-3-yl]-1H-indol-5-yl)-amino)-essigsäure
(4) {(3,5-Dichlor-phenylsulphonyl)-[1-(6-methyl-pyridazin-3-yl)-1H-indol-5-yl]-aminoessigsäure
(5) ((3-Brom-5-methyl-phenylsulphonyl)-{1-[6-(2-dimethylamino-ethylamino)-pyridazin-3-yl]-1H-indol-5-yl)-amino)-essigsäure
(6) {(3,5-Dichlor-phenylsulphonyl)-[1-(6-piperazin-1-yl-pyridazin-3-yl)-1H-indol-5-yl]-amino}-essigsäure
(7) [[1-(6-[1,4]Diazepan-1-yl-pyridazin-3-yl)-1H-indol-5-yl]-(3,5-dichlorphenylsulphonyl)-amino]-essigsäure
(8) {(3,5-Dichlor-phenylsulphonyl)-[1-(4-methyl-pyridin-2-yl)-1H-indol-5-yl]-aminoj-essigsäure
(9) ((3,5-Dichlor-phenylsulphonyl)-{1-[6-(2-dimethylamino-ethylamino)-pyridazin-3-yl]-3-methyl-1H-indol-5-yl)-amino)-essigsäure
(10) [(3,5-Dichlor-phenylsulphonyl)-(1-{6-[methyl-(tetrahydro-pyran-4-yl)-amino]-pyridazin-3-yl}-1H-indol-5-yl)-amino]-essigsäure
(11) ((3,5-Dichlor-phenylsulphonyl)-(1-[5-(3-oxo-piperazin-1-yl)-pyrazin-2-yl]-1H-indol-5-yl)-amino)-essigsäure
(12) [[6-Ethyl-1-(6-methyl-pyridazin-3-yl)-1H-indol-5-yl]-(3,5-dichlor-phenylsulphonyl)-amino]-essigsäure
(13) [[1-(4-Amino-pyridin-2-yl)-1H-indol-5-yl]-(3,5-dichlor-phenylsulphonyl)-amino]-essigsäure
(14) ((3,5-Dichlor-phenylsulphonyl)-{1-[6-(3-hydroxy-propyl)-pyridazin-3-yl]-1H-indol-5-yl)-amino)-essigsäure
(15) [{1-[6-(4-Acetyl-piperazin-1-yl)-pyridazin-3-yl]-1H-indol-5-yl}-(3,5-dichlor-phenylsulphonyl)-amino]-essigsäure
(16) {(3,5-Dichlor-phenylsulphonyl)-[1-(4-methylcarbamoyl-pyridin-2-yl)-1H-indol-5-yl]-amino}-essigsäure
(17) {(3,5-Dichlor-phenylsulphonyl)-[3-methyl-1-(6-methyl-pyridazin-3-yl)-1H-indol-5-yl]-amino}-essigsäure
(18) {(3,5-Dichlor-phenylsulphonyl)-[1-(4-methanesulphinyl-pyridin-2-yl)-1H-indol-5-yl]-amino}-essigsäure
(19) [[1-(4-Cyclopropylcarbamoyl-pyridin-2-yl)-1H-indol-5-yl]-(3,5-dichlorphenylsulphonyl)-amino]-essigsäure
(20) {(2,6-Dichlor-pyridin-4-sulphonyl)-[1-(4-methylcarbamoyl-pyridin-2-yl)-1H-indol-5-yl]-amino}-essigsäure,
und die Enantiomeren, Mischungen davon und Salze davon.

8. Physiologisch akzeptable Salze der Verbindungen nach den Ansprüchen 1 bis 7 mit anorganischen oder organischen Säuren oder Basen.

9. Pharmazeutische Zusammensetzungen, enthaltend eine Verbindung nach mindestens einem der Ansprüche 1 bis 7 oder ein Salz nach Anspruch 8, gegebenenfalls zusammen mit ein oder mehr inerten Trägern und/oder Verdünnungsmitteln.

10. Verwendung einer Verbindung nach mindestens einem der Ansprüche 1 bis 7 oder eines Salzes nach Anspruch 8 zur Herstellung einer pharmazeutischen Zusammensetzung, die für die Behandlung von Diabetes mellitus Typ I und Typ II geeignet ist.

11. Verfahren zur Herstellung einer pharmazeutischen Zusammensetzung nach Anspruch 9, **dadurch gekennzeichnet, dass** eine Verbindung nach mindestens einem der Ansprüche 1 bis 7 oder ein Salz nach Anspruch 8 in ein oder mehr inerte Träger und/oder Verdünnungsmittel durch ein nicht-chemisches Verfahren einbezogen wird.

12. Verfahren zur Herstellung der Verbindungen der allgemeinen Formel I nach den Ansprüchen 1 bis 8, **dadurch gekennzeichnet, dass**
a) eine Verbindung der allgemeinen Formel 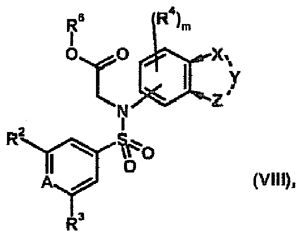 worin R² bis R⁵, m, X, Y, Z und A wie in Anspruch 1 definiert sind und R⁶ eine Carboxyschutzgruppe bezeichnet, entschützt wird und die so erhaltene Säure wird gegebenenfalls durch Alkylierung in eine Verbindung der allgemeinen Formel (I) nach Anspruch 1 umgewandelt und
b) wenn gewünscht wird jede Schutzgruppe, die verwendet wird, um reaktive Gruppen während der Reaktionen zu schützen, nachher oder gleichzeitig abgespalten, und/oder
c) eine so erhaltene Verbindung der allgemeinen Formel I wird in ihre Stereoisomeren aufgetrennt, und/oder
d) eine so erhaltene Verbindung der allgemeinen Formel I wird in die Salze davon, insbesondere zur pharmazeutischen Verwendung in die physiologisch akzeptablen Salze davon, mit anorganischen oder organischen Säuren oder Basen umgewandelt.

## Revendications

1. Arylsulfonylglycines de formule générale 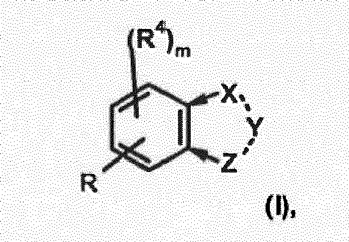 dans laquelle
R représente un groupe de formule 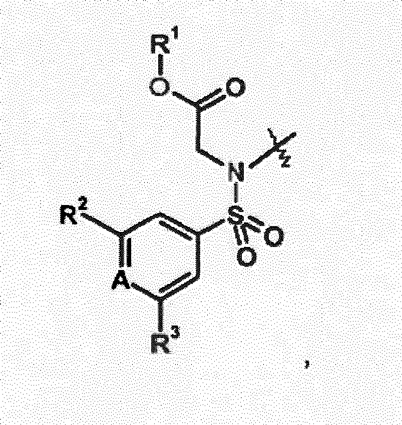 dans laquelle
R¹ représente H, un groupe alkyle en C₁ à C₆ ou un groupe de formule 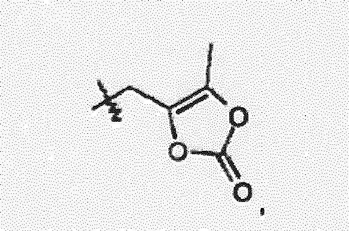 dans laquelle le groupe alkyle en C₁ à C₆ mentionné ci-avant dans ce document pour R¹ peut être substitué par un groupe alkyl en C₁ à C₆-carbonyloxy, alcoxy en C₁ à C₆-carbonyloxy, alcoxy en C₁ à C₆, hydroxy, amino, alkyl en C₁ à C₃-amino, di-(alkyl en C₁ à C₃)-amino, pyrrolidin-1-yle, pipéridin-1-yle, morpholinyl-4-yle, pipérazin-1-yle, 4-(alkyl en C₁ à C₃)-pipérazin-1-yle, aminocarbonyl, alkyl en C₁ à C₃-aminocarbonyle, di-(alkyle en C₁ à C₃) - aminocarbonyle, pyrrolidin-1-yl-carbonyle, pipéridin-1-yl-carbonyle, morpholin-4-yl-carbonyle, pipérazin-1-yl-carbonyle, 4-(alkyl en C₁ à C₃)-pipérazin-1-yl-carbonyle, tétrahydrofuran-3-yl-oxy, alkyl en C₁ à C₃-amino-alkyl en C₁ à C₃-oxy, di-(alkyl en C₁ à C₃) -amino-alkyl en C₁ à C₃-oxy, pyrrolidin-1-yl-alkyl en C₁ à C₃-oxy, pipéridin-1-yl-alkyl en C₁ à C₃-oxy, morpholin-4-yl-alkyl en C₁ à C₃-oxy, pipérazin-1-yl-alkyl en C₁ à C₃-oxy ou 4-(alkyl eb C₁ à C₃-pipérazin-1-yl-alkyl en C₁ à C₃-oxy,
R² et R³ indépendamment l'un de l'autre représentent H, un atome d'halogène, un groupe alkyle en C₁ à C₃, perfluoroalkyle en C₁ à C₃, perfluoroalcoxy en C₁ à C₃, alcoxy en C₁ à C₃, cyano, nitro ou hydroxy,
et
A représente CH ou N,
m représente 0, 1 ou 2,
R⁴ représente un atome d'halogène, un groupe alkyle en C₁ à C₃, perfluoroalkyle en C₁ à C₃, perfluoroalcoxy en C₁ à C₃, tandis que, si m représente le nombre 2, les groupes R⁴ peuvent être identiques ou différents
et le groupe hétérocyclique 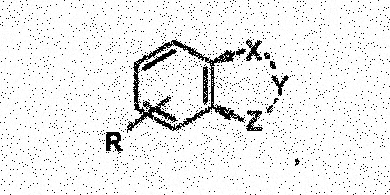 qui peut être substitué par R⁴ tel que décrit ci-avant dans ce document, représente un groupe de formule 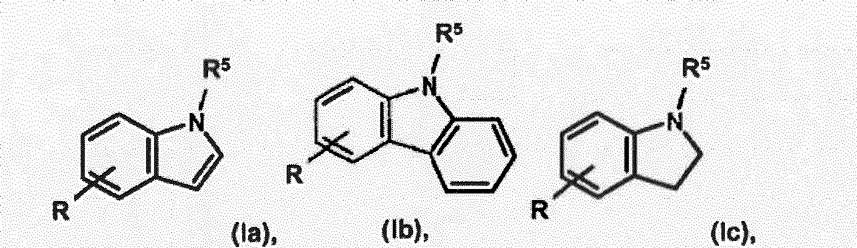 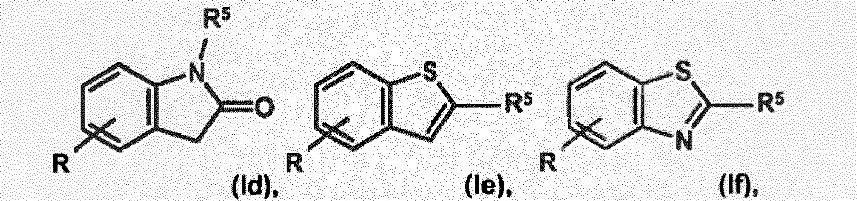 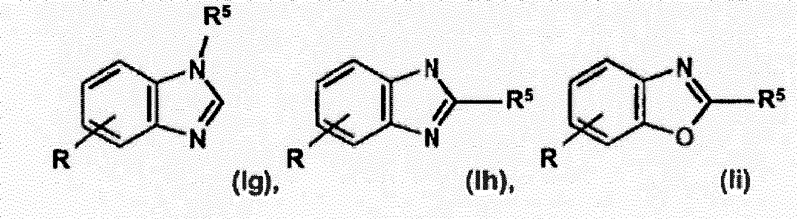 ou 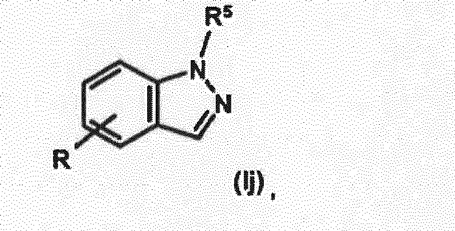
où les hétérocycles susmentionnés des formules (Ia), (Ic), (Id), (Ie), (Ig) et (Ij) peuvent être éventuellement substitués au niveau des atomes de carbone du cycle 5 par un ou deux groupes choisis parmi un atome d'halogène, un groupe alkyle en C₁ à C₃, cyano, perfluoroalkyle en C₁ à C₃, cycloalkyle en C₃ à C₆, alcynyle en C₂ à C₄, alcényle en C₂ à C₄, alkyl en C₁ à C₃-carbonyle, perfluoroalkyl en C₁ à C₃-carbonyle, carboxyle, aminométhyle, alkyl en C₁ à C₃-aminométhyle, di-(alkyl en C₁ à C₃)-aminométhyle, aminocarbonyle, alkyl en C₁ à C₃-aminocarbonyle ou di- (alkyl en C₁ à C₃)-aminocarbonyle, où les groupes peuvent être identiques ou différents et chaque atome de carbone ne peut porter qu'un group, et
où R⁵ représente un groupe 1H-pyrimidin-2,4-dionyle, 2H-pyridazin-3-onyle ou 1H-pyridin-2-onyle éventuellement monosubstitué ou disubstitué par un ou deux groupes méthyle ou
un système de cycle de 5 à 14 chaînons monocyclique ou bicyclique, qui peut contenir 0 à 4 hétéroatomes choisis parmi N, O ou S, où pas plus d'un atome d'oxygène et/ou un atome de soufre ne peut être présent, est aromatique, saturé ou partiellement insaturé et peut être monosubstitué, disubstitué ou trisubstitué indépendamment l'un de l'autre par un groupe choisi parmi
un atome d'halogène, un groupe cyano, nitro,
alkyle en C₁ à C₆, cycloalkyle en C₃ à C₆, perfluoroalkyle en C₁ à C₃, alcynyle en C₂ à C₆, alcényle en C₂ à C₆,
hydroxy, alcoxy en C₁ à C₆, cycloalcoxy en C₃ à C₆, perfluoroalcoxy en C₁ à C₃,
carboxyle, alkyl en C₁ à C₃-carbonyle, alcoxy en C₁ à C₄-carbonyle, cycloalcoxy en C₃ à C₆-carbonyle, aminocarbonyle, alkyl en C₁ à C₆-aminocarbonyle, di-(alkyl en C₁ à C₆)-aminocarbonyle, N-(alkyl en C₁ à C₆)-N-(alcoxy en C₁ à C₆)-aminocarbonyle, cycloalkyl en C₃ à C₆-aminocarbonyle, N-(cycloalkyl en C₃ à C₆)-N-(alkyl en C₁ à C₆)-aminocarbonyle, pyrrolidin-1-yl-carbonyle, pipéridin-1-yl-carbonyle, morpholin-4-yl-carbonyle, pipérazin-1-yl-carbonyle, 4-(alkyl en C₁ à C₃)-pipérazin-1-yl-carbonyle, 4-(alkyl en C₁ à C₃-carbonyl)-pipérazin-1-yl-carbonyle, 4-(cycloalkyl en C₃ à C₆-carbonyl)-pipérazin-1-yl-carbonyle, 4-(alkyl en C₁ à C₃-sulfonyl)-pipérazin-1-yl-carbonyle, 4-(cycloalkyl en C₃ à C₆-sulfonyl)-pipérazin-1-yl-carbonyle,
alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle, cycloalkyl en C₃ à C₆-sulfonyle,
amino, alkyl en C₁ à C₆-amino, di-(alkyl en C₁ à C₆)-amino, cycloalkyl en C₃ à C₆-amino, N-(cycloalkyl en C₃ à C₆)-N-(alkyl en C₁ à C₆)-amino, (alkyl en C₁ à C₄-carbonyl)-amino, (cycloalkyl en C₃ à C₆-carbonyl)-amino, (alkyl en C₁ à C₄-carbonyl)-(alkyl en C₁ à C₃)-amino, (cycloalkyl en C₃ à C₆-carbonyl)-(alkyl en C₁ à C₃)-amino,
pyrrolidin-1-yle, pipéridin-1-yle, 3-amino-pipéridin-1-yle, 4-amino-pipéridin-1-yle, morpholin-4-yle, thiomorpholin-4-yle, 1-axo-1λ⁴-thiomorpholin-4-yle, 1,1-dioxo-1λ⁶-thiomorpholin-4-yle, pipérazin-1-yle, homopipérazin-1-yle, 4-(alkyl en C₁ à C₃)-pipérazin-1-yle, 4-(alkyl en C₁ à C₃-carbonyl)-pipérazin-1-yle, 4-(cycloalkyl en C₃ à C₆-carbonyl)-pipérazin-1-yle, 4-(alkyl en C₁ à C₃-sulfonyl)-pipérazin-1-yle, 4-(cycloalkyl en C₃ à C₆-sulfonyl)-pipérazin-1-yle,
tétrahydrofuran-3-yl-oxy, tétrahydrofuran-3-yl-amino, tétrahydropyran-4-yl-oxy, tétrahydropyran-4-yl-amino, N-tétrahydropyran-4-yl-N-méthyl-amino, 2-oxo-imidazolidin-1-yle, 2-oxo-tétrahydropyrimidin-1-yle et hétéroaryle,
où les groupes alkyl en C₁ à C₆, cycloalkyle en C₃ à C₆, alcynyle en C₂ à C₆, alcényle en C₂ à C₆, alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle, cycloalkyl en C₃ à C₆-sulfonyle, alcoxy en C₁ à C₆, cycloalcoxy en C₃ à C₆, alkyl en C₁ à C₆-amino, di-(alkyl en C₁ à C₆)-amino, cycloalkyl en C₃ à C₆-amino, N-(cycloalkyl en C₃ à C₆)-N-(alkyl en C₁ à C₆)-amino, (alkyl en C₁ à C₄-carbonyl)-amino, (cycloalkyl en C₃ à C₆-carbonyl)-amino, (alkyl en C₁ à C₄-carbonyl)-(alkyl en C₁ à C₃)-amino et (cycloalkyl en C₃ à C₆-carbonyl)-(alkyl en C₁ à C₃)-amino susmentionnés dans la définition de R⁵ peuvent être monosubstitués ou disubstitués indépendamment l'un de l'autre dans le squelette carboné par un groupe choisi parmi
un groupe cyano, hydroxy, alcoxy en C₁ à C₃, tétrahydro-pyran-2-yloxy,
amino, alkyl en C₁ à C₆-amino, di-(alkyl en C₁ à C₆)-amino, cycloalkyl en C₃ à C₆-amino, N-(cycloalkyl en C₃ à C₆)-N-(alkyl en C₁ à C₆)-amino, (alkyl en C₁ à C₄-carbonyl)-amino, (cycloalkyl en C₃ à C₆-carbonyl)-amino, (alkyl en C₁ à C₄-carbonyl)-(alkyl en C₁ à C₃)-amino, (cycloalkyl en C₃ à C₆-carbonyl)-(alkyl en C₁ à C₃)-amino, (alkyl en C₁ à C₄-sulfonyl)-amino, (cycloalkyl en C₃ à C₆-sulfonyl)-amino, (alkyl en C₁ à C₄-sulfonyl)-(alkyl en C₁₃ à C₃)-amino, (cycloalkyl en C₃ à C₆-sulfonyl)-(alkyl en C₁ à C₃)-amino,
pyrrolidin-1-yle, pipéridin-1-yle, morpholin-4-yle, pipérazin-1-yle, 4-(alkyl en C₁ à C₃)-pipérazin-1-yle,
carboxyle, aminocarbonyle, alkyl en C₁ à C₆-aminocarbonyle, di-(alkyl en C₁ à C₆)-aminocarbonyle, alcoxy en C₁ à C₂-carbonyle, cycloalkyl en C₃ à C₆₋aminocarbonyle, N-(cycloalkyl en C₃ à C₆)-N-(alkyl en C₁ à C₆)-amino-carbonyle, pyrrolidin-1-yl-carbonyle, pipéridin-1-yl-carbonyle, morpholin-4-yl-carbonyle, pipérazin-1-yl-carbonyle, 4-(alkyl en C₁ à C₃)-pipérazin-1-yl-carbonyle,
alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle et cycloalkyl en C₃ à C₆-sulfonyle, où les substituants ne doivent pas être liés à un atome de carbone commun, et
où les groupes pyrrolidin-1-yle et pipéridin-1-yle susmentionnés dans la définition de R⁵ peuvent être substitués par un groupe amino ou hydroxy, et
où les groupes alkyl en C₁ à C₆-aminocarbonyle, di-(alkyl en C₁ à C₆)-aminocarbonyle, cycloalkyl en C₃ à C₆-aminocarbonyle, N-(cycloalkyl en C₃ à C₆)-N-(alkyl en C₁ à C₆)-aminocarbonyle mentionnés ci-avant dans ce document pour R⁵ peuvent être chacun monosubstitués ou disubstitués indépendamment l'un de l'autre dans le squelette carboné par un groupe choisi parmi
un groupe cyano, hydroxy, alcoxy en C₁ à C₃, amino, alkyl en C₁ à C₃-amino, di-(alkyl en C₁ à C₃)-amino, cycloalkyl en C₃ à C₆-amino, N-(cycloalkyl en C₃ à C₆)-N-(alkyl en C₁ à C₆)-amino, (alkyl en C₁ à C₄-carbonyl)-amino, (cycloalkyl en C₃ à C₆-carbonyl)-amino, (alkyl en C₁ à C₄-carbonyl)-(alkyl en C₁ à C₃)-amino, (cycloalkyl en C₃ à C₆-carbonyl)-(alkyl en C₁ à C₃)-amino, (alkyl en C₁ à C₄-sulfonyl)-amino, (cycloalkyl en C₃ à C₆-sulfonyl)-amino, (alkyl en C₁ à C₄-sulfonyl)-(alkyl en C₁ à C₃)-amino, (cycloalkyl en C₃ à C₆-sulfonyl)-(alkyl en C₁ à C₃)-amino,
pyrrolidin-1-yle, pipéridin-1-yle, morpholin-4-yle, pipérazin-1-yle, 4-(alkyl en C₁ à C₃)-pipérazin-1-yle,
alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle et cycloalkyl en C₃ à C₆-sulfonyle, où les substituants ne doivent pas être liés à un atome de carbone commun, et
où le groupe hétéroaryle mentionné ci-avant dans ce document pour R⁵ représente un système aromatique de cinq chaînons monocyclique comportant 1 à 4 hétéroatomes choisis parmi N, O ou S, où pas plus d'un atome d'oxygène et/ou un atome de soufre ne peut être présent, ou un système aromatique de six chaînons comportant 1 à 3 atomes d'azote et peut être monosubstitué ou disubstitué indépendamment l'un de l'autre par un atome d'halogène, un groupe cyano, hydroxy, amino, alkyle en C₁ à C₃ ou alkyl en C₁ à C₃-oxycarbonyle, et
où dans les groupes morpholin-4-yle et pipérazin-1-yle susmentionnés dans chaque cas, une unité de méthylène peut être remplacée par un groupe carbonyle ou sulfonyle,
et les tautomères, stéréoisomères, mélanges de celles-ci et les sels de celles-ci.

2. Composés de formule générale I selon la revendication 1, dans laquelle
R représente un groupe de la formule mentionnée dans la revendication 1, dans laquelle
R¹ représente H, un groupe alkyle en C₁ à C₆ ou un groupe de formule 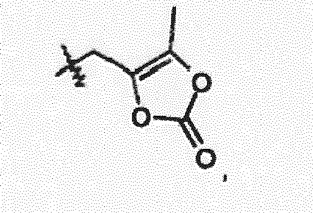 dans laquelle le groupe alkyle en C₁ à C₆ mentionné ci-avant dans ce document pour R¹ peut être substitué par un groupe alkyl en C₁ à C₆-carbonyloxy, alcoxy en c₁ à C₆-carbonyloxy, alcoxy en C₁ à C₆, hydroxy, amino, alkyl en C₁ à C₃-amino, di- (alkyl en C₁ à C₃)-amino, pyrrolidin-1-yle, pipéridin-1-yle, morpholinyl-4-yle, pipérazin-1-yle, 4-(alkyl en C₁ à C₃)-pipérazin-1-yle, aminocarbonyle, alkyl en C₁ à C₃-aminocarbonyle, di-(alkyle en C₁ à C₃)-aminocarbonyle, pyrrolidin-1-yl-carbonyle, pipéridin-1-yl-carbonyle, morpholin-4-yl-carbonyle, pipérazin-1-yl-carbonyle ou 4-(alkyl en C₁ à C₃)-pipérazin-1-yl-carbonyle,
R² et R³ indépendamment l'un de l'autre représentent un atome d'halogène, un groupe alkyle en C₁ à C₃, perfluoroalkyle en C₁ à C₃, alcoxy en C₁ à C₂ ou cyano et
A représente CH ou N,
m représente 0, 1 ou 2,
R⁴ représente un atome d'halogène, un groupe alkyle en C₁ à C₃, trifluorométhyle ou cyano, tandis que, si m représente le nombre 2, les groupes R⁴ peuvent être identiques ou différents,
et le groupe hétérocyclique 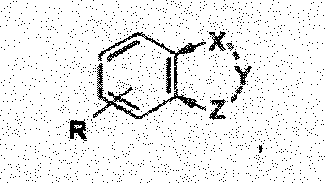 qui peut être substitué par R⁴ tel que décrit ci-lavant dans ce document, représente un groupe de formule 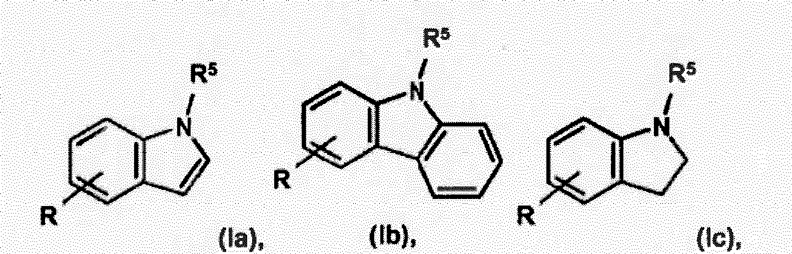 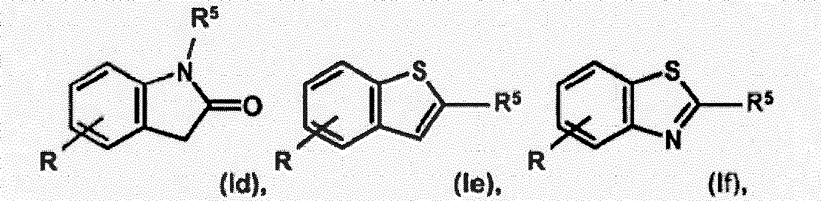 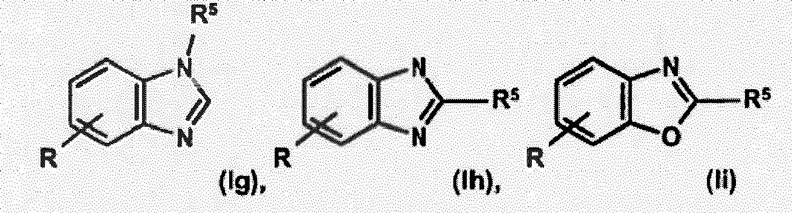 ou 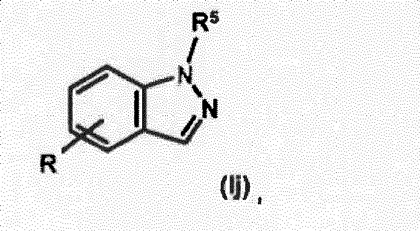
où les hétérocycles susmentionnés des formules (Ia), (Ic), (Id), (Ie), (Ig) et (Ij) peuvent être éventuellement substitués au niveau des atomes de carbone du cycle 5 par un ou deux groupes choisis parmi un atome d'halogène, un groupe alkyle en C₁ à C₃, cyano, perfluoroalkyle en C₁ à C₃, cycloalkyle en C₃ à C₆, alkyl en C₁ à C₃-carbonyle, perfluoroalkyl en C₁ à C₃-carbonyle, aminocarbonyle, alkyl en C₁ à C₃-aminocarbonyle ou di-(alkyl en C₁ à C₃)-aminocarbonyle, où les groupes peuvent être identiques ou différents et chaque atome de carbone porte au plus un groupe, et
où R⁵ représente un groupe 1,3-diméthyl-1H-pyrimidin-2,4-dion-5-yle, 1H-pyrimidin-2,4-dion-6-yle, 1H-pyrimidin-2,4-dion-5-yle, 2H-pyridazin-3-on-6-yle, 1H-pyridin-2-on-3-yle, 1H-pyridin-2-on-5-yle, 1H-pyridin-2-on-4-yle ou
un système de cycle de 5 à 14 chaînons monocyclique ou bicyclique, qui peut contenir 0 à 4 hétéroatomes choisis parmi N, O ou S, où pas plus d'un atome d'oxygène et/ou un atome de soufre ne peut être présent, est aromatique, saturé ou partiellement insaturé et peut être monosubstitué, disubstitué ou trisubstitué indépendamment l'un de l'autre par un groupe choisi parmi
un atome d'halogène, un groupe cyano, alkyle en C₁ à C₆, cycloalkyle en C₃ à C₆, perfluoroalkyle en C₁ à C₃, alcynyle en C₂ à C₆, alcényle en C₂ à C₆,
hydroxy, alcoxy en C₁ à C₆, cycloalcoxy en C₃ à C₆, trifluorométhoxy,
carboxyle, alkyl en C₁ à C₃-carbonyle, alcoxy en C₁ à C₄-carbonyle, cyclopropoxy-carbonyle, aminocarbonyle, alkyl en C₁ à C₆-aminocarbonyle, di-(alkyl en C₁ à C₆)-aminocarbonyle, N-(alkyl en C₁ à C₃)-N-(alcoxy en C₁ à C₃)-aminocarbonyle, cycloalkyl en C₃ à C₆-aminocarbonyle, N-(cycloalkyl en C₃ à C₆)-N-(alkyl en c₁ à C₃)-aminocarbonyle, pyrrolidin-1-yl-carbonyle, pipéridin-1-yl-carbonyle, morpholin-4-yl-carbonyle, pipérazin-1-yl-carbonyle, 4-(alkyl en C₁ à C₃)-pipérazin-1-yl-carbonyle, 4-(alkyl, en C₁ à C₃-carbonyl)-pipérazin-1-yl-carbonyle, 4-(cycloalkyl en C₃ à C₆-carbonyl)-pipérazin-1-yl-carbonyle, 4-(alkyl en C₁ à C₃-sulfonyl)-pipérazin-1-yl-carbonyle, 4-(cycloalkyl en C₃ à C₆-sulfonyl)-pipérazin-1-yl-carbonyle,
alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle, cycloalkyl en C₃ à C₆-sulfonyle, amino, alkyl en C₁ à C₆-amino, di-(alkyl en C₁ à C₆)-amino, cycloalkyl en C₃ à C₆-amino, N-(cycloalkyl en C₃ à C₆)-N-(alkyl en C₁ à C₃)-amino, (alkyl en C₁ à C₄-carbonyl)-amino, (cycloalkyl en C₃ à C₆-carbonyl)-amino, (alkyl en C₁ à C₄-carbonyl)-(alkyl en C₁ à C₃)-amino, (cycloalkyl en C₁ à C₆-carbonyl)-(alkyl en C₁ à C₃)-amino, pyrrolidin-1-yle, pipéridin-1-yle, 3-amino-pipéridin-1-yle, 4-amino-pipéridin-1-yle, morpholin-4-yle, thiomorpholin-4-yle, 1-oxo-1λ⁴-thiomorpholin-4-yle, 1,1-dioxo-1λ⁶-thiomorpholin-4-yle, pipérazin-1-yle, homopipérazin-1-yle, 4-(alkyl en C₁ à C₃)-pipérazin-1-yle, 4-(alkyl en C₁ à C₃-carbonyl)-pipérazin-1-yle, 4-(cycloalkyl en C₃ à C₆-carbonyl)-pipérazin-1-yle, 4-(alkyl en C₁ à C₃-sulfonyl)-pipérazin-1-yle, 4-(cycloalkyl en C₃ à C₆-sulfonyl)-pipérazin-1-yle,
tétrahydrofuran-3-yl-oxy, tétrahydrofuran-3-yl-amino, tétrahydropyran-4-yl-oxy, tétrahydropyran-4-yl-amino, N-tétrahydropyran-4-yl-N-méthyl-amino, 2-oxo-imidazolidinyle, 2-oxo-tétrahydropyrimidinyle et hétéroaryle,
où les groupes alkyle en C₁ à C₆, cycloalkyle en C₃ à C₆, alcynyle en C₂ à C₆, alcényle en C₂ à C₆, alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle, cycloalkyl en C₃ à C₆-sulfonyle, alcoxy en C₁ à C₆, cycloalcoxy en C₃ à C₆, alkyl en C₁ à C₆-amino, di-(alkyl en C₁ à C₆) -amino, cycloalkyl en C₃ à C₆-amino, N-(cycloalkyl en C₃ à C₆)-N-(alkyl en C₁ à C₆)-amino, (alkyl en C₁ à C₄-carbonyl)-amino, (cycloalkyl en C₃ à C₆-carbonyl)-amino, (alkyl en C₁ à C₄-carbonyl)-(alkyl en C₁ à C₃)-amino et (cycloalkyl en C₃ à C₆-carbonyl)-(alkyl en C₁ à C₃)-amino susmentionnés dans la définition de R⁵ peuvent être chacun monosubstitués ou disubstitués indépendamment l'un de l'autre dans le squelette carboné par un groupe choisi parmi
un groupe cyano, hydroxy, alcoxy en C₁ à C₃, tétrahydro-pyran-2-yloxy,
amino, alkyl en C₁ à C₆-amino, di-(alkyl en C₁ à C₆)-amino, cycloalkyl en C₃ à C₆-amino, N-(cycloalkyl en C₃ à C₆)-N-(alkyl en C₁ à C₆)-amino, (alkyl en C₁ à C₄-carbonyl)-amino, (cycloalkyl en C₃ à C₆-carbonyl)-amino, (alkyl en C₁ à C₄-carbonyl)-(alkyl en C₁ à C₃)-amino, (cycloalkyl en C₃ à C₆-carbonyl)-(alkyl en C₁ à C₃)-amino, (alkyl en C₁ à C₄-sulfonyl)-amino, (cycloalkyl en C₃ à C₆-sulfonyl)-amino, (alkyl en C₁ à C₄-sulfonyl)-(alkyl en C₁ à C₃)-amino, (cycloalkyl en C₃ à C₆-sulfonyl)-(alkyl en C₁ à C₃)-amino,
pyrrolidin-1-yle, pipéridin-1-yle, morpholin-4-yle, pipérazin-1-yle, 4-(alkyl en C₁ à C₃)-pipérazin-1-yle,
carboxyle, alcoxy en C₁ à C₂-aminocarbonyle, aminocarbonyle, alkyl en C₁ à C₆-aminocarbonyle, di-(alkyl en C₁ à C₆)-aminocarbonyle, cycloalkyl en C₃ à C₆-aminocarbonyle, N-(cycloalkyl en C₃ à C₆)-N-(alkyl en C₁ à C₃)-aminocarbonyle, pyrrolidin-1-yl-carbonyle, pipéridin-1-yl-carbonyle, morpholin-4-yl-carbonyle, pipérazin-1-yl-carbonyle, 4-(alkyl en C₁ à C₃)-pipérazin-1-yl-carbonyle,
alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle et cycloalkyl en C₃ à C₆-sulfonyle, où les substituants ne doivent pas être liés à un atome de carbone commun, et
où les groupes pyrrolidin-1-yle et pipéridin-1-yle susmentionnés dans la définition de R⁵ peuvent être substitués par un groupe amino ou hydroxy, et
où les groupes alkyl en C₁ à C₆-aminocarbonyle, di-(alkyl en C₁ à C₆)-aminocarbonyle, cycloalkyl en C₃ à C₆-aminocarbonyle, N-(cycloalkyl en C₃ à C₆)-N-(alkyl en C₁ à C₃)-aminocarbonyle mentionnés ci-avant dans ce document pour R⁵ peuvent être chacun monosubstitués ou disubstitués indépendamment l'un de l'autre dans le squelette carboné par un groupe choisi parmi
un groupe amino, hydroxy, alcoxy en C₁ à C₃, alkyl en C₁ à C₃-amino, di-(alkyl en C₁ à C₃)-amino, cycloalkyl en C₃ à C₆-amino, N-(cycloalkyl en C₃ à C₆)-N-(méthyl)-amino, (alkyl en C₁ à C₄-carbonyl)-amino, (cycloalkyl en C₃ à C₆-carbonyl)-amino, (alkyl en C₁ à C₄-carbonyl)-(méthyl)-amino, (cycloalkyl en C₃ à C₆-carbonyl)-(méthyl)-amino, (alkyl en C₁ à C₄-sulfonyl)-amino, (cycloalkyl en C₃ à C₆-sulfonyl)-amino, (alkyl en C₁ à C₄-sulfonyl)-(méthyl)-amino ou (cycloalkyl en C₃ à C₆-sulfonyl)-(méthyl)-amino,
pyrrolidin-1-yle, pipéridin-1-yle, morpholin-4-yle, pipérazin-1-yle, 4-(alkyl en C₁ à C₃)-pipérazin-1-yle,
alkyl en C₁ à C₃-sulfanyle, cycloalkyl en C₃ à C₆-sulfanyle, alkyl en C₁ à C₃-sulfinyle, cycloalkyl en C₃ à C₆-sulfinyle, alkyl en C₁ à C₃-sulfonyle et cycloalkyl en C₃ à C₆-sulfonyle, où les substituants ne doivent pas être liés à un atome de carbone commun, et
où le groupe hétéroaryle mentionné ci-avant dans ce document pour R⁵ représente un système aromatique de cinq chaînons monocyclique comportant 1 à 3 hétéroatomes choisis parmi N, O ou S, où pas plus d'un atome d'oxygène et/ou un atome de soufre ne peut être présent, ou représente un système aromatique de cinq chaînons monocyclique comportant 4 atomes d'azote ou un système aromatique de six chaînons comportant 1 à 3 atomes d'azote et peut être monosubstitué ou disubstitué indépendamment l'un de l'autre par un atome de fluor, de chlore, un groupe cyano, alkyle en C₁ à C₃ ou alkyl en C₁ à C₃-oxycarbonyle, et
où dans les groupes morpholin-4-yle et pipérazin-1-yle susmentionnés dans chaque cas, une unité de méthylène peut être remplacée par un groupe carbonyle ou sulfonyle,
et les tautomères, stéréoisomères, mélanges de ceux-ci et les sels de ceux-ci.

3. Composés de formule générale I selon la revendication 2, dans laquelle
R représente un groupe de la formule mentionnée dans la revendication 1, dans laquelle
R¹ représente H, un groupe alkyle en C₁ à C₄ ou un groupe de formule 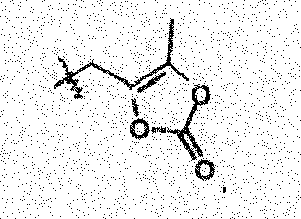 dans laquelle le groupe alkyle en C₁ à C₄ mentionné ci-avant dans ce document pour R¹ peut être substitué par un groupe alcoxy en C₁ à C₄, hydroxy, di-(alkyl en C₁ à C₃)-amino, pyrrolidin-1-yle, pipéridin-1-yle, morpholinyl-4-yle, pipérazin-1-yle ou 4-(méthyl)-pipérazin-1-yle,
R² et R³ indépendamment l'un de l'autre représentent un atome de chlore, de brome ou un groupe alkyle en C₁ à C₂ et
A représente CH ou N,
m représente 0 ou 1,
R⁴ représente un atome de fluor, de chlore, un groupe méthyle ou éthyle,
et le groupe hétérocyclique 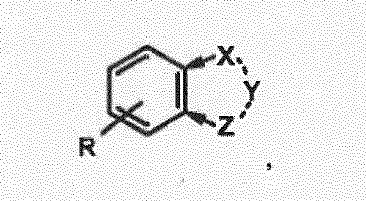 qui peut être substitué par R⁴ tel que décrit ci-avant dans ce document, représente un groupe de formule 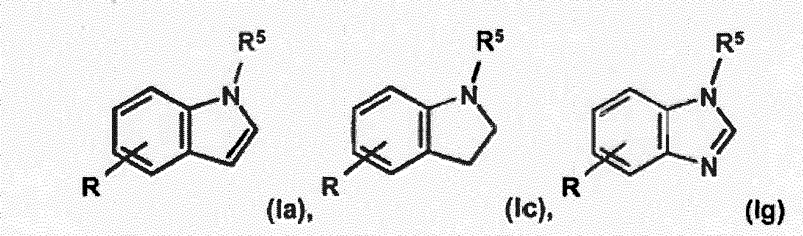 ou 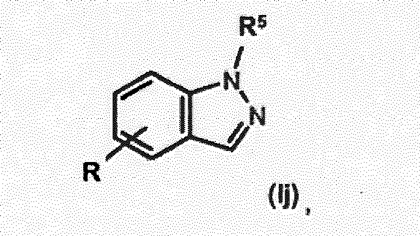 alors que les hétérocycles mentionnés ci-avant dans ce document peuvent être éventuellement substitués au niveau des atomes de carbone du cycle 5 par un ou deux groupes choisis parmi un atome de chlore, de brome, d'iode, un groupe alkyle en C₁ à C₃, cyano et trifluorométhyle, où les groupes peuvent être identiques ou différents et chaque atome de carbone porte au plus un groupe, et
où R⁵ représente un groupe 1,3-diméthyl-1H-pyrimidin-2,4-dion-5-yle, 1H-pyrimidin-2,4-dion-6-yle, 1H-pyrimidin-2,4-dion-5-yle, 2H-pyridazin-3-on-6-yle, 1H-pyridin-2-on-3-yle, 1H-pyridin-2-on-5-yle ou 1H-pyridin-2-on-4-yle,
phényle, pyridazin-3-yle, pyrimidin-2-yle, pyrimidin-4-yle, pyrimidin-5-yle, pyrazin-2-yle, 1,3,5-triazin-2-yle, pyridin-2-yle, pyridin-4-yle, imidazol-2-yle, imidazol-4-yle, pyrazol-3-yle, pyrazol-4-yle, thiazol-2-yle, [1,3,4]thiadiazol-2-yle, thiophén-2-yle, thiophén-3-yle, naphtalin-1-yle, naphtalin-2-yle, purin-6-yle, purin-2-yle, 1-imidazo[1,2-a]pyrazin-6-yle, quinoléin-6-yle, quinoléin-8-yle, quinoléin-2-yle ou isoquinoléin-1-yle, chacun pouvant être monosubstitué ou disubstitué indépendamment l'un de l'autre par un groupe choisi parmi
un atome de fluor, de chlore, un groupe alkyle en C₁ à C₄, cyclopropyle, trifluorométhyle, cyano, hydroxy, alcoxy en C₁ à C₃, cyclopropoxy,
carboxyle, alkyl en C₁ à C₂-carbonyle, alcoxy en C₁ à C₂-carbonyle, aminocarbonyl, alkyl en C₁ à C₄-aminocarbonyle, di-(alkyl en C₁ à C₂)-aminocarbonyle, N-méthoxy-N-méthyl-aminocarbonyle, cyclopropyl-aminocarbonyle, N-(cyclopropyl)-N-(méthyl)-aminocarbonyle, pyrrolidin-1-yl-carbonyle, pipéridin-1-yl-carbonyle, morpholin-4-yl-carbonyle, pipérazin-1-yl-carbonyle, 4-(methyl)-pipérazin-1-yl-carbonyle, 4-(alkyl en C₁ à C₃-carbonyl)-pipérazin-1-yl-carbonyle, 4-(cycloalkyl en C₃ à C₆-carbonyl)-pipérazin-1-yl-carbonyle, 4-(alkyl en C₁ à C₃-sulfonyl)-pipérazin-1-yl-carbonyle, 4-(cycloalkyl en C₃ à C₆-sulfonyl)-pipérazin-1-yl-carbonyle,
alkyl en C₁ à C₂-sulfanyle, cyclopropylsulfanyle, alkyl en C₁ à C₂-sulfinyle, cyclopropyl-sulfinyle, alkyl en C₁ à C₂-sulfonyle, cyclopropylsulfonyle,
amino, alkyl en C₁ à C₄-amino, di-(alkyl en C₁ à C₃)-amino, cyclopropyl-amino, N-(cyclopropyl)-N-(méthyl)-amino, alkyl en C₁ à C₃-carbonyl-amino,
pyrrolidin-1-yle, pipéridin-1-yle, 3-amino-pipéridin-1-yle, 4-amino-pipéridin-1-yle, morpholin-4-yle, thiomorpholin-4-yle, 1-oxo-1λ⁴-thiomorpholin-4-yle, 1,1-dioxo-1λ⁶-thiomorpholin-4-yle, pipérazin-1-yle, homopipérazin-1-yle, 4-(méthyl)-pipérazin-1-yle, 4-(alkyl en C₁ à C₂-carbonyl)-pipérazin-1-yle, 4-(alkyl en C₁ à C₂-sulfonyl)-pipérazin-1-yle,
tétrahydrofuran-3-yl-oxy, tétrahydrofuran-3-yl-amino, tétrahydropyran-4-yl-oxy, tétrahydropyran-4-yl-amino, N-tétrahydropyran-4-yl-N-méthyl-amino, 2-oxo-imidazolidinyle, 2-oxotétrahydropyrimidinyle, imidazol-2-yle, 1-méthyl-imidazol-2-yle, thiazol-2-yle, 4-éthoxycarbonyl-thiazol-2-yle, 3-éthoxycarbonyl-isoxazol-5-yle, oxazol-2-yle, 2,4-dihydroxy-pyrimidin-5-yle, 1,2,4-triazol-3-yle et tétrazol-5-yle, ou
où les groupes alkyle en C₁ à C₄, alcoxy en C₁ à C₃; alkyl en C₁ à C₄-amino, di-(alkyl en C₁ à C₃)-amino et alkyl en C₁ à C₃-carbonyl-amino mentionnés ci-avant dans ce document pour R⁵ peuvent être chacun monosubstitués ou disubstitués indépendamment l'un de l'autre dans le squelette carboné par un groupe choisi parmi
un groupe cyano, hydroxy, alcoxy en C₁ à C₂, tétrahydro-pyran-2-yloxy,
amino, alkyl en C₁ à C₃-amino, di-(alkyl en C₁ à C₃)-amino, (alkyl en C₁ à C₃-carbonyl)-amino, (alkyl en C₁ à C₃-sulfonyl)-amino, pyrrolidin-1-yle, pipéridin-1-yle, morpholin-4-yle, pipérazin-1-yle, 4-(méthyl)-pipérazin-1-yle,
carboxyle, alcoxy en C₁ à C₂-carbonyle, aminocarbonyle, alkyl en C₁ à C₃-aminocarbonyle, di-(alkyl en C₁ à C₃)-aminocarbonyle et cycloalkyl en C₃ à C₆-aminocarbonyl, où les substituants ne doivent pas être liés à un atome de carbone commun, et
où les groupes alkyl en C₁ à C₄-aminocarbonyle et di-(alkyl en C₁ à C₂)-aminocarbonyle mentionnés ci-avant dans ce document pour R⁵ peuvent être chacun monosubstitués ou disubstitués indépendamment l'un de l'autre dans le squelette carboné par un groupe amino, hydroxy, alcoxy en C₁ à C₃, alkyl en C₁ à C₃-amino ou di-(alkyl en C₁ à C₃)-amino, où les substituants ne doivent pas être liés à un atome de carbone commun, et
où dans les groupes morpholin-4-yle et pipérazin-1-yle susmentionnés dans chaque cas, une unité de méthylène peut être remplacée par un groupe carbonyle,
et les tautomères, stéréoisomères, mélanges de ceux-ci et les sels de ceux-ci.

4. Composés de formule générale I selon la revendication 3, dans laquelle
R représente un groupe de la formule mentionnée dans la revendication 1, dans laquelle
R¹ représente H ou un groupe alkyle en C₁ à C₃ éventuellement substitué par un groupe di-(alkyl en C₁ à C₃)-amino,
R² et R³ indépendamment l'un de l'autre représentent un atome de chlore, de brome ou un groupe méthyle et
A représente CH ou N,
m représente 0 ou 1,
R⁴ représente un atome de chlore, un groupe méthyle ou éthyle,
et le groupe hétérocyclique 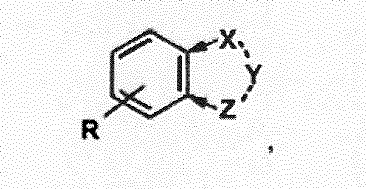 qui peut être substitué par R⁴ tel que décrit ci-avant dans ce document, représente un groupe de formule 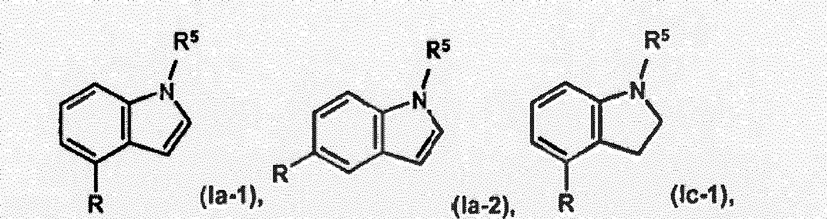 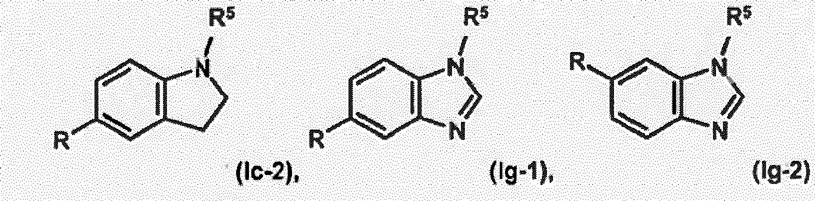 ou 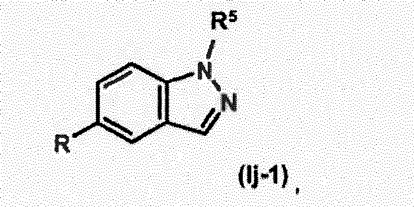 alors que les hétérocycles mentionnés ci-avant dans ce document peuvent être éventuellement substitués au niveau des atomes de carbone du cycle 5 par un ou deux groupes choisis parmi un atome de chlore, de brome, d'iode, un groupe alkyle en C₁ à C₂, cyano et trifluorométhyle, où les groupes peuvent être identiques ou différents et chaque atome de carbone porte au plus un groupe, et
R⁵ est défini comme dans la revendication 3,
et les tautomères, stéréoisomères, mélanges de ceux-ci et les sels de ceux-ci.

5. Composés de formule générale I selon la revendication 4, dans laquelle
R représente un groupe de la formule mentionnée dans la revendication 1, dans laquelle
R¹ représente H, un groupe méthyle, éthyle ou 2-diméthylamino-éthyle,
R² et R³ indépendamment l'un de l'autre représentent un atome de chlore, de brome ou un groupe méthyle et
A représente CH ou N,
m représente 0 ou 1,
R⁴ représente un atome de chlore, un groupe méthyle ou éthyle,
et le groupe hétérocyclique 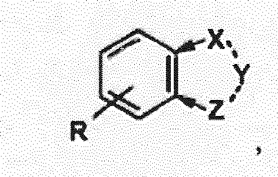 qui peut être substitué par R⁴ tel que décrit ci-avant dans ce document, représente un groupe de formule 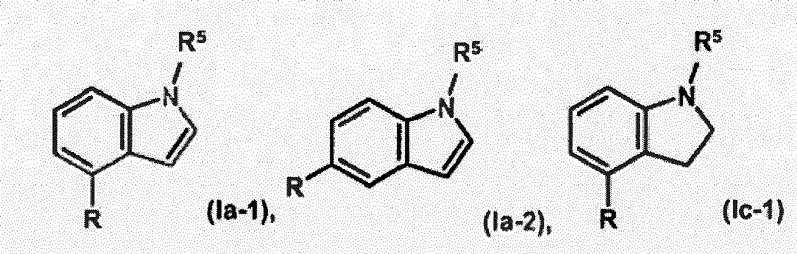 ou 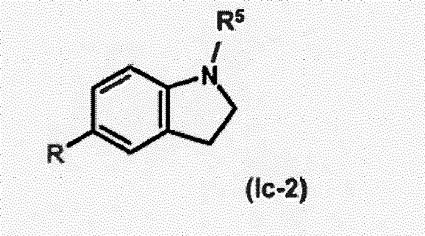
alors que les hétérocycles mentionnés ci-avant dans ce document peuvent être éventuellement substitués au niveau des atomes de carbone du cycle 5 par un ou deux groupes méthyle ou éthyle, où les groupes peuvent être identiques ou différents et chaque atome de carbone porte au plus un groupe, et où
R⁵ représente un groupe 1,3-diméthyl-1H-pyrimidin-2,4-dion-5-yle, 1H-pyrimidin-2,4-dion-6-yle, 1H-pyrimidin-2,4-dion-5-yle, 2H-pyridazin-3-on-6-yle, 1H-pyridin-2-on-3-yle, 1H-pyridin-2-on-5-yle, 1H-pyridin-2-on-4-yle ou
phényle, pyridazin-3-yle, pyrimidin-2-yle, pyrimidin-4-yle, pyrimidin-5-yle, pyrazin-2-yle, pyridin-2-yle, pyridin-4-yle, imidazol-2-yle, imidazol-4-yle, pyrazol-3-yle, pyrazol-4-yle, 1,3,5-triazin-2-yle, thiazol-2-yle, [1,3,4]thiadiazol-2-yle, thiophén-2-yle, thiophén-3-yle, purin-6-yle, purin-2-yle ou 1-imidazo-[1,2-a]pyrazin-6-yle, chacun pouvant être monosubstitué ou disubstitué indépendamment l'un de l'autre par un groupe choisi parmi
un atome de chlore, un groupe cyano, méthyle, aminométhyle, morpholin-4-ylméthyle, hydroxyméthyle, 3-hydroxypropyle, trifluorométhyle,
hydroxy, méthoxy, 2-hydroxyéthoxy, 2-aminoéthoxy, 2-diméthylaminoéthoxy, 2-méthyl-sulfonylamino-éthoxy, 2'-acétylamïno-éthoxy, 2,3-dihydroxy-propoxy,
carboxyle, acétyle, éthylcarbonyle, aminocarbonyle, méthyl-aminocarbonyle, diméthyl-aminocarbonyle, N-méthoxy-N-méthyl-aminocarbonyle, 2-diméthylamino-éthyl-aminocarbonyle, 2-hydroxy-éthyl-aminocarbonyle, 2-méthoxy-éthyl-aminocarbonyle, cyclo-aminocarbonyle,
morpholin-4-yl-carbonyle, pipérazin-1-yl-carbonyle, méthoxy-carbonyle,
méthylsulfanyle, méthylsulfinyle, éthylsulfinyle, méthylsulfonyle,
amino, méthyl-amino, acétylamino, 2-amino-éthyl-amino, 2-diméthylaminoéthyl-amino, 2-hydroxyéthyl-amino, 2-(méthylamino)-éthylamino, N-carboxyméthyl-N-[2-(diméthylamino)-éthyl]-amino, 2-(acétylamino)éthyl-amino, 2-(méthylsulfonylamino)-éthyl-amino,
2-(pyrrolidin-1-yl)-éthyl₋amino, 2-(pipéridin-1-yl)-éthyl-amino, 2-(tétrahydro-pyran-2-yloxy)-éthylamino, 3-aminopropyl-amino, 3-(méthylamino)-propylamino, 2-amino-2-méthyl-propyl-amino, 1,3-dihydroxy-2-propyl-amino, 3-(acétylamino)-propyl-amino, 3-(méthylsulfonylamino)-propyl-amino, diméthyl-amino, N-méthyl-N-2-aminoéthyl-amino, N,N-bis-2-(hydroxyéthyl)-amino, N-méthyl-N-3-aminopropyl-amino, N-méthyl-N-[3-(acétylamino)-propyl]-amino, N-méthyl-N-[3-(méthylsulfonylamino)-propyl]-amino, cyclopropyl-amino,
morpholin-4-yle, thiomorpholin-4-yle, 1-oxo-1λ⁴-thiomorpholin-4-yle, 1,1-dioxo-1λ⁶-thio-morpholin-4-yle, 3-amino-pipéridin-1-yle, pipérazin-1-yle, homopipérazin-1-yle, 4-acétyl-pipérazin-1-yle, 4-méthylsulfonylpipérazin-1-yle,
tétrahydrofuran-3-yl-oxy, tétrahydrofuran-3-yl-amino, tétrahydropyran-4-yl-oxy, tétrahydropyran-4-yl-amino, N-tétrahydropyran-4-yl-N-méthyl-amino, 2-oxo-imidazolidinyle, imidazol-2-yle, 1-méthyl-imidazol-2-yle, thiazol-2-yle, 4-éthoxy-carbonylthiazol-2-yle, 3-éthoxycarbonyl-isoxazol-5-yle et oxazol-2-yle,
où dans les groupes morpholin-4-yle et pipérazin-1-yle susmentionnés dans chaque cas, une unité de méthylène peut être remplacée par un groupe carbonyle,
et les tautomères, stéréoisomères, mélanges de ceux-ci et les sels de ceux-ci.

6. Composés de formule générale I selon la revendication 5, dans laquelle
R représente un groupe de la formule mentionnée dans la revendication 1, dans laquelle
R¹ représente un atome d'hydrogène,
R² et R³ dans chaque cas représentent un atome de chlore et
A représente CH,
m représente 0 et
et le groupe hétérocyclique 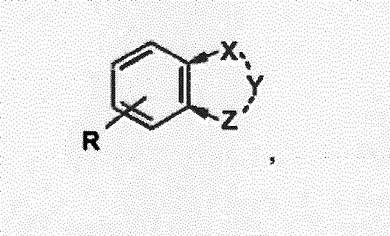 représente un groupe de formule 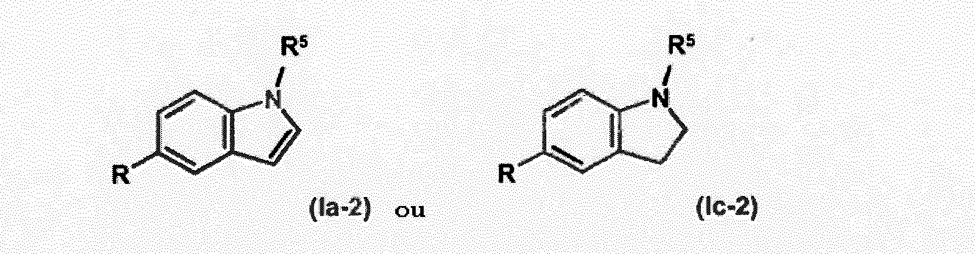
alors que les hétérocycles mentionnés ci-avant dans ce document peuvent être éventuellement substitués au niveau des atomes de carbone du cycle 5 par un ou deux groupes méthyle, chaque atome de carbone porte au plus un groupe, et où
R⁵ représente un groupe phényle, pyridazin-3-yle, pyrimidin-2-yle, pyrimidin-4-yle, pyrimidin-5-yle, pyrazin-2-yle, pyridin-2-yle ou pyridin-4-yle, chacun pouvant être substitué par un groupe choisi parmi
un groupe cyano, méthyle, aminométhyle, hydroxyméthyle, 3-hydroxypropyle,; trifluorométhyle,
hydroxy, méthoxy, 2-hydroxyéthoxy, 2-aminoéthoxy, 2-(diméthyl-amino)éthoxy, 2-(méthylsulfonyl)-amino-éthoxy, 2-(acétalamino)-éthoxy, 2,3-dihydroxy-propoxy,
carboxyle, acétyle, éthylcarbonyle, aminocarbonyle, méthyl-aminocarbonyle, diméthyl-aminocarbonyle, N-méthoxy-N-méthyl-aminocarbonyle, 2-(diméthylamino)-éthyl-aminocarbonyle, 2-hydroxy-éthyl;-aminocarbonyle, 2-méthoxy-éthyl-aminocarbonyle, cyclopropyl-aminocarbonyle,
morpholin-4-yl-carbonyle, pipérazin-1-yl-carbonyle, méthoxy-carbonyle,
méthylsulfanyle, méthylsulfinyle, éthylsulfinyle, méthylsulfonyle,
amino, méthyl-amino, acétylamino, 2-amino-éthyl-amino, 2-(diméthylamino)-éthyl-amino, 2-hydroxyéthyl-amino, 2-(méthylamino)-éthylamino, N-carboxyméthyl-N-[2-(diméthylamino)-éthyl]-amino, 2-(acétylamino)-éthyl-amino, 2-(méthylsulfonylamino)-éthyl-amino, 2-(pyrrolidin-1-yl)-éthyl-amino, 2-(pipéridin-1-yl)-éthyl-amino, 3-aminopropyl-amino, 3-(méthylamino)-propyl-amino, 3-(acétylamino)-propyl-amino, 3-(méthylsulfonylamino)-propyl-amino, diméthyl;-amino, N-méthyl-N-(2-aminoéthyl)-amino, N,N-bis-(2-hydroxyéthyl)-amino, N-méthyl-N-(3-aminopropyl)-amino, N-méthyl-N-[3-(acétylamino)-propyl]-amino, N-méthyl-N-[3-(méthylsulfonylamino)-propyl]-amino, cyclopropyl-amino,
morpholin-4-yle, thiomorpholin-4-yle, 1-oxo-1λ⁴-thiomorpholin-4-yle, 1,1-dioxo-1λ⁶-thio-morpholin-4-yle, 3-amino-pipéridin-1-yle, pipérazin-1-yle, homopipérazin-1-yle, 4-acétyl-pipérazin-1-yle, 4-méthylsulfonylpipérazin-1-yle,
tétrahydrofuran-3-yl-oxy, tétrahydrofuran-3-yl-amino, tétrahydropyran-4-yl-oxy, tétrahydropyran-4-yl-amino, N-tétrahydropyran-4-yl-N-méthyl-amino, 2-oxo-imidazolidinyle, imidazol-2-yle, 1-méthylimidazol-2-yle, thiazol-2-yle, 4-éthoxy-carbonylthiazol-2-yle, 3-éthoxycarbonyl-isoxazol-5-yle et oxazol-2-yle,
et les tautomères, stéréoisomères, mélanges de ceux-ci et les sels de ceux-ci.

7. Composés suivants de formule générale I selon la revendication 1 :
(1) l'acide ((3,5-dichloro-phénylsulfonyl)-{1-[5-(2-diméthylamino₋éthylamino)-pyrazin-2-yl]-1H-indol-5-yl}-amino)-acétique
(2) l'acide {(3,5-dichloro-phénylsulfonyl)-[1-(6-pipérazin-1-yl-pyridazin-3-yl)-2,3-dihydro-1H-indol-5-yl]-amino}-acétique
(3) l'acide ((3,5-dichloro-phénylsulfonyl)-{1-L6-(2'-diméthylamino-éthylamino)-pyridazin-3-yl]-1H-indol-5-yl}-amino)-acétique
(4) l'acide {(3,5-dichlora-phénylsulfonyl)-[1-(6-méthyl-pyridazin-3-yl)-1H-indol-5-yl]-amino}-acétique
(5) l'acide ((3-bromo-5-méthyl-phénylsulfonyl)-{1-[6-(2-diméthylamino-éthylamino)-pyridazin-3-yl]-1H-indol-5-yl}-amino)-acétique
(6) l'acide {(3,5-dichloro-phénylsulfonyl)-[1-(6-pipérazin-1-yl-pyridazin-3-yl)-1H-indol-5-yl]-amino}-acétique
(7) l'acide [[1-(6-[1,4]diazépan-1-yl-pyridazin-3-yl)-1H-indol-5-yl]-(3,5-dichlorophénylsulfonyl)-amino]-acétique
(8) l'acide {(3,5-dichloro-phénylsulfonyl)-[1-(4-méthyl-pyridin-2-yl)-1H-indol-5-yl]-amino}-acétique
(9) l'acide ((3,5-dichloro-phénylsulfonyl)-{1-[6-(2-diméthylamino-éthylamino)-pyridazin-3-yl]-3-méthyl-1H-indol-5-yl}-amino)-acétique
(10) l'acide [(3,5-dichloro-phénylsulfonyl)-(1-{6-[méthyl-(tétrahydro-pyran-4-yl)-amino]-pyridazin-3-yl}-1H-indol-5-yl)-amino]-acétique
(11) l'acide ((3,5-dichloro-phénylsulfonyl)-{1-[5-(3-oxo-pipérazin-1-yl)-pyrazin-2-yl]-1H-indol-5-yl}-amino)-acétique
(12) l'acide [[6-éthyl-1-(6-méthyl-pyridazin-3-yl)-1H-indol-5-yl]-(3,5-dichlorophénylsulfonyl)-amino]-acétique
(13) l'acide [[1-(4-amino-pyridin-2-yl)-1H-indol-5-yl]-(3,5-dichloro-phénylsulfonyl)-amino]-acétique
(14) l'acide ((3,5-dichloro-phénylsulfonyl)-{1-[6-(3-hydroxy-propyl)-pyridazin-3-yl]-1H-indol-5-yl}-amino)-acétique
(15) l'acide [{1-[6-(4-acétyl-pipérazin-1-yl)-pyridazin-3-yl]-1H-indol-5-yl}-(3,5-dichloro-phénylsulfonyl)-amino]-acétique
(16) l'acide {(3,5-dichloro-phénylsulfonyl)-[1-(4-méthylcarbamoyl-pyridin-2-yl)-1H-indol-5-yl]-amino}-acétique
(17) l'acide {(3,5-dichloro-phénylsulfonyl)-[3-méthyl-1-(6-méthyl-pyridazin-3-yl)-1H-indol-5-yl]-aminol-acétique
(18) l'acide {(3,5-dichloro-phénylsulfonyl)-[1-(4-méthanesulfinyl-pyridin-2-yl)-1H-indol-5-yl]-amino}-acétique
(19) l'acide [[1-(4-cyclopropylcarbamoyl-pyridin-2-yl)-1H-indol-5-yl]-(3,5-dichlorophénylsulfonyl)-amino]-acétique
(20) l'acide {(2,6-dichloro-pyridin-4-sulfonyl)-[1-(4-méthylcarbamoyl-pyridin-2-yl)-1Hindol-5-yl]-amino}-acétique,
et les énantiomères, les mélanges de ceux-ci et les sels de ceux-ci.

8. Sels physiologiquement acceptables des composés selon les revendications 1 à 7 avec des acides ou des bases inorganiques ou organiques.

9. Compositions pharmaceutiques, contenant un composé selon au moins l'une des revendications 1 à 7 ou un sel selon la revendication 8 éventuellement conjointement avec un ou plusieurs supports et/ou diluants inertes.

10. Utilisation d'un composé selon au moins l'une des revendications 1 à 7 ou d'un sel selon la revendication 8 pour la préparation d'une composition pharmaceutique qui est appropriée pour le traitement du diabète de type I et de type II.

11. Procédé de préparation d'une composition pharmaceutique selon la revendication 9, **caractérisé en ce qu'**un composé selon au moins l'une des revendications 1 à 7 ou un sel selon la revendication 8 est incorporé dans un ou plusieurs supports et/ou diluants inertes par un procédé non chimique.

12. Procédé de préparation des composés de formule générale I selon les revendications 1 à 8, **caractérisé en ce que**
a) un composé de formule générale 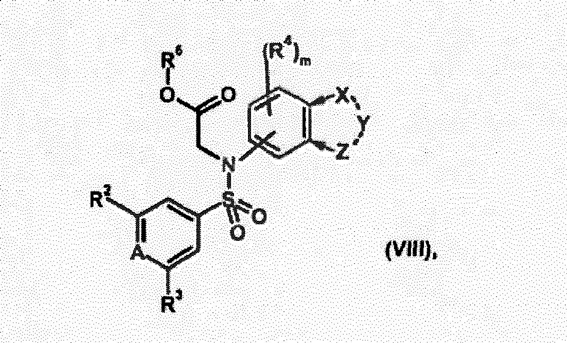 dans laquelle R² à R⁵, m, X, Y, Z et A sont définis comme il a été mentionné dans la revendication 1 et R⁶ représente un groupe carboxy-protecteur, est déprotégé et l'acide ainsi obtenu est éventuellement converti par alkylation en un composé de formule générale (I) selon la revendication 1 et
b) si c'est souhaité, tout groupe protecteur utilisé pour protéger les groupes réactifs durant les réactions est clivé par la suite ou simultanément et/ou
c) un composé de formule générale I ainsi obtenu est résolu en ses stéréo-isomères et/ou
d) un composé de formule générale I ainsi obtenu est converti en ses sels, particulièrement pour un usage pharmaceutique en ses sels physiologiquement acceptables avec un acide ou une base inorganique ou organique.
